# EUROPEAN PATENT SPECIFICATION

(11) **EP 3 430 008 B1**
(45) Date of publication and mention of the grant of the patent: **18.05.2022**
(21) Application number: 17767452.0
(22) Date of filing: 15.03.2017
(51) Int. Cl.: C07D 471/04, A61P 35/00, A61K 31/519

(54) **HISTONE DEMETHYLASE INHIBITORS**
HISTONDEMETHYLASEINHIBITOREN
INHIBITEURS DE L'HISTONE DÉMÉTHYLASE

(30) Priority: 15.03.2016 US 201615071050
(43) Date of publication of application: 23.01.2019
(73) Proprietor: Celgene Quanticel Research, Inc., San Diego, CA 92121 (US)
(72) Inventor: BOLOOR, Amogh, San Diego, California 92116 (US); KANOUNI, Toufike, Rancho Santa Fe, CA 92091 (US); STAFFORD, Jeffrey, Alan, San Diego, California 92130 (US); VEAL, James, Marvin, Apex, North Carolina 27502 (US); WALLACE, Michael, Brennan, San Diego, California 92117 (US)
(74) Representative: Richly & Ritschel Patentanwälte PartG mbB
(86) International application number: PCT/US2017/022546
(87) International publication number: WO 2017/161012

(56) References cited:
- WO-A1-2014/100463
- WO-A1-2014/164708
- WO-A1-2016/044429
- US-A1- 2014 213 591
- US-A1- 2014 371 195
- US-A1- 2015 376 169
- US-A1- 2016 039 808

## Description

### BACKGROUND

A need exists in the art for an effective treatment of cancer and neoplastic disease. WO 2016/044429 A1 discloses histone demethylase inhibitors.

### BRIEF SUMMARY OF THE INVENTION

The invention is directed to a substituted pyrido[3,4-d]pyrimidin-4-one derivative compound selected from the group consisting of:
2-(4-((4-isopropylphenyl)(1-methylpiperidin-4-yl)amino)phenoxy)pyrido [3,4 d] pyrimidin-4-ol;
2-(4-((4-isopropylphenyl)(propyl)amino)phenoxy)pyrido[3,4-d]pyrimidin-4-ol;
2-(4-((4-isopropylphenyl)amino)phenoxy)pyrido[3,4-d]pyrimidin-4-ol;
2-(4-(isopropyl(4-isopropylphenyl)amino)phenoxy)pyrido[3,4-d]pyrimidin-4-ol;
2-(4-(isobutyl(4-isopropylphenyl)amino)phenoxy)pyrido[3,4-d]pyrimidin-4-ol;
2-(4-((4-isopropylphenyl)(1-methylpiperidin-3-yl)amino)phenoxy)pyrido[3,4d] pyrimidin-4-ol;
2-(4-(cyclopentyl(4-isopropylphenyl)amino)phenoxy)pyrido[3,4-d]pyrimidin-4-ol;
2-(4-((4-isopropylphenyl)(pyrrolidin-3-yl)amino)phenoxy)pyrido[3,4-d]pyrimidin-4-ol;
2-(4-((4-isopropylphenyl)(1-methylpyrrolidin-3-yl)amino)phenoxy)pyrido[3,4 d] pyrimidin-4-ol;
2-(4-(5-methylindolin-1-yl)phenoxy)pyrido[3,4-d]pyrimidin-4-ol;
2-(4-(5-methyl-1H-indol-1-yl)phenoxy)pyrido[3,4-d]pyrimidin-4-ol;
2-(4-((4-isopropylphenyl)(piperidin-3-yl)amino)phenoxy)pyrido[3,4-d]pyrimidin-4-ol;
2-(4-((4-isopropylphenyl)(methyl)amino)-3-methylphenoxy)pyrido[3,4-d]pyrimidin-4-ol;
2-(4-((3-(4-isopropylpiperazin-1-yl)phenyl)(methyl)amino)phenoxy)pyrido[3,4-d] pyrimidin-4-ol;
2-(4-(methyl(3-(4-methylpiperazin-1-yl)phenyl)amino)-3 - (trifluoromethyl)phenoxy)pyrido[3,4-d] pyrimidin-4-ol;
2-(3-methyl-4-(methyl(3-(4-methylpiperazin-1-yl)phenyl)amino)phenoxy)pyrido[3,4 d] pyrimidin-4-ol;
2-(4-((4-ethyl-3-methoxyphenyl)(methyl)amino)phenoxy)pyrido[3,4-d]pyrimidin-4-ol;
2-(4-(methyl(3-(4-methylpiperazin-1-yl)-4-(2,2,2 trifluoroethoxy)phenyl)amino)phenoxy) pyrido[3,4-d]pyrimidin-4-ol;
2-(4-((4-ethyl-2-methoxyphenyl)(methyl)amino)phenoxy)pyrido[3,4-d]pyrimidin-4-ol;
2-(4-((3-(4-ethylpiperazin-1-yl)phenyl)(methyl)amino)phenoxy)pyrido[3,4-d] pyrimidin-4-ol;
2-(4-(methyl(3-(1-methyl-1,2,3,6-tetrahydropyridin-4-yl)phenyl)amino)phenoxy)pyrido[3,4-d] pyrimidin-4-ol;
2-(4-(methyl(3-(1-methylpiperidin-4-yl)phenyl)amino)phenoxy)pyrido[3,4-d] pyrimidin-4-ol;
2-(4-(ethyl(3-(4-methylpiperazin-1-yl)phenyl)amino)phenoxy)pyrido[3,4-d]pyrimidin-4-ol;
2-(4-((cyclopropylmethyl)(3-(4-methylpiperazin-1-yl)phenyl)amino)phenoxy)pyrido[3,4-d] pyrimidin-4-ol;
2-(4-((4-isopropylphenyl)(methyl)amino)-3-(trifluoromethyl)phenoxy)pyrido[3,4-d] pyrimidin-4-ol;
2-(4-((4-ethylphenyl)(methyl)amino)-3-methylphenoxy)pyrido[3,4-d]pyrimidin-4-ol;
2-(4-((2-methoxy-5-(4-methylpiperazin-1-yl)phenyl)(methyl)amino)phenoxy)pyrido[3,4-d] pyrimidin-4-ol;
2-(4-((2-ethoxy-5-(4-methylpiperazin-1-yl)phenyl)(methyl)amino)phenoxy)pyrido[3,4-d] pyrimidin-4-ol;
2-(4-(methyl(2-methyl-5-(4-methylpiperazin-1-yl)phenyl)amino)phenoxy)pyrido[3,4 d] pyrimidin-4-ol;
2-(4-((4-(tert-butyl)phenyl)(methyl)amino)-3-methylphenoxy)pyrido[3,4-d]pyrimidin-4-ol; and
2-(3-methyl-4-(methyl(4-propylphenyl)amino)phenoxy)pyrido[3,4-d]pyrimidin-4-ol.

A pharmaceutical composition may comprise a compound as described above.

The compound as described above or the pharmaceutical composition as described above may be for use in the therapy of cancer

The compound as described above or the pharmaceutical composition as described above may be for use in the therapy of prostate cancer, breast cancer, bladder cancer, lung cancer and/or melanoma.

### DETAILED DESCRIPTION

As used herein and in the appended claims, the singular forms "a," "and," and "the" include plural referents unless the context clearly dictates otherwise. Thus, for example, reference to "an agent" includes a plurality of such agents, and reference to "the cell" includes reference to one or more cells (or to a plurality of cells) and equivalents thereof known to those skilled in the art, and so forth. When ranges are used herein for physical properties, such as molecular weight, or chemical properties, such as chemical formulae, all combinations and subcombinations of ranges and specific embodiments therein are intended to be included. The term "about" when referring to a number or a numerical range means that the number or numerical range referred to is an approximation within experimental variability (or within statistical experimental error), and thus the number or numerical range may vary between 1% and 15% of the stated number or numerical range. The term "comprising" (and related terms such as "comprise" or "comprises" or "having" or "including") is not intended to exclude that in other certain embodiments, for example, an embodiment of any composition of matter, composition, method, or process, or the like, described herein, may "consist of" or "consist essentially of" the described features.

The compounds disclosed herein may contain one or more asymmetric centers and may thus give rise to enantiomers, diastereomers, and other stereoisomeric forms that may be defined, in terms of absolute stereochemistry, as (*R*)- or (*S*)-. Unless stated otherwise, it is intended that all stereoisomeric forms of the compounds disclosed herein are contemplated by this disclosure. When the compounds described herein contain alkene double bonds, and unless specified otherwise, it is intended that this disclosure includes both *E* and Z geometric isomers (e.g., *cis* or *trans*)*.* Likewise, all possible isomers, as well as their racemic and optically pure forms, and all tautomeric forms are also intended to be included. The term "geometric isomer" refers to *E* or Z geometric isomers (e.g., *cis* or *trans*) of an alkene double bond. The term "positional isomer" refers to structural isomers around a central ring, such as *ortho-, meta-,* and *para-* isomers around a benzene ring.

A "tautomer" refers to a molecule wherein a proton shift from one atom of a molecule to another atom of the same molecule is possible. The compounds presented herein may, in certain embodiments, exist as tautomers. In circumstances where tautomerization is possible, a chemical equilibrium of the tautomers will exist. The exact ratio of the tautomers depends on several factors, including physical state, temperature, solvent, and pH. Some examples of tautomeric equilibrium include:

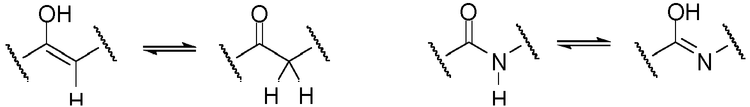

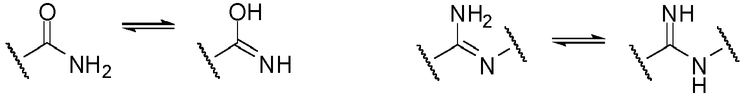

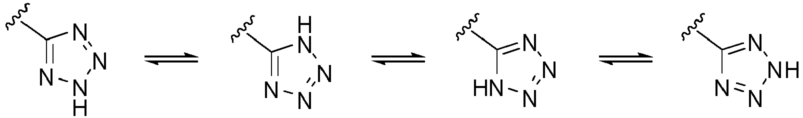

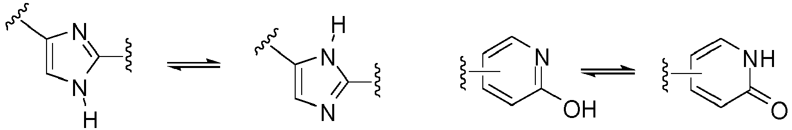

"Pharmaceutically acceptable salt" includes both acid and base addition salts. A pharmaceutically acceptable salt of any one of the substituted pyrido[3,4-d]pyrimidin-4-one derivative compounds described herein is intended to encompass any and all pharmaceutically suitable salt forms. Preferred pharmaceutically acceptable salts of the compounds described herein are pharmaceutically acceptable acid addition salts and pharmaceutically acceptable base addition salts.

"Pharmaceutically acceptable acid addition salt" refers to those salts which retain the biological effectiveness and properties of the free bases, which are not biologically or otherwise undesirable, and which are formed with inorganic acids such as hydrochloric acid, hydrobromic acid, sulfuric acid, nitric acid, phosphoric acid, hydroiodic acid, hydrofluoric acid, phosphorous acid, and the like. Also included are salts that are formed with organic acids such as aliphatic mono- and dicarboxylic acids, phenyl-substituted alkanoic acids, hydroxy alkanoic acids, alkanedioic acids, aromatic acids, aliphatic and. aromatic sulfonic acids, etc. and include, for example, acetic acid, trifluoroacetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, and the like. Exemplary salts thus include sulfates, pyrosulfates, bisulfates, sulfites, bisulfites, nitrates, phosphates, monohydrogenphosphates, dihydrogenphosphates, metaphosphates, pyrophosphates, chlorides, bromides, iodides, acetates, trifluoroacetates, propionates, caprylates, isobutyrates, oxalates, malonates, succinate suberates, sebacates, fumarates, maleates, mandelates, benzoates, chlorobenzoates, methylbenzoates, dinitrobenzoates, phthalates, benzenesulfonates, toluenesulfonates, phenylacetates, citrates, lactates, malates, tartrates, methanesulfonates, and the like. Also contemplated are salts of amino acids, such as arginates, gluconates, and galacturonates (*see, e.g.,* Berge et al., Pharmaceutical Salts, J. Pharma. Sci., 66:1-19 (1997), which is hereby incorporated by reference in its entirety). Acid addition salts of basic compounds may be prepared by contacting the free base forms with a sufficient amount of the desired acid to produce the salt according to methods and techniques with which a skilled artisan is familiar.

"Pharmaceutically acceptable base addition salt" refers to those salts that retain the biological effectiveness and properties of the free acids, which are not biologically or otherwise undesirable. These salts are prepared from addition of an inorganic base or an organic base to the free acid. Pharmaceutically acceptable base addition salts may be formed with metals or amines, such as alkali and alkaline earth metals or organic amines. Salts derived from inorganic bases include, but are not limited to, sodium, potassium, lithium, ammonium, calcium, magnesium, iron, zinc, copper, manganese, aluminum salts and the like. Salts derived from organic bases include, but are not limited to, salts of primary, secondary, and tertiary amines, substituted amines including naturally occurring substituted amines, cyclic amines and basic ion exchange resins, for example, isopropylamine, tri-methylamine, diethylamine, triethylamine, tripropylamine, ethanolamine, diethanolamine, 2-dimethyl-aminoethanol, 2-diethylaminoethanol, dicyclohexylamine, lysine, arginine, histidine, caffeine, procaine, *N*,*N*-dibenzylethylenediamine, chloroprocaine, hydrabamine, choline, betaine, ethylene-diamine, ethylenedianiline, *N*-methylglucamine, glucosamine, methylglucamine, theobromine, purines, piperazine, piperidine, *N*-ethylpiperidine, polyamine resins and the like. *See* Berge et al., *supra.*

As used herein, "treatment" or "treating," or "palliating" or "ameliorating" are used interchangeably herein. These terms refers to an approach for obtaining beneficial or desired results including but not limited to therapeutic benefit and/or a prophylactic benefit. By "therapeutic benefit" is meant eradication or amelioration of the underlying disorder being treated. Also, a therapeutic benefit is achieved with the eradication or amelioration of one or more of the physiological symptoms associated with the underlying disorder such that an improvement is observed in the patient, notwithstanding that the patient may still be afflicted with the underlying disorder. For prophylactic benefit, the compositions may be administered to a patient at risk of developing a particular disease, or to a patient reporting one or more of the physiological symptoms of a disease, even though a diagnosis of this disease may not have been made.

### Substituted Pyrido[3,4-d]pyrimidin-4-one Derivative Compounds

Substituted pyrido[3,4-d]pyrimidin-4-one derivative compounds are described herein that inhibit a histone demethylase enzyme. These compounds, and compositions comprising these compounds, are useful for the treatment of cancer and neoplastic disease. The compounds described herein are useful for treating prostate cancer, breast cancer, bladder cancer, lung cancer and/or melanoma and the like.

The invention provides a compound, or pharmaceutically acceptable salt thereof, chosen from:
2-(4-((4-isopropylphenyl)(1-methylpiperidin-4-yl)amino)phenoxy)pyrido[3,4-d] pyrimidin-4-ol;
2-(4-((4-isopropylphenyl)(propyl)amino)phenoxy)pyrido[3,4-d]pyrimidin-4-ol;
2-(4-((4-isopropylphenyl)amino)phenoxy)pyrido[3,4-d]pyrimidin-4-ol;
2-(4-(isopropyl(4-isopropylphenyl)amino)phenoxy)pyrido[3,4-d]pyrimidin-4-ol;
2-(4-(isobutyl(4-isopropylphenyl)amino)phenoxy)pyrido[3,4-d]pyrimidin-4-ol;
2-(4-((4-isopropylphenyl)(1-methylpiperidin-3-yl)amino)phenoxy)pyrido[3,4-d] pyrimidin-4-ol;
2-(4-(cyclopentyl(4-isopropylphenyl)amino)phenoxy)pyrido[3,4-d]pyrimidin-4-ol;
2-(4-((4-isopropylphenyl)(pyrrolidin-3-yl)amino)phenoxy)pyrido[3,4-d]pyrimidin-4-ol;
2-(4-((4-isopropylphenyl)(1-methylpyrrolidin-3-yl)amino)phenoxy)pyrido[3,4-d] pyrimidin-4-ol;
2-(4-(5-isopropylindolin-1-yl)phenoxy)pyrido[3,4-d]pyrimidin-4-ol;
2-(4-(5-isopropyl-1H-indol-1-yl)phenoxy)pyrido[3,4-d]pyrimidin-4-ol;
2-(4-((4-isopropylphenyl)(piperidin-3-yl)amino)phenoxy)pyrido[3,4-d]pyrimidin-4-ol;
2-(4-((4-isopropylphenyl)(methyl)amino)-3-methylphenoxy)pyrido[3,4-d]pyrimidin-4-ol;
2-(4-((3-(4-isopropylpiperazin-1-yl)phenyl)(methyl)amino)phenoxy)pyrido[3,4-d] pyrimidin-4-ol;
2-(4-((3-(4-isopropylpiperazin-1-yl)phenyl)(methyl)amino)phenoxy)pyrido[3,4-d] pyrimidin-4-ol;
2-(4-(methyl(3-(4-methylpiperazin-1-yl)phenyl)amino)-3 - (trifluoromethyl)phenoxy)pyrido [3,4-d]pyrimidin-4-ol;
2-(3-methyl-4-(methyl(3-(4-methylpiperazin-1-yl)phenyl)amino)phenoxy)pyrido[3,4-d] pyrimidin-4-ol;
2-(4-((4-ethyl-3-methoxyphenyl)(methyl)amino)phenoxy)pyrido[3,4-d]pyrimidin-4-ol;
2-(4-(methyl(3-(4-methylpiperazin-1-yl)-4-(2,2,2-trifluoroethoxy)phenyl)amino)phenoxy) pyrido[3,4-d]pyrimidin-4-ol;
2-(4-((4-ethyl-2-methoxyphenyl)(methyl)amino)phenoxy)pyrido[3,4-d]pyrimidin-4-ol;
2-(4-((3-(4-ethylpiperazin-1-yl)phenyl)(methyl)amino)phenoxy)pyrido[3,4-d] pyrimidin-4-ol;
2-(4-(methyl(3-(1-methyl-1,2,3,6-tetrahydropyridin-4-yl)phenyl)amino)phenoxy)pyrido [3,4-d]pyrimidin-4-ol;
2-(4-(methyl(3-(1-methylpiperidin-4-yl)phenyl)amino)phenoxy)pyrido[3,4-d] pyrimidin-4-ol;
2-(4-(ethyl(3-(4-methylpiperazin-1-yl)phenyl)amino)phenoxy)pyrido[3,4-d]pyrimidin-4-ol;
2-(4-((cyclopropylmethyl)(3-(4-methylpiperazin-1-yl)phenyl)amino)phenoxy)pyrido[3,4-d] pyrimidin-4-ol;
2-(4-((4-isopropylphenyl)(methyl)amino)-3-(trifluoromethyl)phenoxy)pyrido[3,4-d] pyrimidin-4-ol;
2-(4-((4-ethylphenyl)(methyl)amino)-3-methylphenoxy)pyrido[3,4-d]pyrimidin-4-ol;
2-(4-((2-methoxy-5-(4-methylpiperazin-1-yl)phenyl)(methyl)amino)phenoxy)pyrido[3,4-d] pyrimidin-4-ol;
2-(4-((2-ethoxy-5-(4-methylpiperazin-1-yl)phenyl)(methyl)amino)phenoxy)pyrido[3,4-d] pyrimidin-4-ol;
2-(4-(methyl(2-methyl-5-(4-methylpiperazin-1-yl)phenyl)amino)phenoxy)pyrido[3,4-d] pyrimidin-4-ol;
2-(4-((4-(tert-butyl)phenyl)(methyl)amino)-3-methylphenoxy)pyrido[3,4-d]pyrimidin-4-ol; and
2-(3-methyl-4-(methyl(4-propylphenyl)amino)phenoxy)pyrido[3,4-d]pyrimidin-4-ol.

The compound disclosed herein may have a structure provided in Table 1. Examples 1-118 are reference examples.

| Table 1 | | |
|---|---|---|
| Ex. | Structure | Name |
| 1 | 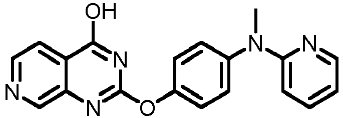 | 2-[4-(methyl-pyridin-2-yl-amino)-phenoxy]-pyrido [3,4-d]pyrimidin -4-ol |
| 2 | 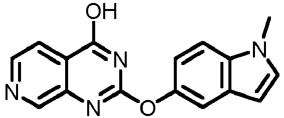 | 2-(1-methyl-1H-indol-5-yloxy)-pyrido [3,4-d]pyrimidin-4-ol |
| 3 | 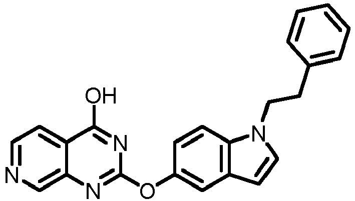 | 2-(1-phenethyl-1H-indol-5-yloxy)-pyrido[3,4-d]pyrimidin -4-ol |
| 4 | 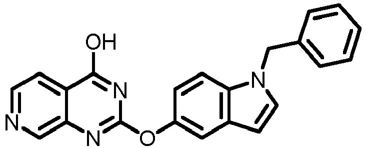 | 2-(1-benzyl-1H-indol-5-yloxy)-pyrido [3,4-d]pyrimidin-4-ol |
| 5 | 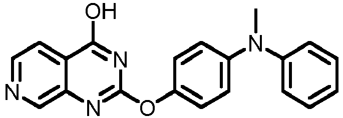 | 2-[4-(methyl-phenyl-amino)-phenoxy]-pyrido[3,4-d]pyrimidin -4-ol |
| 6 | 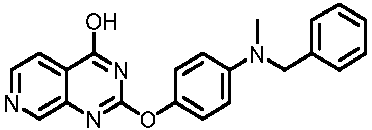 | 2-[4-(benzyl-methyl-amino)-phenoxy]-pyrido[3,4-d]pyrimidin -4-ol |
| 7 | 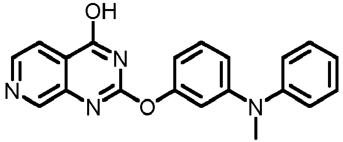 | 2-[3-(methyl-phenyl-amino)-phenoxy]-pyrido[3,4-d]pyrimidin -4-ol |
| 8 | 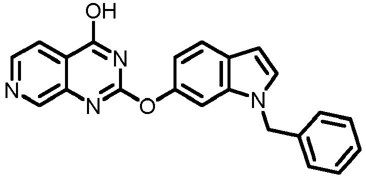 | 2-(1-benzyl-1H-indol-6-yloxy)-pyrido [3,4-d]pyrimidin-4-ol |
| 9 | 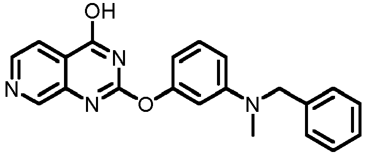 | 2-[3-(benzyl-methyl-amino)-phenoxy]-pyrido[3,4-d]pyrimidin -4-ol |
| 10 | 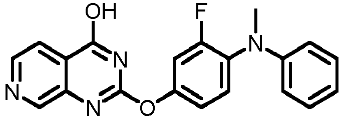 | 2-[3-fluoro-4-(methyl-phenyl-amino)-phenoxy]-pyrido[3,4-d]pyrimidin-4-ol |
| 11 | 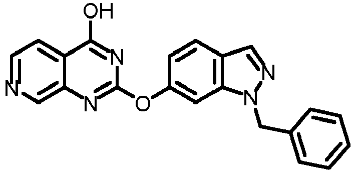 | 2-(1-benzyl-1H-indazol-6-yloxy)-pyrido[3,4-d]pyrimidin -4-ol |
| 12 | 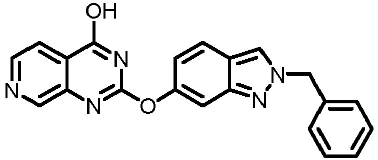 | 2-(2-benzyl-2H-indazol-6-yloxy)-pyrido[3,4-d]pyrimidin -4-ol |
| 13 | 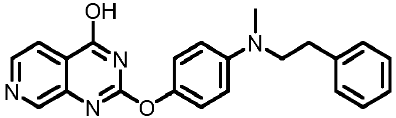 | 2-{4-[methyl(2-phenylethyl)amino]phenoxy}pyridino [3,4-d]pyrimidin-4-ol |
| 14 | 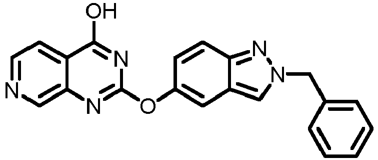 | 2-[2-benzyl-2H-indazol-5-yloxy]pyridino[3,4-d]pyrimidin-4-ol |
| 15 | 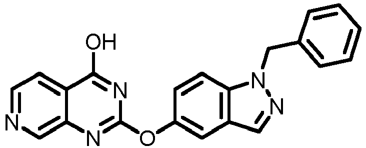 | 2-(1-benzyl-1H-indazol-5-yloxy)-pyridino[3,4-d]pyrimidin -4-ol |
| 16 | 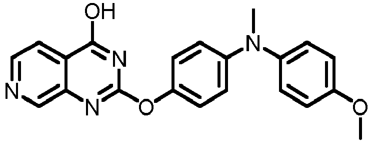 | 2-{4-[(4-methoxy-phenyl)-methyl-amino]-phenoxy }-pyrido[3,4-d]pyrimidin-4-ol |
| 17 | 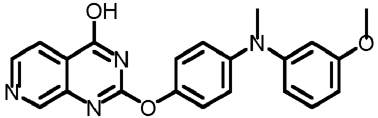 | 2-{4- [(3 -methoxy-phenyl)-methyl-amino] - phenoxy}-pyrido[3,4-d]pyrimidin-4-ol |
| 18 | 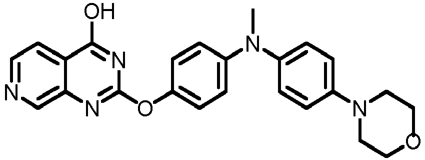 | 2-{4- [methyl-(4-morpholin-4-yl-phenyl)-amino] -phenoxy}-pyrido [3,4-d]pyrimidin-4-ol |
| 19 | 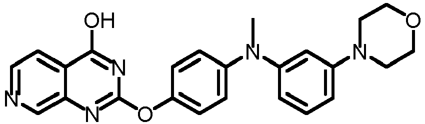 | 2-{4- [methyl-(3 -morpholin-4-yl-phenyl)-amino] -phenoxy}-pyrido [3,4-d]pyrimidin-4-ol |
| 20 | 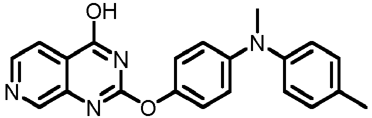 | 2-[4-(methyl-p-tolyl-amino)-phenoxy]-pyrido[3,4-d]pyrimidin -4-ol |
| 21 | 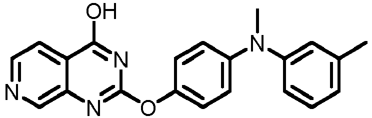 | 2-[4-(methyl-m-tolyl-amino)-phenoxy]-pyrido[3,4-d]pyrimidin -4-ol |
| 22 | 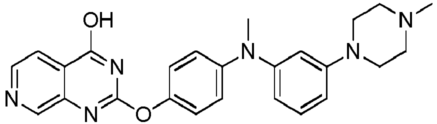 | 2-(4-{methyl-[3-(4-methyl-piperazin-1-yl)-phenyl] -amino } -phenoxy)-pyrido [3,4-d] pyrimidin-4-ol |
| 23 | 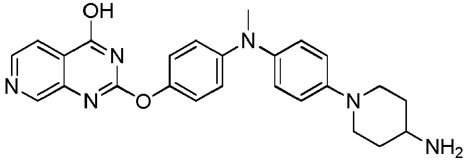 | 2-(4-{[4-(4-amino-piperidin-1-yl)-phenyl]-methyl-amino}-phenoxy)-pyrido [3,4-d]pyrimidin-4-ol |
| 24 | 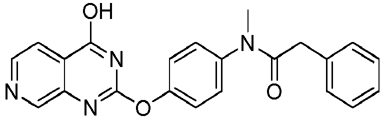 | N-[4-(4-hydroxy-pyrido[3,4-d] pyrimidin-2-yloxy)-phenyl]-N-methyl-2-phenyl-acetamide |
| 25 | 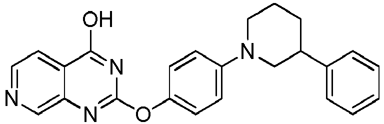 | 2- [4-(3 -phenyl-piperidin-1-yl)-phenoxy] - pyrido[3,4-d]pyrimidin -4-ol |
| 26 | 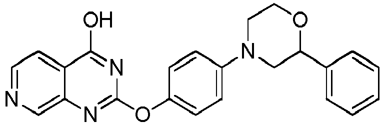 | 2-[4-(2-phenyl-morpholin-4-yl)-phenoxy]-pyrido[3,4-d]pyrimidin -4-ol |
| 27 | 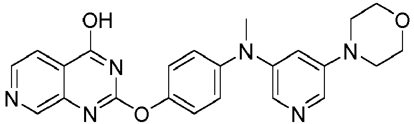 | 2-{4- [methyl-(5 -morpholin-4-yl-pyridin-3 - yl)-amino] -phenoxy } -pyrido [3,4-d]pyrimidin-4-ol |
| 28 | 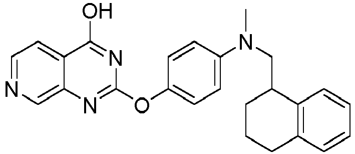 | 2-{4-[methyl-(1,2,3,4-tetrahydro-naphthalen-1-ylmethyl)-amino] -phenoxy } - pyrido[3,4-d]pyrimidin -4-ol |
| 29 | 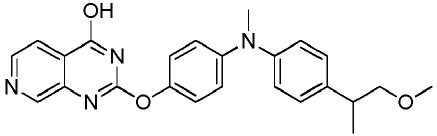 | 2-(4-{[4-(2-methoxy-1-methyl-ethyl)-phenyl]-methyl-amino}-phenoxy)-pyrido[3,4-d]pyrimidin -4-ol |
| 30 | 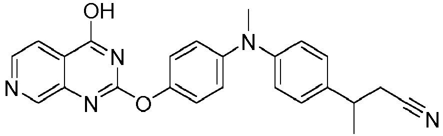 | 3-(4-{[4-(4-hydroxy-pyrido[3,4-d]pyrimidin-2-yloxy)-phenyl]-methyl-amino}-phenyl)-butyronitrile |
| 31 | 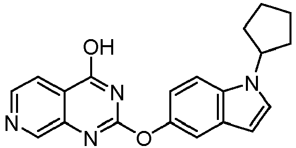 | 2-(1-cyclopentyl-1H-indol-5-yloxy)-pyrido[3,4-d]pyrimidin -4-ol |
| 32 | 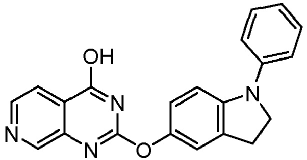 | 2-(1-phenyl-2,3-dihydro-1H-indol-5-yloxy)-pyrido[3,4-d]pyrimidin -4-ol |
| 33 | 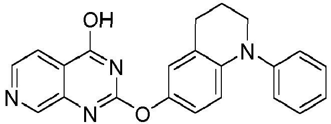 | 2-(1-phenyl-1,2,3,4-tetrahydro-quinolin-6-yloxy)-pyrido[3,4-d]pyrimidin-4-ol |
| 34 | 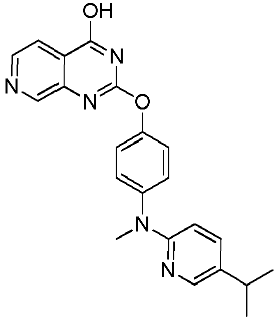 | 2-{4-[(5-isopropyl-pyridin-2-yl)-methyl-amino] -phenoxy }-pyrido [3,4-d]pyrimidin-4-ol |
| 35 | 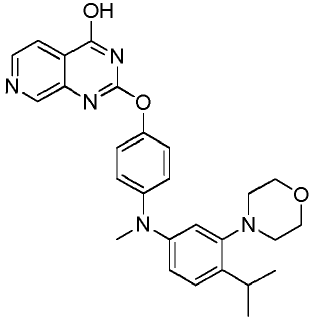 | 2-{4-[(4-isopropyl-3-morpholin-4-yl-phenyl)-methyl-amino] -phenoxy } - pyrido[3,4-d]pyrimidin -4-ol |
| 36 | 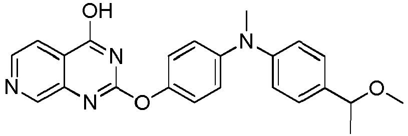 | 2-(4-{[4-(1-methoxy-ethyl)-phenyl]-methyl-amino}-phenoxy)-pyrido [3,4-d]pyrimidin-4-ol |
| 37 | 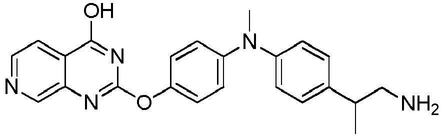 | 2-(4- {[4-(2-amino-1-methyl-ethyl)-phenyl]-methyl-amino}-phenoxy)-pyrido[3,4-d]pyrimidin -4-ol |
| 38 | 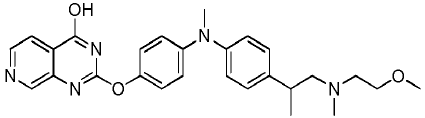 | 2-{4-[(4-{2-[(2-methoxy-ethyl)-methyl-amino]-1-methyl-ethyl}-phenyl)-methyl-amino] -phenoxy } -pyrido [3,4-d]pyrimidin-4-ol |
| 39 | 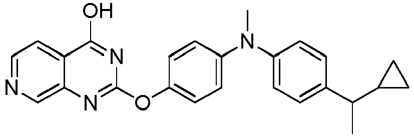 | 2-(4- {[4-(1-cyclopropyl-ethyl)-phenyl]-methyl-amino}-phenoxy)-pyrido [3,4-d]pyrimidin-4-ol |
| 40 | 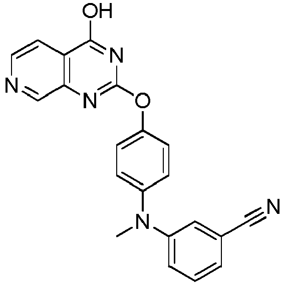 | 3-{[4-(4-hydroxy-pyrido[3,4-d]pyrimidin-2-yloxy)-phenyl]-methyl-amino}-benzonitrile |
| 41 | 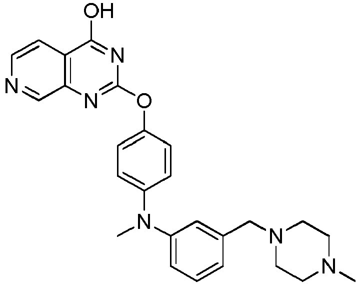 | 2-(4-{methyl-[3-(4-methyl-piperazin-1-ylmethyl)-phenyl]-amino}-phenoxy)-pyrido[3,4-d]pyrimidin -4-ol |
| 42 | 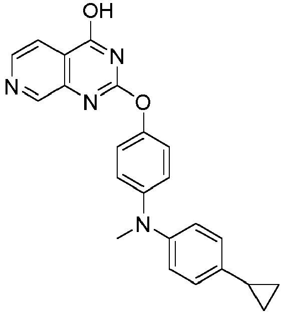 | 2-{4- [(4-cyclopropyl-phenyl)-methyl-amino] -phenoxy } -pyrido [3,4-d]pyrimidin-4-ol |
| 43 | 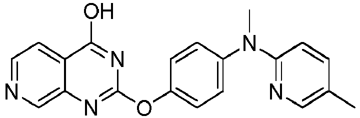 | 2-[4-[methyl-(5-methylpyridin-2-yl)amino]phenoxy]pyrido[3,4-d] pyrimidin-4-ol |
| 44 | 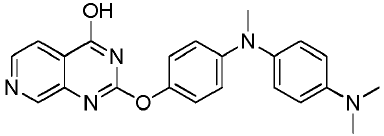 | 2-[4-[4-(dimethylamino)-N-methylanilino]phenoxy]pyrido [3,4-d]pyrimidin-4-ol |
| 45 | 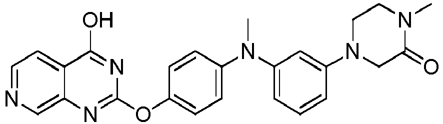 | 4-[3-[4-(4-hydroxypyrido[3,4-d]pyrimidin-2-yl)oxy-N-methylanilino]phenyl]-1-methylpiperazin-2-one |
| 46 | 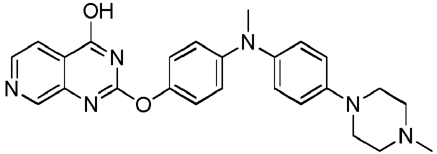 | 2-[4-[N-methyl-4-(4-methylpiperazin-1-yl)anilino]phenoxy]pyrido [3,4-d]pyrimidin-4-ol |
| 47 | 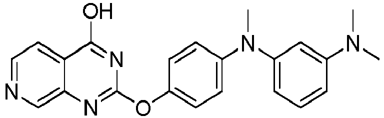 | 2-[4-[3-(dimethylamino)-N-methylanilino]phenoxy]pyrido [3,4-d]pyrimidin-4-ol |
| 48 | 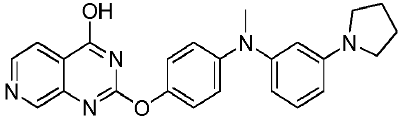 | 2-[4-(N-methyl-3-pyrrolidin- 1-ylanilino)phenoxy]pyrido [3,4-d]pyrimidin-4-ol |
| 49 | 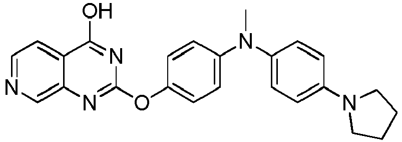 | 2-[4-(N-methyl-4-pyrrolidin-1-ylanilino)phenoxy]pyrido [3,4-d]pyrimidin-4-ol |
| 50 | 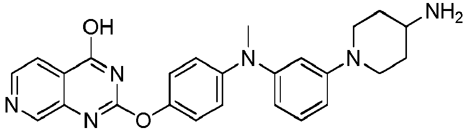 | 2-[4-[3-(4-aminopiperidin-1-yl)-N-methylanilino]phenoxy]pyrido [3,4-d]pyrimidin-4-ol |
| 51 | 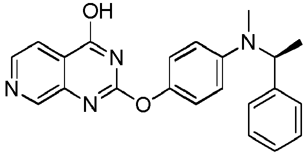 | 2-[4-[methyl-[(1S)-1-phenylethyl]amino]phenoxy]pyri do [3,4-d]pyrimidin-4-ol |
| 52 | 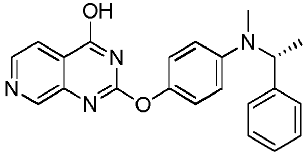 | 2-[4-[methyl-[(1R)-1-phenylethyl]amino]phenoxy]pyri do [3,4-d]pyrimidin-4-ol |
| 53 | 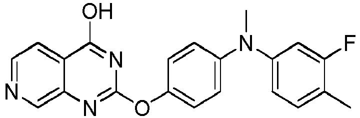 | 2-[4-(3-fluoro-N,4-dimethylanilino)phenoxy]pyrido [3,4-d]pyrimidin-4-ol |
| 54 | 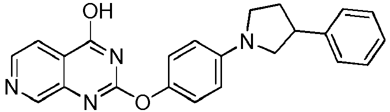 | 2-[4-(3-phenylpyrrolidin-1-yl)phenoxy]pyrido[3,4-d]pyrimidin-4-ol |
| 55 | 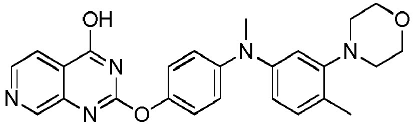 | 2-[4-(N,4-dimethyl-3-morpholin-4-ylanilino)phenoxy] pyrido[3,4-d]pyrimidin -4-ol |
| 56 | 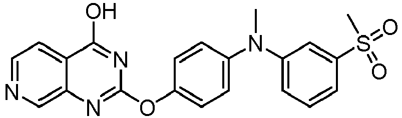 | 2-[4-(N-methyl-3-methylsulfonylanilino) phenoxy]pyrido[3,4-d]pyrimidin-4-ol |
| 57 | 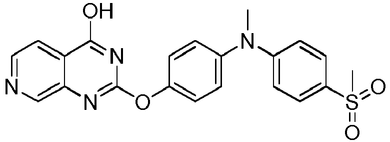 | 2-[4-(N-methyl-4-methylsulfonylanilino) phenoxy]pyrido[3,4-d]pyrimidin-4-ol |
| 58 | 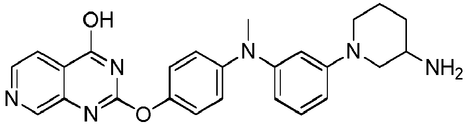 | 2-[4-[3-(3-aminopiperidin-1-yl)-N-methylanilino]phenoxy]pyrido [3,4-d]pyrimidin-4-ol |
| 59 | 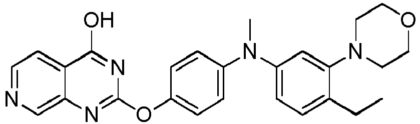 | 2-[4-(4-ethyl-N-methyl-3-morpholin-4-ylanilino)phenoxy] pyrido[3,4-d]pyrimidin -4-ol |
| 60 | 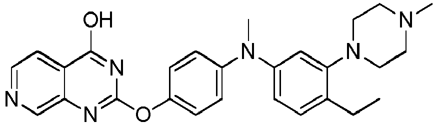 | 2-[4-[4-ethyl-N-methyl-3-(4-methylpiperazin-1-yl)anilino] phenoxy]pyrido[3,4-d]pyrimidin-4-ol |
| 61 | 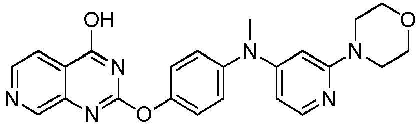 | 2-[4-[methyl-(2-morpholin-4-yl pyridin-4-yl)amino]phenoxy]pyrido [3,4-d]pyrimidin-4-ol |
| 62 | 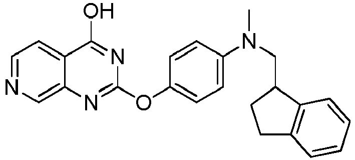 | 2-[4-[2,3-dihydro-1H-inden-1-ylmethyl(methyl)amino]phenoxy] pyrido[3,4-d]pyrimidin -4-ol |
| 63 | 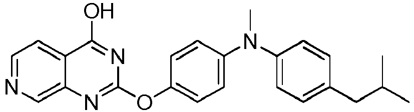 | 2-[4-[N-methyl-4-(2-methylpropyl)anilino] phenoxy]pyrido[3,4-d]pyrimidin-4-ol |
| 64 | 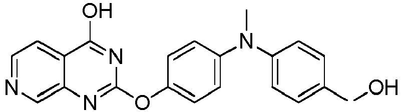 | 2-[4-[4-(2-hydroxypropan-2-yl)-N-methylanilino]phenoxy] pyrido[3,4-d]pyrimidin -4-ol |
| 65 | 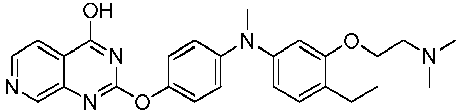 | 2-[4-[3-[2-(dimethylamino)ethoxy]-4-ethyl-N-methylanilino]phenoxy] pyrido[3,4-d]pyrimidin -4-ol |
| 66 | 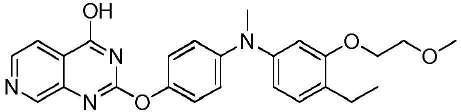 | 2-[4-[4-ethyl-3-(2-methoxyethoxy)-N-methylanilino]phenoxy]pyrido [3,4-d]pyrimidin-4-ol |
| 67 | 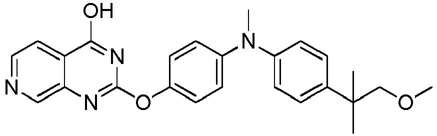 | 2-[4-[4-(1-methoxy-2-methylpropan-2-yl)-N-methylanilino]phenoxy]pyrido [3,4-d]pyrimidin-4-ol |
| 68 | 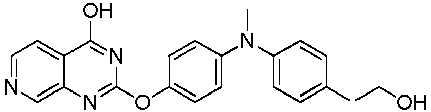 | 2-[4-[4-(1-hydroxy-2-methylpropan-2-yl)-N-methylanilino]phenoxy]pyrido [3,4-d]pyrimidin-4-ol |
| 69 | 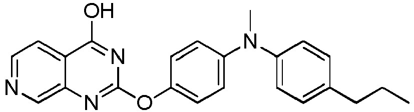 | 2-[4-(N-methyl-4-propylanilino)phenoxy]pyrido [3,4-d]pyrimidin-4-ol |
| 70 | 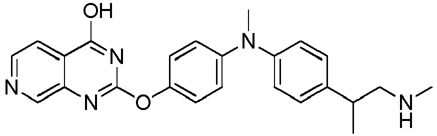 | 2-[4-[N-methyl-4-[1-(methylamino)propan-2-yl]anilino]phenoxy]pyrido [3,4-d]pyrimidin-4-ol |
| 71 | 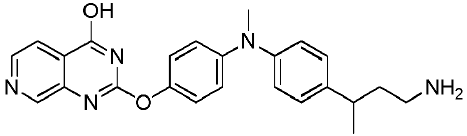 | 2-[4-[4-(4-aminobutan-2-yl)-N-methylanilino]phenoxy]pyrido [3,4-d]pyrimidin-4-ol |
| 72 | 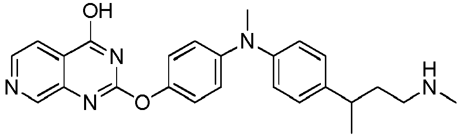 | 2-[4-[N-methyl-4-[4-(methylamino)butan-2-yl] anilino]phenoxy]pyrido [3,4-d]pyrimidin-4-ol |
| 73 | 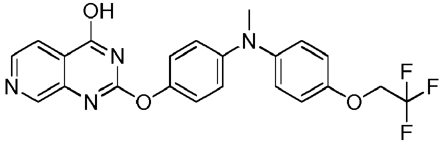 | 2-[4-[N-methyl-4-(2,2,2-trifluoroethoxy)anilino]phenoxy]pyrido [3,4-d]pyrimidin-4-ol |
| 74 | 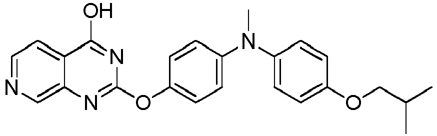 | 2-[4-[N-methyl-4-(2-methylpropoxy)anilino]phenoxy]pyrido [3,4-d]pyrimidin-4-ol |
| 75 | 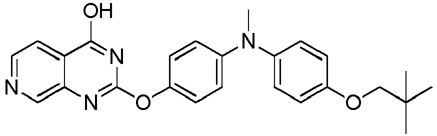 | 2-[4-[4-(2,2-dimethylpropoxy)-N-methylanilino]phenoxy]pyrido [3,4-d]pyrimidin-4-ol |
| 76 | 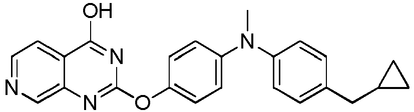 | 2-[4-[4-(cyclopropylmethyl)-N-methylanilino]phenoxy]pyrido [3,4-d]pyrimidin-4-ol |
| 77 | 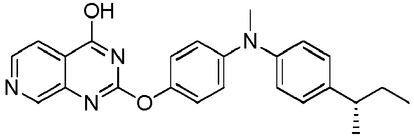 | 2-[4-[4-[(2S)-butan-2-yl]-N-methylanilino]phenoxy]pyrido [3,4-d]pyrimidin-4-ol |
| 78 | 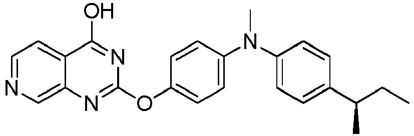 | 2-[4-[4-[(2R)-butan-2-yl]-N-methylanilino]phenoxy]pyrido [3,4-d]pyrimidin-4-ol |
| 79 | 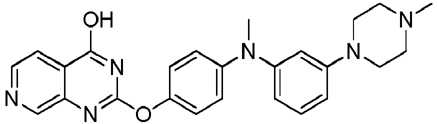 | 2-[4-[N-methyl-3-(4-methylpiperazin-1-yl)anilino]phenoxy]pyrido[3,4-d] pyrimidin-4-ol |
| 80 | 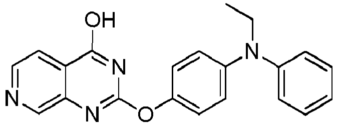 | 2-[4-(N-ethylanilino)phenoxy]pyrido [3,4-d]pyrimidin-4-ol |
| 81 | 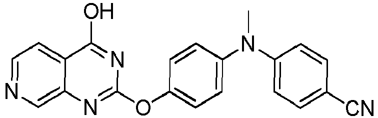 | 4-[4-(4-hydroxypyrido[3,4-d]pyrimidin-2-yl)oxy-N-methylanilino]benzonitrile |
| 82 | 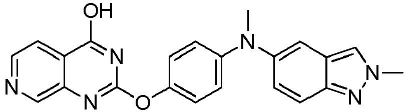 | 2-[4-[methyl-(2-methylindazol-5-yl)amino]phenoxy]pyrido[3,4-d] pyrimidin-4-ol |
| 83 | 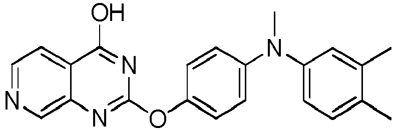 | 2-[4-(N,3,4-trimethylanilino) phenoxy]pyrido[3,4-d]pyrimidin-4-ol |
| 84 | 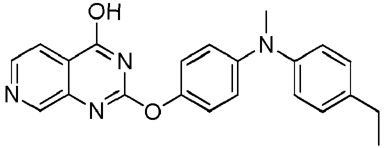 | 2-[4-(4-ethyl-N-methylanilino) phenoxy]pyrido[3,4-d]pyrimidin-4-ol |
| 85 | 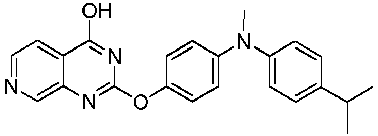 | 2-[4-(N-methyl-4-propan-2-ylanilino) phenoxy]pyrido[3,4-d]pyrimidin-4-ol |
| 86 | 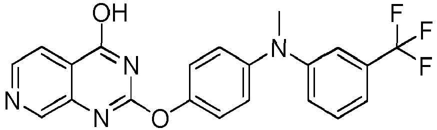 | 2-[4-[N-methyl-3-(trifluoromethyl)anilino] phenoxy]pyrido[3,4-d]pyrimidin-4-ol |
| 87 | 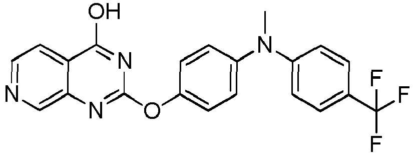 | 2-[4-[N-methyl-4-(trifluoromethyl)anilino] phenoxy]pyrido[3,4-d]pyrimidin-4-ol |
| 88 | 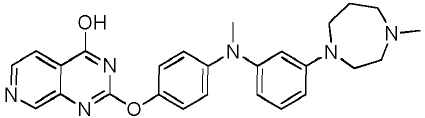 | 2-[4-[N-methyl-3-(4-methyl-1,4-diazepan-1-yl)anilino]phenoxy]pyrido [3,4-d]pyrimidin-4-ol |
| 89 | 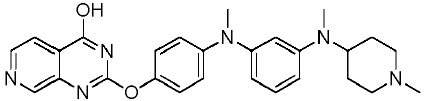 | 2-[4-[N-methyl-3-[methyl-(1-methyl piperidin-4-yl)amino]anilino] phenoxy]pyrido[3,4-d]pyrimidin-4-ol |
| 90 | 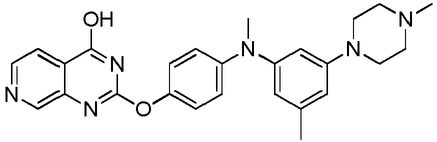 | 2-[4-[N,3-dimethyl-5-(4-methylpiperazin-1-yl)anilino]phenoxy]pyrido [3,4-d]pyrimidin-4-ol |
| 91 | 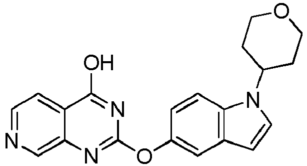 | 2-[1-(oxan-4-yl)indol-5-yl]oxypyrido [3,4-d]pyrimidin-4-ol |
| 92 | 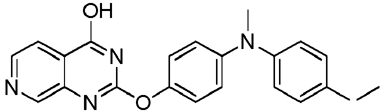 | 2-[4-(4-tert-butyl-N-methylanilino) phenoxy]pyrido[3,4-d]pyrimidin-4-ol |
| 93 | 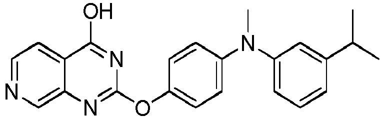 | 2- [4-(N-methyl-3 -prop an-2-ylanilino) phenoxy]pyrido[3,4-d]pyrimidin-4-ol |
| 94 | 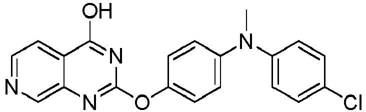 | 2-[4-(4-chloro-N-methylanilino) phenoxy]pyrido[3,4-d]pyrimidin-4-ol |
| 95 | 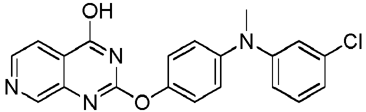 | 2-[4-(3-chloro-N-methylanilino) phenoxy]pyrido[3,4-d]pyrimidin-4-ol |
| 96 | 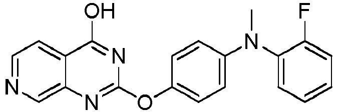 | 2-[4-(3-fluoro-N-methylanilino) phenoxy]pyrido[3,4-d]pyrimidin-4-ol |
| 97 | 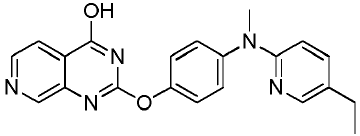 | 2-[4-[(5-ethylpyridin-2-yl)-methylamino] phenoxy]pyrido[3,4-d]pyrimidin-4-ol |
| 98 | 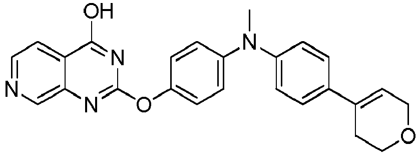 | 2-[4-[4-(3,6-dihydro-2H-pyran-4-yl)-N-methylanilino]phenoxy]pyrido [3,4-d]pyrimidin-4-ol |
| 99 | 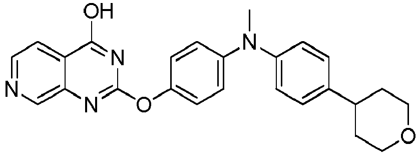 | 2-[4-[N-methyl-4-(oxan-4-yl)anilino] phenoxy]pyrido[3,4-d]pyrimidin-4-ol |
| 100 | 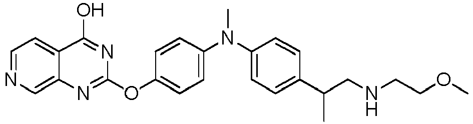 | 2-[4-[4-[1-(2-methoxyethylamino)propan-2-yl]-N-methylanilino]phenoxy]pyrido [3,4-d]pyrimidin-4-ol |
| 101 | 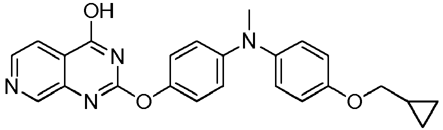 | 2-[4-[4-(cyclopropylmethoxy)-N-methylanilino]phenoxy]pyrido [3,4-d]pyrimidin-4-ol |
| 102 | 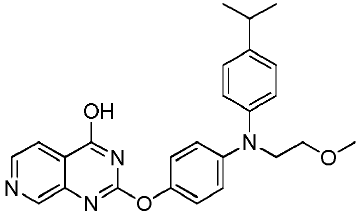 | 2-[4-[N-(2-methoxyethyl)-4-propan-2-ylanilino]phenoxy]pyrido [3,4-d]pyrimidin-4-ol |
| 103 | 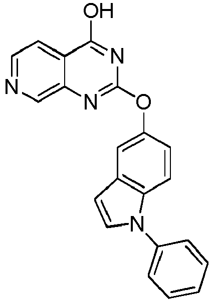 | 2-(1-phenylindol-5-yl)oxypyrido [3,4-d]pyrimidin-4-ol |
| 104 | 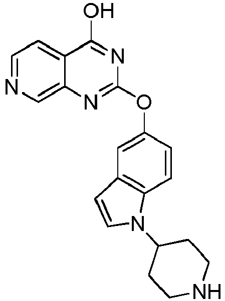 | 2-(1-piperidin-4-ylindol-5-yl)oxypyrido[3,4-d]pyrimidin-4-ol |
| 105 | 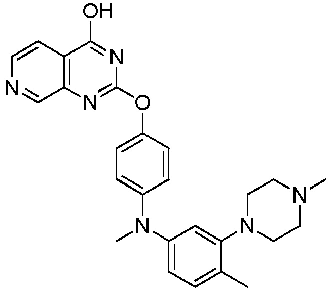 | 2-[4-[N,4-dimethyl-3-(4-methylpiperazin-1-yl)anilino]phenoxy]pyrido[3,4-d] pyrimidin-4-ol |
| 106 | 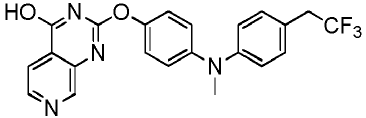 | 2-[4-[N-methyl-4-(2,2,2-trifluoroethyl)anilino]phenoxy]pyrido [3,4-d]pyrimidin-4-ol |
| 107 | 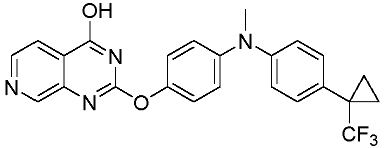 | 2-[4-[N-methyl-4-[1-(trifluoromethyl) cyclopropyl]anilino]phenoxy] pyrido[3,4-d]pyrimidin -4-ol |
| 108 | 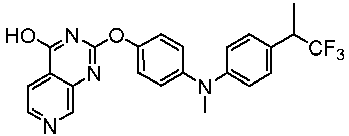 | 2-[4-[N-methyl-4-(1,1,1-trifluoropropan-2-yl)anilino]phenoxy]pyrido[3,4-d] pyrimidin-4-ol |
| 109 | 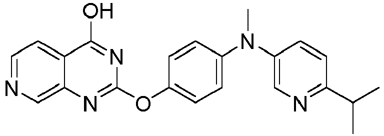 | 2-[4-[methyl-(6-propan-2-ylpyridin-3-yl)amino]phenoxy]pyrido[3,4-d] pyrimidin-4-ol |
| 110 | 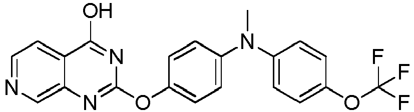 | 2-[4-[N-methyl-4-(trifluoromethoxy)anilino]phenoxy]pyrido [3,4-d]pyrimidin-4-ol |
| 111 | 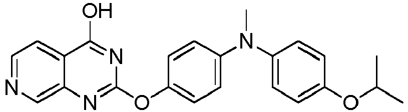 | 2-[4-(N-methyl-4-propan-2-yloxyanilino)phenoxy]pyrido [3,4-d]pyrimidin-4-ol |
| 112 | 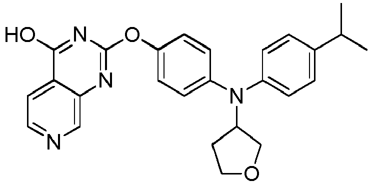 | 2-[4-[N-(oxolan-3-yl)-4-propan-2ylanilino] phenoxy]pyrido[3,4-d]pyrimidin-4-ol |
| 113 | 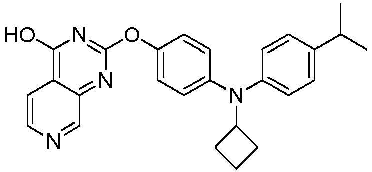 | 2-[4-(N-cyclobutyl-4-propan-2-ylanilino) phenoxy]pyrido[3,4-d]pyrimidin-4-ol |
| 114 | 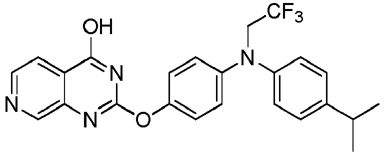 | 2-{4-[(4-isopropyl-phenyl)-(2,2,2-trifluoroethyl)-amino] -phenoxy } -pyrido [3,4-d]pyrimidin-4-ol |
| 115 | 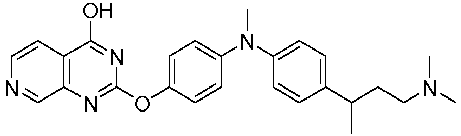 | 2-(4-{[4-(3 -dimethylamino-1-methylpropyl)-phenyl]-methyl-amino}-phenoxy)-pyrido[3,4-d]pyrimidin -4-ol |
| 116 | 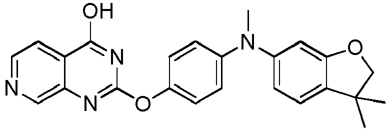 | (3,3 -dimethyl-2,3-dihydro-benzofuran-6-yl)-methyl-[4-(4-methyl-pyrido[3,4-d]pyrimidin-2-yloxy)-phenyl]-amine |
| 117 | 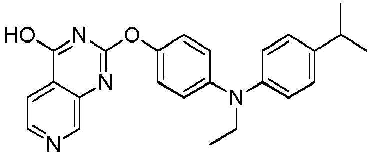 | 2-(4-(ethyl(4-isopropylphenyl)amino) phenoxy)pyrido[3,4-d]pyrimidin-4-ol |
| 118 | 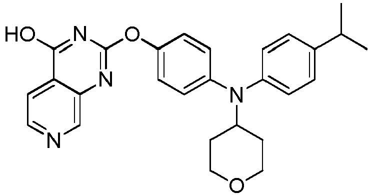 | 2-(4-((4-isopropylphenyl)(tetrahydro-2H-pyran-4-yl)amino)phenoxy)pyrido [3,4-d]pyrimidin-4-ol |
| 119 | 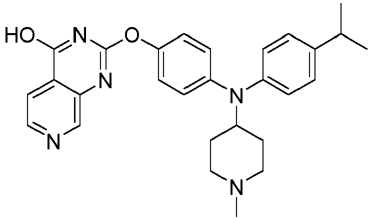 | 2-(4-((4-isopropylphenyl)(1-methyl piperidin-4-yl)amino)phenoxy)pyrido [3,4-d]pyrimidin-4-ol |
| 120 | 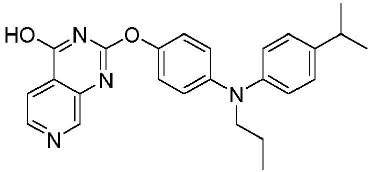 | 2-(4-((4-isopropylphenyl)(propyl)amino) phenoxy)pyrido[3,4-d]pyrimidin-4-ol |
| 121 | 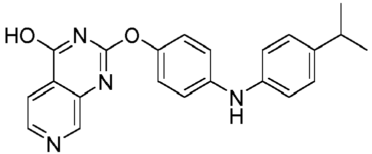 | 2-(4-((4-isopropylphenyl)amino) phenoxy)pyrido[3,4-d]pyrimidin-4-ol |
| 122 | 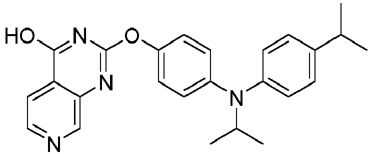 | 2-(4-(isopropyl(4-isopropylphenyl)amino) phenoxy)pyrido[3,4-d]pyrimidin-4-ol |
| 123 | 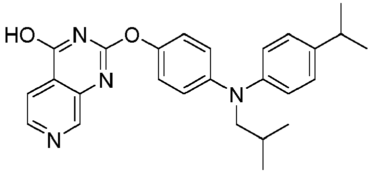 | 2-(4-(isobutyl(4-isopropylphenyl)amino) phenoxy)pyrido[3,4-d]pyrimidin-4-ol |
| 124 | 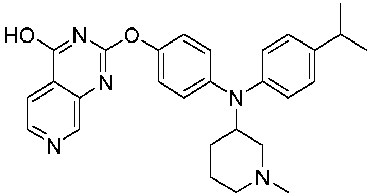 | 2-(4-((4-isopropylphenyl)(1-methyl piperidin-3-yl)amino)phenoxy) pyrido[3,4-d]pyrimidin -4-ol |
| 125 | 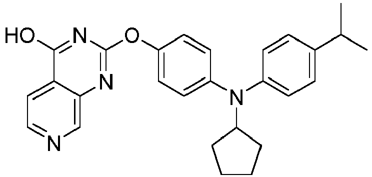 | 2-(4-(cyclopentyl(4-isopropylphenyl) amino) phenoxy)pyrido[3,4-d] pyrimidin-4-ol |
| 126 | 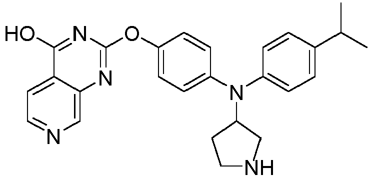 | 2-(4-((4-isopropylphenyl) (pyrrolidin-3-yl)amino)phenoxy) pyrido[3,4-d]pyrimidin -4-ol |
| 127 | 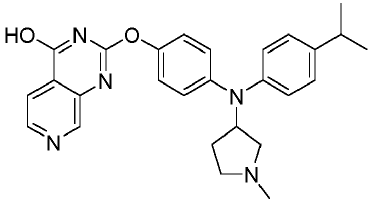 | 2-(4-((4-isopropylphenyl) (1-methylpyrrolidin-3 -yl)amino) phenoxy)pyrido[3,4-d]pyrimidin-4-ol |
| 128 | 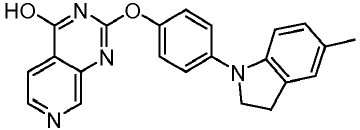 | 2-(4-(5-methylindolin-1-yl)phenoxy) pyrido[3,4-d]pyrimidin -4-ol |
| 129 | 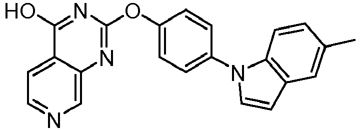 | 2-(4-(5-methyl-1H-indol-1-yl)phenoxy) pyrido[3,4-d]pyrimidin -4-ol |
| 130 | 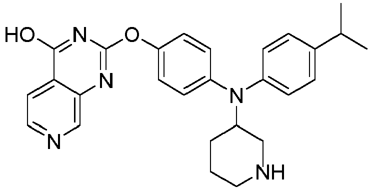 | 2-(4-((4-isopropylphenyl) (piperidin-3 -yl)amino)phenoxy) pyrido[3,4-d]pyrimidin -4-ol |
| 131 | 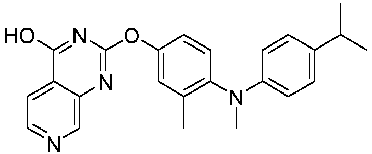 | 2-(4-((4-isopropylphenyl)(methyl)amino)-3-methylphenoxy)pyrido[3,4-d]pyrimidin-4-ol |
| 132 | 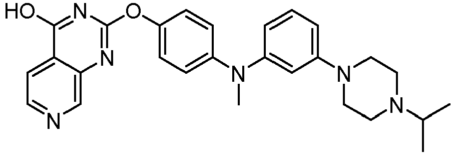 | 2-(4-((3 -(4-isopropylpiperazin- 1-yl)phenyl)(methyl)amino)phenoxy) pyrido[3,4-d]pyrimidin -4-ol |
| 133 | 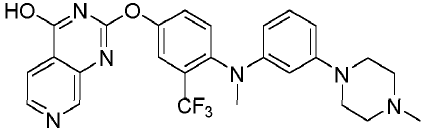 | 2-(4-(methyl(3-(4-methylpiperazin-1-yl)phenyl)amino)-3 -(trifluoromethyl) phenoxy)pyrido[3,4-d]pyrimidin-4-ol |
| 134 | 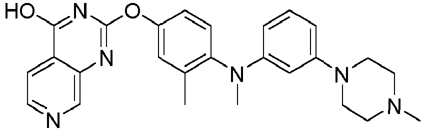 | 2-(3 -methyl-4-(methyl(3 -(4-methyl-piperazin-1-yl)phenyl)amino)phenoxy) pyrido[3,4-d]pyrimidin -4-ol |
| 135 | 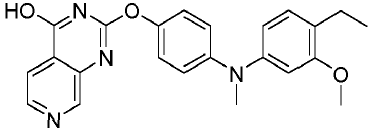 | 2-(4-((4-ethyl-3-methoxyphenyl) (methyl)amino)phenoxy) pyrido[3,4-d]pyrimidin -4-ol |
| 136 | 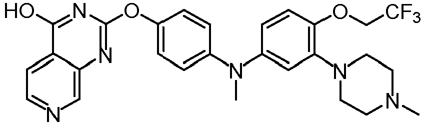 | -(4-(methyl(3-(4-methylpiperazin- 1-yl)-4-(2,2,2-trifluoroethoxy)phenyl)amino) phenoxy)pyrido[3,4-d]pyrimidin-4-ol |
| 137 | 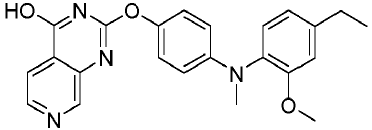 | 2-(4-((4-ethyl-2-methoxyphenyl) (methyl)amino)phenoxy)pyrido [3,4-d]pyrimidin-4-ol |
| 138 | 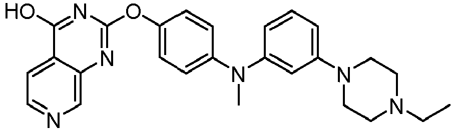 | 2-(4-((3 -(4-ethylpiperazin-1-yl) phenyl)(methyl)amino)phenoxy) pyrido[3,4-d]pyrimidin -4-ol |
| 139 | 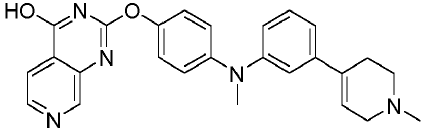 | 2-(4-(methyl(3-(1-methyl-1,2,3,6-tetrahydropyridin-4-yl)phenyl)amino) phenoxy)pyrido[3,4-d]pyrimidin-4-ol |
| 140 | 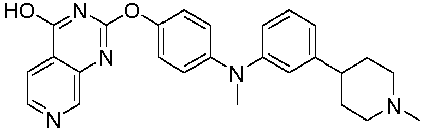 | 2-(4-(methyl(3-(1-methylpiperidin-4-yl)phenyl)amino)phenoxy) pyrido[3,4-d]pyrimidin -4-ol |
| 141 | 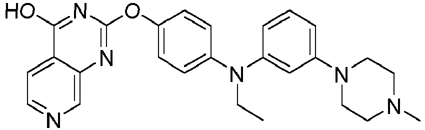 | 2-(4-(ethyl(3-(4-methylpiperazin-1-yl)phenyl)amino)phenoxy)pyrido [3,4-d]pyrimidin-4-ol |
| 142 | 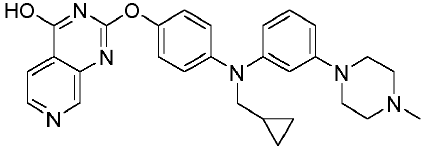 | 2-(4-((cyclopropylmethyl)(3-(4-methyl piperazin- 1-yl)phenyl)amino)phenoxy) pyrido[3,4-d]pyrimidin -4-ol |
| 143 | 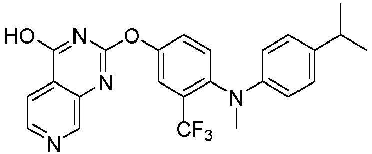 | 2-(4-((4-isopropylphenyl)(methyl) amino)-3-(trifluoromethyl)phenoxy) pyrido[3,4-d]pyrimidin -4-ol |
| 144 | 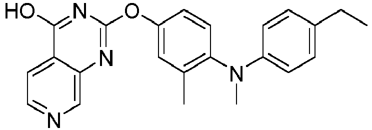 | 2-(4-((4-ethylphenyl)(methyl)amino)-3-methylphenoxy)pyrido[3,4-d]pyrimidin-4-ol |
| 145 | 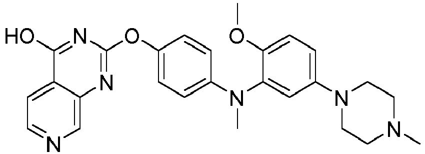 | 2-(4-((2-methoxy-5-(4-methylpiperazin-1-yl) phenyl)(methyl)amino)phenoxy) pyrido[3,4-d]pyrimidin -4-ol |
| 146 | 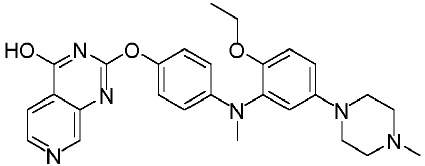 | 2-(4-((2-ethoxy-5-(4-methylpiperazin-1-yl) phenyl)(methyl)amino)phenoxy) pyrido[3,4-d]pyrimidin -4-ol |
| 147 | 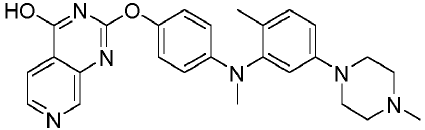 | 2-(4-(methyl(2-methyl-5-(4-methylpiperazin-1-yl)phenyl)amino) phenoxy)pyrido[3,4-d]pyrimidin-4-ol |
| 148 | 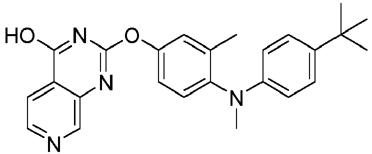 | 2-(4-((4-(tert-butyl)phenyl)(methyl) amino)-3 -methylphenoxy)pyrido [3,4-d]pyrimidin-4-ol |
| 149 | 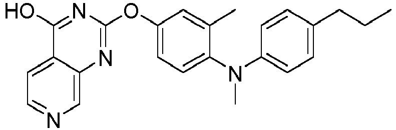 | 2-(3 -methyl-4-(methyl(4-propylphenyl)amino)phenoxy) pyrido[3,4-d]pyrimidin -4-ol |

In some embodiments, the compound disclosed herein has a structure provided in Table 2.

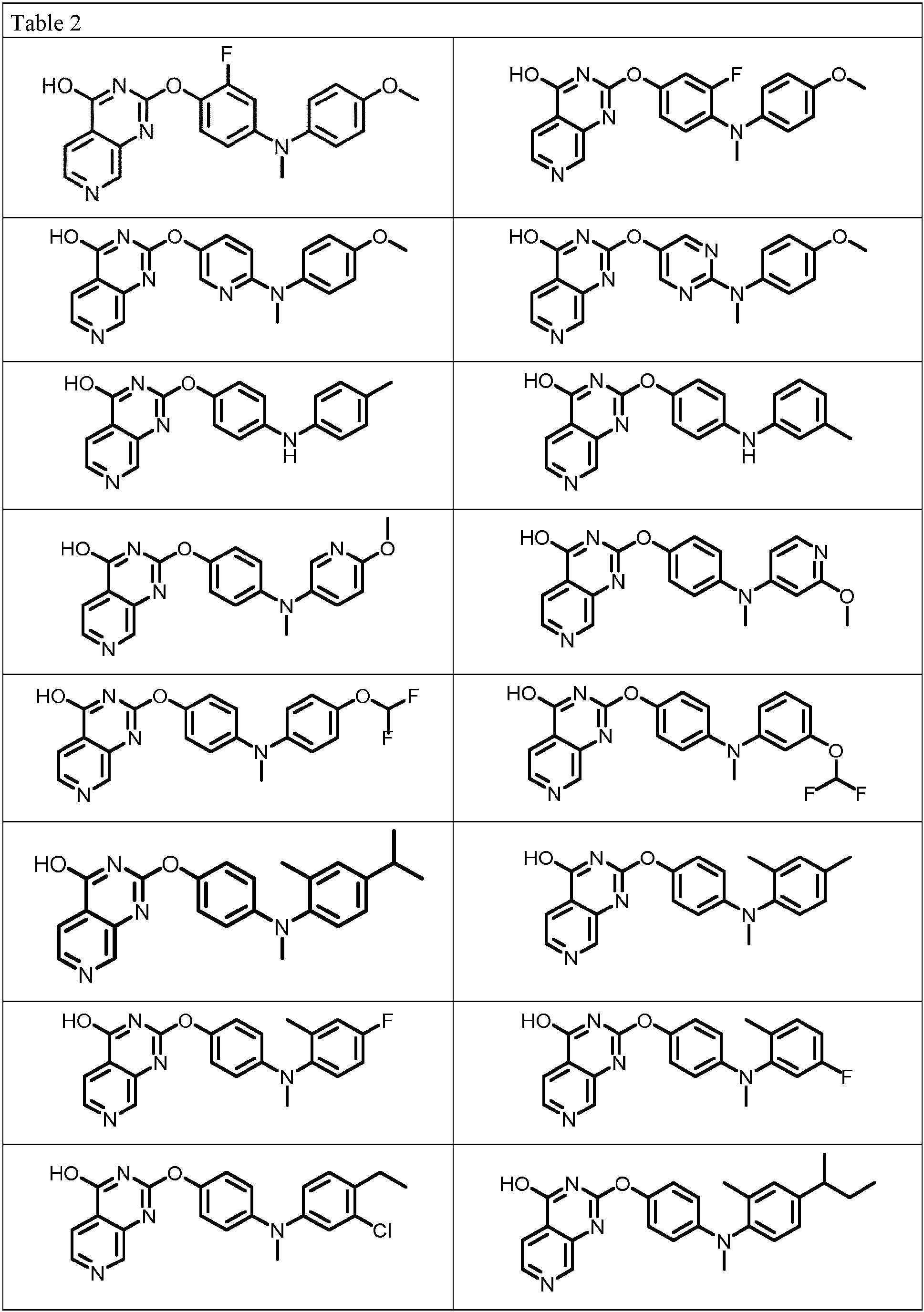

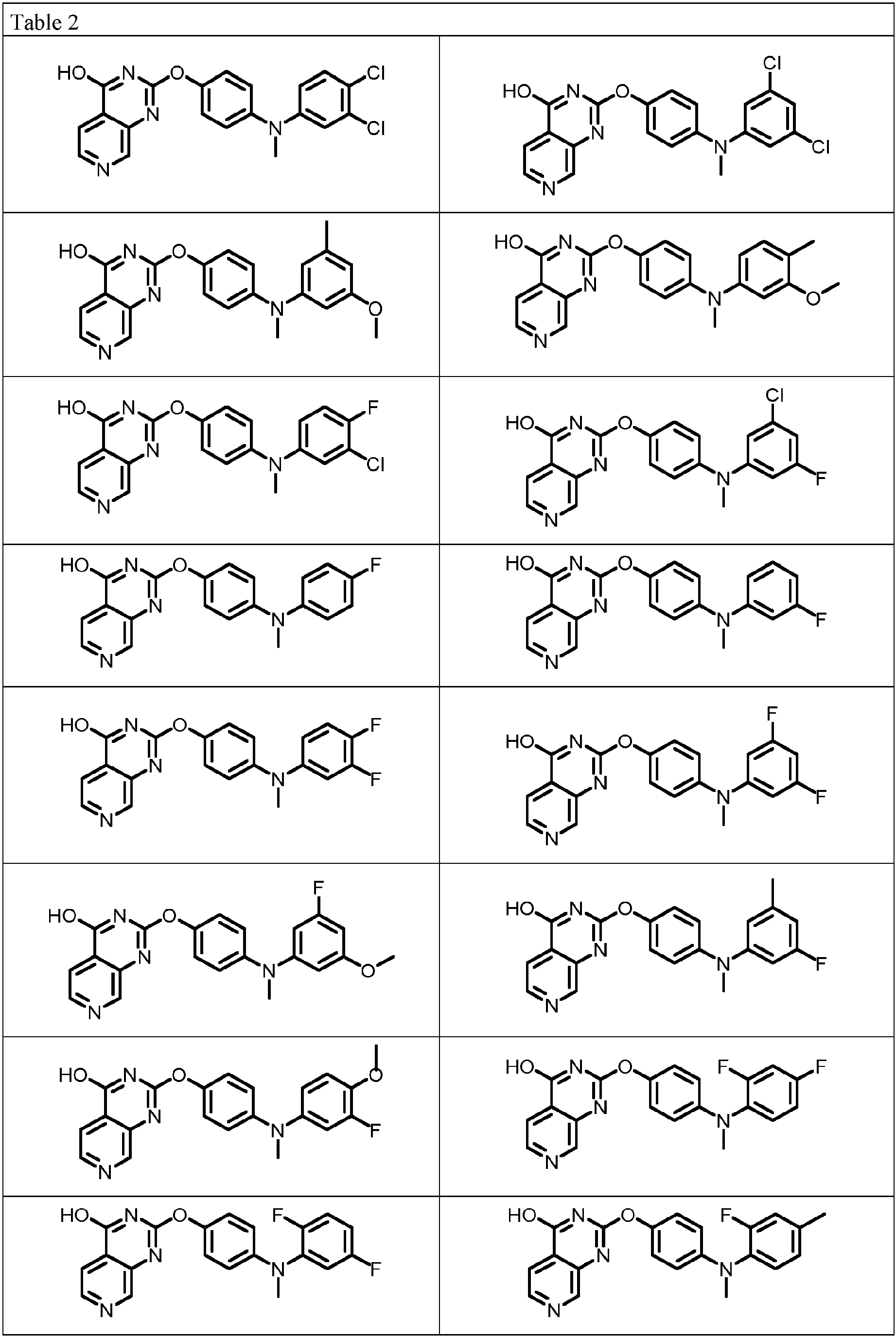

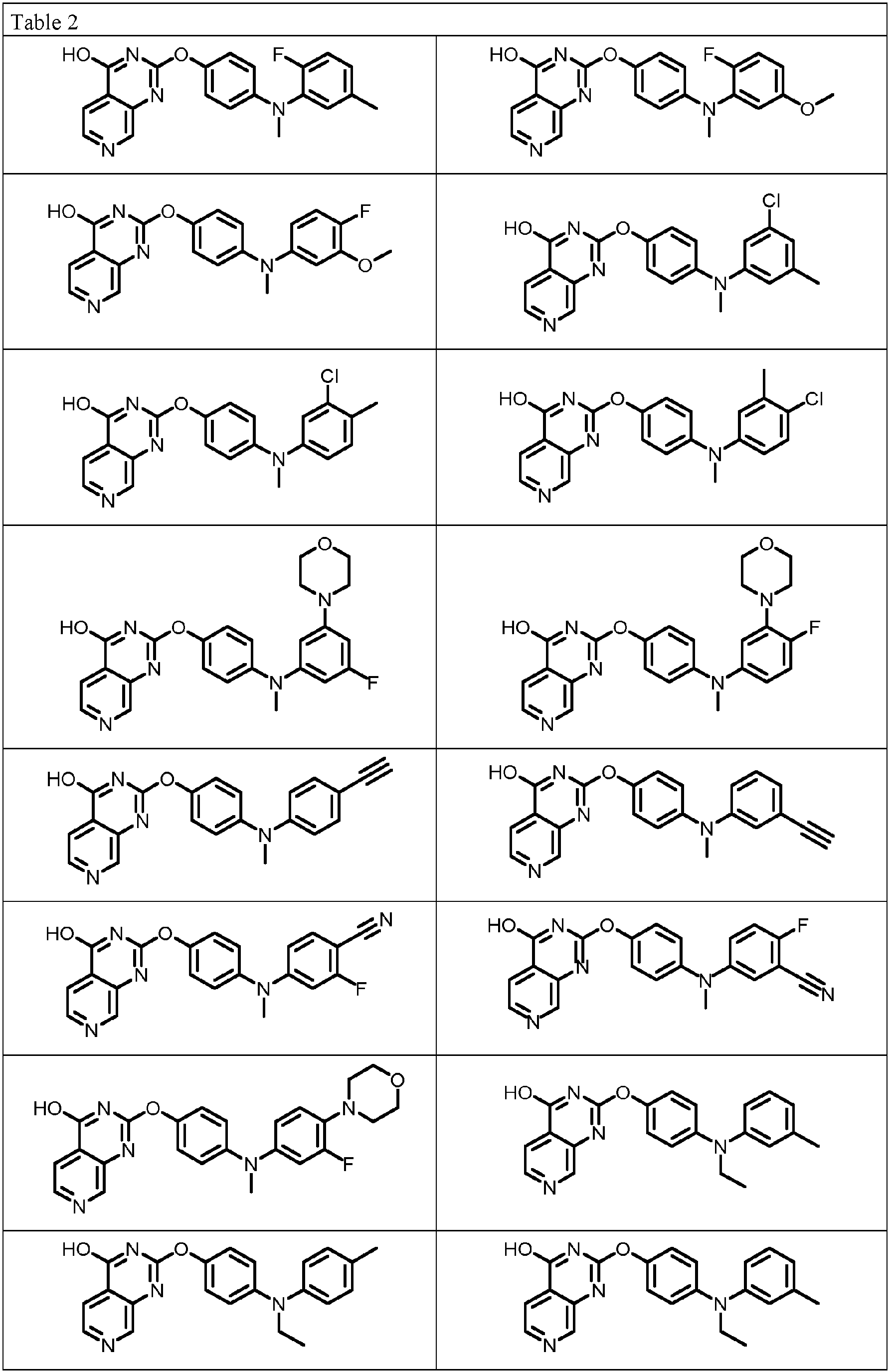

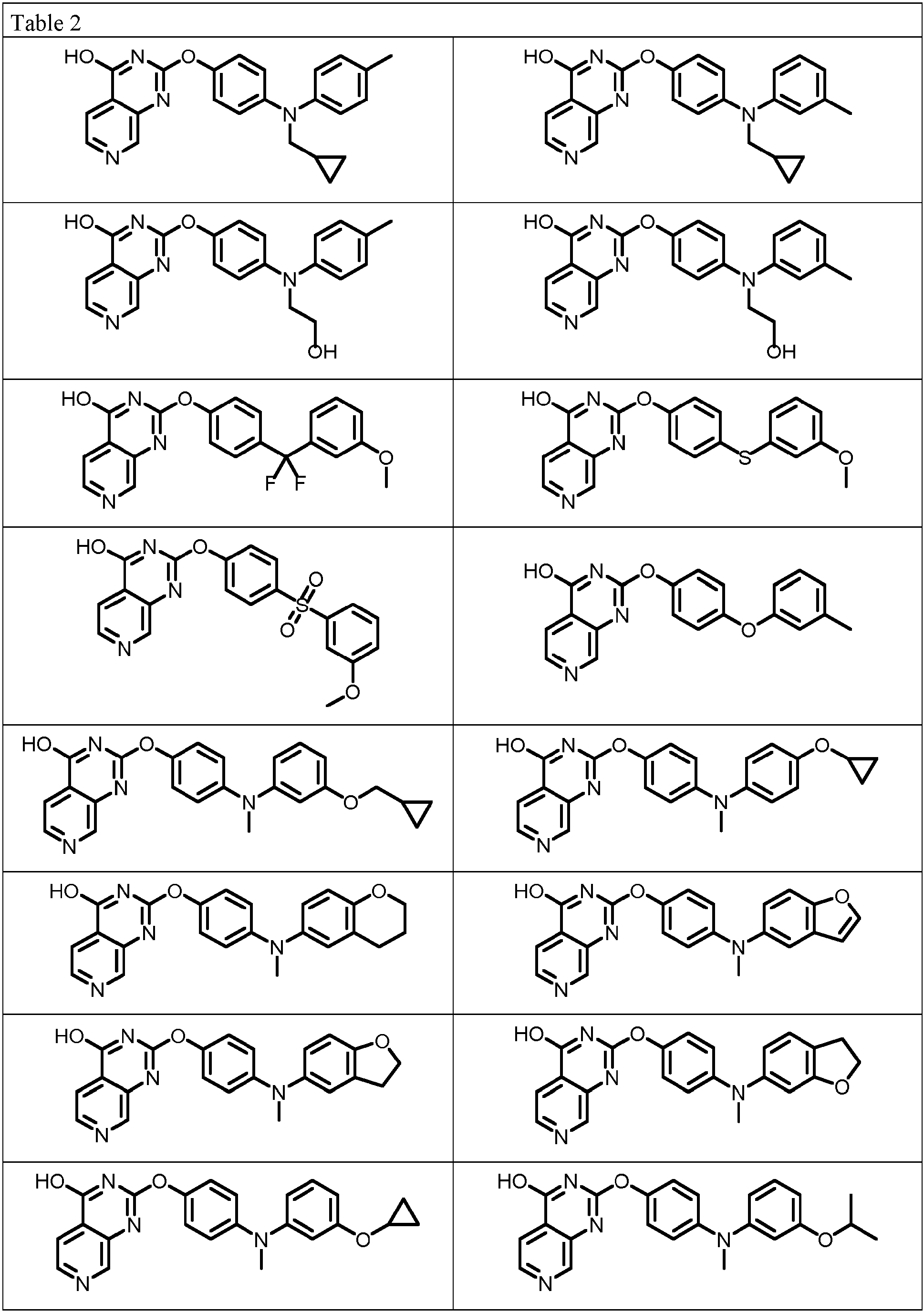

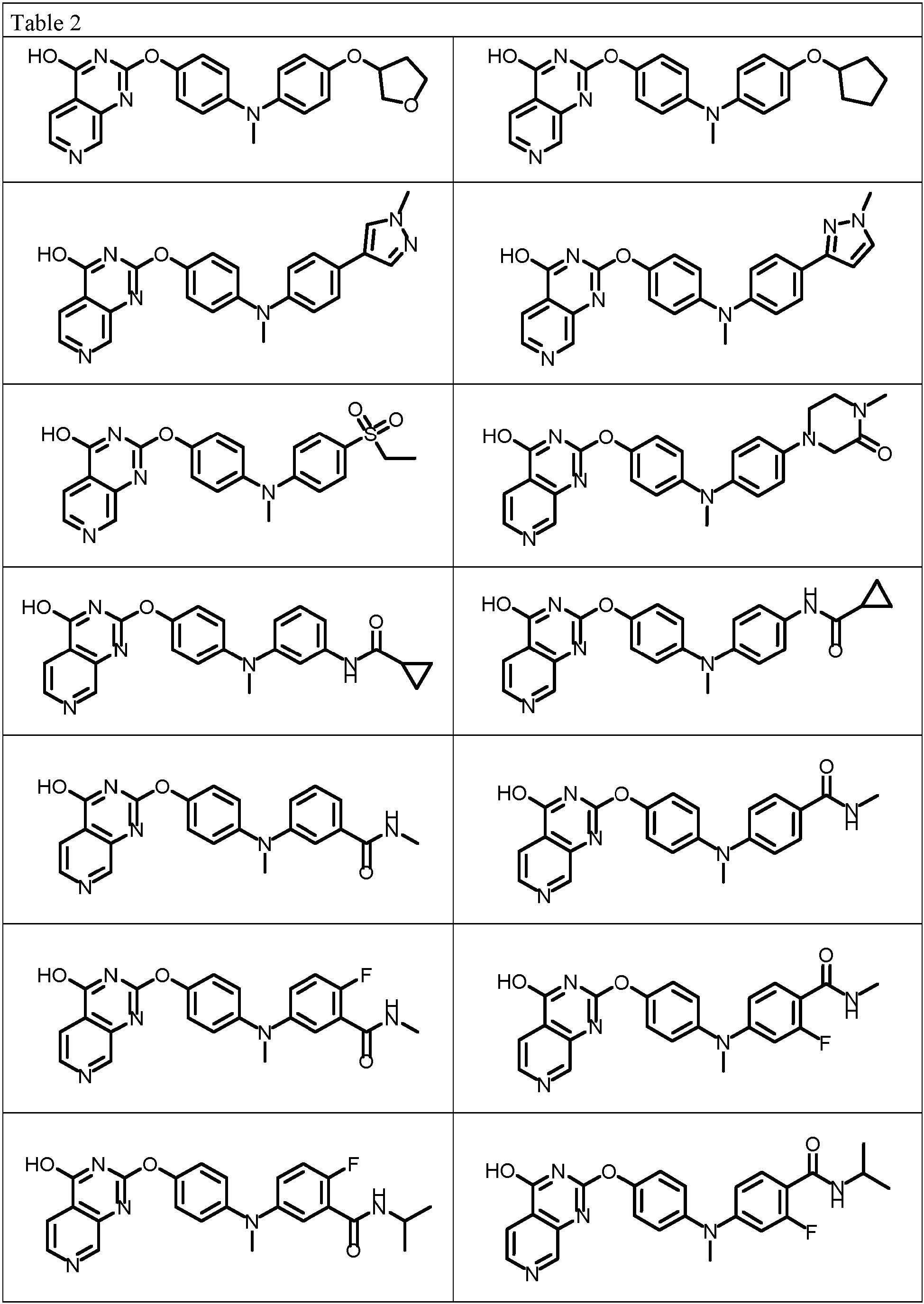

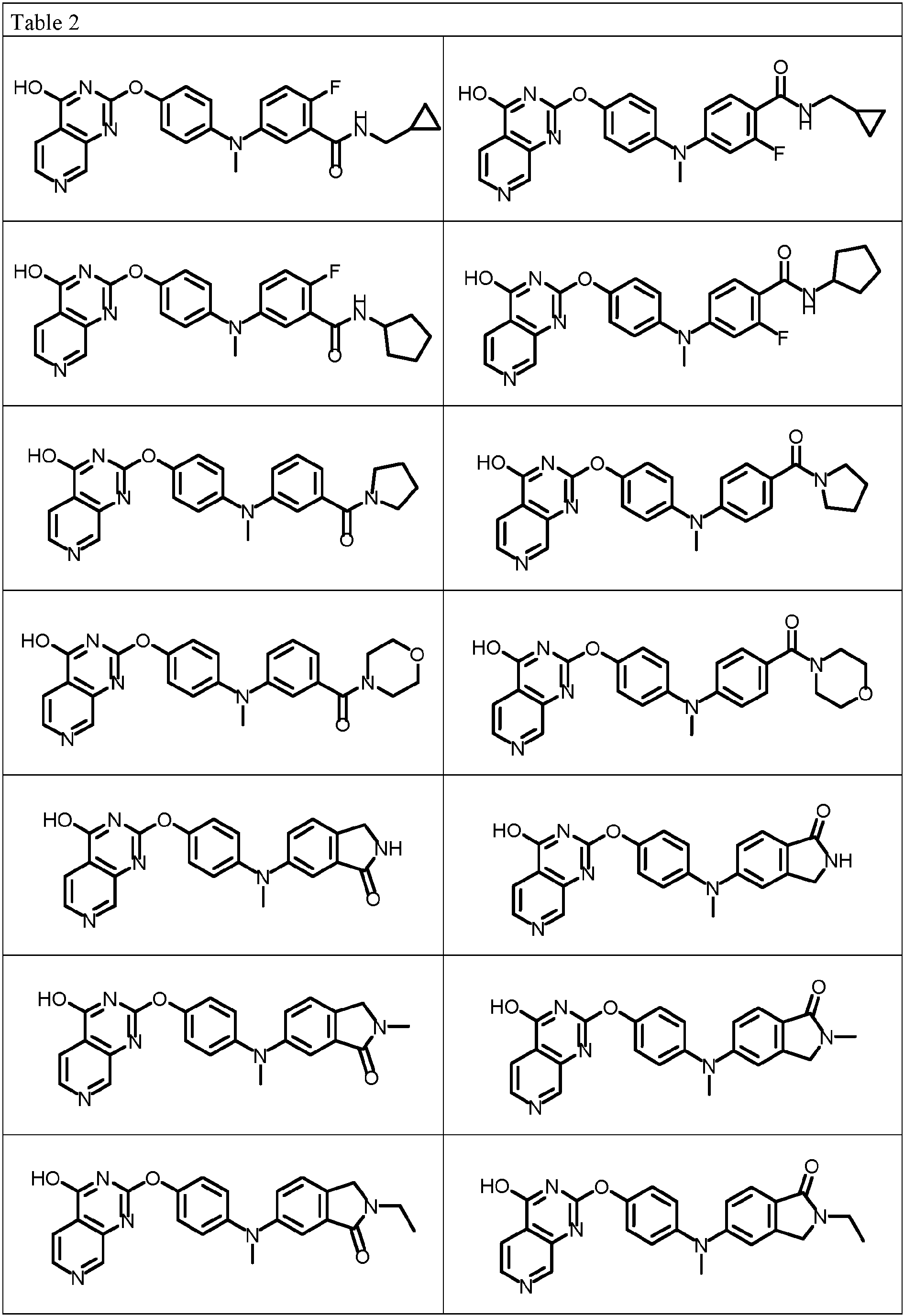

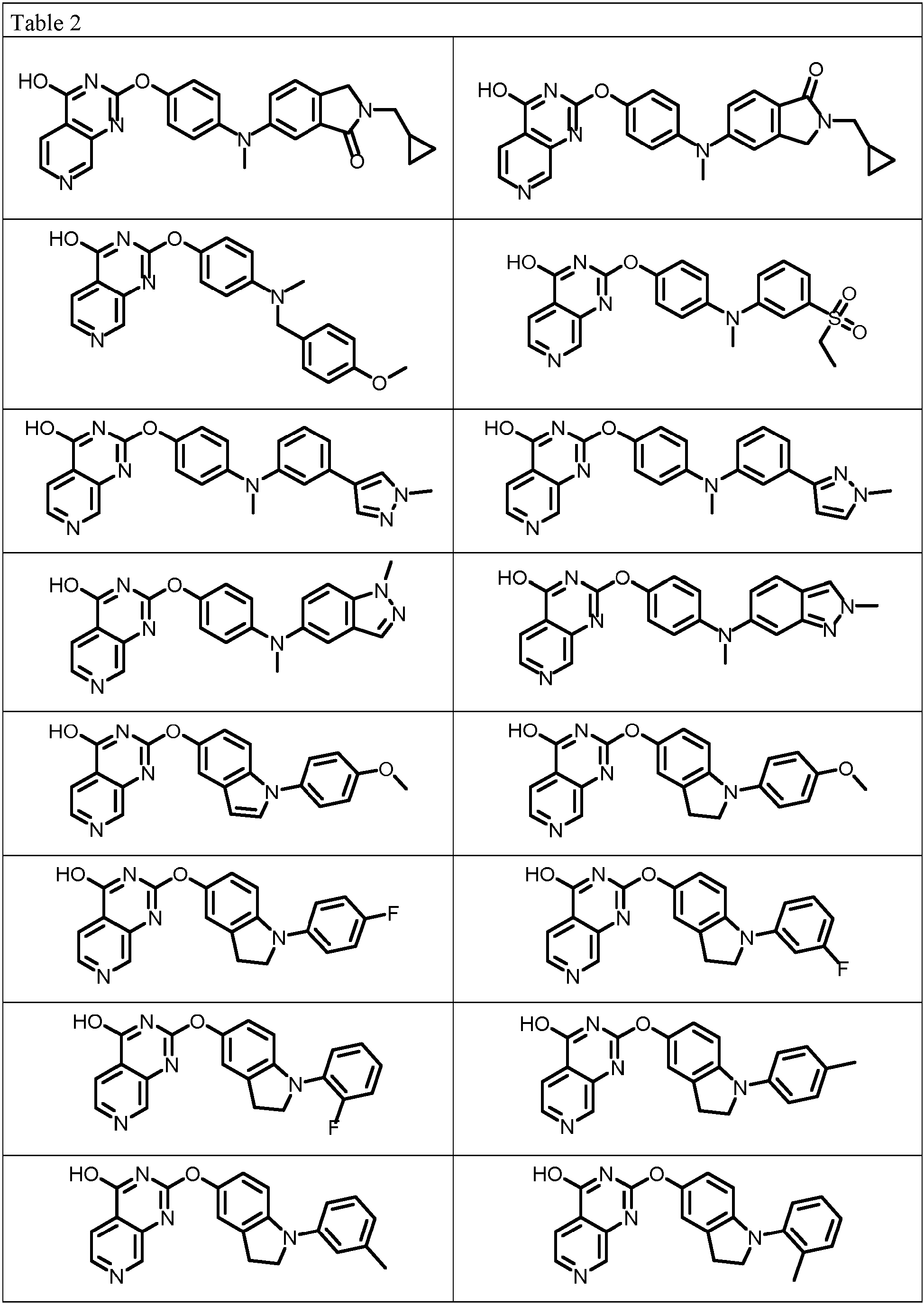

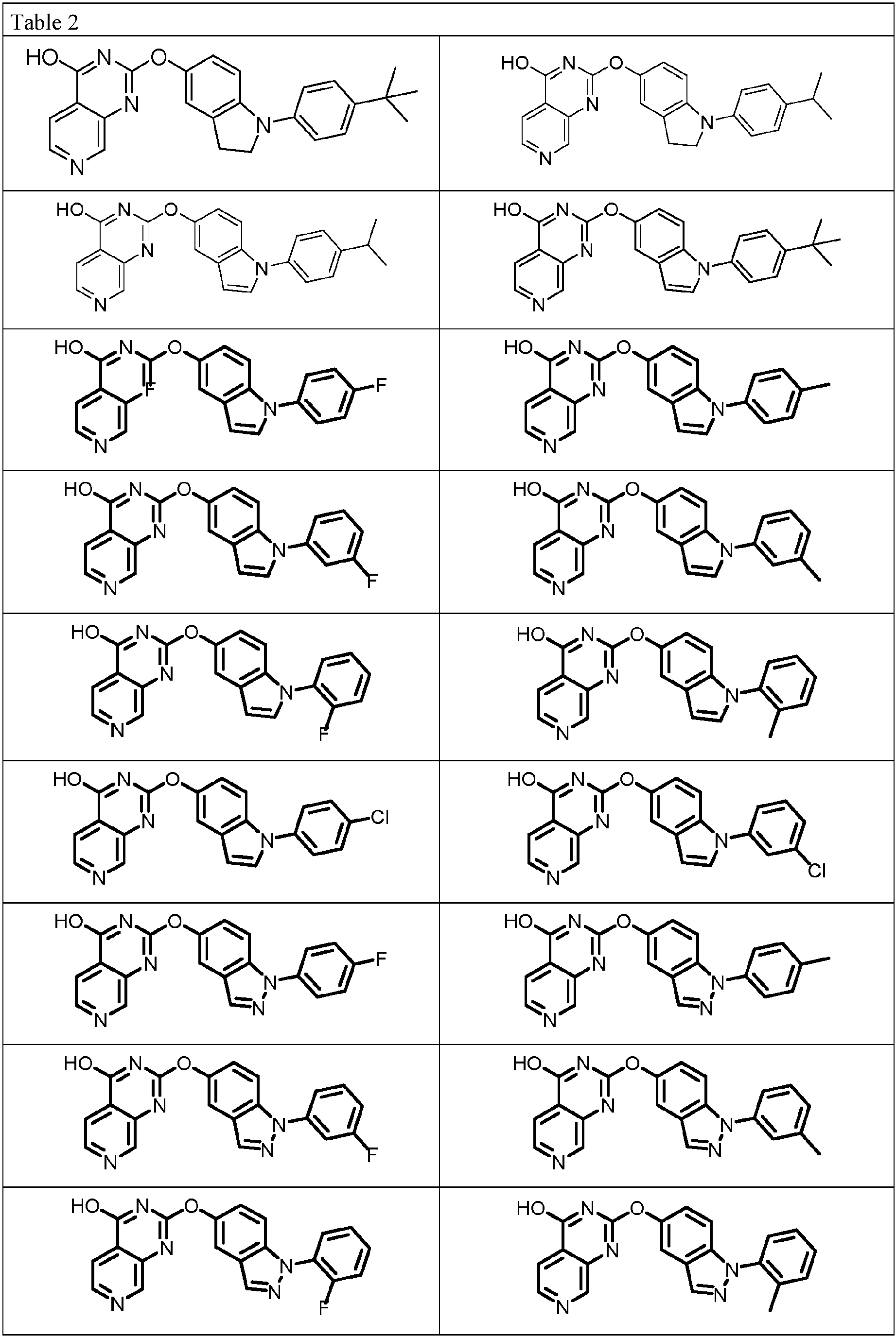

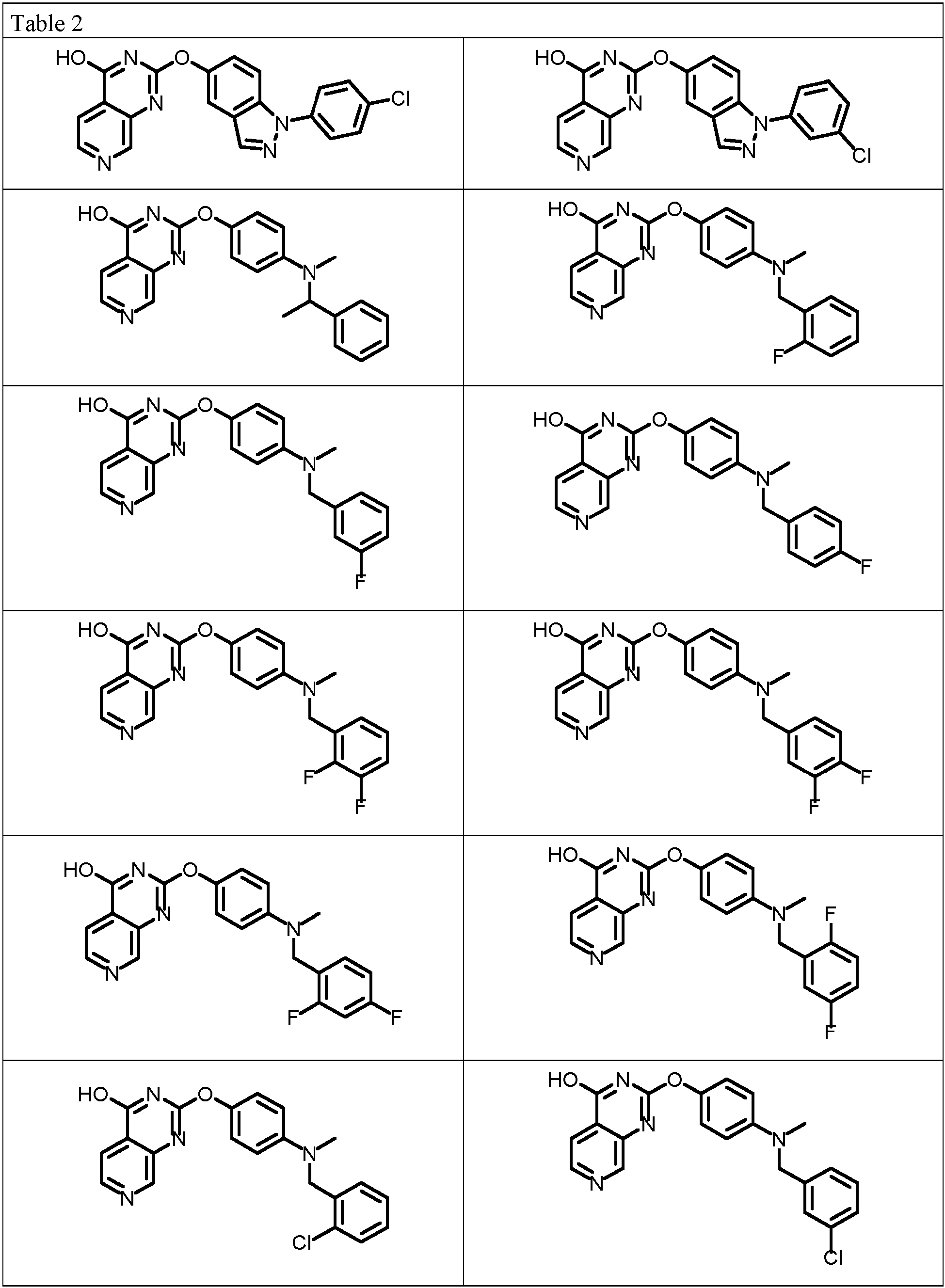

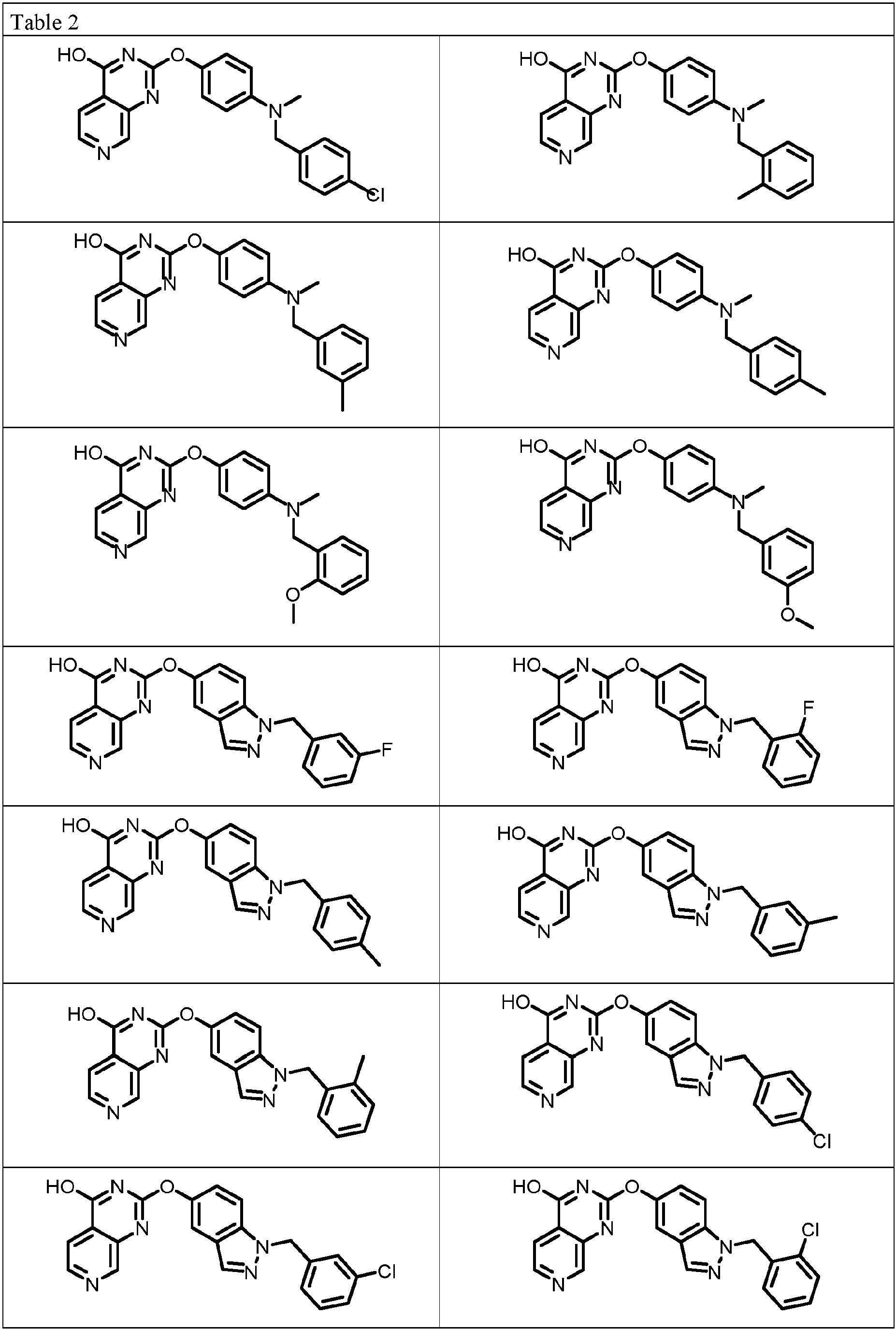

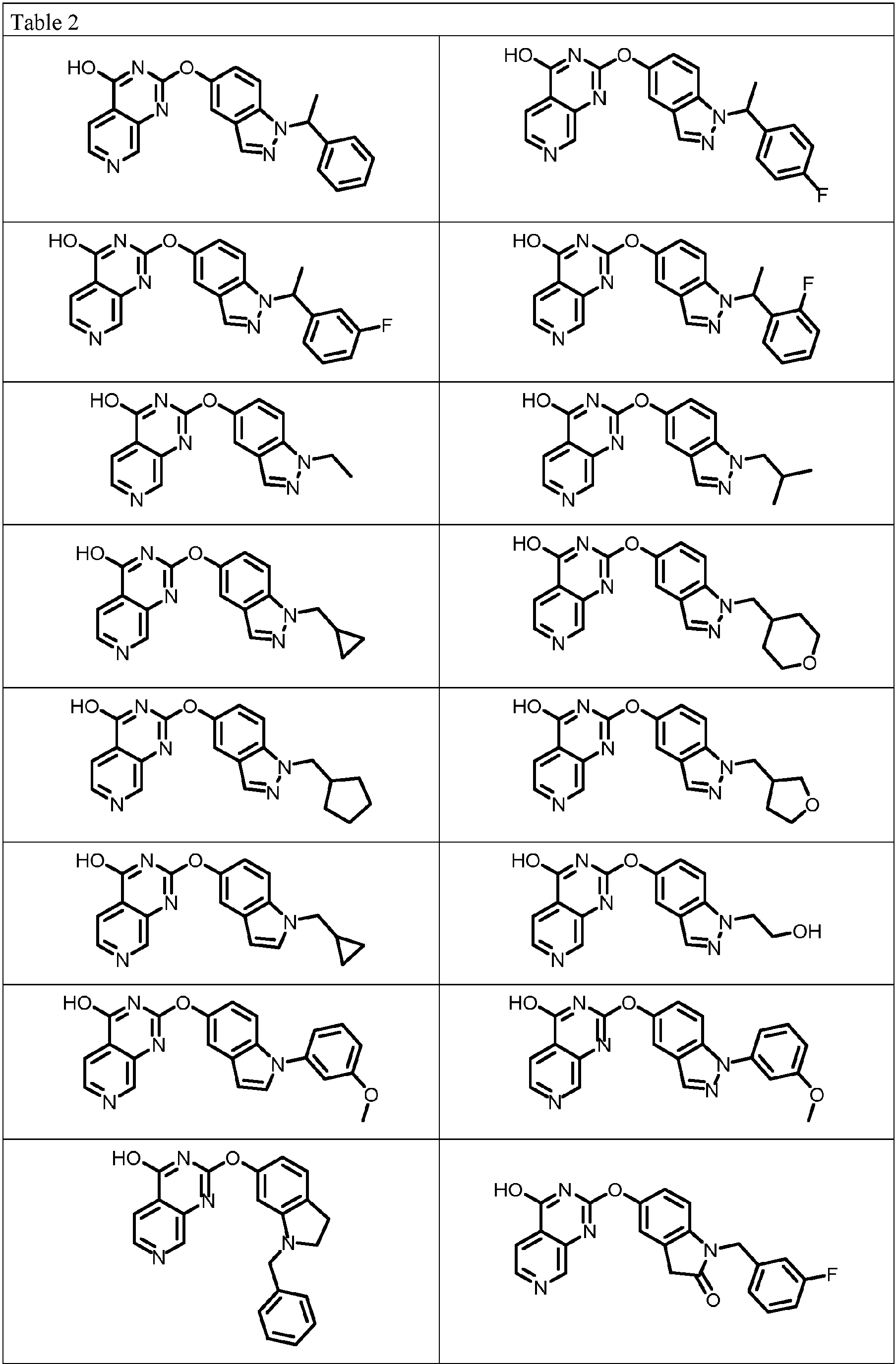

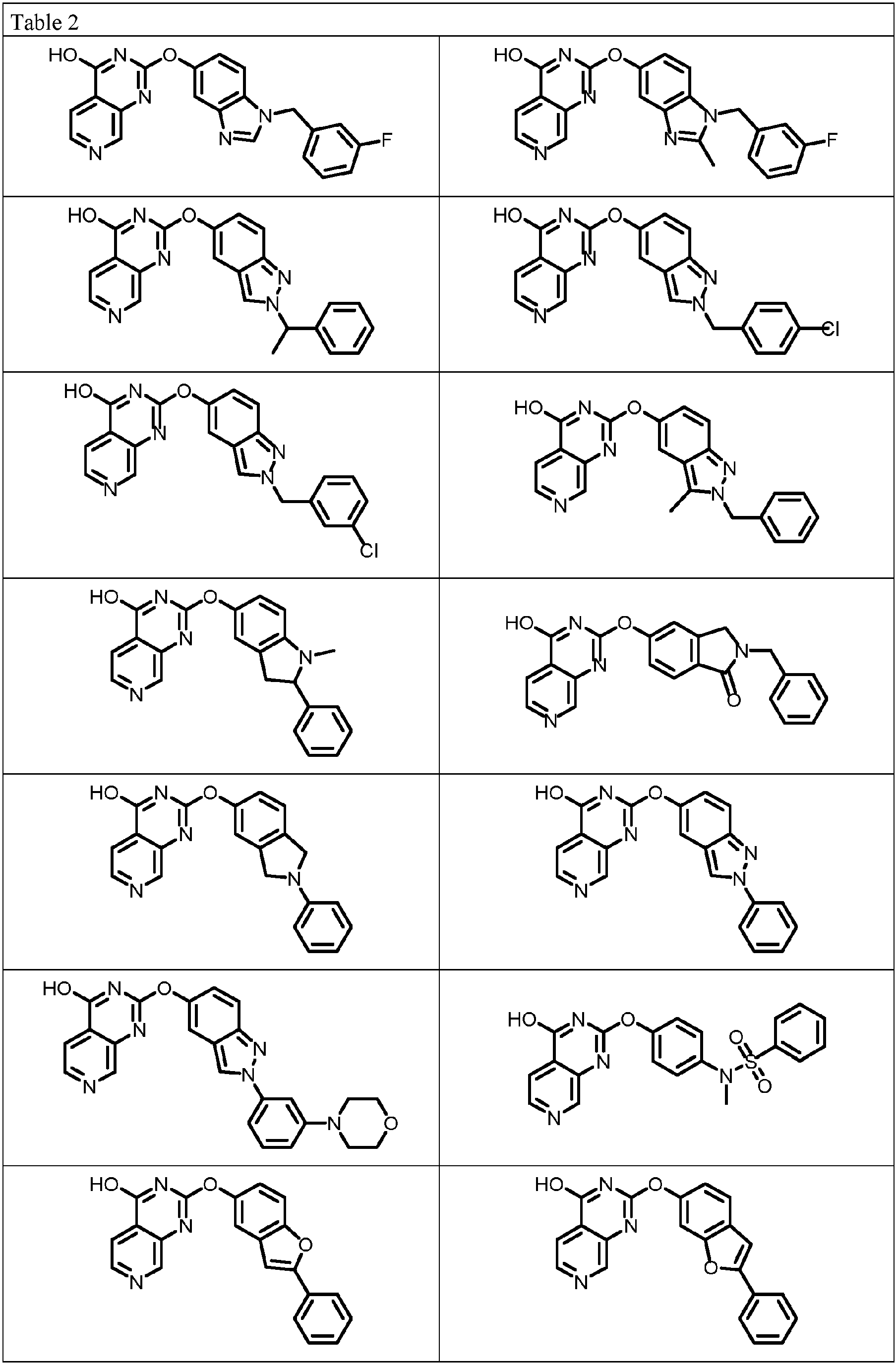

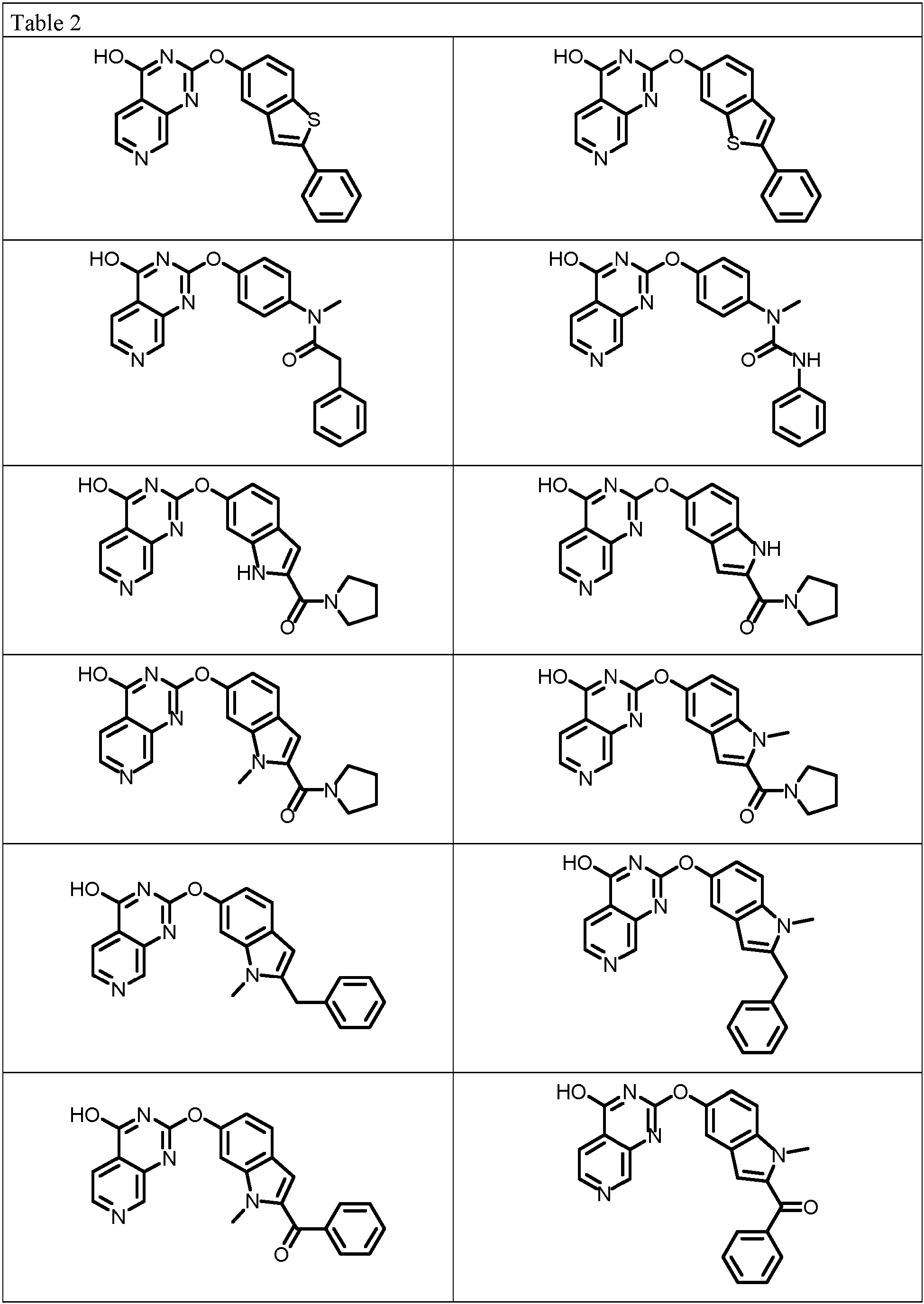

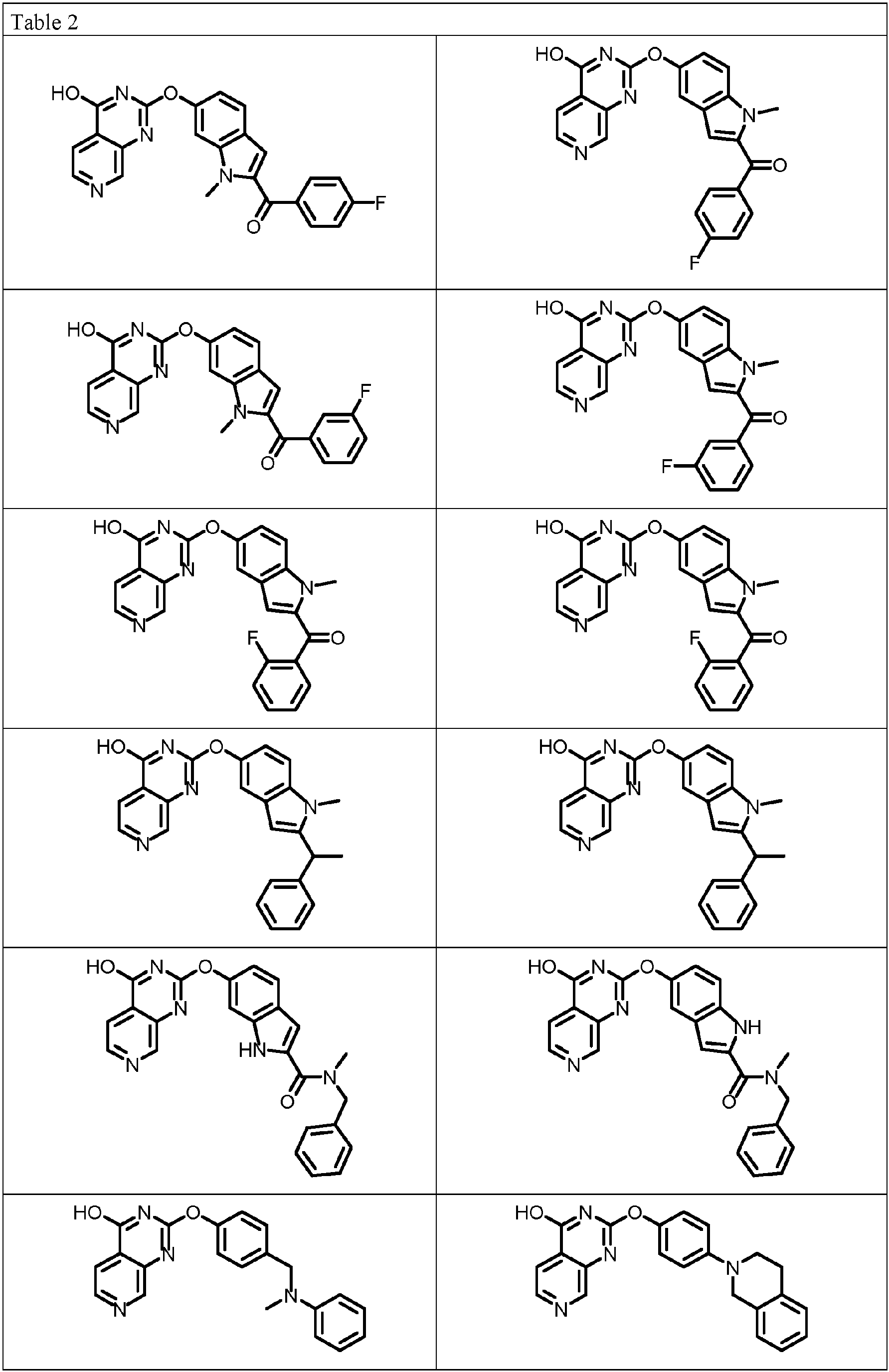

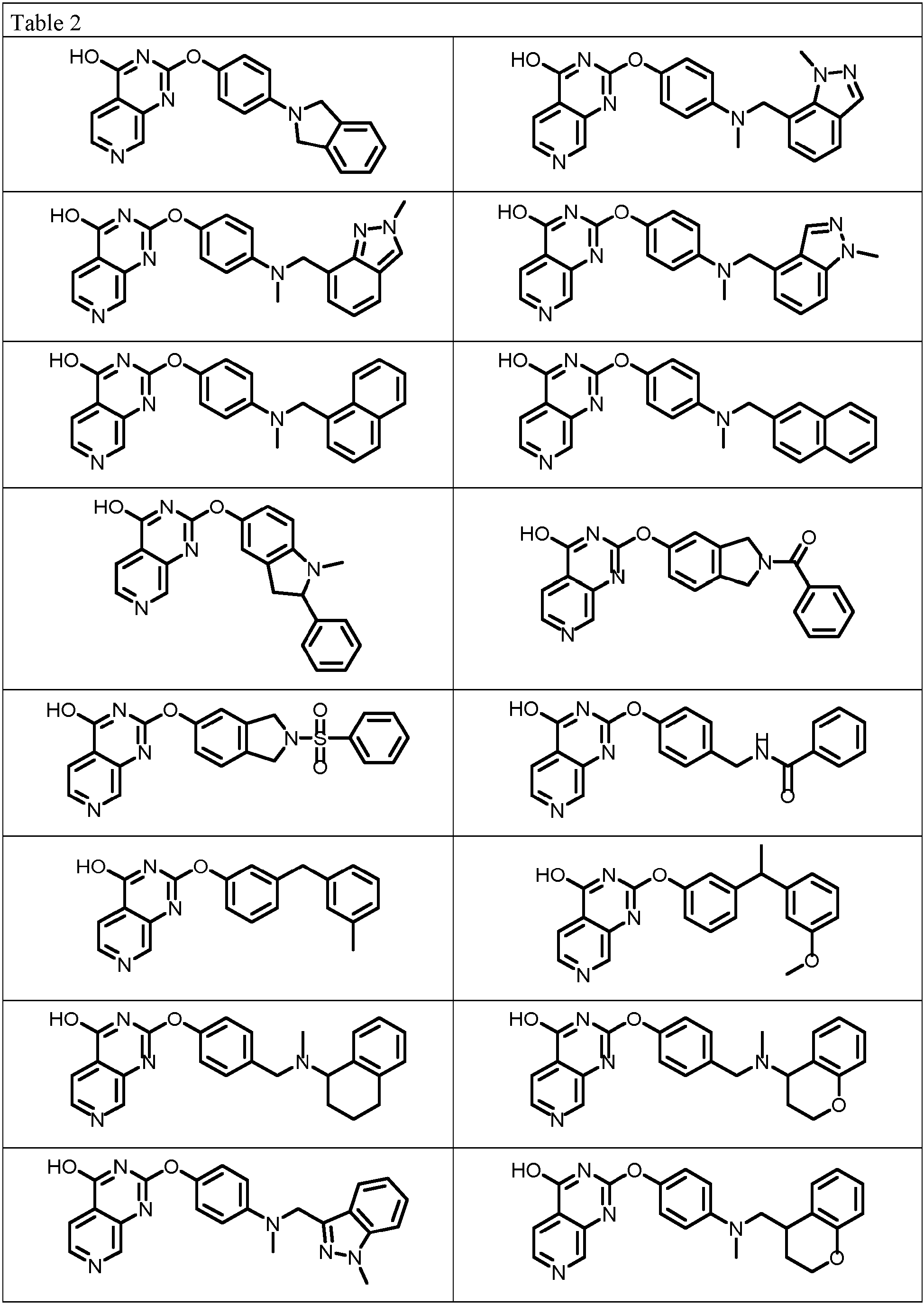

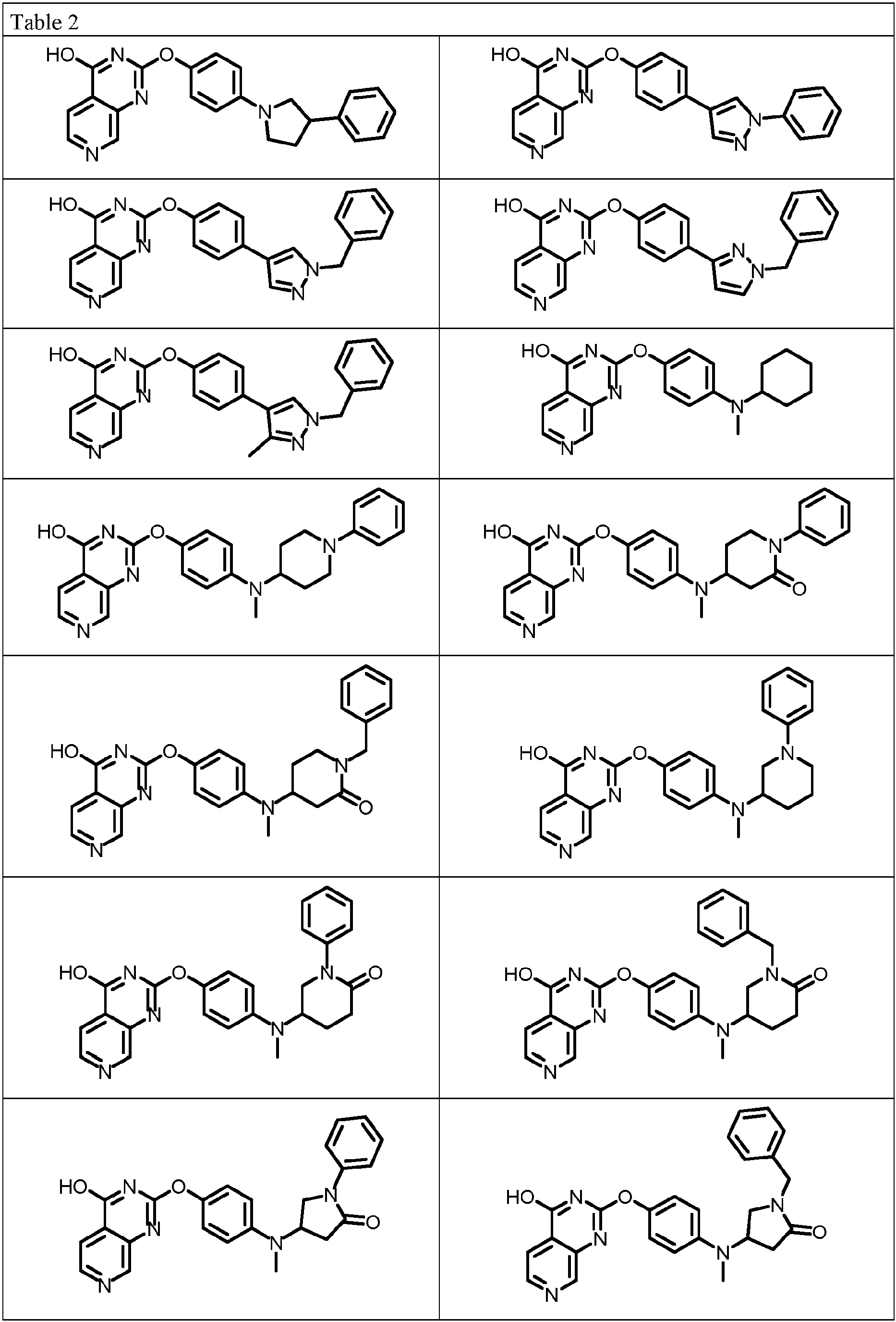

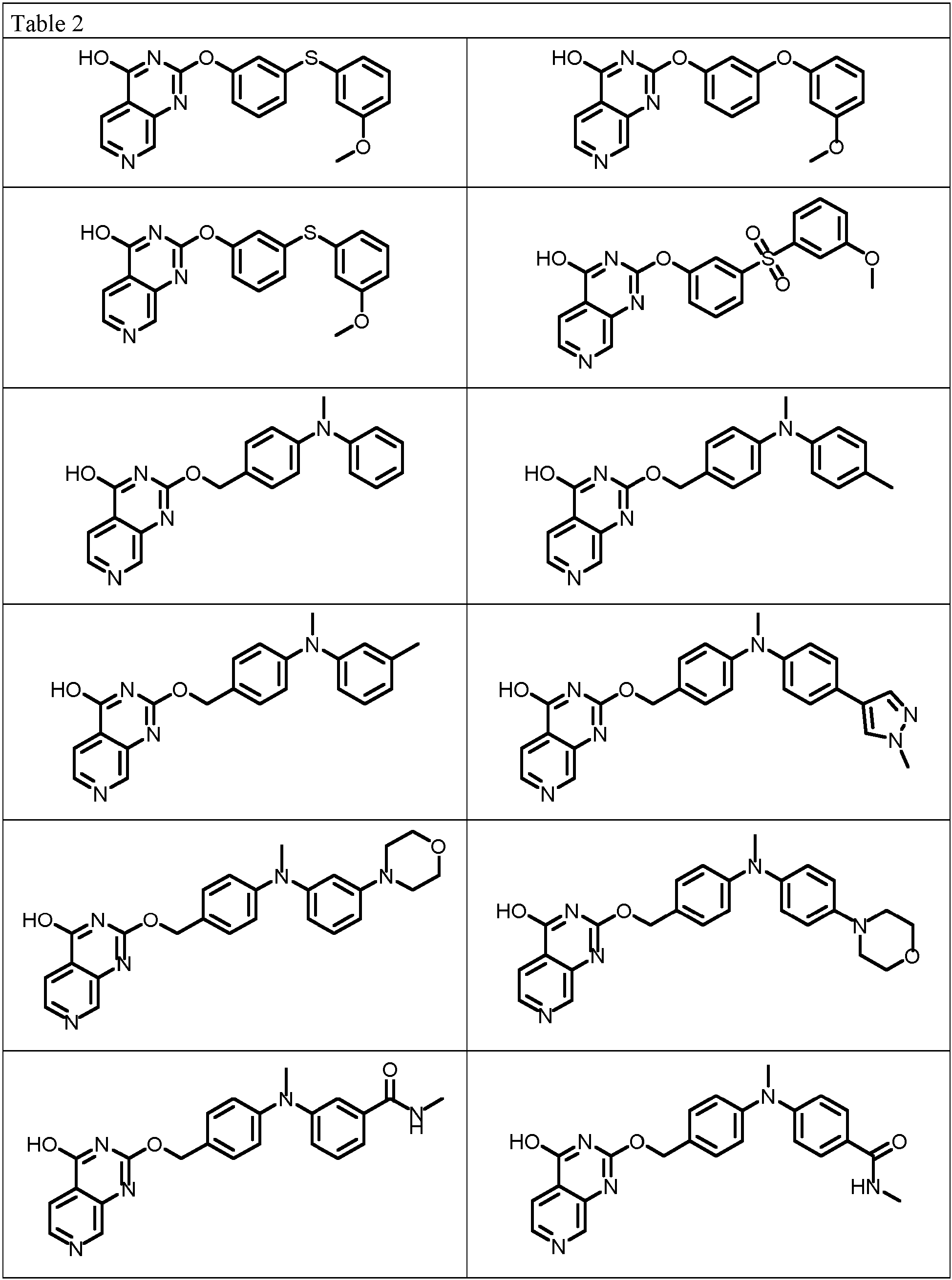

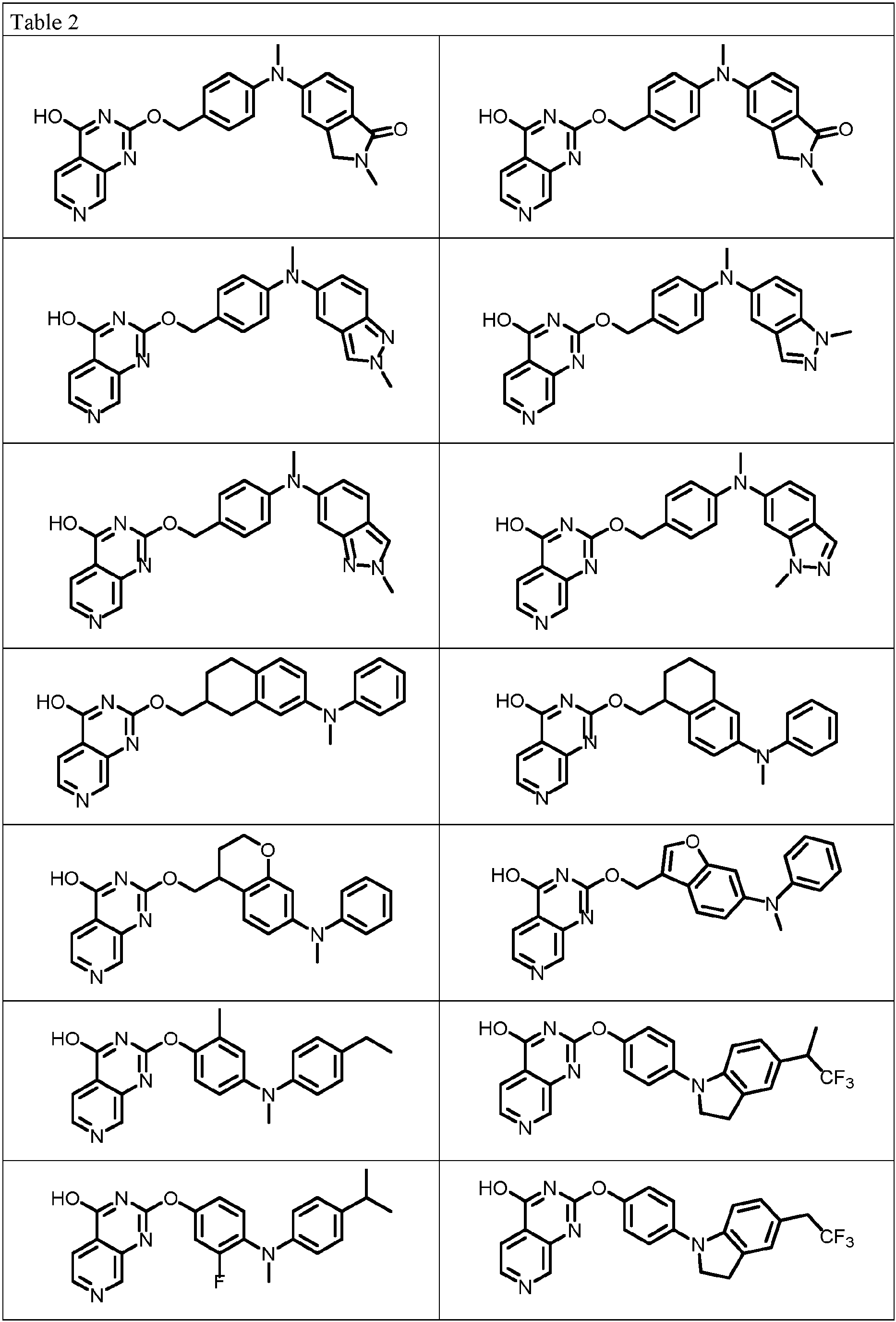

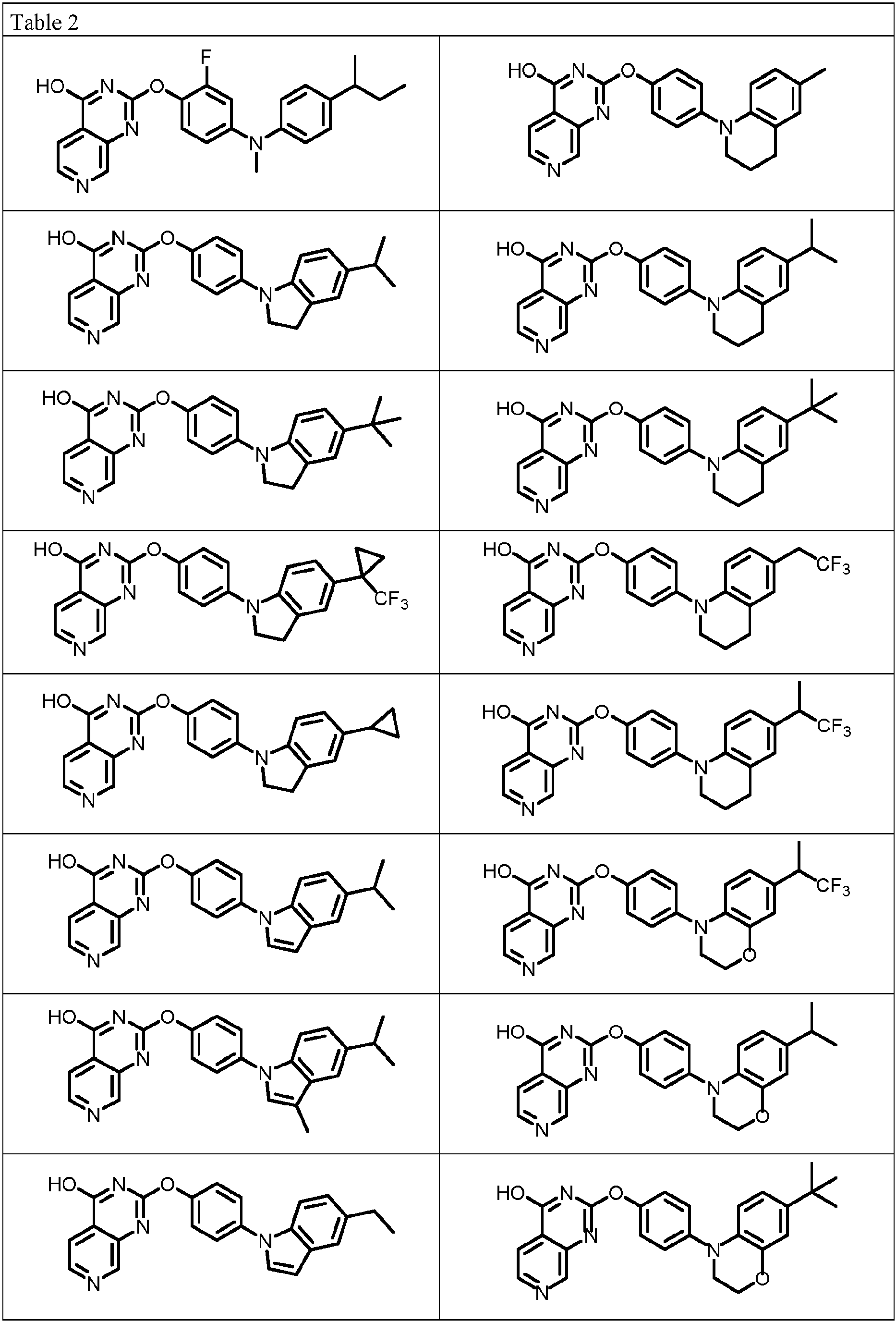

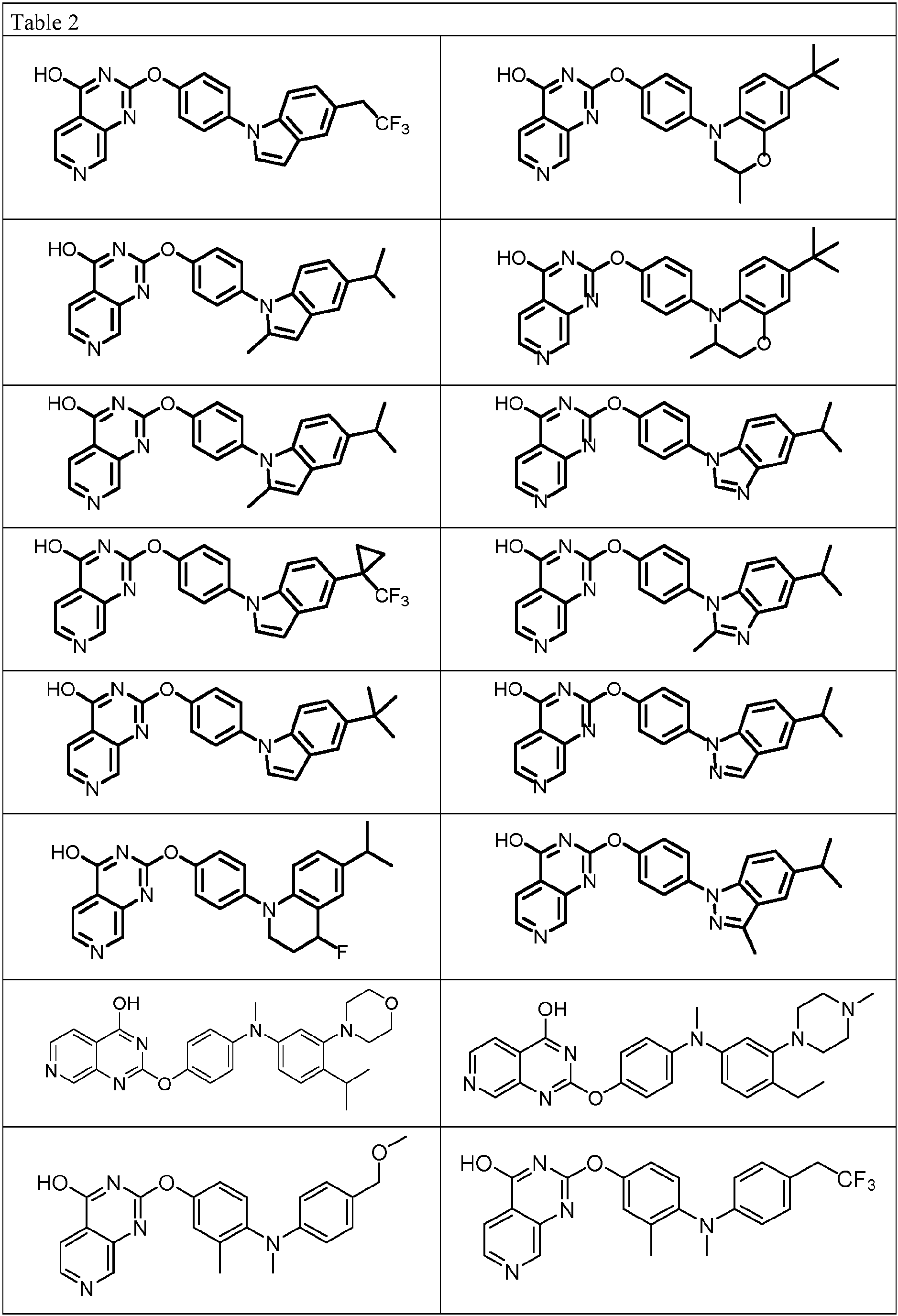

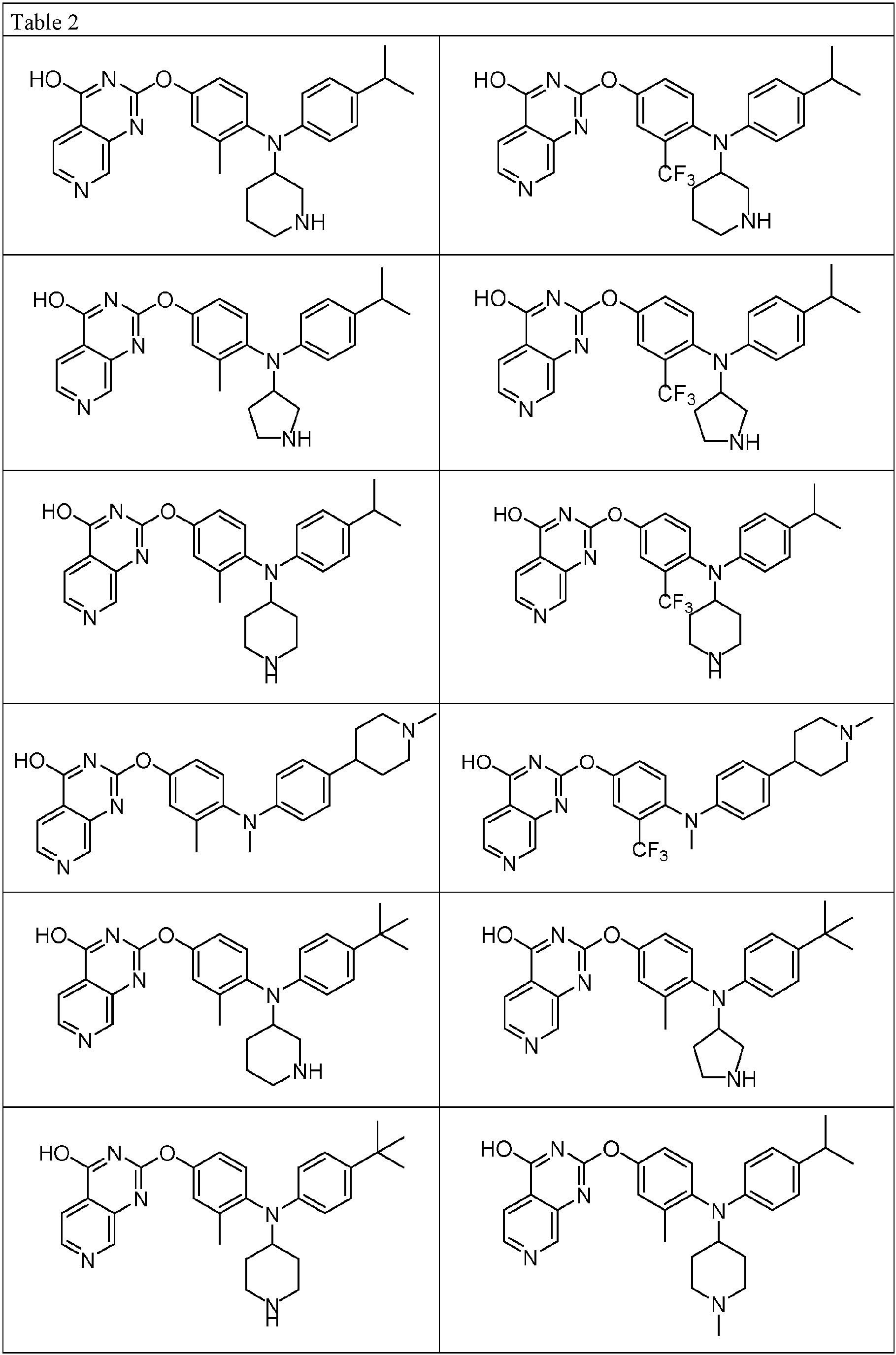

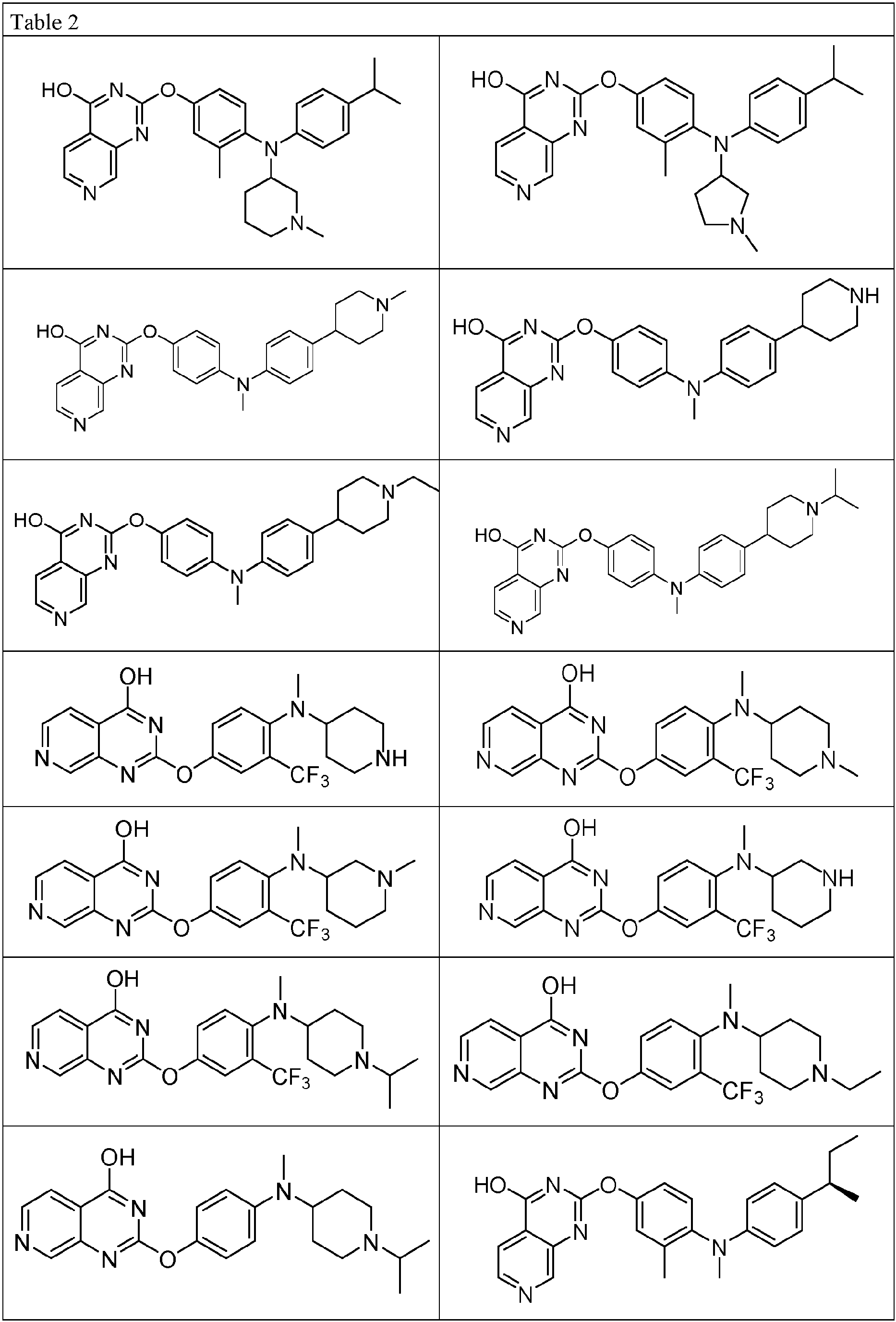

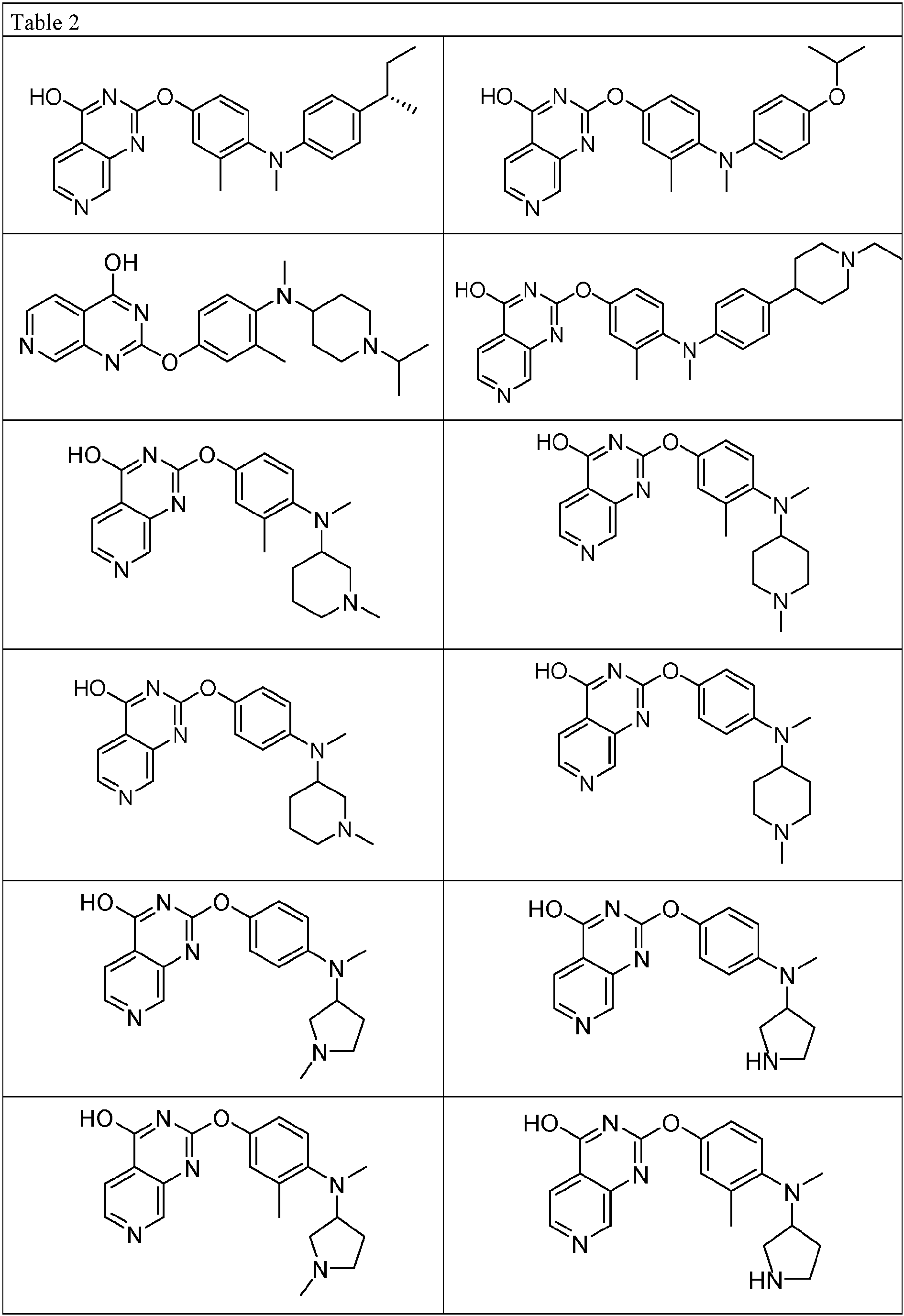

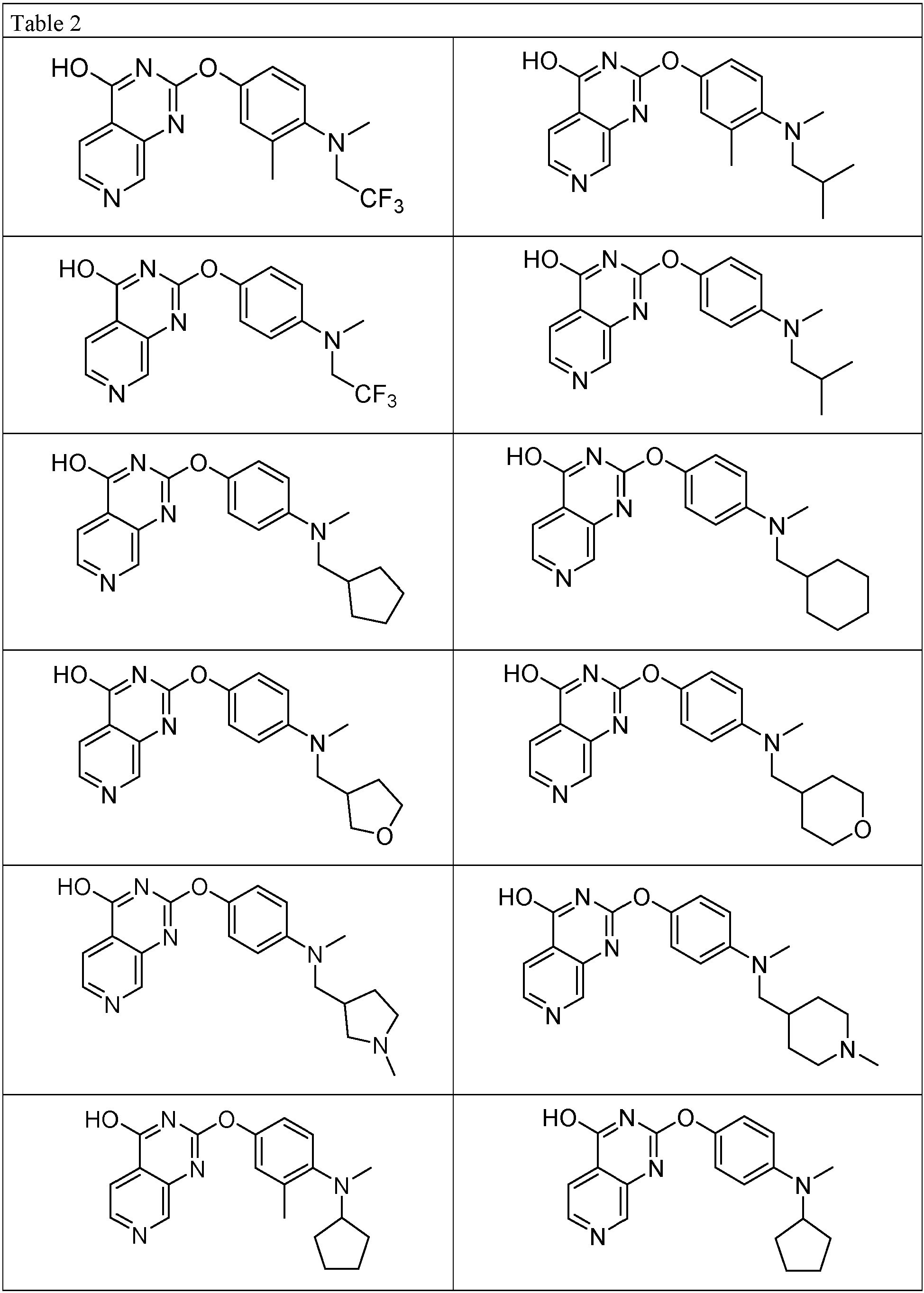

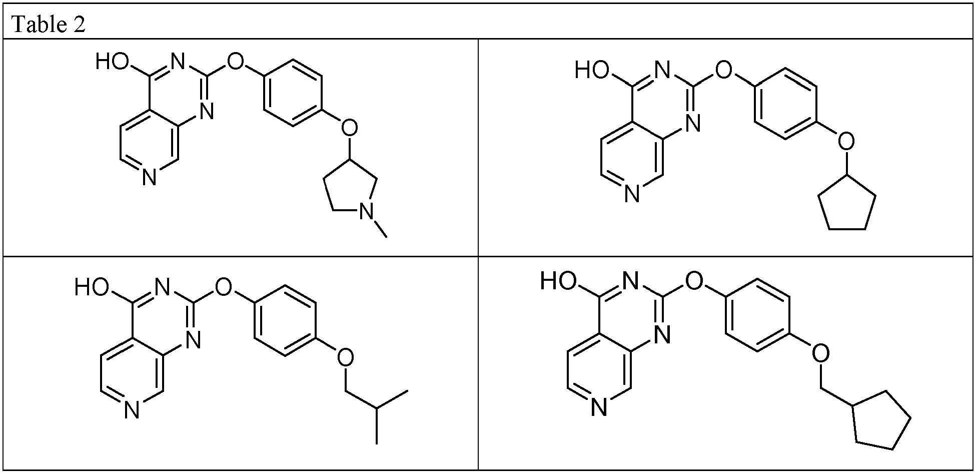

### Preparation of the Substituted Pyrido[3,4-d]pyrimidin-4-one Derivative Compounds

The compounds used in the reactions described herein are made according to organic synthesis techniques known to those skilled in this art, starting from commercially available chemicals and/or from compounds described in the chemical literature. "Commercially available chemicals" are obtained from standard commercial sources including Acros Organics (Pittsburgh, PA), Aldrich Chemical (Milwaukee, WI, including Sigma Chemical and Fluka), Apin Chemicals Ltd. (Milton Park, UK), Avocado Research (Lancashire, U.K.), BDH Inc. (Toronto, Canada), Bionet (Cornwall, U.K.), Chemservice Inc. (West Chester, PA), Crescent Chemical Co. (Hauppauge, NY), Eastman Organic Chemicals, Eastman Kodak Company (Rochester, NY), Fisher Scientific Co. (Pittsburgh, PA), Fisons Chemicals (Leicestershire, UK), Frontier Scientific (Logan, UT), ICN Biomedicals, Inc. (Costa Mesa, CA), Key Organics (Cornwall, U.K.), Lancaster Synthesis (Windham, NH), Maybridge Chemical Co. Ltd. (Cornwall, U.K.), Parish Chemical Co. (Orem, UT), Pfaltz & Bauer, Inc. (Waterbury, CN), Polyorganix (Houston, TX), Pierce Chemical Co. (Rockford, IL), Riedel de Haen AG (Hanover, Germany), Spectrum Quality Product, Inc. (New Brunswick, NJ), TCI America (Portland, OR), Trans World Chemicals, Inc. (Rockville, MD), and Wako Chemicals USA, Inc. (Richmond, VA).

Methods known to one of ordinary skill in the art are identified through various reference books and databases. Suitable reference books and treatise that detail the synthesis of reactants useful in the preparation of compounds described herein, or provide references to articles that describe the preparation, include for example, SYNTHETIC ORGANIC CHEM. (John Wiley & Sons, Inc., New York); Sandler et al., ORGANIC FUNCTIONAL GROUP PREP. (2nd Ed., Acad. Press, NY, 1983); House, MODERN SYNTHETIC REACTIONS (2nd Ed., W.A. Benjamin, Inc., Menlo Park, CA 1972); Gilchrist, HETEROCYCLIC CHEM. (2nd Ed., John Wiley & Sons, New York, 1992); March, ADVANCED ORGANIC CHEM.: REACTIONS, MECHANISMS & STRUCTURE (4th Ed., Wiley-Intersci., New York, 1992). Additional suitable reference books and treatise that detail the synthesis of reactants useful in the preparation of compounds described herein, or provide references to articles that describe the preparation, include for example, Fuhrhop and Penzlin, ORGANIC SYNTHESIS: CONCEPTS, METHODS, STARTING MATERIALS, SECOND, REVISED & ENLARGED ED. (John Wiley & Sons ISBN: 3-527-29074-5, 1994); Hoffman, ORGANIC CHEMISTRY, INTERMEDIATE TEXT (Oxford Univ. Press, ISBN 0-19-509618-5, 1996); Larock, COMPREHENSIVE ORGANIC TRANSFORMATIONS: A GUIDE TO FUNCTIONAL GROUP PREPARATIONS (2nd Ed., Wiley-VCH, ISBN: 0-471-19031-4, 1999); Otera (ed.), MODERN CARBONYL CHEMISTRY (Wiley-VCH, ISBN: 3-527-29871-1, 2000; Patai, PATAI'S 1992 GUIDE TO THE CHEMISTRY OF FUNCTIONAL GROUPS (Interscience ISBN: 0-471-93022-9); Solomons, ORGANIC CHEMISTRY (7th Ed. John Wiley & Sons, ISBN: 0-471-19095-0, 2000); Stowell, INTERMEDIATE ORGANIC CHEMISTRY (2nd Ed.,Wiley-Interscience, ISBN: 0-471-57456-2,1993); INDUSTRIAL ORGANIC CHEM.: STARTING MATS. & INTERMEDIATES: ULLMANN'S ENCYCLO. (John Wiley & Sons, ISBN: 3-527-29645-X, 1999) (in eight volumes); ORGANIC REACTIONS (1942-2000) (John Wiley & Sons) (in over 55 volumes); and CHEM. OF FUNCT. GROUPS (John Wiley & Sons) (in 73 volumes).

Specific and analogous reactants may also be identified through the indices of known chemicals prepared by the Chemical Abstract Service of the American Chemical Society, which are available in most public and university libraries, as well as through on-line databases (the American Chemical Society, Washington, DC, may be contacted for more details). Chemicals that are known but not commercially available in catalogs may be prepared by custom chemical synthesis houses, where many of the standard chemical supply houses (e.g., those listed above) provide custom synthesis services. A reference for the preparation and selection of pharmaceutical salts of the substituted pyrido[3,4-d]pyrimidin-4-one derivative compounds described herein is Stahl & Wermuth, HANDBOOK OF PHARM. (Verlag Helvetica Chimica Acta, Zurich, 2002).

The substituted pyrido[3,4-d]pyrimidin-4-one derivative compounds are prepared by the general synthetic routes described below in Scheme 1.

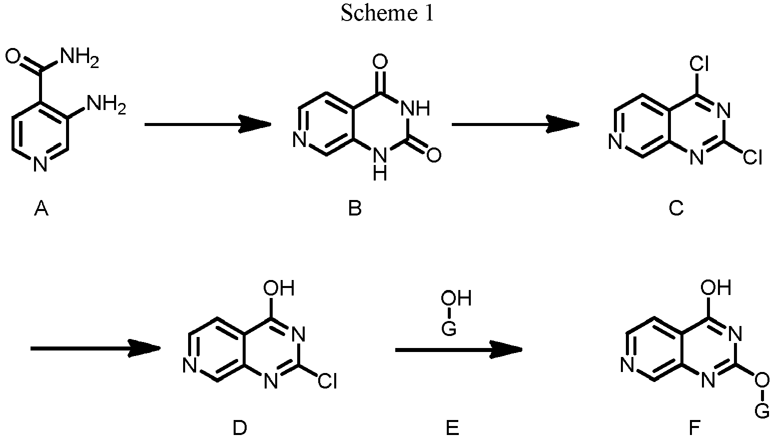

Referring to Scheme 1, compound A is converted to compound B by condensation with urea. The azaquinazolinedione compound B is converted to compound C using an appropriate chlorinating agent, such as POCl₃. Compound C is selectively hydrolyzed to form compound D under a variety of basic conditions, such as hydrolysis in a NaOH solution. Nucleophilic substitution of the chloride in compound D is carried out with an alcohol, such as G-OH, under a variety of basic conditions to form compound F. For example, compound D can be treated with the sodium salt of the alcohol E. Additionally, compound D can be heated with the alcohol or phenol G-OH in the presence of CuI and CsCO₃ in an appropriate solvent to form compound F.

In each of the above reaction procedures or schemes, the various substituents may be selected from among the various substituents otherwise taught herein.

### Pharmaceutical Compositions

In certain embodiments, a substituted pyrido[3,4-d]pyrimidin-4-one derivative compound as described by Formula (I)-(XI) is administered as a pure chemical. In other embodiments, the substituted pyrido[3,4-d]pyrimidin-4-one derivative compound as described by Formula (I)-(XI) is combined with a pharmaceutically suitable or acceptable carrier (also referred to herein as a pharmaceutically suitable (or acceptable) excipient, physiologically suitable (or acceptable) excipient, or physiologically suitable (or acceptable) carrier) selected on the basis of a chosen route of administration and standard pharmaceutical practice as described, for example, in REMINGTON: SCIENCE & PRACTICE OF PHARMACY (Gennaro, 21st Ed. Mack Pub. Co., Easton, PA, 2005), the disclosure of which is hereby incorporated herein by reference, in its entirety.

Accordingly, provided herein is a pharmaceutical composition comprising at least one substituted pyrido[3,4-d]pyrimidin-4-one derivative compound, or a stereoisomer, pharmaceutically acceptable salt, hydrate, solvate, or N-oxide thereof, together with one or more pharmaceutically acceptable carriers. The carrier(s) (or excipient(s)) is acceptable or suitable if the carrier is compatible with the other ingredients of the composition and not deleterious to the recipient (i.e., the subject) of the composition.

One embodiment provides a pharmaceutical composition comprising a pharmaceutically acceptable carrier and a compound of Formula (I) or a pharmaceutically acceptable salt thereof. One embodiment provides a pharmaceutical composition comprising a pharmaceutically acceptable carrier and a compound of Formula (II) or a pharmaceutically acceptable salt thereof. One embodiment provides a pharmaceutical composition comprising a pharmaceutically acceptable carrier and a compound of Formula (III) or a pharmaceutically acceptable salt thereof. One embodiment provides a pharmaceutical composition comprising a pharmaceutically acceptable carrier and a compound of Formula (IV) or a pharmaceutically acceptable salt thereof. One embodiment provides a pharmaceutical composition comprising a pharmaceutically acceptable carrier and a compound of Formula (V) or a pharmaceutically acceptable salt thereof. One embodiment provides a pharmaceutical composition comprising a pharmaceutically acceptable carrier and a compound of Formula (VI) or a pharmaceutically acceptable salt thereof. One embodiment provides a pharmaceutical composition comprising a pharmaceutically acceptable carrier and a compound of Formula (VII) or a pharmaceutically acceptable salt thereof. One embodiment provides a pharmaceutical composition comprising a pharmaceutically acceptable carrier and a compound of Formula (VIII) or a pharmaceutically acceptable salt thereof. One embodiment provides a pharmaceutical composition comprising a pharmaceutically acceptable carrier and a compound of Formula (IX) or a pharmaceutically acceptable salt thereof. One embodiment provides a pharmaceutical composition comprising a pharmaceutically acceptable carrier and a compound of Formula (X) or a pharmaceutically acceptable salt thereof. One embodiment provides a pharmaceutical composition comprising a pharmaceutically acceptable carrier and a compound of Formula (XI) or a pharmaceutically acceptable salt thereof.

In certain embodiments, the substituted pyrido[3,4-d]pyrimidin-4-one derivative compound as described by Formula (I)-(XI) is substantially pure, in that it contains less than about 5%, or less than about 1%, or less than about 0.1%, of other organic small molecules, such as contaminating intermediates or by-products that are created, for example, in one or more of the steps of a synthesis method.

Suitable oral dosage forms include, for example, tablets, pills, sachets, or capsules of hard or soft gelatin, methylcellulose or of another suitable material easily dissolved in the digestive tract. Suitable nontoxic solid carriers can be used which include, for example, pharmaceutical grades of mannitol, lactose, starch, magnesium stearate, sodium saccharin, talcum, cellulose, glucose, sucrose, magnesium carbonate, and the like. *See*, *e.g.*, REMINGTON, 2005.

The dose of the composition comprising at least one substituted pyrido [3,4-d]pyrimidin-4-one derivative compound as described herein may differ, depending upon the patient's (e.g., human) condition, that is, stage of the disease, general health status, age, and other factors that a person skilled in the medical art will use to determine dose.

Pharmaceutical compositions may be administered in a manner appropriate to the disease to be treated (or prevented) as determined by persons skilled in the medical arts. An appropriate dose and a suitable duration and frequency of administration will be determined by such factors as the condition of the patient, the type and severity of the patient's disease, the particular form of the active ingredient, and the method of administration. In general, an appropriate dose and treatment regimen provides the composition(s) in an amount sufficient to provide therapeutic and/or prophylactic benefit (e.g., an improved clinical outcome, such as more frequent complete or partial remissions, or longer disease-free and/or overall survival, or a lessening of symptom severity. Optimal doses may generally be determined using experimental models and/or clinical trials. The optimal dose may depend upon the body mass, weight, or blood volume of the patient.

Oral doses can typically range from about 1.0 mg to about 1000 mg, one to four times, or more, per day.

### Histone Demethylase

Chromatin is the complex of DNA and protein that makes up chromosomes. Histones are the major protein component of chromatin, acting as spools around which DNA winds. Changes in chromatin structure are affected by covalent modifications of histone proteins and by non-histone binding proteins. Several classes of enzymes are known which can covalently modify histones at various sites.

Proteins can be post-translationally modified by methylation on amino groups of lysines and guanidino groups of arginines or carboxymethylated on aspartate, glutamate, or on the C-terminus of the protein. Post-translational protein methylation has been implicated in a variety of cellular processes such as RNA processing, receptor mediated signaling, and cellular differentiation. Post-translational protein methylation is widely known to occur on histones, such reactions known to be catalyzed by histone methyltransferases, which transfer methyl groups from S-adenyosyl methionine (SAM) to histones. Histone methylation is known to participate in a diverse range of biological processes including heterochromatin formation, X-chromosome inactivation, and transcriptional regulation (Lachner et al., (2003) J. Cell Sci. 116:2117-2124; Margueron et al., (2005) Curr. Opin. Genet. Dev. 15:163-176).

Unlike acetylation, which generally correlates with transcriptional activation, whether histone methylation leads to transcription activation or repression depends on the particular site of methylation and the degree of methylation (e.g., whether a particular histone lysine residue is mono-, di-, or tri-methylated). Generally, however, methylation on H3K9, H3K27 and H4K20 is linked to gene silencing, while methylation on H3K4, H3K36, and H3K79 is generally associated with active gene expression. In addition, tri- and di-methylation of H3K4 generally marks the transcriptional start sites of actively transcribed genes, whereas monomethylation of H3K4 is associated with enhancer sequences.

A "demethylase" or "protein demethylase," as referred to herein, refers to an enzyme that removes at least one methyl group from an amino acid side chain. Some demethylases act on histones, e.g., act as a histone H3 or H4 demethylase. For example, an H3 demethylase may demethylate one or more of H3K4, H3K9, H3K27, H3K36 and/or H3K79. Alternately, an H4 demethylase may demethylate histone H4K20. Demethylases are known which can demethylate either a mono-, di- and/or a tri-methylated substrate. Further, histone demethylases can act on a methylated core histone substrate, a mononucleosome substrate, a dinucleosome substrate and/or an oligonucleosome substrate, peptide substrate and/or chromatin (e.g., in a cell-based assay).

The first lysine demethylase discovered was lysine specific demethylase 1 (LSD1/KDM1), which demethylates both mono- and di-methylated H3K4 or H3K9, using flavin as a cofactor. A second class of Jumonji C (JmjC) domain containing histone demthylases were predicted, and confirmed when a H3K36 demethylase was found using a formaldehyde release assay, which was named JmjC domain containing histone demethylase 1 (JHDM1/KDM2A).

More JmjC domain-containing proteins were subsequently identified and they can be phylogenetically clustered into seven subfamilies: JHDM1, JHDM2, JHDM3, JMJD2, JARID, PHF2/PHF8, UTX/UTY, and JmjC domain only.

### JMJD2 Family

The JMJD2 family of proteins are a family of histone-demethylases known to demethylate tri- and di-methylated H3-K9, and were the first identified histone tri-methyl demethylases. In particular, ectopic expression of JMJD2 family members was found to dramatically decrease levels of tri-and di-methylated H3-K9, while increasing levels of mono-methylated H3- K9, which delocalized Heterochromatin Protein 1 (HP1) and reduced overall levels of heterochromatin in vivo. Members of the JMJD2 subfamily of jumonji proteins include JMJD2C and its homologues JMJD2A, JMJD2B, JMJD2D and JMJD2E. Common structural features found in the JMJD2 subfamily of Jumonji proteins include the JmjN, JmjC, PHD and Tdr sequences.

JMJD2C, also known as GASC1 and KDM4C, is known to demethylate tri-methylated H3K9 and H3K36. Histone demethylation by JMJD2C occurs via a hydroxylation reaction dependent on iron and α-ketoglutarate, wherein oxidative decarboxylation of α-ketoglutarate by JMJD2C produces carbon dioxide, succinate, and ferryl and ferryl subsequently hydroxylates a methyl group of lysine H3K9, releasing formaldehyde. JMJD2C is known to modulate regulation of adipogenesis by the nuclear receptor PPARγ and is known to be involved in regulation of self-renewal in embryonic stem cells.

### JARID Family

As used herein, a "JARID protein" includes proteins in the JARID 1 subfamily (e.g., JARID 1A, JARID 1B, JARID 1C and JARID 1D proteins) and the JARID2 subfamily, as well as homologues thereof. A further description and listing of JARID proteins can be found in Klose et al. (2006) Nature Reviews/Genetics 7:715-727. The JARID1 family contains several conserved domains: JmjN, ARID, JmjC, PHD and a C5HC2 zing finger.

JARID 1A, also called KDM5A or RBP2, was initially found as a binding partner of retinoblastoma (Rb) protein. JARID1A was subsequently found to function as a demethylase of tri- and di-methylated H3K4 , and has been found to promote cell growth, while inhibiting senescence and differentiation. For instance, abrogation of JARID1A from mouse cells inhibits cell growth, induces senescence and differentiation, and causes loss of pluripotency of embryonic stem cells in vitro. JARID1A has been found to be overexpressed in gastric cancer and the loss of JARID1A has been found to reduce tumorigenesis in a mouse cancer model. Additionally, studies have demonstrated that loss of the retinoblastome binding protein 2 (RBP2) histone demethylase suppresses tumorigenesis in mice lacking *Rb1* or *Men1* (Lin etal. Proc. Natl. Acad. Sci. USA, August 16, 2011, 108(33),13379-86; doi: 10.1073/pnas.1110104108) and lead to the conclusion that RBP2-inhibitory drugs would have anti-cancer activity.

JARID1B, also referred to as KDM5B and PLU1, was originally found in experiments to discover genes regulated by the HER2 tyrosine kinase. JARID 1B has consistently been found to be expressed in breast cancer cell lines, although restriction of JARID 1B has been found in normal adult tissues, with the exception of the testis. In addition, 90% of invasive ductal carcinomas have been found to express JARID1B. In addition, JARID1B has been found to be up-regulated in prostate cancers, while having more limited expression in benign prostate, and has also been found to be up-regulated in bladder cancer and lung cancer (both SCLC and NSCLC). JARID1B has also been found to repress tumor suppressor genes such as BRCA1, CAV1 and 14-3-3σ, and knockdown of JARID1B was found to increase the levels of tri-methylated H3K4 at these genes.

Other embodiments and uses will be apparent to one skilled in the art in light of the present disclosures. The following examples are provided merely as illustrative of various embodiments and shall not be construed to limit the invention in any way.

### EXAMPLES

### I. Chemical Synthesis

Unless otherwise noted, reagents and solvents were used as received from commercial suppliers. Anhydrous solvents and oven-dried glassware were used for synthetic transformations sensitive to moisture and/or oxygen. Yields were not optimized. Reaction times are approximate and were not optimized. Column chromatography and thin layer chromatography (TLC) were performed on silica gel unless otherwise noted. Spectra are given in ppm (δ) and coupling constants, J are reported in Hertz. For proton spectra the solvent peak was used as the reference peak. Examples 1-118 are reference examples.

### Preparation I: 2-Chloropyrido[3,4-d]pyrimidin-4-ol

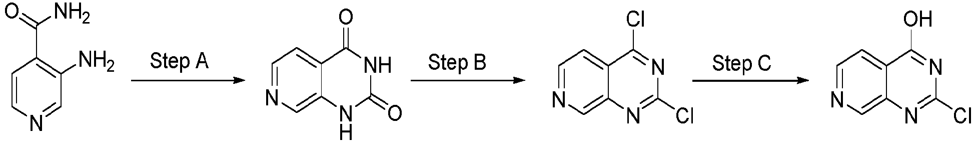

### Step A: Pyrido[3,4-d]pyridine-2,4(1H,3H)-dione

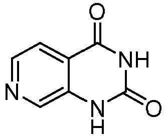

To a solution of 3-aminopyridine-4-carboxamide (5 g, 36.5 mmol) in THF (100 mL) was added triphosgene (11.9 g, 40.1 mmol) and TEA (7.4 g, 73 mmol). The reaction mixture was refluxed for 2h. The solution was concentrated *in vacuo* and the residue was triturated in water. The solid was filtered and washed with water and THF. The solid was dried to afford 4.1g (70%) of the title compound. ¹H NMR (400 MHz, DMSO-*d₆*): δ 11.62 (s, 1H), 11.58 (s, 1H), 8.66 (s, 1H), 8.40 (d, 1H, *J* = 5.2Hz), 7.80 (d, 1H, *J* = 5.2 Hz).

### Step B: 2,4-Dichloropyrido[3,4-d]pyrimidine

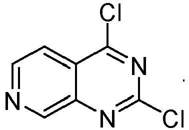

To a mixture of pyrido[3,4-*d*]pyridine-2,4(1H,3H)-dione (2 g, 12.3 mmol) in toluene (50 mL) was added DIEA (3.15 g, 25 mmol) and POCl₃ (9.5 g, 61.4 mmol). The reaction mixture was refluxed overnight. The solution was concentrated *in vacuo* and the residue was taken in ethyl acetate and washed with aq. NaHCO₃ and brine. The organics were dried and concentrated. The residue was purified by silica gel chromatography (25% EA:PE) to afford 1g (41%) of the title compound. ¹H NMR (400 MHz, DMSO-*d₆*): δ 9.50 (s, 1H), 8.90 (d, 1H, *J* = 5.2Hz), 8.02 (d, 1H, *J* = 5.2Hz).

### Step C: 2-Chloropyrido[3,4-d]pyrimidin-4-ol

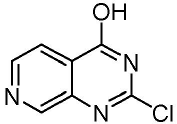

To a solution of 2,4-dichloropyrido[3,4-d]pyrimidine (1 g, 5 mmol) in THF (20 mL) was added a solution of NaOH (0.5 g, 12.5 mmol) in water (20 mL). The reaction mixture was stirred at r.t. for 2 h. The solution was adjusted to pH=2 using 5N HCl and the resulting precipitate was filtered and washed with water and THF, and dried to afford 0.8g (88%) of the title compound. ¹H NMR (400 MHz, DMSO-*d₆*): δ 13.61 (s, 1H), 8.99 (s, 1H), 8.69 (d, 1H, *J* = 5.2 Hz ), 7.94 (d, 1H, *J* = 5.2 Hz).

### Preparation 1A: (4-Methoxy-phenyl)-pyridin-2-yl-amine

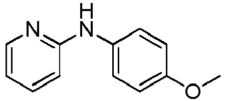

A mixture of 2-bromo-pyridine (2.00 g, 12.7 mmol), 4-methoxy-phenylamine (1.56 g, 12.7 mmol), Pd(OAc)₂ (290 mg, 1.27 mmol), BINAP (791 mg, 1.27 mmol) and *t*-BuOK (2.84 g, 25.3 mmol) in toluene (30 mL) was stirred under nitrogen atmosphere for 4 h at 125 °C. The reaction mixture was concentrated. The residue was purified by silica gel chromatography (PE:EA 15:1) to give 1.78 g (70%) of the title compound. ¹H NMR (400 MHz, CDCl₃): δ 3.81 (s, 3H), 6.47 (s, 1H), 6.67-6.68 (m, 2H), 6.88-6.92 (m, 2H), 7.21-7.26 (m, 2H), 7.41-7.45 (m, 1H), 8.14 (d, *J* = 4.0 Hz, 1H). [M+H] Calc'd for C₁₉H₁₅N₅O₂, 201; Found, 201.

### Preparation 1B: (4-Methoxy-phenyl)-methyl-pyridin-2-yl-amine

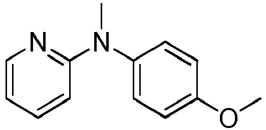

To a solution of (4-methoxy-phenyl)-pyridin-2-yl-amine (1.78 g, 8.9 mmol) in DMF (20 mL) was added *t*-BuOK (2.0 g, 17.8 mmol) at 0 °C. After stirring for 30 min, MeI (2.53 g, 17.8 mmol) was added dropwise over 10 min and the reaction mixture was stirred at rt overnight. The reaction mixture was diluted with water (100 mL), extracted with DCM (30 mL^{∗}3). The combined organic layers were washed with water (150 mL^{∗}3), brine (150 mL), dried over Na₂SO₄, and concentrated to give 1.47g (77%) of the title product. ¹H NMR (400 MHz, CDCl₃): δ 3.42 (s, 3H), 3.83 (s, 3H), 6.37 (d, *J=* 8.8 Hz, 1H), 6.54-6.57 (m, 1H), 6.93-6.95 (m, 2H), 7.16-7.19 (m, 2H), 7.26-7.28 (m, 1H), 8.19-8.21 (m, 1H). [M+H] Calc'd for C₁₃H₁₄N₂O, 215; Found, 215.

### Preparation 1C: 4-(Methyl-pyridin-2-yl-amino)-phenol

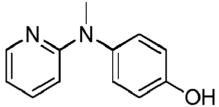

To a solution of (4-methoxy-phenyl)-methyl-pyridin-2-yl-amine (600 mg, 2.8 mmol) in DCM (12 mL) was added dropwise BBr₃ (28 mL, 28 mmol, 1M in DCM) at 0 °C. The reaction mixture was stirred for 30 min and carefully quenched with MeOH at 0 °C. The volatiles were concentrated *in vacuo.* The residue was dissolved in DCM (20 mL), washed with saturated NaHCO₃ solution (50 mL) and brine (50 mL), dried over Na₂SO₄, and concentrated to give 450 mg (80%) of the title product. ¹H NMR (400 MHz, CDCl₃): δ 3.41 (s, 3H), 6.41 (d, *J* = 8.8 Hz, 1H), 6.56-6.59 (m, 1H), 6.85-6.87 (m, 2H), 7.08-7.11 (m, 2H), 7.29-7.33 (m, 1H), 8.17-8.19 (m, 1H). [M+H] Calc'd for C₁₂H₁₂N₂O, 200; Found, 201.

### Example 1: 2-[4-(Methyl-pyridin-2-yl-amino)-phenoxy]-pyrido[3,4-d]pyrimidin-4-ol

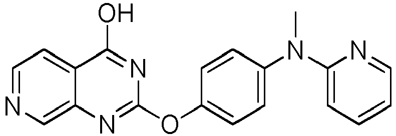

A mixture of 2-chloro-pyrido[3,4-d]pyrimidin-4-ol (408 mg, 2.25 mmol), 4-(methyl-pyridin-2-yl-amino)-phenol (450 mg, 2.25 mmol), CS₂CO₃ (734 mg, 2.25 mmol), and CuI (428 mg, 2.25 mmol) in DMF (5 mL) was stirred at 130 °C under nitrogen atmosphere overnight. The reaction mixture was concentrated. The residue was purified by HPLC to obtain 156 mg (20%) of the title product. ¹H NMR (400 MHz, DMSO-*d₆*): δ 3.43 (s, 3H), 6.63 (d, *J* = 7.2 Hz, 1H), 6.70-6.73 (m, 2H), 7.38-7.40 (m, 4H), 7.48-7.52 (m, 1H), 8.02-8.11 (m, 1H), 8.17 (d, *J* = 4.0 Hz, 1H), 8.62-8.7 (m, 1H), 13.14 (s, 1H). [M+H] Calc'd for C₁₉H₁₅N₅O₂, 346; Found, 346.

### Preparation 2A: 5-Benzyloxy-1-methyl-1H-indole

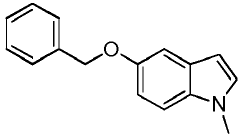

To a solution of 5-benzyloxy-1H-indole (2.23 g, 10 mmol) in DMF (20 mL) at 0°C was added NaH (480 mg, in mineral oil, 60%, 12 mmol) in portions and the mixture was stirred for 30 min. MeI was then added and the mixture was stirred at rt overnight. The reaction mixture was diluted with water (100 mL) and extracted with DCM (30 mL^{∗}3). Organics were dried over Na₂SO₄ and concentrated. The residue was purified by silica gel chromatography (PE:EA 50:1) to give 1.97 g (83%) of the title product. ¹H NMR (400 MHz, CDCl₃): δ 3.77 (s, 3H), 5.11 (s, 2H), 6.39 (d, *J*= 3.2 Hz, 1H), 6.97 (dd, *J* = 2.4, 8.8 Hz, 1H), 7.01 (d, *J* = 3.2 Hz, 1H), 7.17 (d, *J* = 2.0 Hz, 1H), 7.22 (*J* = 10.8 Hz, 1H), 7.31 (t, *J* = 7.2 Hz, 1H), 7.39 (t, *J* = 7.2 Hz, 2H), 7.48 (d, *J* = 7.2 Hz, 2H). [M+H] Calc'd for C₁₆H₁₅NO, 238; Found, 238.

### Preparation 2B: 1-Methyl-1H-indol-5-ol

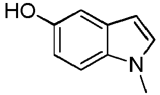

A mixture of 5-benzyloxy-1-methyl-1H-indole (1.97 g, 8.31 mmol) and Pd/C (0.5 g, 10% wet) in EtOH was stirred overnight under H₂ atmosphere. The reaction mixture was filtered through a Celite pad. The filtrate was concentrated to give 1.2g (98%) of the title product. ¹H NMR (400 MHz, CDCl₃): δ 3.75 (s, 3H), 6.35 (d, *J* = 2.8 Hz, 1H), 6.80 (dd, *J* = 2.4, 8.8 Hz, 1H), 7.01-7.03 (m, 2H), 7.17 (d, *J* = 8.8 Hz, 1H). [M+H] Calc'd for C₉H₉NO, 148; Found, 148.

### Example 2: 2-(1-Methyl-1H-indol-5-yloxy)-pyrido[3,4-d]pyrimidin-4-ol

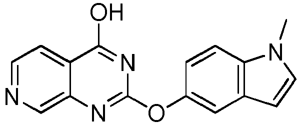

The title compound was prepared in 2.5% yield from 2-chloro-pyrido[3,4-d]pyrimidin-4-ol and 1-methyl-1H-indol-5-ol according to the preparation of Example 1. ¹H NMR (400 MHz, DMSO-*d₆*): δ 3.83 (s, 3H), 6.45 (d, *J* = 2.8 Hz,1H), 7.07 (d, *J* = 7.6 Hz, 1H), 7.40-7.43 (m, 2H), 7.48 (d, *J=* 8.8 Hz, 1H), 7.84 (d, *J=* 4.8 Hz, 1H), 8.44 (d, *J=* 4.4 Hz, 1H), 8.59 (s, 1H). [M+H] Calc'd for C₁₆H₁₂N₄O₂, 293; Found, 293.

### Preparation 3A: 5-Benzyloxy-1-phenethyl-1H-indole

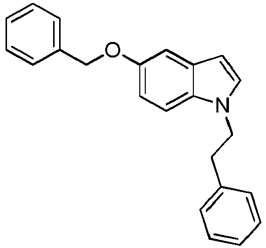

A mixture of 5-benzyloxy-1H-indole (3.0 g, 13.5 mmol), (2-bromo-ethyl)-benzene (3.0 g, 16.1 mmol) and KOH (2.7 g, 40.4 mmol) in DMSO (25 mL) was stirred at 100°C overnight. The reaction mixture was diluted with water (50 mL) and extracted with EA (20 mL^{∗}3). The organic layers were combined, washed with water (30 mL^{∗}3), washed with brine, dried over Na₂SO₄, and concentrated. The residue was purified by silica gel chromatography (PE:EA 50:1) to give 1.9 g (43%) of the title product. ¹H NMR (400 MHz, CDCl₃): δ 3.08 (t, *J=* 7.2 Hz, 2H), 4.29 (t, *J=* 7.2 Hz, 2H), 5.10 (s, 2H), 6.33 (d, *J=* 2.8 Hz, 1H), 6.88 (d, *J* = 2.8 Hz, 1H), 6.94 (dd, *J* = 2.8, 8.8 Hz, 1H), 7.07 (d, *J* = 6.8 Hz, 2H), 7.16 (d, *J* = 2.4 Hz, 1H), 7.21-7.26 (m, 4H), 7.31 (t, *J* = 7.2 Hz, 1H), 7.38 (t, *J* = 7.2 Hz, 2H), 7.47 (d, *J* = 7.6 Hz, 2H). [M+H] Calc'd for C₂₃H₂₁NO, 328; Found, 328.

### Preparation 3B: 1-Phenethyl-1H-indol-5-ol

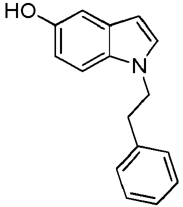

A mixture of 5-benzyloxy-1-phenethyl-1H-indole (1.9 g, 5.8 mmol) and Pd/C (0.5 g, 10% wet) in EtOH was stirred under H₂ atmosphere overnight. The reaction mixture was filtered through a Celite pad. The filtrate was concentrated to give 1.2 g (87%) of the title product. ¹H NMR (400 MHz, CDCl₃): δ 3.08 (t, *J=* 7.2 Hz, 2H), 4.29 (t, *J* = 7.2 Hz, 2H), 6.29 (d, *J=* 2.8 Hz, 1H), 6.79 (dd, *J=* 2.4, 8.8 Hz, 1H), 6.88 (d, *J=* 2.8 Hz, 1H), 7.02 (d, *J=* 2.4 Hz, 1H), 7.06-7.08 (m, 2H), 7.16-7.29 (m, 5H). [M+H] Calc'd for C₁₆H₁₅NO, 238; Found, 238.

### Example 3: 2-(1-Phenethyl-1H-indol-5-yloxy)-pyrido[3,4-d]pyrimidin-4-ol

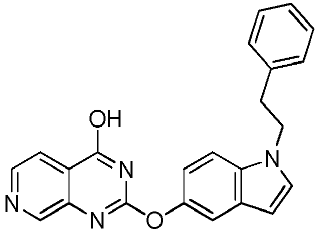

The title compound was prepared in 6% yield from 2-chloro-pyrido[3,4-d] pyrimidin-4-ol and 1-phenethyl-1H-indol-5-ol according to the procedure for the preparation of Example 1. ¹H NMR (400 MHz, DMSO-*d₆*): δ 3.10 (t, *J=* 7.0 Hz, 2H), 4.44 (t, *J=* 7.0 Hz, 2H), 6.43 (s, 1H), 7.06 (d, *J* = 7.6 Hz, 1H), 7.21-7.26 (m, 5H), 7.39 (s, 1H), 7.44 (s, 1H), 7.56 (d, *J* = 8.8 Hz, 1H), 7.87 (t, *J=* 2.0 Hz, 1H), 8.48 (d, *J=* 4.8 Hz, 1H), 8.63 (s, 1H). [M+H] Calc'd for C₂₃H₁₈N₄O₂, 383; Found, 383.

### Preparation 4A: 1-Benzyl-5-methoxy-1H-indole

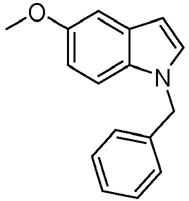

To a solution of 5-methoxy-1H-indole (1.50 g, 10 mmol) in DMF (20 mL) at 0°C was added NaH (480 mg, in mineral oil, 60%, 12 mmol) in portions, and the mixture was stirred for 30 min. BnBr (2.09 g, 12 mmol) was then added and the mixture was stirred at rt overnight. The reaction mixture was diluted with water (100 mL) and extracted with DCM (30 mL^{∗}3). Organics were dried over Na₂SO₄ and concentrated. The residue was purified by silica gel chromatography (PE:EA 20:1) to give 2.40 g (99%) of the title product. [M+H] Calc'd for C₁₆H₁₅NO, 238; Found, 238.

### Example 4B: 1-Benzyl-1H-indol-5-ol

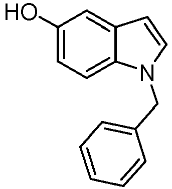

To a solution of 1-benzyl-5-methoxy-1H-indole (2.40 g, 10 mmol) in DCM (20 mL) was added BBr₃ (40 mL, 1.0 M in DCM, 40 mmol) in portions at 0 °C, and the mixture was stirred at rt for 2 h. The reaction mixture was diluted with water (30 mL), basified to pH 5 with sat. Na₂CO₃, and extracted with DCM (30 mL^{∗}3). Organics were dried over Na₂SO₄ and concentrated. The residue was purified by silica gel chromatography (PE:EA 10:1) to give 1.20 g (53%) of the title product. [M+H] Calc'd for C₁₅H₁₃NO, 224; Found, 224.

### Example 4: 2-(1-Benzyl-1H-indol-5-yloxy)-pyrido[3,4-d]pyrimidin-4-ol

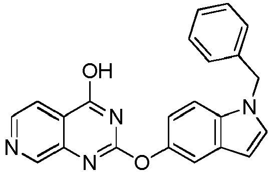

The title compound was prepared in 19% yield from 2-chloro-pyrido [3,4-d]pyrimidin-4-ol and 1-benzyl-1H-indol-5-ol according to the procedure for the preparation of Example 1. ¹H NMR (300 MHz, DMSO-*d₆*): δ 5.45 (s, 2H), 6.53 (d, *J=* 2.1 Hz, 1H), 7.04-7.08 (m, 1H), 7.24-7.36 (m, 5H),7.49-7.55 (m, 2H), 7.63 (d, *J=* 1.5 Hz, 1H), 7.85-7.89 (m, 1H), 8.15 (brs, 2H), 13.04 (s, 1H). [M+H] Calc'd for C₂₂H₁₆N₄O₂, 369; Found, 369.

### Preparation 5A: 4-(Methyl-phenyl-amino)-phenol

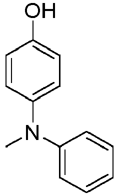

To a suspension of 4-bromo-phenol (2.00 g, 12 mmol), methyl-phenyl-amine (1.48 g, 14 mmol), Pd₂(dba)₃ (267 mg, 0.29 mmol) and 2-dicyclohexyphosphino-biphenyl (121 mg, 0.35 mmol) in toluene (40 mL) was added LiHMDS (25 mL, 1.0 M in THF, 25 mmol) in portions under nitrogen atmosphere. The reaction mixture was stirred at 65 °C overnight, acidified to pH 6 with IN HCl, washed with brine (50 mL), dried over Na₂SO₄, filtered, and concentrated. The residue was purified by silica gel column (PE:EA 10:1) to give 2g (87%) of the title product. [M+H] Calc'd for C₁₃H₁₃NO, 200; Found, 200.

### Example 5: 2-[4-(Methyl-phenyl-amino)-phenoxy]-pyrido[3,4-d]pyrimidin-4-ol

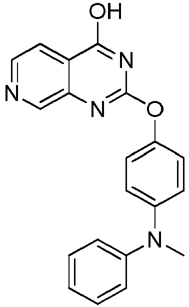

The title compound was prepared in 18% yield from 2-chloro-pyrido[3,4-d]pyrimidin-4-ol and 4-(methyl-phenyl-amino)-phenol according to the procedure for the preparation of Example 1. ¹H NMR (400 MHz, DMSO-*d₆*): δ 3.30 (s, 3H), 6.97-7.08 (m, 5H), 7.23-7.25 (m, 2H), 7.30-7.34 (m, 2H), 7.90-7.92 (m, 1H), 8.51-8.78 (m, 2H), 13.06 (s, 1H). [M+H] Calc'd for C₂₀H₁₆N₄O₂, 345; Found, 345.

### Preparation 6A: 4-(Benzyl-methyl-amino)-phenol

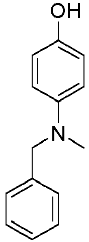

The title compound was prepared in 41% yield from 4-bromo-phenol and benzyl-methyl-amine according to the procedure of Preparation 5A. [M+H] Calc'd for C₁₄H₁₅NO, 214; Found, 214.

### Example 6: 2-[4-(Benzyl-methyl-amino)-phenoxy]-pyrido[3,4-d]pyrimidin-4-ol

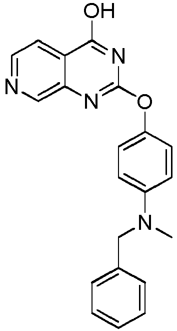

The title compound was prepared in 5% yield from 2-chloro-pyrido[3,4-d] pyrimidin-4-ol and 4-(benzyl-methyl-amino)-phenol according to the procedure for the preparation of Example 1. ¹H NMR (300 MHz, DMSO-*d₆*): δ 3.04 (s, 3H), 4.56 (s, 2H), 6.75-6.77 (m, 2H), 7.09-7.12 (m, 2H), 7.20-7.36 (m, 5H), 7.91-7.95 (m, 1H), 8.47-8.96 (m, 2H), 12.98 (s, 1H). [M+H] Calc'd for C₂₁H₁₈N₄O₂, 359; Found, 359.

### Preparation 7A: 3-(Methyl-phenyl-amino)-phenol

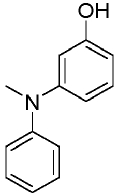

The title compound was prepared in 78% yield from 3-bromo-phenol and methyl-phenyl-amine according to the procedure of Preparation 5A. [M+H] Calc'd for C₁₃H₁₃NO, 200; Found, 200.

### Example 7: 2-[3-(Methyl-phenyl-amino)-phenoxy]-pyrido[3,4-d]pyrimidin-4-ol

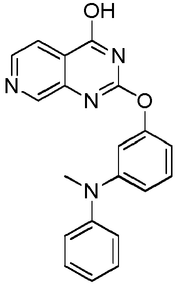

The title compound was prepared in 26% yield from 2-chloro-pyrido [3,4-d]pyrimidin-4-ol and 3-(methyl-phenyl-amino)-phenol according to the procedure for the preparation of Example 1. ¹H NMR (400 MHz, DMSO-*d₆*): δ 3.31 (s, 3H), 6.79-6.86 (m, 3H), 7.05 (t, *J=* 7.2 Hz, 1H), 7.17-7.19 (m, 2H), 7.32-7.36 (m, 3H), 7.88-7.95 (m, 1H), 8.45-8.73 (m, 2H), 13.03 (s, 1H). [M+H] Calc'd for C₂₀H₁₆N₄O₂, 345; Found, 345.

### Example 8: 2-(1-Benzyl-1H-indol-6-yloxy)-pyrido[3,4-d]pyrimidm-4-ol

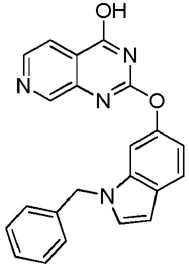

The title compound was prepared in 16% yield from 2-chloro-pyrido [3,4-d]pyrimidin-4-ol and 1-benzyl-1H-indol-6-ol according to the procedure for the preparation of Example 1. ¹H NMR (400 MHz, DMSO-*d₆*): δ 5.41 (s, 2H), 6.55 (d, *J* = 2.8 Hz, 1H), 6.98-7.01 (m, 1H), 7.23-7.32 (m, 5H), 7.50-7.52 (m, 1H), 7.57-7.62 (m, 2H), 7.87-7.94 (m, 1H), 8.52-8.63 (m, 2H), 13.07 (s, 1H). [M+H] Calc'd for C₂₂H₁₆N₄O₂, 369; Found, 369.

### Preparation 9A: 3-(Benzyl-methyl-amino)-phenol

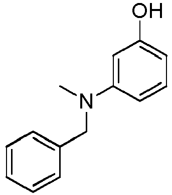

The title compound was prepared in 24% yield from 3-bromo-phenol and benzyl-methyl-amine according to the procedure of Preparation 5A. [M+H] Calc'd for C₁₄H₁₅NO, 214; Found, 214.

### Example 9: 2-[3-(Benzyl-methyl-amino)-phenoxy]-pyrido[3,4-d]pyrimidin-4-ol

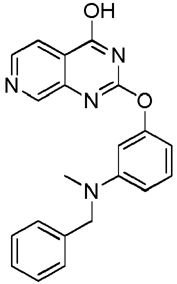

The title compound was prepared in 19% yield from 2-chloro-pyrido [3,4-d]pyrimidin-4-ol and 3-(benzyl-methyl-amino)-phenol according to the procedure for the preparation of Example 1. ¹H NMR (400 MHz, DMSO-*d₆*): δ 3.03 (s, 3H), 4.59 (s, 2H), 6.55-6.57 (m, 1H), 6.64-6.69 (m, 2H), 7.23-7.32 (m, 7H), 7.92-8.20 (br, 2H), 13.02 (s, 1H). [M+H] Calc'd for C₂₁H₁₈N₄O₂, 359; Found, 359.

### Preparation 10A: 3-Fluoro-4-(methyl-phenyl-amino)-phenol

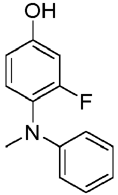

The title compound was prepared in 18% yield from 4-bromo-3-fluoro-phenol and methyl-phenyl-amine according to the procedure of Preparation 5A. [M+H] Calc'd for C₁₃H₁₂FNO, 218; Found, 218.

### Example 10: 2-[3-Fluoro-4-(methyl-phenyl-amino)-phenoxy]-pyrido[3,4-d]pyrimidin-4-ol

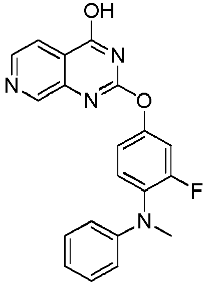

The title compound was prepared in 4% yield from 2-chloro-pyrido[3,4-d] pyrimidin-4-ol and 3-fluoro-4-(methyl-phenyl-amino)-phenol according to the procedure for the preparation of Example 5. ¹H NMR (400 MHz, DMSO-*d₆*): δ 3.32 (s, 3H), 6.68-6.77 (m, 5H), 7.17-7.20 (m, 4H), 7.34-7.42 (m, 2H). [M+H] Calc'd for C₂₀H₁₅FN₄O₂, 363; Found, 363.

### Preparation 11A: 1-Benzyl-6-methoxy-1H-indazole

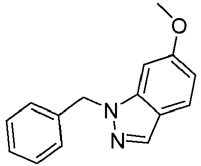

The title compound was prepared in 62% yield from 6-methoxy-1H-indazole and bromomethyl-benzene according to the procedure of Preparation 4A. ¹H NMR (400 MHz, CDCl₃): δ 3.85 (s, 3H), 5.53 (s, 2H), 6.76-6.78 (m, 1H), 6.98 (s, 1H), 7.26-7.37 (m, 5H), 7.47-7.49 (m, 1H), 7.78 (s, 1H). [M+H] Calc'd for C₁₅H₁₄N₂O, 239; Found, 239.

### Preparation 11B: 1-Benzyl-1H-indazol-6-ol

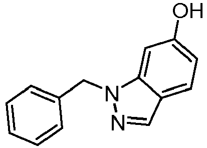

The title compound was prepared in 69% yield from 1-benzyl-6-methoxy-1H-indazole according to the procedure of Preparation 4B. ¹H NMR (400 MHz, DMSO-*d₆*): δ 5.51 (s, 2H), 6.65-6.68 (m, 1H), 6.79 (s, 1H), 7.15-7.32 (m, 5H), 7.54 (d, *J* = 8.8 Hz, 1H), 7.91 (s, 1H), 9.63 (s, 1H). [M+H] Calc'd for C₁₄H₁₂N₂O, 225; Found, 225.

### Example 11: 2-(1-Benzyl-1H-indazol-6-yloxy)-pyrido[3,4-d]pyrimidin-4-ol

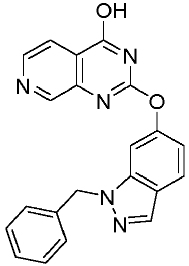

The title compound was prepared in 16% yield from 2-chloro-pyrido [3,4-d]pyrimidin-4-ol and 1-benzyl-1H-indazol-6-ol according to the procedure for the preparation of Example 1. ¹H NMR (300 MHz, DMSO-*d₆*): δ 5.64 (s, 2H), 7.13-7.16 (m, 1H), 7.25-7.33 (m, 5H), 7.79-7.91 (m, 3H), 8.17 (m, 1H), 8.44-8.78 (m, 2H), 13.20 (s, 1H). [M+H] Calc'd for C₂₁H₁₅N₅O₂, 370; Found, 370.

### Preparation 12A: 2-Benzyl-6-methoxy-1H-indazole

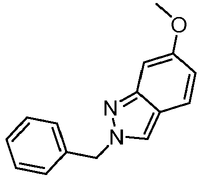

The title compound was prepared in 31% yield from 6-methoxy-1H-indazole and bromomethyl-benzene according to the procedure of Preparation 4A. [M+H] Calc'd for C₁₅H₁₄N₂O, 239; Found, 239.

### Preparation 12B: 2-Benzyl-1H-indazol-6-ol

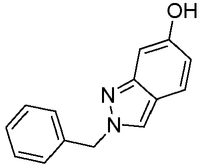

The title compound was prepared in 74% yield from 2-benzyl-6-methoxy-1H-indazole according to the procedure of Preparation 4B. [M+H] Calc'd for C₁₄H₁₂N₂O, 225; Found, 225.

### Example 12: 2-(2-Benzyl-2H-indazol-6-yloxy)-pyrido[3,4-d]pyrimidin-4-ol

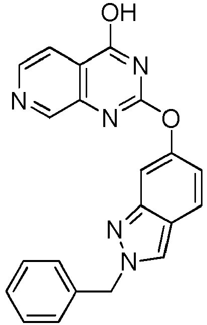

The title compound was prepared in 1% yield from 2-chloro-pyrido[3,4-d]pyrimidin-4-ol and 2-benzyl-1H-indazol-6-ol according to the procedure for the preparation of Example 1. ¹H NMR (300 MHz, DMSO-*d₆*): δ 5.66 (s, 2H), 7.01-7.05 (m, 1H), 7.29-7.37 (m, 5H), 7.52 (s, 1H), 7.79-7.88 (m, 2H), 8.49-8.65 (m, 3H), 13.11 (s, 1H). [M+H] Calc'd for C₂₁H₁₅N₅O₂, 370; Found, 370.

### Preparation 13A: 4-[Methyl(2-phenylethyl)amino]phenol

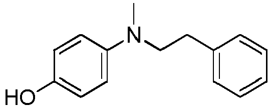

To a solution of 4-bromophenol (2.0 g, 11.56 mmol) and methyl(2-phenylethyl)amine (1.88 g, 13.9 mmol) in toluene (20 mL) was added Pd₂(dba)₃ (110 mg, 0.12 mmol) and dicyclohexyl(2-phenylphenyl)phosphine (98 mg, 0.28 mmol), then LiHMDS (25.4 mL, 25.4 mmol) was added under nitrogen. The mixture was stirred overnight at 65 °C under nitrogen. The reaction mixture was filtered and concentrated. The residue was purified by prep-HPLC to give the 1.52 g (58%) of the title product. [M+H] Calc'd for C₁₅H₁₇NO, 228; Found, 228.

### Example 13: 2-{4-[Methyl(2-phenylethyl)amino]phenoxy}pyridino[3,4-d]pyrimidin-4-ol

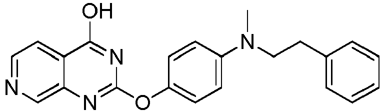

The title compound was prepared in 17% yield from 2-chloro-pyrido[3,4-d]pyrimidin-4-ol and 4-[methyl(2-phenylethyl)amino]phenol according to the procedure for the preparation of Example 1. ¹H NMR (400 MHz, DMSO-*d₆*): δ 2.82 (t, *J* = 7.6 Hz, 2H), 2.90 (s, 3H), 3.56 (t, *J=* 7.8 Hz, 2H), 6.76 (d, *J=* 9.2 Hz, 2H), 7.13 (d, *J=* 8.8 Hz, 2H), 7.20-7.23 (m, 1H), 7.28-7.31 (m, 4H), 7.86 (d, *J=* 4.8 Hz, 1H), 8.49 (d, *J=* 4.8 Hz, 1H), 8.68 (s, 1H). [M+H] Calc'd for C₂₂H₂₀N₄O₂, 373; Found, 373.

### Preparation 14A and 15A: 5-Methoxy-2-benzyl-2H-indazole and 5-methoxy-1-benzyl-1H-indazole

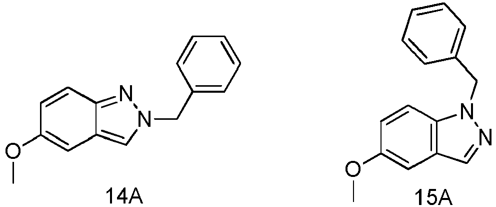

To a solution of 5-methoxy-1H-indazole (1.0 g, 6.76 mmol) in DMF (10 mL) was added Cs₂CO₃ (2.2 g, 6.76 mmol) and BnBr (1.38 g, 8.1 mmol). The mixture was stirred at rt for 3 h, diluted with water (100 mL), and extracted with EA. Organics were washed with brine, dried over Na₂SO₄, and concentrated. The residue was purified by prep-HPLC to give 576 mg of the product 14A (54%) and 863 mg of the product 15A (36%). 14A: ¹H NMR (400 MHz, CDCl₃): δ 3.81 (s, 3H), 5.56 (s, 2H), 6.84 (d, *J=* 2.4 Hz, 1H), 6.99 (dd, *J=* 2.0, 7.2 Hz, 1H), 7.25-7.28 (m, 2H), 7.31-7.37 (m, 3H), 7.61 (d, *J* = 9.6 Hz, 1H), 7.75 (s, 1H). 15A: ¹H NMR (400 MHz, CDCl₃): δ 3.84 (s, 3H), 5.57 (s, 2H), 7.00 (dd, *J=* 2.8, 6.4 Hz, 1H), 7.08 (d, *J=* 2.0 Hz, 1H), 7.17-7.19 (m, 1H), 7.21-7.29 (m, 5H), 7.94 (s, 1H).

### Preparation 14B: 2-Benzyl-2H-indazol-5-ol

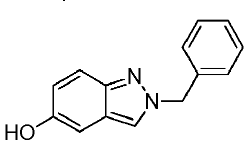

The title compound was prepared in 47% yield from 5-methoxy-2-benzyl-2H-indazole according to the procedure of Preparation 1C. [M+H] Calc'd for C₁₄H₁₂N₂O, 225; Found 225.

### Example 14: 2-[2-Benzyl-2H-indazol-5-yloxy]pyridino[3,4-d]pyrimidin-4-ol

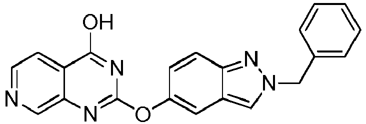

The title compound was prepared in 16% yield from 2-chloro-pyrido[3,4-d]pyrimidin-4-ol and 2-benyl-2H-indazol-5-ol according to the procedure for the preparation of Example 1. ¹H NMR (400 MHz, DMSO-*d₆*): δ 5.66 (s, 2H), 7.19 (d, *J=* 7.2 Hz, 1H), 7.32-7.37 (m, 5H), 7.59 (d, *J=* 1.6 Hz, 1H), 7.66 (d, *J=* 9.6 Hz, 1H), 7.84 (d, *J=* 4.8 Hz, 1H), 8.45 (d, *J=* 4.8 Hz, 1H), 8.52 (s, 1H), 8.62 (s, 1H). [M+H] Calc'd for C₂₁H₁₅N₅O₂, 370; Found, 370.

### Preparation 15B: 1-Benzyl-1H-indazol-5-ol

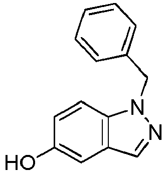

The title compound was prepared in 75% yield from 5-methoxy-1-benzyl-1H-indazole according to the procedure of Preparation 1C. [M+H] Calc'd for C₁₄H₁₂N₂O, 225; Found, 225.

### Example 15: 2-(1-Benzyl-1H-indazol-5-yloxy)-pyridino[3,4-d]pyrimidin-4-ol

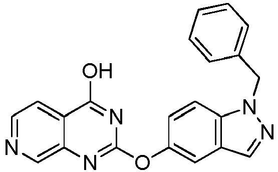

The title compound was prepared in 16% yield from 2-chloro-pyrido [3,4-d]pyrimidin-4-ol and 1-benzyl-1H-indazol-5-ol according to the procedure for the preparation of Example 1. ¹H NMR (300 MHz, DMSO-*d₆*): δ 5.71 (s, 2H), 7.22-7.41 (m, 9H), 7.78-7.83 (m, 2H), 7.16 (s, 1H), 13.12 (s, 1H). [M+H] Calc'd for C₂₁H₁₅N₅O₂, 370; Found, 370.

### Preparation 16A: 4-[(4-Methoxy-phenyl)-methyl-amino]-phenol

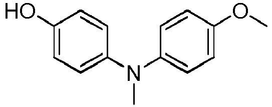

The title compound was prepared in 34% yield from 4-bromo-phenol and benzyl-methyl-amine according to the Preparation 5A. [M+H] Calc'd for C₁₄H₁₅NO₂, 230; Found, 230.

### Example 16: 2-{4-[(4-Methoxy-phenyl)-methyl-amino]-phenoxy}-pyrido[3,4-d]pyrimidin-4-ol

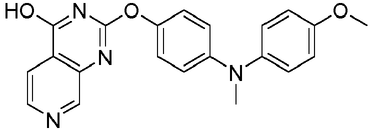

The title compound was prepared in 15% yield from 2-chloro-pyrido[3,4-d]pyrimidin-4-ol and 1-benzyl-1H-indazol-5-ol according to the procedure for the preparation of Example 1. ¹H NMR (400 MHz, DMSO-*d₆*): δ 3.26 (s, 3H), 3.73 (s, 3H), 6.76 (d, *J=* 9.2 Hz, 2H), 6.97 (d, *J=* 9.2 Hz, 2H), 7.12-7.16 (m, 4H), 7.89 (s, 1H), 8.52 (s, 1H), 8.75 (s, 1H), 13.02 (s, 1H). [M+H] Calc'd for C₂₁H₁₈N₄O₃, 375; Found, 375.

### Preparation 17A: 4-[(3-Methoxy-phenyl)-methyl-amino]-phenol

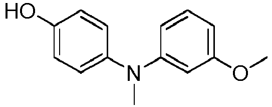

The title compound was prepared in 34% yield from 4-bromo-phenol and benzyl-methyl-amine according to the procedure of Preparation 5A. [M+H] Calc'd for C₁₄H₁₅NO₂, 230; Found, 230.

### Example 17: 2-{4-[(3-Methoxy-phenyl)-methyl-amino]-phenoxy}-pyrido[3,4-d]pyrimidin-4-ol

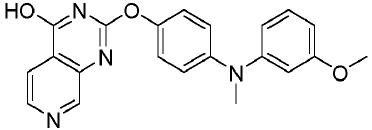

The title compound was prepared in 5% yield from 2-chloro-pyrido[3,4-d] pyrimidin-4-ol and 1-benzyl-1H-indazol-5-ol according to the procedure for preparation of Example 1. ¹H NMR (400 MHz, DMSO-*d₆*): δ 3.29 (s, 3H), 3.73 (s, 3H), 6.54-6.61 (m, 3H), 7.08-7.27 (m, 5H), 7.89 (s, 1H), 8.52 (s, 1H), 8.71 (s, 1H), 13.08 (s, 1H). [M+H] Calc'd for C₂₁H₁₈N₄O₃, 375; Found, 375.

### Preparation 18A: Methyl-(4-morpholin-4-yl-phenyl)-amine

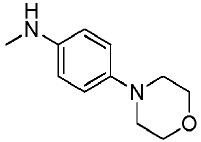

A solution of 4-morpholin-4-yl-phenylamine (4 g, 22.5 mmol) in HCOOH (40 mL) was heated at 110 °C overnight. The solution was diluted with water (100 mL) and extracted with DCM (30 mL x 3). Organics were washed with H₂O and brine, dried over Na₂SO₄, and concentrated *in vacuo.* To the residue in THF (100 mL) at 0°C was added a 2M LiAlH₄ solution (33 mL), and the mixture was stirred at rt for 2 h. H₂O (3 mL) was added at 0 °C, followed by 10% NaOH solution (6 mL). The mixture was filtered and the filtrate was extracted with DCM (50 mL x 2). The extracts were washed with brine, dried over Na₂SO₄, concentrated *in vacuo.* The residue was purified by silica gel chromatography (PE:EA 2:1) to give 4 g (93%) of the title compound. [M+H] Calc'd for C₁₁H₁₆N₂O, 193; Found, 193.

### Preparation 18B: 4- [Methyl-(4-morpholin-4-yl-phenyl)-amino] -phenol

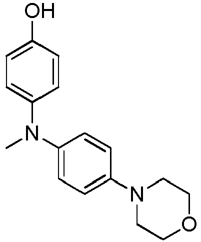

The title compound was prepared in 34% yield from 4-bromo-phenol and methyl-(4-morpholin-4-yl-phenyl)-amine according to the procedure of Preparation 5A. [M+H] Calc'd for C₁₇H₂₀N₂O₂, 285; Found, 285.

### Example 18: 2-{4-[Methyl-(4-morpholin-4-yl-phenyl)-amino]-phenoxy}-pyrido[3,4-d]pyrimidin-4-ol

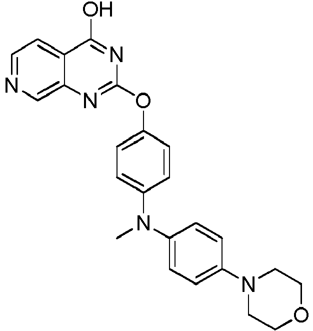

The title compound was prepared in 2% yield from 2-chloro-pyrido[3,4-d] pyrimidin-4-ol and 4-[methyl-(4-morpholin-4-yl-phenyl)-amino]-phenol according to the procedure for the preparation of Example 1. ¹H NMR (300 MHz, DMSO-*d*₆): δ 3.05-3.10 (m, 4H), 3.19 (s, 3H), 3.73-3.78 (m, 4H), 6.75 (d, *J* = 9.2 Hz, 2H), 6.97 (d, *J* = 9.2 Hz, 2H), 7.06 - 7.12 (m, 4H), 7.86 (d, *J* = 5.2 Hz, 1H), 8.49-8.53 (m, 1H), 8.69 (s, 1H), 13.03 (s, 1H). [M+H] Calc'd for C₂₄H₅₅N₅O₃, 430; Found, 430.

### Preparation 19A: Methyl-(3-morpholin-4-yl-phenyl)-amine

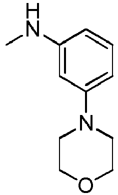

The title compound was prepared in 90% yield from 3-morpholin-4-yl-phenylamine according to the procedure of Preparation 18. [M+H] Calc'd for C₁₁H₁₆N₂O, 193; Found, 193.

### Preparation 19B: 4- [Methyl-(3 -morpholin-4-yl-phenyl)-amino] -phenol

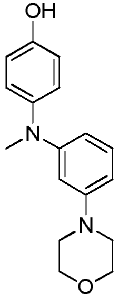

The title compound was prepared in 45% yield from 4-bromo-phenol and methyl-(3-morpholin-4-yl-phenyl)-amine according to the procedure of preparation of 5A. [M+H] Calc'd for C₁₇H₂₀N₂O₂, 285; Found, 285.

### Example 19: 2-{4-[Methyl-(3-morpholin-4-yl-phenyl)-amino]-phenoxy}-pyrido[3,4-d]pyrimidin-4-ol

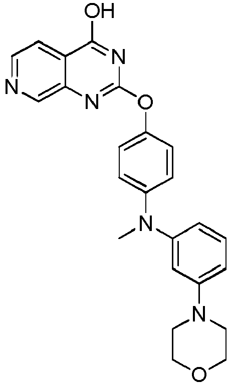

The title compound was prepared in 3% yield from 2-chloro-pyrido[3,4-d] pyrimidin-4-ol and 4-[methyl-(3-morpholin-4-yl-phenyl)-amino]-phenol according to the procedure for the preparation of Example 1. ¹H NMR (300 MHz, DMSO-*d*₆): δ 3.06-3.09 (m, 4H), 3.29 (s, 3H), 3.71-3.73 (m, 4H), 6.53-6.55 (m, 1H), 6.62-6.64 (m, 2H), 6.97-6.99 (m, 2H), 7.16-7.21 (m, 3H), 7.87 (d, *J* = 5.2 Hz, 1H), 8.51 (d, *J* = 4.8 Hz, 1H), 8.69 (s, 1H), 13.03 (s, 1H). [M+H] Calc'd for C₂₄H₅₅N₅O₃, 430; Found, 430.

### Preparation 20: 4-(Methyl-p-tolyl-amino)-phenol

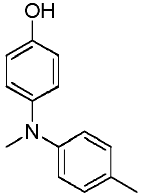

The title compound was prepared in 53% yield from 4-bromo-phenol and methyl-*p*-tolyl-amine according to the procedure of preparation 5A. [M+H] Calc'd for C₁₄H₁₅NO, 214; Found 214.

### Example 20: 2-[4-(Methyl-p-tolyl-amino)-phenoxy]-pyrido[3,4-d]pyrimidin-4-ol

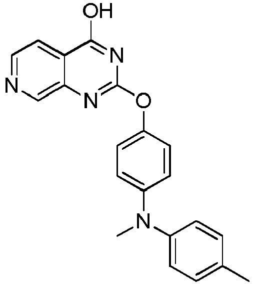

The title compound was prepared in 18% yield from 2-chloro-pyrido [3,4-d]pyrimidin-4-ol and 4-(methyl-*p*-tolyl-amino)-phenol according to the procedure for the preparation of Example 1. ¹H NMR (400 MHz, DMSO-*d*₆): δ 2.33 (s, 3H), 3.26 (s, 3H), 6.90 (d, *J* = 8.8 Hz, 2H), 6.99 (d, *J* = 9.6 Hz, 2H), 7.15-7.22 (m, 4H), 7.86 (d, *J* = 5.2 Hz, 1H), 8.50 (d, *J* = 4.8 Hz, 1H), 8.69 (s, 1H), 13.07 (s, 1H). [M+H] Calc'd for C₂₁H₁₈N₄O₂, 359; Found, 359.

### Preparation 21: 4-(Methyl-m-tolyl-amino)-phenol

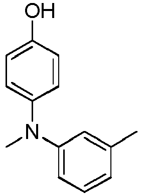

The title compound was prepared in 53% yield from 4-bromo-phenol and methyl-*m*-tolyl-amine according to the procedure of Preparation 5A. [M+H] Calc'd for C₁₄H₁₅NO, 214; Found 214.

### Example 21: 2-[4-(Methyl-m-tolyl-amino)-phenoxy]-pyrido[3,4-d]pyrimidin-4-ol

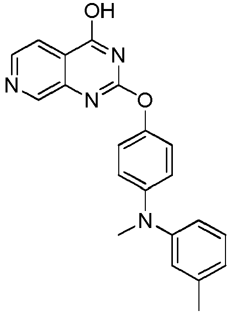

The title compound was prepared in 15% yield from 2-chloro-pyrido[3,4-d]pyrimidin-4-ol and 4-(methyl-*m*-tolyl-amino)-phenol according to the procedure for the preparation of Example 1. ¹H NMR (400 MHz, DMSO-*d*₆): δ 2.28 (s, 3H), 3.29 (s, 3H), 6.82-6.88 (m, 3H), 7.01 (d, *J* = 8.8 Hz, 2H), 7.19-7.23 (m, 3H), 7.87 (d, *J* = 4.8 Hz, 1H), 8.51 (d, *J* = 4.8 Hz, 1H), 8.71 (s, 1H). [M+H] Calc'd for C₂₁H₁₈N₄O₂, 359; Found, 359.

### Preparation 22A: (3-chloro-phenyl)-(4-methoxy-phenyl)-methyl-amine

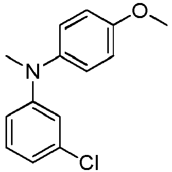

To a solution of (3-chloro-phenyl)-methyl-amine (10 g, 71 mmol) in toluene was added 1-bromo-4-methoxy-benzene (14.0 g, 74.5 mmol), biphenyl-2-yl-dicyclohexyl-phosphane (270 mg, 0.71 mmol), Pd₂(dba)₃ (650 mg, 0.71 mmol), *t*-BuOK (12.0 g, 106.5 mmol) and the mixture was refluxed overnight under nitrogen atmosphere. The reaction mixture was cooled to RT and the solvent was concentrated. The residue was purified by silica gel chromatography (PE:EA, 50:1) to give 15 g (86%) of the title compound. [M+H] Calc'd for C₁₄H₁₄ClNO, 248; Found, 248.

### Preparation 22B: (4-methoxy-phenyl)-methyl-[3-(4-methyl-piperazin-1-yl)-phenyl]-amine

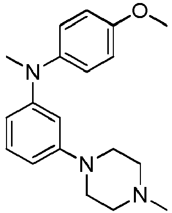

To a solution of ((3-chloro-phenyl)-(4-methoxy-phenyl)-methyl-amine (2.0 g, 8.1 mmol) in toluene was added 1-methyl-piperazine (0.97 g, 9.7 mmol), biphenyl-2-yl-dicyclohexyl-phosphane (155 mg, 0.4 mmol), Pd₂(dba)₃ (450 mg, 0.48 mmol), *t*-BuOK (2.5 g, 22.3 mmol) and the mixture was refluxed overnight under nitrogen atmosphere. The reaction mixture was cooled to RT and the solvent was concentrated. The residue was purified by silica gel chromatography (DCM:MeOH, 20:1) to give 1.1 g (48%) of the title compound. [M+H] Calc'd for C₁₉H₂₅N₃O, 312; Found, 312.

### Preparation 22C: 4-{methyl-[3-(4-methyl-piperazin-1-yl)-phenyl] -amino}-phenol

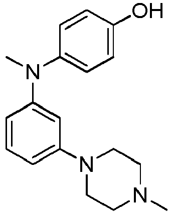

To a solution of (4-methoxy-phenyl)-methyl-[3-(4-methyl-piperazin-1-yl)-phenyl]-amine (1.0 g, 3.2 mmol) in DCM (10 mL) was added BBr₃ (20 mL, 1M) at -20 °C and the mixture was stirred at RT for 1h. The mixture was then warmed to 0 °C, quenched with MeOH and the PH of the solution was neutralized with aqueous NaHCO₃. The organic layer was washed brine, dried over Na₂SO₄ and concentrated. The residue was purified by silica gel chromatography (DCM:MeOH, 10:1) to give 370 mg (39%) of the title compound. [M+H] Calc'd for C₁₈H₂₃N₃O, 298; Found, 298.

### Example 22: 2-(4- {methyl-[3-(4-methyl-piperazin-1-yl)-phenyl]-amino}-phenoxy)-pyrido [3,4-d] pyrimidin-4-ol

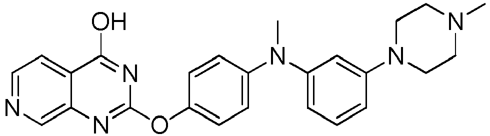

A mixture of 2-chloro-pyrido[3,4-d]pyrimidin-4-ol (200 mg, 1.1 mmol), 4-{methyl-[3-(4-methyl-piperazin-1-yl)-phenyl] -amino}-phenol (370 mg, 1.24 mmol), Cs₂CO₃ (400 mg, 1.22 mmol), and CuI (230 mg, 1.22 mmol) in DMF (10 mL) was stirred overnight at 130°C under nitrogen atmosphere. The reaction mixture was concentrated and the residue was purified by prep-HPLC to give 30 mg (6%) of the title product. ¹H NMR (400 MHz, DMSO-*d₆*): δ 2.24 (s, 3H), 2.47-2.49 (m, 4H), 3.10-3.12 (m, 4H), 3.26 (s, 3H), 6.49 (d, *J* = 10.0 Hz, 1H), 6.60 (d, *J* = 9.2 Hz, 2H), 6.96-7.17 (m, 5H), 7.84(d, *J* = 6.8 Hz, 1H), 8.46 (d, *J* = 6.8 Hz, 1H), 8.67 (s, 1H). [M+H] Calc'd for C₂₅H₂₆N₆O₂, 443; Found, 443.

### Preparation 23A: (1-{3-[(4-methoxy-phenyl)-methyl-amino]-phenyl}-piperidin-4-yl)-carbamic acid tert-butyl ester

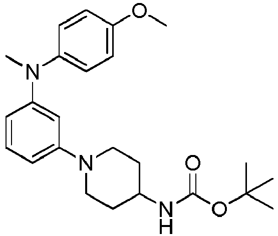

To a solution of ((3-chloro-phenyl)-(4-methoxy-phenyl)-methyl-amine (3.0 g, 12.1 mmol) in toluene was added piperidin-4-yl-carbamic acid *tert*-butyl ester (3.65 g, 18.2 mmol), S-Phos (250 mg, 0.61 mmol), Pd₂(dba)₃ (670 mg, 0.73 mmol), *t*-BuOK (2.74 g, 24.5 mmol) and the mixture was stirred overnight at 95 °C under nitrogen atmosphere. The reaction mixture was cooled to RT and the solvent was concentrated. The residue was purified by silica gel chromatography (DCM:MeOH, 10:1) to give 0.65 g (27%) of the title compound. [M+H] Calc'd for C₂₄H₃₃N₃O₃, 412; Found, 412.

### Preparation 23B: 4-{[3-(4-amino-piperidin-1-yl)-phenyl]-methyl-amino}-phenol

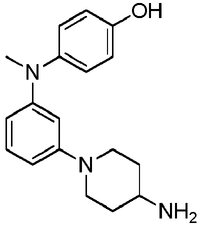

To a solution of (1-{3-[(4-methoxy-phenyl)-methyl-amino]-phenyl}-piperidin-4-yl)-carbamic acid tert-butyl ester (650 mg, 1.58 mmol) in DCM (10 mL) was added BBr₃ (10 mL, 1 M) at -20°C and the mixture was stirred at RT for 1h. The mixture was then warmed to 0°C, quenched with MeOH and the PH of the solution was neutralized with aqueous NaHCO₃. The residue was purified by silica gel chromatography (DCM:MeOH, 10:1) to give 360 mg (77%) of the title compound. [M+H] Calc'd for C₁₈H₂₃N₃O, 298; Found, 298.

### Preparation 23B: (1-{3-[(4-hydroxy-phenyl)-methyl-amino] -phenyl}-piperidin-4-yl)-carbamic acid tert-butyl ester

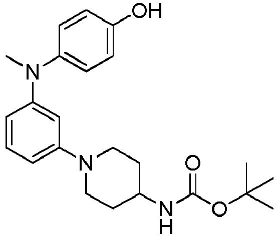

To a solution of compound 4-{[3-(4-amino-piperidin-1-yl)-phenyl]-methyl-amino}-phenol (360 mg, 1.21 mmol) in MeOH (5 mL) was added was added 1N NaOH (2.42 mL) and (Boc)₂O (287 mg, 1.32 mmol) and the mixture was stirred at RT for 2h. The reaction mixture was diluted with water and extracted with DCM (3x). The organics were concentrated and the residue was purified by silica gel chromatography (PE:EA, 5:1) to give 400 mg (83%) of the title compound. [M+H] Calc'd for C₂₃H₃₁N₃O₃, 398; Found, 398.

### Example 23: 2-(4-{[4-(4-amino-piperidin-1-yl)-phenyl]-methyl-amino}-phenoxy)-pyrido[3,4-d]pyrimidin-4-ol

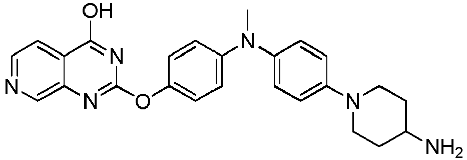

A mixture of 2-chloro-pyrido[3,4-d]pyrimidin-4-ol (150 mg, 0.83 mmol), (1-{3-[(4-hydroxy-phenyl)-methyl-amino]-phenyl}-piperidin-4-yl)-carbamic acid tert-butyl ester (400 mg, 1.24 mmol), Cs₂CO₃ (300 mg, 0.92 mmol), and CuI (175 mg, 0.92 mmol) in DMF (10 mL) was stirred overnight at 130°C under nitrogen atmosphere. The reaction mixture was concentrated. The residue was purified by silica gel chromatography (DCM:MeOH, 10:1) to give 30 mg of boc protected product, which was subsequently taken in DCM (10 mL). TFA (2 mL) was added to the solution and the mixture was stirred at RT for 1h. The solvent was concentrated and the residue was purified by preparative HPLC to give 10 mg (3%) of the title product. [M+H] Calc'd for C₂₅H₂₆N₆O₂, 443; Found, 443.

### Preparation 24A: N-(4-methoxy-phenyl)-N-methyl-2-phenyl-acetamide

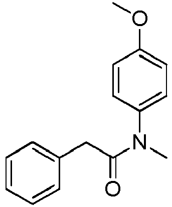

Phenyl-acetyl chloride (2.0 g, 1.31 mmol) was added at 0 °C to a solution of (4-methoxy-phenyl)-methyl-amine (1.5 g, 1.1mmol) and TEA (2.2 g, 2.2 mmol) in DCM (20 mL). The mixture was stirred at RT for 2h and quenched with NH₄Cl. The mixture was concentrated and the residue was purified by silica gel chromatography (PE:EA, 5:1) to give 2.0 g (71%) of the title compound. [M+H] Calc'd for C₁₆H₁₇NO₂, 256; Found, 256.

### Preparation 24B: N-(4-hydroxy-phenyl)-N-methyl-2-phenyl-acetamide

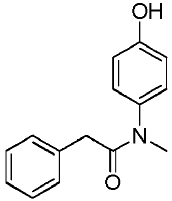

To a solution of N-(4-methoxy-phenyl)-N-methyl-2-phenyl-acetamide (2.0 g, 10 mmol) in DCM (20 mL) was added BBr₃ (40 mL, 1.0 M in DCM, 40 mmol) in portions at 0°C, and the mixture was stirred at RT for 2 h. The reaction mixture was diluted with water (30 mL), basified to pH 5 with sat. Na₂CO₃ and extracted with DCM (30 mL^{∗}3). The combined organics were dried over Na₂SO₄ and concentrated. The residue was purified by silica gel chromatography (PE:EA, 2:1) to give 1.5 g (94%) of the title product. [M+H] Calc'd for C₁₅H₁₅NO₂, 242; Found, 242.

### Example 24: N-[4-(4-hydroxy-pyrido[3,4-d]pyrimidin-2-yloxy)-phenyl]-N-methyl-2-phenyl-acetamide

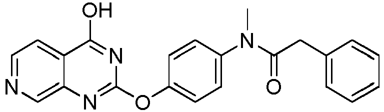

The title compound was prepared in 28% yield from 2-chloro-pyrido[3,4-d]pyrimidin-4-ol and N-(4-hydroxy-phenyl)-N-methyl-2-phenyl-acetamide according to the procedure for the preparation of Example 1. ¹H NMR (400 MHz, DMSO-*d*₆): δ 3.20 (s, 2H), 3.44 (s, 3H), 7.05-7.40 (m, 9H), 7.87 (d, *J* = 6.8 Hz, 1H), 8.50 (d, *J* = 6.8 Hz, 1H), 8.67 (s, 1H), 13.07 (s, 1H). [M+H] Calc'd for C₂₂H₁₈N₄O₃, 387; Found, 387.

### Preparation 25A: 1-(-methoxy-phenyl)-3-phenyl-piperidine

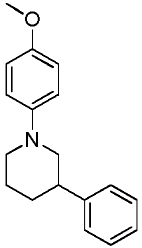

To a solution of 1-bromo-4-methoxy-benzene (0.5 g, 2.9 mmol) in toluene was added 3-phenyl-piperidine (0.5 g, 3.2 mmol), S-Phos (60 mg, 0.15 mmol), Pd₂(dba)₃ (160 mg, 0.17 mmol), *t*-BuOK (0.81 g, 7.23 mmol) and the mixture was stirred overnight at 95 °C under nitrogen atmosphere. The reaction mixture was cooled to RT and the solvent was concentrated. The residue was purified by silica gel chromatography (PE:EA, 10:1) to give 0.5 g (52%) of the title compound. [M+H] Calc'd for C₁₈H₂₁NO, 268; Found, 268.

### Preparation 25B: 4-(3-phenyl-piperidin-1-yl)-phenol

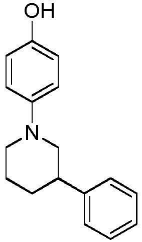

The title compound was prepared in 53% yield from 1-(4-methoxy-phenyl)-3-phenyl-piperidine according to the procedure of Preparation 22C. [M+H] Calc'd for C₁₇H₁₉NO, 254; Found 254.

### Example 25: 2-[4-(3-phenyl-piperidin-1-yl)-phenoxy]-pyrido[3,4-d]pyrimidin-4-ol

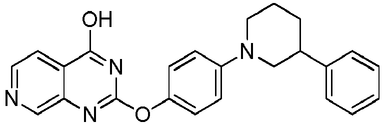

The title compound was prepared in 15% yield from 2-chloro-pyrido[3,4-d]pyrimidin-4-ol and 4-(3-phenyl-piperidin-1-yl)-phenol according to the procedure for the preparation of Example 1. ¹H NMR (400 MHz, DMSO-*d*₆): δ 1.62-1.94 (m, 4H), 2.72-2.83 (m, 3H), 3.69-3.76 (m, 2H), 7.00 (d, *J* = 12.0Hz, 2H), 7.13 (d, *J* = 12.0Hz, 2H), 7.19-7.35 (m, 5H), 7.84 (d, *J* = 7.6 Hz, 1H), 8.47 (d, *J* = 7.2 Hz, 1H), 8.65(s, 1H). [M+H] Calc'd for C₂₄H₂₂N₄O₂, 399; Found, 399.

### Preparation 26A: 4-(4-benzyloxy-phenyl)-2-phenyl-morpholine

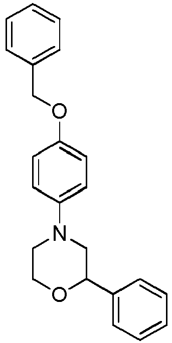

The title compound was prepared in 80% yield from 2-phenylmorpholine and 1-(benzyloxy)-4-bromobenzene according to the procedure of Preparation 25A. [M+H] Calc'd for C₂₃H₂₃NO₂, 346 Found, 346.

### Preparation 26B: 4-(2-phenyl-morpholin-4-yl)-phenol

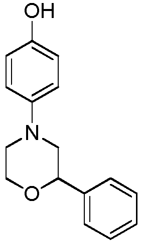

To a solution of 4-(4-benzyloxy-phenyl)-2-phenyl-morpholine (1.5 g, 3.9 mmol) in MeOH (20 mL) was added Pd/C (0.15 g), and the mixture was stirred overnight under H₂ atmosphere. The reaction mixture was filtered and the filtrate was concentrated to give 0.99 g (99%) of the title compound. [M+H] Calc'd for C₁₆H₁₇NO₂, 256; Found, 256.

### Example 26: 2-[4-(2-phenyl-morpholin-4-yl)-phenoxy]-pyrido[3,4-d]pyrimidin-4-ol

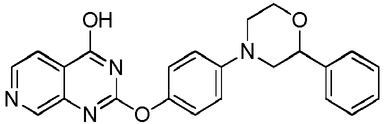

The title compound was prepared in 32% yield from 2-chloro-pyrido[3,4-d]pyrimidin-4-ol and 4-(2-phenyl-morpholin-4-yl)-phenol according to the procedure for the preparation of Example 1. ¹H NMR (400 MHz, DMSO-*d*₆): δ 2.25-2.63 (m, 1H), 2.79-2.87 (m, 1H), 3.27-3.86 (m, 3H), 4.09-4.14 (m, 1H), 4.62-4.66 (m, 1H), 7.06 (d, *J* = 12.8Hz, 2H), 7.17 (d, *J* = 12.4Hz, 2H), 7.29-7.48 (m, 5H), 7.84 (d, *J* = 7.6 Hz, 1H), 8.47 (d, *J* = 7.2 Hz, 1H), 8.65 (s, 1H), 13.07 (s, 1H). [M+H] Calc'd for C₂₃H₂₀N₄O₃, 401; Found, 401.

### Preparation 27A: (5-bromo-pyridin-3-yl)-methyl-carbamic acid tert-butyl ester

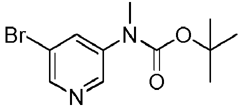

A solution of (5-bromo-pyridin-3-yl)-carbamic acid tert-butyl ester (3.4 g, 12.5 mmol) in THF (10 mL) was added to a solution of NaH (0.75 g, 18.8 mmol, 60% in mineral oil) in THF (15 mL) and the mixture was stirred at RT for 10 min. CH₃I (2.13 g, 15.0 mmol) was added and the mixture was stirred at RT for 2h. The reaction was quenched with aqueous NH₄Cl and extracted with EtOAc (3x). The organics were combined, dried over Na₂SO₄ and concentrated. The residue was purified by silica gel chromatography (PE:EA, 10:1) to give 2.7 g (76%) of the title compound. [M+H] Calc'd for C₁₁H₁₅BrN₂O₂, 287; Found, 287.

### Preparation 27B: methyl-(5-morpholin-4-yl-pyridin-3-yl)-amine

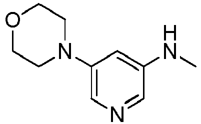

To a solution of (5-bromo-pyridin-3-yl)-methyl-carbamic acid tert-butyl ester (200 mg, 0.7 mmol) in toluene was added morpholine (120 mg, 1.4 mmol), BINAP (87 mg, 0.14 mmol), Pd(OAc)₂ (16 mg, 0.07 mmol), Cs₂CO₃ (2.5 g, 2.1 mmol) and the mixture was refluxed overnight under N₂ atmosphere. The solvent was concentrated and the residue was purified by silica gel chromatography (PE:EA, 1:1) to give boc-protected product, which was dissolved in DCM (2 mL). TFA was added to the solution and the mixture was stirred at RT for 1h. The solution was concentrated to give 100 mg (74%) of the title compound as the TFA salt. [M+H] Calc'd for C₁₀H₁₅N₃O, 194; Found, 194.

### Preparation 27C: 4- [methyl-(5 -morpholin-4-yl-pyridin-3 -yl)-amino] -phenol

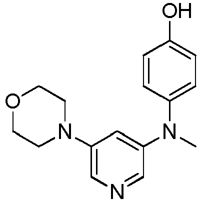

The title compound was prepared in 34% yield from 4-bromo-phenol and methyl-(5-morpholin-4-yl-pyridin-3-yl)-amine according to the procedure of Preparation 5A. [M+H] Calc'd for C₁₆H₁₉N₃O₂, 286; Found, 286.

### Example 27: 2-{4-[methyl-(5-morpholin-4-yl-pyridin-3-yl)-amino]-phenoxy}-pyrido[3,4-d]pyrimidin-4-ol

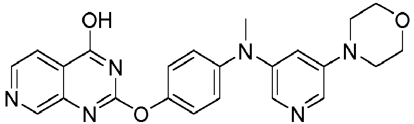

The title compound was prepared in 10% yield from 2-chloro-pyrido[3,4-d]pyrimidin-4-ol and 4-[methyl-(5-morpholin-4-yl-pyridin-3-yl)-amino]-phenol according to the procedure for the preparation of Example 1. ¹H NMR (400 MHz, DMSO-*d*₆): δ 3.12-3.13 (m, 4H), 3.20 (s, 3H), 3.71-3.73 (m, 4H), 6.90 (s, 1H), 7.07-7.09 (m, 2H), 7.23-7.26 (m, 2H), 7.75 (s, 1H), 7.86-7.90 (m, 2H), 8.49 (s, 1H), 8.67(s, 1H). [M+H] Calc'd for C₂₃H₂₂N₆O₃, 431; Found, 431.

### Preparation 28A: 1,2,3,4-tetrahydro-naphthalene-1-carboxylic acid (4-methoxy-phenyl)-methyl-amide

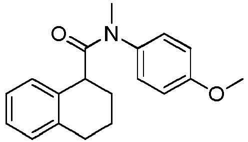

SOCl₂ (2 mL) was added to a solution of 1,2,3,4-tetrahydro-naphthalene-1-carboxylic acid (0.6 g, 3.4 mmol) in DCM (10 mL) and the mixture was refluxed for 2h. The solvent was concentrated and the residue was added to a mixture of (4-methoxy-phenyl)-methylamine (470 mg, 3.4 mmol) and TEA (2 mL) in DCM (10 mL). The reaction mixture was stirred at RT for 5h and quenched with IN HCl (10 mL). The organic layer was separated, washed with water, washed with brine, dried over Na₂SO₄ and concentrated. The residue was purified by silica gel chromatography (PE:EA, 4:1) to give 630 mg (63%) of the title compound. [M+H] Calc'd for C₁₉H₂₁NO₂, 296; Found, 296.

### Preparation 28B: (4-methoxy-phenyl)-methyl-(1,2,3,4-tetrahydro-naphthalen-1-ylmethyl)-amine

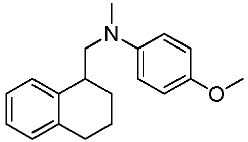

To a solution of 1,2,3,4-tetrahydro-naphthalene-1-carboxylic acid (4-methoxyphenyl)-methyl-amide (600 mg, 2.0 mmol) in THF (10 mL) was added BH₃ (8 mL, IN in Me₂S). The mixture was stirred at 40 °C for 2h, cooled to RT and quenched with MeOH. The solution was adjusted to PH=9 with IN NaOH and extracted with EA (3x). The organics were combined, washed with water, washed with brine, dried over Na₂SO₄ and concentrated. The residue was purified by silica gel chromatography (PE:EA, 10:1) to give 300 mg (53%) of the title compound. [M+H] Calc'd for C₁₉H₂₃NO, 282; Found, 282.

### Preparation 28C: 4-[methyl-(1,2,3,4-tetrahydro-naphthalen-1-ylmethyl)-amino]-phenol

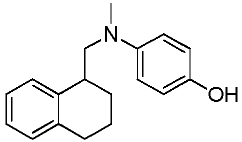

The title compound was prepared in 82% yield from 4-bromo-phenol and (4-methoxy-phenyl)-methyl-(1,2,3,4-tetrahydro-naphthalen-1-ylmethyl)-amine according to the procedure of Preparation 5A. [M+H] Calc'd for C₁₈H₂₁NO, 268; Found, 268.

### Example 28: 2-{4-[methyl-(1,2,3,4-tetrahydro-naphthalen-1-ylmethyl)-amino]-phenoxy}-pyrido[3,4-d]pyrimidin-4-ol

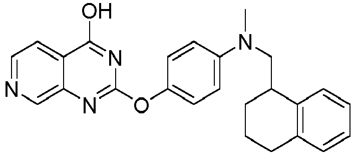

The title compound was prepared in 20% yield from 2-chloro-pyrido[3,4-d]pyrimidin-4-ol and 4-[methyl-(1,2,3,4-tetrahydro-naphthalen-1-ylmethyl)-amino]-phenol according to the procedure for the preparation of Example 1. ¹H NMR (400 MHz, DMSO-*d*₆): δ 1.65-1.89 (m, 4H), 2.67-2.74 (m, 2H), 2.96 (s, 3H), 3.29-3.56 (m, 3H), 6.78 (d, *J* = 11.2Hz, 2H), 7.07-7.22 (m, 6H), 7.86 (s, 1H), 8.49 (s, 1H), 8.67(s, 1H), 12.99 (s, 1H). [M+H] Calc'd for C₂₅H₂₄N₄O₂, 413; Found, 413.

### Preparation 29A: 2-(4-bromo-phenyl)-propionic acid methyl ester

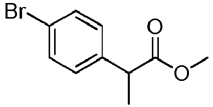

To a solution of (4-bromo-phenyl)-acetic acid methyl ester (1.0 g, 4.37 mmol) in THF was added LDA (4.4 mL, IN) at -78 °C under N₂ atmosphere and the mixture was stirred at this temperature for 10 min. CH₃I (0.74 g, 5.2 mmol) was added and the mixture was stirred at RT for 1h, quenched with aqueous NH₄Cl and extracted with EA (3x). The organics were combined, washed with water, washed with brine, dried over Na₂SO₄ and concentrated. The residue was purified by silica gel chromatography (PE:EA, 20:1) to give 0.6 g (57%) of the title compound. [M+H] Calc'd for C₁₀H₁₁BrO₂, 242; Found, 242.

### Preparation 29B: 2-{4-[(4-benzyloxy-phenyl)-methyl-amino]-phenyl}-propionic acid methyl ester

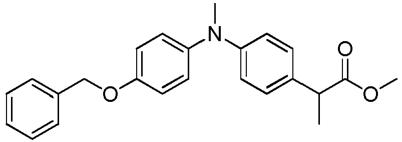

To a solution of 2-(4-bromo-phenyl)-propionic acid methyl ester (0.6 g, 2.47 mmol) in toluene was added (4-benzyloxy-phenyl)-methyl-amine (526 mg, 2.47 mmol), X-Phos (140 mg, 0.29 mmol), Pd(OAc)₂ (33 mg, 0.15 mmol), Cs₂CO₃ (3.2 g, 9.8 mmol) and the mixture was refluxed overnight. The solvent was concentrated and the residue was purified by silica gel chromatography (PE:EA, 10:1) to give 0.7 g (76%) of the title compound. [M+H] Calc'd for C₂₄H₂₅NO₃, 376; Found, 376.

### Preparation 29C: 2-{4-[(4-benzyloxy-phenyl)-methyl-amino]-phenyl}-propan-1-ol

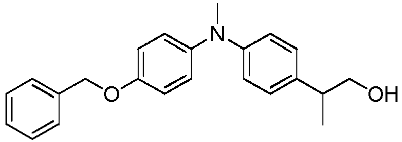

To a solution of 2-{4-[(4-benzyloxy-phenyl)-methyl-amino]-phenyl}-propionic acid methyl ester (0.7 g, 1.87mmol) in THF (15 mL) was added LAH (1.6 mL, 1N) at 0 °C under N₂ atmosphere and the mixture was stirred at this temperature for 2h. The reaction was warmed to RT, quenched with water and extracted with EA (3x). The organics were combined, washed with brine, dried over Na₂SO₄ and concentrated to give 0.6 g (92%) of the title compound. [M+H] Calc'd for C₂₃H₂₅NO₂, 348; Found, 348.

### Preparation 29D: (4-benzyloxy-phenyl)-[4-(2-methoxy-1-methyl-ethyl)-phenyl]-methyl-amine

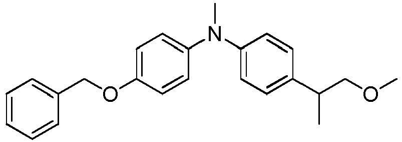

To a solution of 2-{4-[(4-benzyloxy-phenyl)-methyl-amino]-phenyl}-propan-1-ol (1.5 g, 4.3 mmol) in DMF (20 mL) was added NaH (0.32 g, 7.8 mmol, 60% in mineral oil) at 0 °C. After stirring for 30 min, MeI (0.92 g, 6.5 mmol) was added dropwise over 10 min and the reaction mixture was stirred at RT for 3h. The reaction mixture was diluted with water (100 mL), extracted with EA (10 mL^{∗}3). The combined organic were washed with water (150 mL^{∗}3), brine (150 mL), dried over Na₂SO₄, and concentrated to give 1.47 g (77%) of the title product. [M+H] Calc'd for C₂₄H₂₇NO₂, 362; Found, 362.

### Preparation 29E: 4-{[4-(2-methoxy-1-methyl-ethyl)-phenyl]-methyl-amino}-phenol

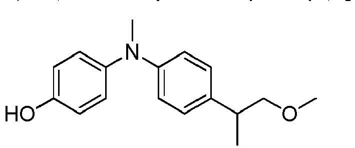

The title compound was prepared in 100% yield from (4-benzyloxy-phenyl)-[4-(2-methoxy-1-methyl-ethyl)-phenyl]-methyl-amine according to the procedure of Preparation 26B. [M+H] Calc'd for C₁₇H₂₁NO₂, 272 Found, 272.

### Example 29: 2-(4-{[4-(2-methoxy-1-methyl-ethyl)-phenyl]-methyl-amino}-phenoxy)-pyrido[3,4-d]pyrimidin-4-ol

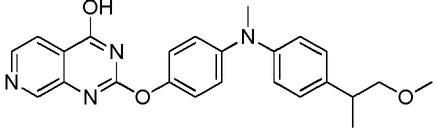

The title compound was prepared in 20% yield from 2-chloro-pyrido[3,4-d]pyrimidin-4-ol and 4-{[4-(2-methoxy-1-methyl-ethyl)-phenyl]-methyl-amino}-phenol according to the procedure for the preparation of Example 1. ¹H NMR (400 MHz, DMSO-*d*₆): δ 1.17 (t, *J* = 9.2 Hz,3 H), 2.91-2.95 (m, 1H), 3.20 (s, 3H), 3.30 (s, 3H), 3.35-3.38 (m, 2H), 6.93 (d, *J* = 12.0 Hz, 2H), 7.01(m, *J* = 11.2 Hz, 2H), 7.16-7.19 (m, 4H), 7.86 (d, *J* = 6.4 Hz, 1H), 8.48 (d, *J=* 6.4 Hz, 1H), 8.67(s, 1H). [M+H] Calc'd for C₂₄H₂₄N₄O₃, 417; Found, 417.

### Preparation 30A: methanesulfonic acid 2-{4-[(4-benzyloxy-phenyl)-methyl-amino]-phenyl}-propyl ester

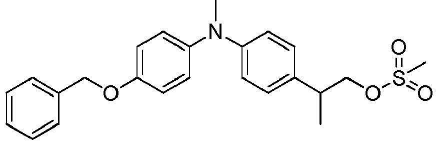

To a solution of 2-{4-[(4-benzyloxy-phenyl)-methyl-amino]-phenyl}-propan-1-ol (1.5 g, 4.32 mmol) in DCM (20 mL) was added TEA (0.72 g, 6.5 mmol) and MsCl (0.65 g, 5.6 mmol) at 0 °C and the mixture was stirred at RT for 2h. The reaction mixture was washed with aqueous NH₄Cl, washed with brine, dried over Na₂SO₄ and concentrated. The residue was purified by silica gel chromatography (PE:EA, 3:1) to give 1.4 g (76%) of the title compound. [M+H] Calc'd for C₂₄H₂₇NO₄S, 426; Found, 426.

### Preparation 30B: 3-{4-[(4-benzyloxy-phenyl)-methyl-amino]-phenyl}-butyronitrile

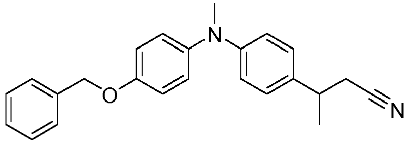

To a solution of methanesulfonic acid 2-{4-[(4-benzyloxy-phenyl)-methyl-amino]-phenyl}-propyl ester (1.35 g, 3.17 mmol) in DMSO (20 mL) was added KCN (0.4 g, 6.34 mmol) and 18-crown-6 (0.84 g, 3.17 mmol), and the mixture was stirred overnight at 65 °C. The reaction mixture was cooled to RT, quenched with water and extracted with EA (3x). The combined organics were washed with brine, dried over Na₂SO₄ and concentrated. The residue was purified by silica gel chromatography (PE:EA, 5:1) to give 0.56 g (50%) of the title compound. [M+H] Calc'd for C₂₄H₂₄N₂O, 357; Found, 357.

### Preparation 30C: 3-{4-[(4-hydroxy-phenyl)-methyl-amino]-phenyl}-butyronitrile

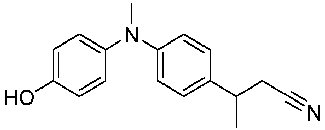

The title compound was prepared in 88% yield from 3-{4-[(4-benzyloxy-phenyl)-methyl-amino]-phenyl}-butyronitrile according to the procedure of Preparation 26B. [M+H] Calc'd for C₁₇H₁₈N₂O, 267 Found, 267.

### Example 30: 3-(4-{[4-(4-hydroxy-pyrido[3,4-d]pyrimidin-2-yloxy)-phenyl]-methyl-amino}-phenyl)-butyronitrile

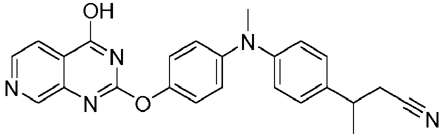

The title compound was prepared in 17% yield from 2-chloro-pyrido [3,4-d]pyrimidin-4-ol and 3-{4-[(4-hydroxy-phenyl)-methyl-amino]-phenyl}-butyronitrile according to the procedure for the preparation of Example 1. ¹H NMR (400 MHz, DMSO-*d*₆): δ 1.28 (d, *J* = 9.2 Hz, 3H), 2.77 (d, *J* = 9.2 Hz, 2H), 3.05-3.09 (m, 1H), 3.27 (s, 3H), 6.95-6.99 (m, 4H), 7.19-7.27 (m, 4H), 7.86 (d, *J* = 6.8 Hz, 1H), 8.49 (d, *J* = 6.4 Hz, 1H), 8.70 (s, 1H). [M+H] Calc'd for C₂₄H₂₁N₅O₂, 412; Found, 412.

### Preparation 31A: 1-cyclopentyl-5-methoxy-1H-indole

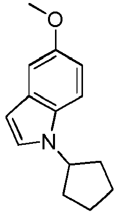

To a solution of 5-methoxy-1H-indole (1.5 g, 10 mmol) in DMF (20 mL) at 0°C was added NaH (480 mg, in mineral oil, 60%, 12 mmol) in portions and the mixture was stirred for 30 min. Bromo-cyclopentane (2.3 g, 15 mmol) was then added and the mixture was stirred overnight at 90°C. The reaction mixture was diluted with water (100 mL) and extracted with DCM (30 mL^{∗}3). The organics were combined, dried over Na₂SO₄ and concentrated. The residue was purified by silica gel chromatography (PE:EA, 20:1) to give 450 mg (21%) of the title product. [M+H] Calc'd for C₁₄H₁₇NO, 216; Found, 216.

### Preparation 31B: 1-cyclopentyl-1H-indol-5-ol

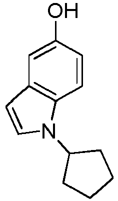

The title compound was prepared in 21% yield from 1-cyclopentyl-5-methoxy-1H-indole according to the procedure for the preparation of Example 4B. [M+H] Calc'd for C₁₃H₁₅NO, 202; Found, 202.

### Example 31: 2-(1-cyclopentyl-1H-indol-5-yloxy)-pyrido[3,4-d]pyrimidin-4-ol

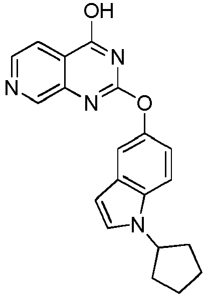

The title compound was prepared in 3% yield from 2-chloro-pyrido[3,4-d]pyrimidin-4-ol and 1-cyclopentyl-1H-indol-5-ol according to the procedure for the preparation of Example 1. ¹H NMR (400 MHz, DMSO-*d*₆): δ 1.72-1.76 (m, 2H), 1.87-1.91 (m, 4H), 2.15-2.21 (m, 2H), 4.92 (t, *J* = 6.4 Hz, 1H), 6.49 (d, *J* = 3.2 Hz, 1H), 7.08 (dd, *J* = 1.6, 7.2 Hz, 1H), 7.45 (d, *J* = 1.6 Hz, 1H), 7.54-7.59 (m, 2H), 7.88 (s, 1H), 8.51 (brs, 1H), 8.64 (brs, 1H). [M+H] Calc'd for C₂₀H₁₈N₄O₂, 347; Found, 347.

### Preparation 32A: 5-benzyloxy-1-phenyl-1H-indole

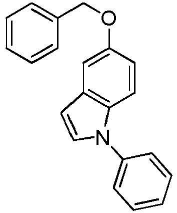

A mixture of iodobenzene (1.83 g, 8.97 mmol), 5-benzyloxy -1H-indole (2.0 g, 8.97 mmol), CuI (171 mg, 0.90 mmol) and Cs₂CO₃ (5.8 g, 17.94 mmol) in DMF (17 mL) was heated overnight at 120 °C. The reaction mixture was filtered and the filtrate was concentrated. The residue was purified by flash chromatography (PE:EA, 15:1 to 10:1) to give 1.29 g (49%) of the title compound. [M+H] Calc'd for C₂₁H₁₇NO, 300; Found, 300.

### Preparation 32B: 1-phenyl-1H-indol-5-ol

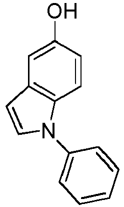

To a solution of 5-benzyloxy-1-phenyl-1H-indole (100 mg, 0.33 mmol) in EtOH (20 mL) was added Pd/C (20 mg) and the mixture was stirred overnight at RT under H₂ atmosphere. The reaction mixture was filtered on celite and concentrated to give 70 mg (100%) of the title crude product. [M+H] Calc'd for C₁₄H₁₁NO, 210; Found, 210.

### Preparation 32C: 1-phenyl-2,3-dihydro-1H-indol-5-ol

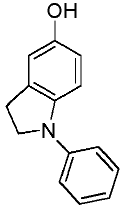

To a solution of 1-phenyl-1H-indol-5-ol (500 mg, 2.4 mmol) in AcOH (10 mL) was added NaBH₃CN (1.2 g, 19.1 mmol) and the reaction mixture was stirred overnight at RT. The reaction was diluted with water, basified to pH 8 with sat. Na₂CO₃ and extracted with EA (3x). The combined organics were washed with brine, dried over Na₂SO₄, filtered and concentrated to give 200 mg (40%) of the title crude product. [M+H] Calc'd for C₁₅H₁₅NO, 226; Found, 226.

### Example 32: 2-(1-phenyl-2,3-dihydro-1H-indol-5-yloxy)-pyrido[3,4-d]pyrimidin-4-ol

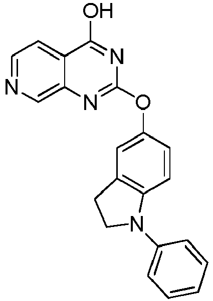

The title compound was prepared in 16% yield from 2-chloro-pyrido [3,4-d]pyrimidin-4-ol and 1-phenyl-2,3-dihydro-1H-indol-5-ol according to the procedure for the preparation of Example 1. ¹H NMR (400 MHz, DMSO-*d*₆): δ 3.14 (t, *J* = 8.0 Hz, 2H), 4.00 (t, *J* = 8.4 Hz, 2H), 6.97 (d, *J* = 6.8 Hz, 2H), 7.11 (d, *J* = 8.4 Hz, 1H), 7.16 (s, 1H), 7.27 (d, *J* = 8.0 Hz, 2H), 7.35-7.39 (m, 2H), 7.87 (d, *J* = 4.8 Hz, 1H), 8.50 (d, *J* = 5.2 Hz, 1H), 8.70 (s, 1H), 13.04 (brs, 1H). [M+H] Calc'd for C₂₁H₁₆N₄O₂, 357; Found, 357.

### Preparation 33A: 6-(tert-butyl-dimethyl-silanyloxy)-1,2,3,4-tetrahydro-quinoline

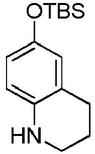

To a solution of 1,2,3,4-tetrahydro-quinolin-6-ol (1.0 g, 6.7 mmol) and imidazole (1.4 g, 20.1 mmol) in DCM (20 mL) was added TBSCl (1.7 g, 7.4 mmol) at ice-bath temperature, and the reaction was stirred at RT for 3h. The reaction was diluted with water and extracted with EA (3x). The combined organics were washed with brine, dried over Na₂SO₄, filtered and concentrated. The residue was purified by silica gel chromatography (PE:EA, 20:1) to give 1.7g (97%) of the title product.

### Preparation 33B: 6-(tert-butyl-dimethyl-silanyloxy)-1-phenyl-1,2,3,4-tetrahydro-quinoline

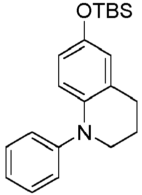

To a suspension of 6-(*tert*-butyl-dimethyl-silanyloxy)-1,2,3,4-tetrahydro-quinoline (100 mg, 0.38 mmol), iodo-benzene (78 mg, 0.38 mmol), BINAP (24 mg, 0.038 mmol) and Cs₂CO₃ (248 mg, 0.76 mmol) in toluene (10 mL) was added Pd₂(dba)₃ (18 mg, 0.019 mmol) under N₂ atmosphere. The reaction was stirred at reflux overnight, filtered and the filtrate was concentrated. The residue was purified by silica gel chromatography (PE) to give 100 mg (78%) of the title product. [M+H] Calc'd for C₂₁H₂₉NOSi, 340; Found, 340.

### Preparation 33C: 1-phenyl-1,2,3,4-tetrahydro-quinolin-6-ol

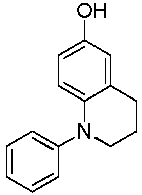

To a solution 6-(*tert*-butyl-dimethyl-silanyloxy)-1-phenyl-1,2,3,4-tetrahydro-quinoline (700 mg, 2.1 mmol) in THF (20 mL) was added TBAF (4.1 mL, 1.0 M in THF, 4.1 mmol) at RT and the reaction was stirred at RT for 30 min. The reaction was diluted with water, extracted with EA (3x). The combined organics were washed with brine, dried over Na₂SO₄, filtered and concentrated. The residue was purified by silica gel chromatography (PE:EA, 6:1) to give 250 mg (54%) of the title product. [M+H] Calc'd for C₁₅H₁₅NO, 226; Found, 226.

### Example 33: 2-(1-phenyl-1,2,3,4-tetrahydro-quinolin-6-yloxy)-pyrido[3,4-d]pyrimidin-4-ol

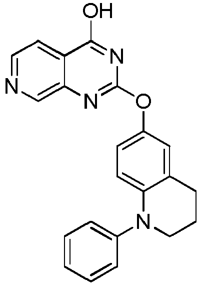

The title compound was prepared in 26% yield from 2-chloro-pyrido [3,4-d]pyrimidin-4-ol and 1-phenyl-1,2,3,4-tetrahydro-quinolin-6-ol according to the procedure for the preparation of Example 1. ¹H NMR (300 MHz, DMSO-*d*₆): δ 1.96-1.98 (m, 2H), 2.80 (t, *J* = 6.0 Hz, 2H), 3.57 (t, *J* = 6.0 Hz, 2H), 6.61 (d, *J* = 9.0 Hz, 1H), 6.84 (d, *J* = 8.4 Hz, 1H), 6.98 (s, 1H), 7.08-7.12 (m, 1H), 7.24 (d, *J* = 7.8 Hz, 2H),7.34-7.39 (m, 2H), 7.84 (d, *J* = 2.7 Hz, 1H), 8.48 (brs, 1H), 8.68 (brs, 1H). [M+H] Calc'd for C₂₂H₁₈N₄O₂, 371; Found, 371.

### Preparation 34A: 2-chloro-5-isopropenyl-pyridine

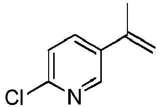

To a suspension of 2-chloro-5-iodo-pyridine (100 mg, 0.42 mmol), 2-isopropenyl-4,4,5,5-tetramethyl-[1,3,2]dioxaborolane (75 mg, 0.42 mmol), S-Phos (17 mg, 0.042 mmol) and K₃PO₄ (178 mg, 0.84 mmol) in toluene (10 mL) was added Pd₂(dba)₃ (8 mg, 0.008 mmol) under N₂ atmosphere. The reaction was stirred at reflux overnight, filtered and the filtrate was concentrated. The residue was purified by silica gel chromatography (PE:EA, 50:1) to give 30 mg (47%) of the title product. [M+H] Calc'd for C₈H₈ClN, 154; Found, 154.

### Preparation 34B: (5-isopropenyl-pyridin-2-yl)-(4-methoxy-phenyl)-methyl-amine

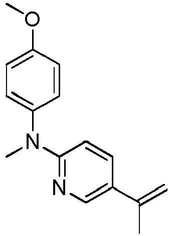

The title compound was prepared in 77% yield from 2-chloro-5-isopropenyl-pyridine and (4-methoxy-phenyl)-methyl-amine according to the procedure of Preparation 34A. [M+H] Calc'd for C₁₆H₁₈N₂O, 255; Found, 255.

### Preparation 34C: (5-isopropyl-pyridin-2-yl)-(4-methoxy-phenyl)-methyl-amine

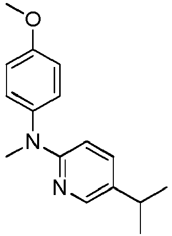

The title compound was prepared in 99% yield from (5-isopropenyl-pyridin-2-yl)-(4-methoxy-phenyl)-methyl-amine according to the procedure of Preparation 32B. [M+H] Calc'd for C₁₆H₂₀N₂O, 257; Found, 257.

### Preparation 34D: 4-[(5-isopropyl-pyridin-2-yl)-methyl-amino]-phenol

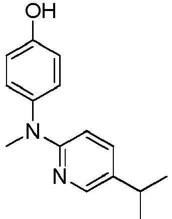

The title compound was prepared in 59% yield from (5-isopropyl-pyridin-2-yl)-(4-methoxy-phenyl)-methyl-amine according to the procedure of Preparation 4B. [M+H] Calc'd for C₁₅H₁₈N₂O, 243; Found, 243.

### Example 34: 2-{4-[(5-isopropyl-pyridin-2-yl)-methyl-amino]-phenoxy}-pyrido[3,4-d]pyrimidin-4-ol

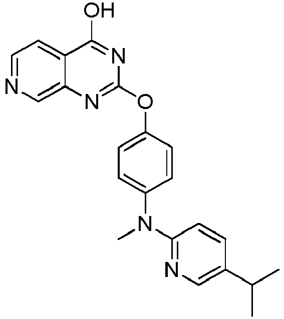

The title compound was prepared in 28% yield from 2-chloro-pyrido [3,4-d]pyrimidin-4-ol and 4-[(5-isopropyl-pyridin-2-yl)-methyl-amino]-phenol according to the procedure for the preparation of Example 1. ¹H NMR (300 MHz, DMSO-*d*₆): δ 1.16 (d, *J* = 6.6 Hz, 6H), 2.77-2.82 (m, 1H), 3.38 (s, 3H), 6.62 (d, *J* = 8.7 Hz, 1H), 7.34 (s, 4H), 7.41 (dd, *J* = 2.7, 9.0 Hz, 1H), 7.87 (d, *J=* 5.1 Hz, 1H), 8.04 (d, *J* = 2.1 Hz, 1H), 8.51 (d, *J* = 3.9 Hz, 1H), 8.72 (s, 1H), 13.12 (s, 1H). [M+H] Calc'd for C₂₂H₂₁N₅O₂, 388; Found, 388.

### Preparation 35A: (4-isopropyl-3-nitro-phenyl)-(4-methoxy-phenyl)-methyl-amine

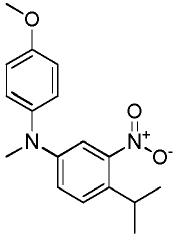

The title compound was prepared in 81% yield from (4-methoxy-phenyl)-methylamine and 4-bromo-1-isopropyl-2-nitro-benzene according to the procedure of Preparation 27B. [M+H] Calc'd for C₁₇H₂₀N₂O₃, 301; Found, 301.

### Preparation 35B: 4-isopropyl-N1-(4-methoxy-phenyl)-N1-methyl-benzene-1,3-diamine

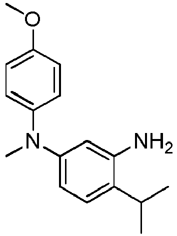

The title compound was prepared in 96% yield from (4-isopropyl-3-nitrophenyl)-(4-methoxy-phenyl)-methyl-amine according to the procedure of Preparation 32B. [M+H] Calc'd for C₁₇H₂₂N₂O, 271; Found, 271.

### Preparation 35C: (4-isopropyl-3-morpholin-4-yl-phenyl)-(4-methoxy-phenyl)-methyl-amine

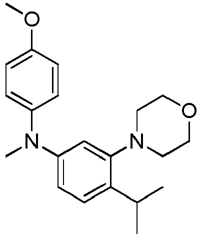

To a suspension of 4-isopropyl-N1-(4-methoxy-phenyl)-N1-methyl-benzene-1,3-diamine (2.0 g, 7.4 mmol), K₂CO₃ (5.1 g, 37.1 mmol) and NaI (2.0 g) in DMF (100 mL) was added 1-chloro-2-(2-chloro-ethoxy)-ethane (1.1 g, 7.4 mmol) and the reaction was stirred overnight at RT. The reaction was diluted with water and extracted with EA (3x). The combined organics were washed with brine, dried over Na₂SO₄, filtered and concentrated. The residue was purified by silica gel chromatography (PE:EA, 20:1) to give 1.5 g (60%) of the title product. [M+H] Calc'd for C₂₁H₂₈N₂O₂, 341; Found, 341.

### Preparation 35D: 4-[(4-isopropyl-3-morpholin-4-yl-phenyl)-methyl-amino]-phenol

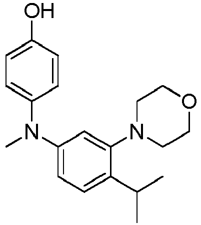

The title compound was prepared in 59% yield from (4-isopropyl-3-morpholin-4-yl-phenyl)-(4-methoxy-phenyl)-methyl-amine according to the procedure of Preparation 4B. [M+H] Calc'd for C₂₀H₂₆N₂O₂, 327; Found, 327.

### Example 35: 2-{4-[(4-isopropyl-3-morpholin-4-yl-phenyl)-methyl-amino]-phenoxy}-pyrido[3,4-d]pyrimidin-4-ol

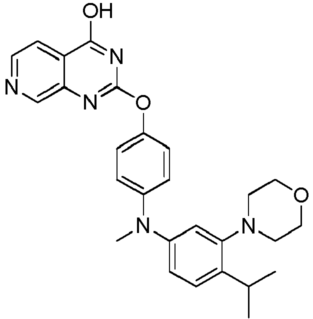

The title compound was prepared in 23% yield from 2-chloro-pyrido [3,4-d]pyrimidin-4-ol and 4-[(5-isopropyl-pyridin-2-yl)-methyl-amino]-phenol according to the procedure for the preparation of Example 1. ¹H NMR (400 MHz, DMSO-*d*₆): δ 1.17 (d, *J* = 6.8 Hz, 6H), 2.78 (t, *J* = 4.0 Hz, 4H), 3.28 (s, 3H), 3.34-3.41 (m, 1H), 3.72 (t, *J* = 4.0 Hz, 4H), 6.84-6.86 (m, 2H), 6.96 (d, *J* = 8.0 Hz, 2H), 7.18-7.25 (m, 3H), 7.88 (d, *J* = 5.2 Hz, 1H), 8.51 (d, *J* = 5.2 Hz, 1H), 8.70 (s, 1H), 13.06 (brs, 1H). [M+H] Calc'd for C₂₇H₂₉N₅O₃, 472; Found, 472.

### Preparation 36A: 1-[4-(4-benzyloxy-phenylamino)-phenyl]-ethanone

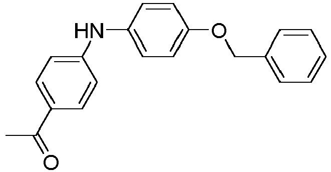

A mixture of 1-(4-bromo-phenyl)-ethanone (10 g, 0.05 mol), 4-benzyloxy-phenylamine (12 g, 0.06 mol), X-Phos (1.2 g, 2.5 mmol), Pd₂(dba)₃ (1.16 g, 1.26 mmol) and K₃PO₄ (16 g, 0.075 mol) in toluene (200 mL) was refluxed overnight under N₂ atmosphere. The reaction mixture was filtered and the filtrate was concentrated. The residue was purified by silica gel chromatography (PE:EA, 5:1) to give 11.7 g of the title product. [M+H] Calc'd for C₂₁H₁₉NO₂, 318; Found, 318.

### Preparation 36B: 1-{4-[(4-benzyloxy-phenyl)-methyl-amino]-phenyl}-ethanone

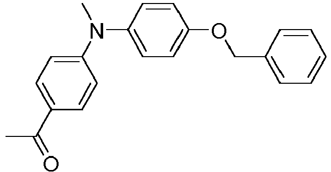

The title compound was prepared in 68% yield from 1-[4-(4-benzyloxy-phenylamino)-phenyl]-ethanone according to the procedure of Preparation 27A. [M+H] Calc'd for C₂₂H₂₁NO₂, 332 Found, 332.

### Preparation 36C: 1-{4-[(4-benzyloxy-phenyl)-methyl-amino]-phenyl}-ethanol

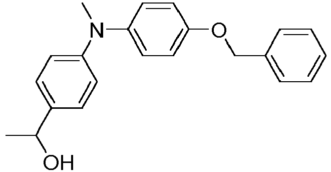

The title compound was prepared in 99% yield from 1-{4-[(4-benzyloxy-phenyl)-methyl-amino]-phenyl}-ethanone according to the procedure of Preparation 29C. [M+H] Calc'd for C₂₂H₂₃NO₂, 334 Found, 334.

### Preparation 36D: (4-benzyloxy-phenyl)-[4-(1-methoxy-ethyl)-phenyl]-methyl-amine

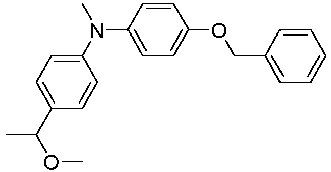

The title compound was prepared in 45% yield from 1-[4-(4-benzyloxy-phenylamino)-phenyl]-ethanone according to the procedure of Preparation 29D. [M+H] Calc'd for C₂₃H₂₅NO₂, 348 Found, 348.

### Preparation 36E: 4-{[4-(1-methoxy-ethyl)-phenyl]-methyl-amino}-phenol

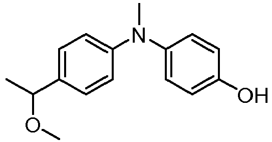

To a solution of (4-benzyloxy-phenyl)-[4-(1-methoxy-ethyl)-phenyl]-methyl-amine (0.947 g, 2.73 mmol) in THF (20 mL) was added Pd/C (0.10 g) and the mixture was stirred overnight under H₂ atmosphere. The reaction mixture was filtered on celite and the filtrate was concentrated to give 0.34 g (50%) of the title compound. [M+H] Calc'd for C₁₆H₁₉NO₂, 258; Found, 258.

### Example 36: 2-(4-{[4-(1-methoxy-ethyl)-phenyl]-methyl-amino}-phenoxy)-pyrido[3,4-d]pyrimidin-4-ol

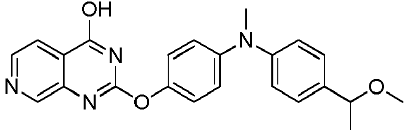

The title compound was prepared in 23% yield from 2-chloro-pyrido [3,4-d]pyrimidin-4-ol and 4-{[4-(1-methoxy-ethyl)-phenyl]-methyl-amino}-phenol according to the procedure for the preparation of Example 1. ¹H NMR (300 MHz, DMSO-*d*₆): δ 1.41 (d, *J* = 6.6 Hz, 3H), 3.07 (s, 3H), 3.27 (s, 3H), 4.23-4.25 (m, 1H), 7.01-7.04 (m, 4H), 7.20-7.24 (m, 4H), 7.86 (d, *J* = 5.1 Hz, 1H), 8.50 (d, *J* = 5.1 Hz, 1H), 8.69 (s, 1H). [M+H] Calc'd for C₂₃H₂₂N₄O₃, 403; Found, 403.

### Preparation 37A: methanesulfonic acid 2-{4-[(4-benzyloxy-phenyl)-methyl-amino]-phenyl}-propyl ester

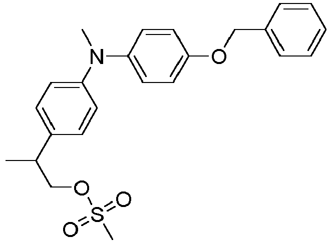

The title compound was prepared in 60% yield from 2-{4-[(4-benzyloxy-phenyl)-methyl-amino]-phenyl}-propan-1-ol according to the procedure of Preparation 30A. [M+H] Calc'd for C₂₄H₂₇NO₄S, 426; Found, 426.

### Preparation 37B: [4-(2-amino-isopropyl)phenyl]methyl[4-(phenylmethoxy)phenyl]amine

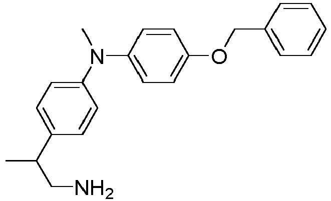

A solution of methanesulfonic acid 2-{4-[(4-benzyloxy-phenyl)-methyl-amino]-phenyl}-propyl ester (3.5 g, 8.24 mmol) in NH₃/THF (20 mL) was stirred overnight at 148 °C in a sealed tube. The reaction mixture was concentrated and the residue was purified by silica gel chromatography (DCM:MeOH, 10:1) to give 1.71 g (60%) of the title product. [M+H] Calc'd for C₂₃H₂₆N₂O, 347; Found, 347.

### Preparation 37C: (2-{4-[(4-benzyloxy-phenyl)-methyl-amino]-phenyl}-propyl)-carbamic acid tert-butyl ester

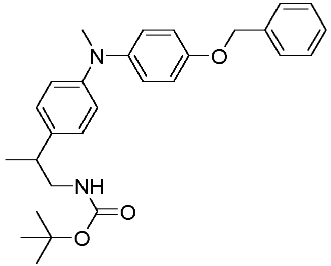

A solution of [4-(2-amino-isopropyl)phenyl]methyl[4-(phenylmethoxy)phenyl]amine (1.7 g, 4.91 mmol), (Boc)₂O (1.29 g, 5.90 mmol) and TEA (744 mg, 7.37 mmol) in THF (20 mL) was stirred for 2h at RT. The reaction mixture was concentrated and the residue was purified by silica gel chromatography (PE:EA, 10:1) to give 1.67 g (76%) of the title product. [M+H] Calc'd for C₂₈H₃₄N₂O₃, 447; Found, 447.

### Preparation 37D: (2-{4-[(4-hydroxy-phenyl)-methyl-amino]-phenyl}-propyl)-carbamic acid tert-butyl ester

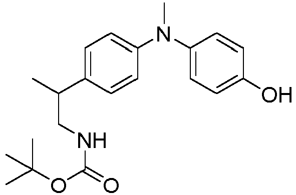

The title compound was prepared in 88% yield from (2-{4-[(4-benzyloxy-phenyl)-methyl-amino]-phenyl}-propyl)-carbamic acid tert-butyl ester according to the procedure of Preparation 36E. [M+H] Calc'd for C₂₁H₂₈N₂O₃, 357; Found, 357.

### Example 37: 2-(4-{[4-(2-amino-1-methyl-ethyl)-phenyl]-methyl-amino}-phenoxy)-pyrido[3,4-d]pyrimidin-4-ol

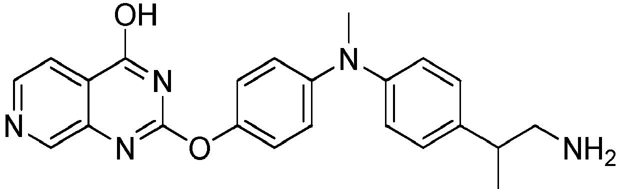

The title compound was prepared in 3.3% yield from 2-chloro-pyrido [3,4-d]pyrimidin-4-ol and (2-{4-[(4-hydroxy-phenyl)-methyl-amino]-phenyl}-propyl)-carbamic acid tert-butyl ester according to the procedure of Example 23. ¹H NMR (300 MHz, CD3OD-*d*₄): δ 1.28 (d, *J* = 6.3 Hz, 3H), 2.73-2.96 (m, 2H), 3.32 (s, 3H), 3.41-3.50 (m, 1H), 7.04-7.08 (m, 4H), 7.17-7.24 (m, 4H), 7.98 (d, *J* = 4.8 Hz, 1H), 8.47 (d, *J* = 4.8 Hz, 1H), 8.73 (s, 1H). [M+H] Calc'd for C₂₃H₂₃N₅O₂, 402; Found, 4 02.

### Preparation 38A: (4-{2-[(2-methoxyethyl)amino]-isopropyl}phenyl)methyl [4-(phenylmethoxy)phenyl]amine

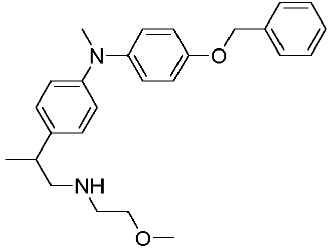

A mixture of methanesulfonic acid 2-{4-[(4-benzyloxy-phenyl)-methyl-amino]-phenyl}-propyl ester (1.22 g, 2.88 mmol), 2-methoxy-ethylamine (1.08 g, 14.4 mmol) and NaHCO₃ (720 mg, 8.64 mmol) in ACN (20 mL) was stirred overnight at 70 °C. The reaction mixture was concentrated and the residue was purified by silica gel chromatography (DCM:MeOH, 10:1) to give 1.14 g (100%) of the title product. [M+H] Calc'd for C₂₆H₃₂N₂O₂, 405; Found, 405.

### Preparation 38B: (4-{2-[(2-methoxyethyl)methylamino]-isopropyl}phenyl)methyl[4-(phenylmethoxy)phenyl]amine

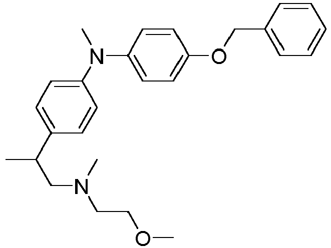

The title compound was prepared in 25% yield from (4-{2-[(2-methoxyethyl)amino]-isopropyl}phenyl)methyl[4 (phenylmethoxy)phenyl]amine according to the procedure of Preparation 27A. [M+H] Calc'd for C₂₇H₃₄N₂O₂, 419 Found, 419.

### Preparation 38C: 4-[(4-{2-[(2-methoxy-ethyl)-methyl-amino]-1-methyl-ethyl}-phenyl) methyl-amino]-phenol

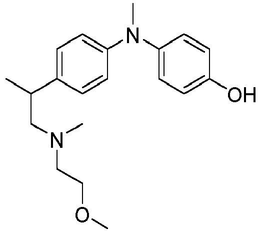

The title compound was prepared in 100% yield from (4-{2-[(2-methoxyethyl) methylamino]-isopropyl}phenyl)methyl[4-(phenylmethoxy)phenyl]amine according to the procedure of Preparation 36E. [M+H] Calc'd for C₂₀H₂₈N₂O₂, 329; Found, 329.

### Example 38: 2-{4-[(4-{2-[(2-methoxy-ethyl)-methyl-amino]-1-methyl-ethyl}-phenyl)-methyl-amino]-phenoxy}-pyrido[3,4-d]pyrimidin-4-ol

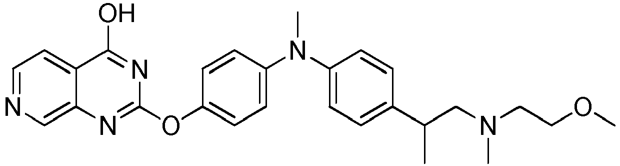

The title compound was prepared in 23% yield from 2-chloro-pyrido [3,4-d]pyrimidin-4-ol and 4-[(4-{2-[(2-methoxy-ethyl)-methyl-amino]-1-methyl-ethyl}-phenyl)-methyl-amino]-phenol according to the procedure for the preparation of Example 1. ¹H NMR (300 MHz, CD3OD-*d*₄): δ 1.12-1.14 (m, 3H), 2.59-2.60 (m, 1H), 2.72 (s, 3H), 3.04-3.66 (m, 12H), 6.94-7.12 (m, 8H), 7.90-7.95 (m, 1H), 8.34-8.35 (m, 1H), 8.73-8.74 (m, 1H). [M+H] Calc'd for C₂₇H₃₁N₅O₃, 474; Found, 474.

### Preparation 39A: 1-(4-bromo-phenyl)-1-cyclopropyl-ethanol

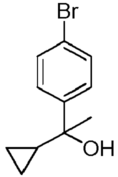

To a solution of (4-bromo-phenyl)-cyclopropyl-methanone (225 mg, 1mmol) in THF (10 mL) was added CH₃MgBr (0.4 mL, 1.1 mmol) at 0°C, and the mixture was stirred for 2h at RT. The reaction was quenched with aqueous NH₄Cl, extracted with EA (3x), dried over Na₂SO₄, filtered and concentrated. The residue was purified by silica gel chromatography (PE:EA, 10:1) to give 179 mg (75%) of the title product. [M+H] Calc'd for C₁₁H₁₃BrO, 241; Found, 241.

### Preparation 39B: 1-bromo-4-(1-cyclopropyl-ethyl)-benzene

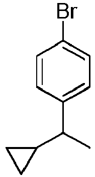

To a solution of 1-(4-bromo-phenyl)-1-cyclopropyl-ethanol (138 mg, 0.575 mmol) in DCM (5 mL) at -78 °C was added under N₂ atmosphere Et₃SiH (87 mg, 0.75 mmol) and TFA (131 mg, 1.15 mmol) and the mixture was stirred overnight at RT. The reaction was quenched with aqueous NaHCO₃, extracted with EA (3x), dried over Na₂SO₄, filtered and concentrated. The residue was purified by silica gel chromatography (PE) to give 120 mg (94%) of the title product. [M+H] Calc'd for C₁₁H₁₃Br, 225; Found, 225.

### Preparation 39C: [4-(1-cyclopropyl-ethyl)-phenyl]-methyl-amine

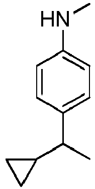

A mixture of 1-bromo-4-(1-cyclopropyl-ethyl)-benzene (3.7 g, 16.52 mmol) and Cu (53 mg, 0.825 mmol) in a methylamine solution (40 wt. % in H₂O, 35 mL) was stirred overnight at 100 °C in a sealed tube. The reaction was cooled to RT, diluted with H₂O and extracted with EA (3x). The combined organics were washed with brine, dried over Na₂SO₄, filtered and concentrated. The residue was purified by silica gel chromatography (PE:EA, 10:1) to give 440 mg (15%) of the title product. [M+H] Calc'd for C₁₂H₁₇N, 176; Found, 176.

### Preparation 39D: 4-{[4-(1-cyclopropyl-ethyl)-phenyl]-methyl-amino } -phenol

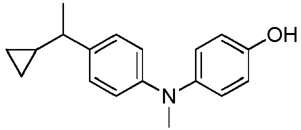

The title compound was prepared in 35% yield from [4-(1-cyclopropyl-ethyl)-phenyl]-methyl-amine according to the procedure of Preparation 5A. [M+H] Calc'd for C₁₈H₂₁NO, 268; Found, 268.

### Example 39: 2-(4-{[4-(1-cyclopropyl-ethyl)-phenyl]-methyl-amino}-phenoxy)-pyrido[3,4-d]pyrimidin-4-ol

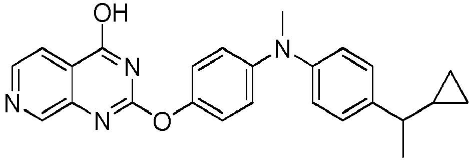

The title compound was prepared in 15% yield from 2-chloro-pyrido[3,4-d]pyrimidin-4-ol and 4-{[4-(1-cyclopropyl-ethyl)-phenyl]-methyl-amino}-phenol according to the procedure for the preparation of Example 1. ¹H NMR (300 MHz, DMSO-*d*₆): δ 0.10-0.20 (m, 2H), 0.35-0.38 (m, 1H), 0.46-0.50 (m, 1H), 0.89-0.93 (m, 1H), 1.25 (d, *J* = 6.9 Hz, 3H), 1.91-1.97 (m, 1H), 3.23 (s, 3H), 6.93 (d, *J* = 9.0 Hz, 2H), 7.04 (d, *J* = 8.7 Hz, 2H), 7.16-7.25 (m, 4H), 7.86 (d, *J* = 4.5 Hz, 1H), 8.49 (d, *J* = 4.5 Hz, 1H), 8.69 (s, 1H), 13.02 (s, 1H). [M+H] Calc'd for C₂₅H₂₄N₄O₂, 413; Found, 413.

### Preparation 40A: 3-(4-methoxy-phenylamino)-benzonitrile

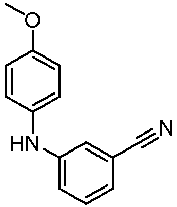

The title compound was prepared in 55% yield from 1-bromo-4-methoxybenzene and 3-amino-benzonitrile according to the procedure of Preparation 1A. [M+H] Calc'd for C₁₄H₁₂N₂O, 225; Found, 225.

### Preparation 40B: 3-[(4-methoxy-phenyl)-methyl-amino]-benzonitrile

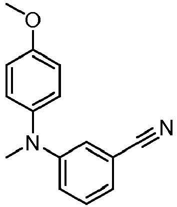

The title compound was prepared in 67% yield from 3-(4-methoxy-phenylamino)-benzonitrile according to the procedure of Preparation 1B. [M+H] Calc'd for C₁₅H₁₄N₂O, 239; Found, 239.

### Preparation 40C: 3-[(4-hydroxy-phenyl)-methyl-amino]-benzonitrile

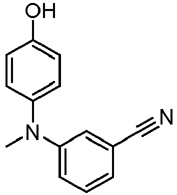

The title compound was prepared in 39% yield from 3-[(4-methoxy-phenyl)-methyl-amino]-benzonitrile according to the procedure of Preparation 4B. [M+H] Calc'd for C₁₄H₁₂N₂O, 225; Found, 225.

### Example 40: 3-{[4-(4-hydroxy-pyrido[3,4-d]pyrimidin-2-yloxy)-phenyl]-methyl-amino}-benzonitrile

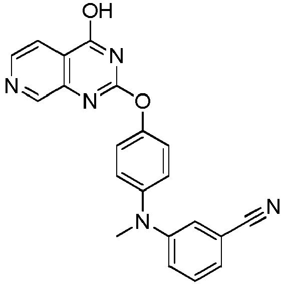

The title compound was prepared in 37% yield from 2-chloro-pyrido [3,4-d]pyrimidin-4-ol and 3-[(4-hydroxy-phenyl)-methyl-amino]-benzonitrile according to the procedure for the preparation of Example 1. ¹H NMR (300 MHz, DMSO- *d*₆): δ 3.30 (s, 3H), 7.16-7.29 (m, 5H), 7.35-7.44 (m, 3H), 7.89 (d, *J* = 5.1 Hz, 1H), 8.53 (d, *J* = 4.8 Hz, 1H), 8.72 (s, 1H). [M+H] Calc'd for C₂₁H₁₅N₅O₂, 370; Found, 370.

### Preparation 41A: 1-methyl-4-(3-nitro-benzyl)-piperazine

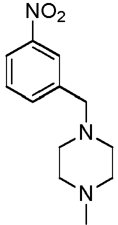

A mixture of 1-bromomethyl-3-nitro-benzene (5.0 g, 23 mmol), 1-methyl-piperazine (2.3 g, 23 mmol) and K₂CO₃ (6.4 g, 46 mmol) in DMF (80 mL) was stirred overnight at RT. The reaction mixture was diluted with water and the mixture was extracted with EA (3x). The combined organics were washed with brine, dried over Na₂SO₄, filtered and concentrated to give 3.2 g (59%) of the title product without further purification. [M+H] Calc'd for C₁₂H₁₇N₃O₂, 236; Found, 236.

### Preparation 41B: 3-(4-methyl-piperazin-1-ylmethyl)-phenylamine

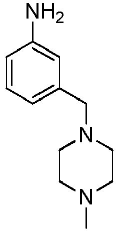

A mixture of 1-methyl-4-(3-nitro-benzyl)-piperazine (3.20 g, 13.6 mmol), Fe (7.60 g, 136 mmol), NH₄Cl (364 mg, 6.81 mmol) in EtOH (40 mL) and H₂O (10 mL) was stirred for 2h at 80°C. The mixture was concentrated, redissolved in methanol and filtered through celite. The filtrate concentrated to give 1.3 g (46%) of the title product without further purification. [M+H] Calc'd for C₁₂H₁₉N₃, 206; Found, 206.

### Preparation 41C: methyl-[3-(4-methyl-piperazin-1-ylmethyl)-phenyl]-amine

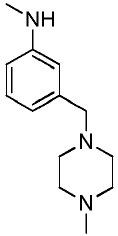

The title compound was prepared in 57% yield from 3-(4-methyl-piperazin-1-ylmethyl)-phenylamine according to the procedure of Preparation 18A.

### Preparation 41D: (4-benzyloxy-phenyl)-methyl-[3-(4-methyl-piperazin-1-ylmethyl)-phenyl]-amine

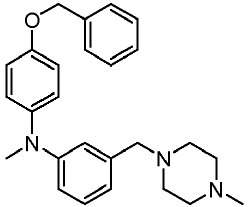

To a solution of methyl-[3-(4-methyl-piperazin-1-ylmethyl)-phenyl]-amine (1.0 g, 4.6 mmol) in toluene (5 mL) was added 1-benzyloxy-4-bromo-benzene (1.26 g, 4.79 mmol), biphenyl-2-yl-dicyclohexyl-phosphane (16 mg, 0.046 mmol), Pd₂(dba)₃ (42 mg, 0.046 mmol) and *t*-BuOK (767 mg, 6.85 mmol), and the mixture was stirred overnight at 110 °C under N₂ atmosphere. The reaction mixture was filtered and concentrated. The residue was purified by silica gel chromatography (PE:EA, 20:1) to give 1.29 g (70%) of the title compound. [M+H] Calc'd for C₂₆H₃₁N₃O, 402; Found, 402.

### Preparation 41E: 4-{methyl-[3-(4-methyl-piperazin-1-ylmethyl)-phenyl]-amino}-phenol

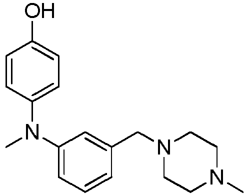

The title compound was prepared in 36% yield from (4-benzyloxy-phenyl)-methyl-[3-(4-methyl-piperazin-1-ylmethyl)-phenyl]-amine according to the procedure of Preparation 36E. [M+H] Calc'd for C₁₉H₂₅N₃O, 312; Found, 312.

### Example 41: 2-(4-{methyl-[3-(4-methyl-piperazin-1-ylmethyl)-phenyl]-amino}-phenoxy)-pyrido[3,4-d]pyrimidin-4-ol

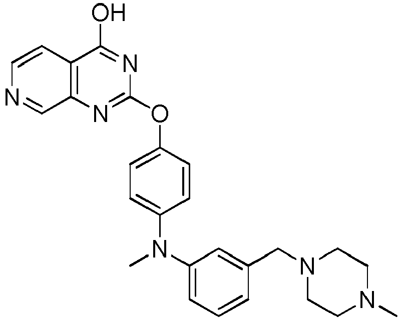

The title compound was prepared in 8% yield from 2-chloro-pyrido[3,4-d] pyrimidin-4-ol and 4-{methyl-[3-(4-methyl-piperazin-1-ylmethyl)-phenyl]-amino}-phenol according to the procedure for the preparation of Example 1. ¹H NMR (300 MHz, DMSO- *d*₆): δ 2.29 (s, 3H), 2.38-2.57 (m, 8H), 3.20 (s, 3H), 3.44 (s, 2H), 6.86-7.04 (m, 5H), 7.16-7.26 (m, 3H), 7.82 (d, *J=* 5.1 Hz, 1H), 8.44 (d, *J=* 5.1 Hz, 1H), 8.67 (s, 1H). [M+H] Calc'd for C₂₆H₂₈N₆O₂, 457; Found, 457.

### Preparation 42A: (4-bromo-phenoxy)-tert-butyl-dimethyl-silane

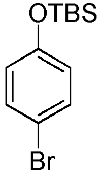

The title compound was prepared in 90% yield from 4-bromo-phenol according to the procedure of Preparation 33A. [M+H] Calc'd for C₁₂H₁₉BrOSi, 288; Found, 288.

### Preparation 42B: [4-(tert-butyl-dimethyl-silanyloxy)-phenyl]-(4-cyclopropyl-phenyl)-methyl-amine

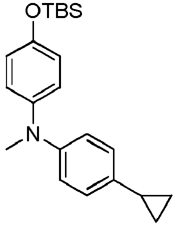

The title compound was prepared in 38% yield from (4-bromo-phenoxy)-*tert-*butyl-dimethyl-silane and (4-cyclopropyl-phenyl)-methyl-amine according to the procedure of Preparation 41D. [M+H] Calc'd for C₂₂H₃₁NOSi, 354; Found, 354.

### Preparation 42C: 4-[(4-cyclopropyl-phenyl)-methyl-amino]-phenol

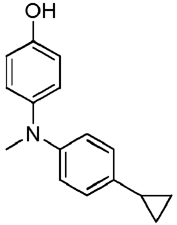

The title compound was prepared in 28% yield from [4-(*tert*-butyl-dimethyl-silanyloxy)-phenyl]-(4-cyclopropyl-phenyl)-methyl-amine according to the procedure of Preparation 33C. [M+H] Calc'd for C₁₆H₁₇NO, 240; Found, 240.

### Example 42: 2-{4-[(4-cyclopropyl-phenyl)-methyl-amino]-phenoxy}-pyrido[3,4-d]pyrimidin-4-ol

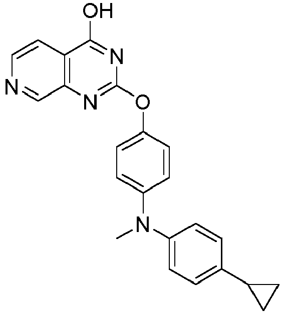

The title compound was prepared in 3% yield from 2-chloro-pyrido[3,4-d] pyrimidin-4-ol and 4-[(4-cyclopropyl-phenyl)-methyl-amino]-phenol according to the procedure for the preparation of Example 1. ¹H NMR (300 MHz, DMSO- *d*₆): δ 0.60-0.63 (m, 2H), 0.86-0.90 (m, 2H), 1.86-1.88 (m, 1H), 3.23 (s, 3H), 6.88-7.16 (m, 8H), 7.84-7.86 (m, 1H), 8.47-8.49 (m, 1H), 8.68 (s, 1H). [M+H] Calc'd for C₂₃H₂₀N₄O₂, 385; Found, 385.

| Ex. | Structure (prepared by procedure of cited Example) | MS (ESI) m/z | NMR spectrum data |
|---|---|---|---|
| 43 | 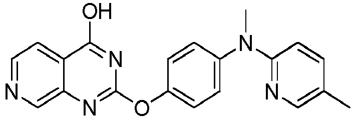 Prepared by the procedure of Example 1 | 360 | ¹H NMR (400 MHz, DMSO-*d*₆): δ 2.15 (s , 3H), 3.36 (s, 3H), 6.61 (d, *J* = 11.6 Hz, 1H), 7.32-7.34 (m, 5H), 7.87 (d, *J* = 6.4 Hz, 1H), 7.99 (s, 1H), 8.50 (d, *J* = 6.8 Hz, 1H), 8.72 (s, 1H). |
| 44 | 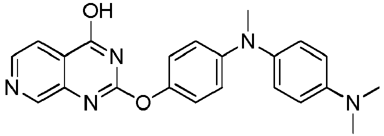 Prepared by the procedure of Example 1 | 388 | ¹H NMR (400 MHz, DMSO-*d*₆): δ 2.89 (s , 6H), 3.20 (s, 3H), 6.69 (d, *J* = 12.4 Hz, 2H), 6.78(d, *J* = 12.4 Hz, 2H), 7.04-7.09 (m, 4H), 7.86 (d, *J* = 6.4 Hz, 1H), 8.50 (d, *J* = 6.8 Hz, 1H), 8.68 (s, 1H). |
| 45 | 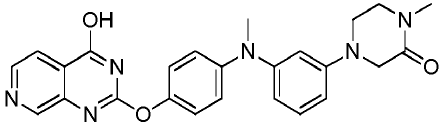 Prepared by the procedure of Example 22 | 457 | ¹H NMR (400 MHz, DMSO-*d*₆): δ 2.89 (s , 3H), 3.41 ( s, 3H), 3.42-3.46 (m, 4H), 3.72 (s, 2H), 6.51 (d, *J* = 7.6 Hz, 1H), 6.60 (d, *J* = 8.4 Hz, 2H), 6.99-7.20 (m, 5H), 7.86 (d, *J* = 5.2 Hz, 1H), 8.48 (d, *J* = 5.6 Hz, 1H), 8.68 (s, 1H). |
| 46 | 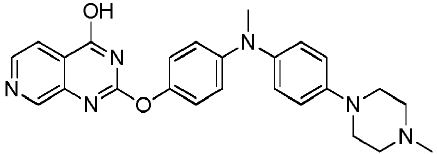 Prepared by the procedure of Example 22 | 443 | ¹H NMR (400 MHz, DMSO-*d*₆): δ 2.23 (s, 3H), 2.47-2.49 (m, 4H), 3.10-3.12 (m, 4H), 3.21 (s, 3H), 6.74 (d, *J* = 12.0 Hz, 2H), 6.95 (d, *J*=12.0 Hz, 2H), 7.02-7.09 (m, 4H), 7.82(d, *J*=6.8 Hz, 1H), 8.45 (d, *J*=6.8 Hz, 1H), 8.67 (s, 1H). |
| 47 | 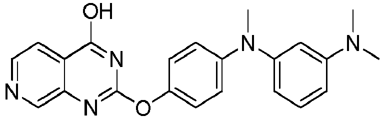 Prepared by the procedure of Example 1 | 388 | ¹H NMR (400 MHz, DMSO-*d*₆): δ 2.87 (s , 6H), 3.32 ( s, 3H), 6.38-6.44 (m, 3H), 6.93-6.96 (m, 2H), 7.13-7.17 (m, 3H), 7.86 (d, *J* = 6.4 Hz, 1H), 8.47 (d, *J* = 6.8 Hz, 1H), 8.68 (s, 1H). |
| 48 | 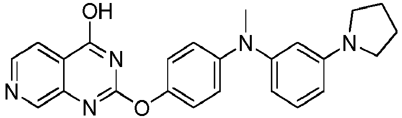 Prepared by the procedure of Example 22 | 414 | ¹H NMR (400 MHz, DMSO-*d*₆): δ 1.96 (m , 4H), 3.20 ( s, 4H), 3.32 (s, 3H), 6.57-6.66 (m, 4H), 7.03-7.08 (m, 4H), 7.86 (d, *J* = 4.8 Hz, 1H), 8.50 (d, *J=* 4.8 Hz, 1H), 8.69 (s, 1H), 12.99 (s, 1H). |
| 49 | 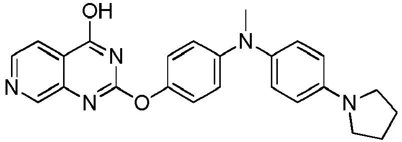 Prepared by the procedure of Example 22 | 414 | ¹H NMR (400 MHz, DMSO-*d*₆): δ 1.96 (m, 4H), 3.20 (m, 4H), 3.32 (s, 3H), 6.25-6.35 (m, 3H), 6.94-7.18 (m, 5H), 7.87 (d, *J* = 4.8 Hz, 1H), 8.51 (d, *J=* 4.8 Hz, 1H), 8.69 (s, 1H), 13.00 (s, 1H). |
| 50 | 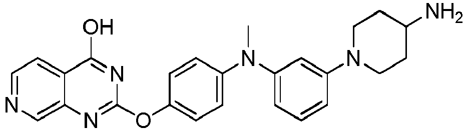 Prepared by the procedure of Example 23 | 443 | ¹H NMR (400 MHz, CD₃OD): δ 1.91-2.95 (m, 2H), 2.19-2.25 (m, 2H), 3.25-3.45 (m, 6H), 3.74-3.78 (m, 2H), 6.81-6.90 (m, 2H), 7.15-7.33 (m, 6H), 8.11 (d, *J* = 6.4Hz, 1H), 8.53 (d, *J* = 6.4Hz, 1H), 8.81 (s, 1H). |
| 51 | 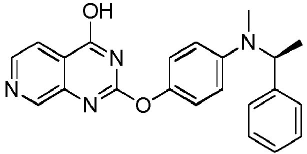 Prepared by the procedure of Example 5 | 373 | ¹H NMR (400 MHz, DMSO-*d*₆): δ 1.50 (d, *J* = 9.2 Hz, 3H ), 2.69 (s, 3H), 5.14-5.16 (m, 1H), 6.85 (d, *J* = 9.6 Hz, 2H), 7.10 (d , *J* = 9.2 Hz, 2H), 7.24-7.37 (m, 5H), 7.80 (d, *J* = 6.8 Hz, 1H), 8.48 (d, *J* = 7.2 Hz, 1H), 8.67 (s, 1H), 13.05 (s, 1H). |
| 52 | 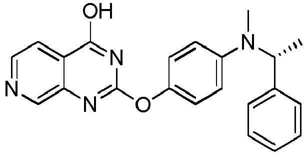 Prepared by the procedure of Example 5 | 373 | ¹H NMR (400 MHz, DMSO-*d*₆): δ 1.50 (d, *J* = 9.2 Hz, 3H ), 2.69 (s, 3H), 5.14-5.16 (m, 1H), 6.85 (d, *J* = 9.6 Hz, 2H), 7.10 (d , *J* = 9.2 Hz, 2H), 7.24-7.37 (m, 5H), 7.80 (d, *J* = 6.8 Hz, 1H), 8.48 (d, *J* = 7.2 Hz, 1H), 8.67 (s, 1H), 13.05 (s, 1H). |
| 53 | 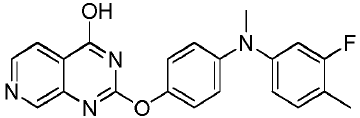 Prepared by the procedure of Example 1 | 377 | ¹H NMR (400 MHz, DMSO-*d*₆): δ 2.14 (s, 3H), 3.25 (s, 3H), 6.69-6.77 (m, 2H), 7.08-7.26 (m, 5H), 7.86 (d, *J* = 6.4 Hz, 1H), 8.48 (d, *J* = 6.8 Hz, 1H), 8.67 (s, 1H). |
| 54 | 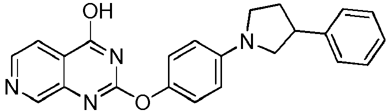 Prepared by the procedure of Example 25 | 385 | ¹H NMR (400 MHz, DMSO-*d*₆): δ 1.96-2.06 (m, 2H), 2.47-2.49 (m, 1H), 3.36-3.53 (m, 3H), 3.67-3.70 (m, 1H), 6.59 (d, *J* = 12.4 Hz, 2H), 7.08 (d, *J* = 12.4 Hz, 2H), 7.22-7.34 (m , 5H), 7.82 (d, *J=* 6.8 Hz, 1H), 8.43 (d, *J* = 6.8 Hz, 1H), 8.63( s, 1H). |
| 55 | 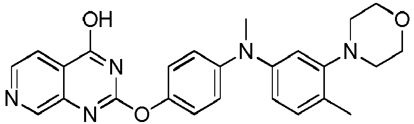 Prepared by the procedure of Example 18 | 444 | ¹H NMR (300 MHz, DMSO-*d*₆): δ 2.19 (s, 3H), 2.77-2.80 (m, 4H), 3.24 (s, 3H), 3.68-3.71 (m, 4H), 6.69-6.73(m, 2H), 6.80 (d, *J=* 12.0 Hz, 2H), 7.09-7.14 (m, 3H), 7.81 (d, *J* = 6.4 Hz, 1H), 8.43 (d, *J* = 6.0 Hz, 1H), 8.65(s, 1H). |
| 56 | 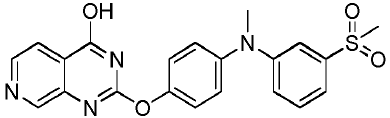 Prepared by the procedure of Example 1 | 423 | ¹H NMR (300 MHz, DMSO-*d*₆): δ 3.18 (s, 3H), 3.35 (s, 3H), 7.17-7.50 (m, 8H), 7.87 (d, *J* = 6.4 Hz, 1H), 8.50 (d, *J* = 6.0 Hz, 1H), 8.70 (s, 1H). |
| 57 | 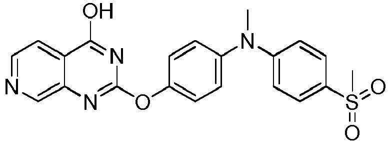 Prepared by the procedure of Example 1 | 423 | ¹H NMR (300 MHz, DMSO-*d*₆): δ 3.08 (s, 3H), 3.35 (s, 3H), 6.89 (d, *J* = 12.0 Hz, 2H), 7.37-7.44 (m, 4H), 7.68 (d, *J* = 12.0 Hz, 2H). 7.87 (d, *J* = 6.4 Hz, 1H), 8.51 (d, *J* = 6.0 Hz, 1H), 8.71(s, 1H). |
| 58 | 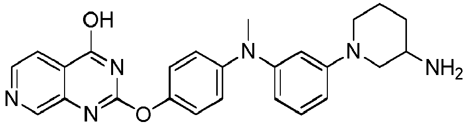 Prepared by the procedure of Example 23 | 443 | ¹H NMR (400 MHz, CD₃OD): δ 1.70-1.81 (m, 2H), 1.93-2.04 (m, 2H), 3.11-3.23 (m, 3H), 3.32 (s, 3H), 3.45-3.49 (m, 2H), 6.66-6.77 (m, 2H), 7.02-7.24 (m, 6H), 8.19(d, *J* = 6.8 Hz, 1H), 8.53 (d, *J* = 6.4 Hz, 1H), 8.83 (s, 1H). |
| 59 | 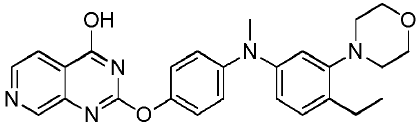 Prepared by the procedure of Example 18 | 458 | ¹H NMR (300 MHz, DMSO-*d*₆): δ 1.17 (t, *J* = 10.0 Hz, 3H), 2.60 (q, *J*=10.0 Hz, 2H ), 2.76-2.79 (m, 4H), 3.30 (s, 3H), 3.68-3.71 (m, 4H), 6.79 (d, *J* = 9.2 Hz, 2H), 6.93 (d, *J* = 9.2 Hz, 2H), 7.14-7.17 (m, 3H), 7.85(d, *J*=6.8 Hz, 1H), 8.49 (d, *J*=7.2 Hz, 1H), 8.67(s, 1H). |
| 60 | 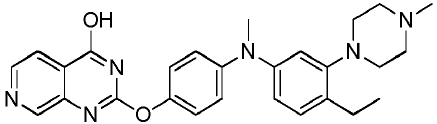 Prepared by the procedure of Example 18 | 471 | ¹H NMR (300 MHz, DMSO-*d*₆): δ 1.16 (t, *J* = 10.0 Hz, 3H), 2.25 (s, 3H), 2.48-2.62 (m, 6H), 2.76-2.79 (m, 4H), 3.25 (s, 3H), 6.77 (d, J=8.0 Hz, 2H), 6.93 (d, *J*=8.0 Hz, 2H), 7.14-7.17 (m, 3H), 7.84 (d, *J*=6.4 Hz, 1H), 8.47 (d, *J*=6.4 Hz, 1H), 8.66 (s, 1H). |
| 61 | 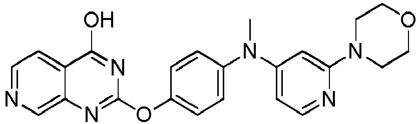 Prepared by the procedure of Example 18 | 431 | ¹H NMR (300 MHz, DMSO-*d*₆): δ 3.28-3.36 (m, 7H), 3.66-3.69 (m, 4H), 6.03-6.07 (m, 2H), 7.32-7.42 (m, 4H), 7.77 (d, *J* = 7.6 Hz, 1H), 7.89 (d, *J=* 7.6 Hz, 1H), 8.53 (d, *J*=7.6 Hz, 1H), 8.71 (s, 1H), 13.15 (s, 1H). |
| 62 | 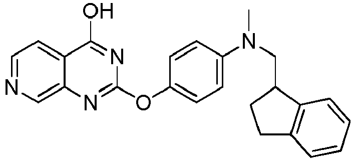 Prepared by the procedure of Example 28 | 399 | ¹H NMR (300 MHz, DMSO-*d*₆): δ 1.82-1.86 (m, 1H), 2.07-2.17 (m, 1H), 2.77-2.84 (m, 1H), 2.95-3.02 (m, 1H), 3.30-3.36 (m, 4H), 3.52-3.55 (m, 1H), 3.67-3.72 (m, 1H), 6.79 (d, *J* = 9.6 Hz, 1H), 7.12-7.30 (m, 6H), 7.80 (d, *J*=5.2 Hz, 1H), 8.50 (d, *J*=5.2 Hz, 1H), 8.68 (s, 1H), 13.00 (s, 1H). |
| 63 | 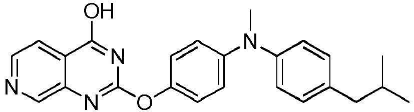 Prepared by the procedure of Example 18 | 401 | ¹H NMR (300 MHz, DMSO-*d*₆): δ 0.85 (d, *J* = 8.4 Hz, 6H), 1.75-1.82 (m, 1H), 2.38 (d, *J* = 7.6 Hz, 2H), 3.22 (s, 3H), 6.93 (d, *J* = 11.6 Hz, 2H), 7.01 (d, *J* = 10.8 Hz, 2H), 7.11 (d, *J* = 11.2 Hz, 2H), 7.17 (d, *J* = 11.6 Hz, 1H), 7.85(d, *J* = 7.2 Hz, 1H), 8.49 (d, *J* = 6.0 Hz, 1H), 8.68 (s, 1H), 13.01 (s, 1H). |
| 64 | 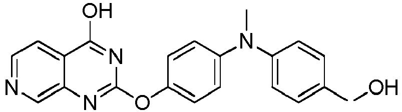 Prepared by the procedure of Example 26 | 403 | ¹H NMR (300 MHz, DMSO-*d*₆): δ 1.40 (s, 6H), 3.26 (s, 3H), 4.93 (s, 1H), 6.93 (d, *J* = 11.6 Hz, 2H), 7.03 (d, *J* = 11.2 Hz, 2H), 7.17 (d, *J* = 12.0 Hz, 2H), 7.41 (d, *J* = 11.6 Hz, 1H), 7.85 (d, *J* = 6.8 Hz, 1H), 8.49 (d, *J* = 6.8 Hz, 1H), 8.68 (s, 1H), 13.01 (s, 1H). |
| 65 | 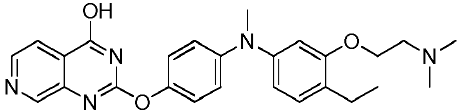 Prepared by the procedure of Example 18 | 460 | ¹H NMR (300 MHz, DMSO-*d*₆): δ 1.12 (t,*J* = 10.0 Hz, 3H), 2.52-2.60 (m, 2H), 2.87 (s, 6H), 3.28 (s, 3H), 3.51 (t, *J* = 6.0 Hz, 2H), 4.26 (t, *J =* 6.0 Hz, 2H), 6.66-6.74 (m, 2H), 6.94 (d, *J =* 11.6 Hz, 2H), 7.10-7.19 (m, 3H), 7.87(d, J= 6.4 Hz, 1H), 8.50(d, *J= 6.0* Hz, 1H), 8.68 (s, 1H). |
| 66 | 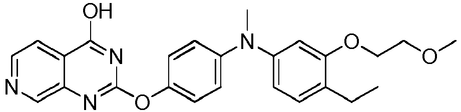 Prepared by the procedure of Example 18 | 447 | ¹H NMR (300 MHz, DMSO-*d*₆): δ 1.11 (t,*J* = 10.0 Hz, 3H), 2.47-2.52 (m, 2H), 3.26 (s, 3H), 3.32 (s, 3H), 3.63 (t, *J =* 6.0 Hz, 2H), 4.03 (t, *J =* 6.0 Hz, 2H), 6.59-6.66 (m, 2H), 6.94 (d, *J =* 11.6 Hz, 2H), 7.06-7.17 (m, 3H), 7.87 (d, *J* = 6.4 Hz, 1H), 8.48(d, J= 6.0 Hz, 1H), 8.67 (s, 1H). |
| 67 | 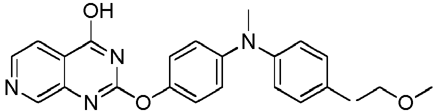 Prepared by the procedure of Example 29 | 431 | ¹H NMR (400 MHz, DMSO-*d*₆): δ 1.22 (s, 6H), 3.20 (s, 3H), 3.30 (s, 3H), 3.32 (s, 2H), 6.93 (d, *J =* 11.6 Hz, 2H), 7.01 (d, *J =* 11.2 Hz, 2H),7.19 (d, *J =* 11.6 Hz, 2H), 7.31 (d, *J =* 11.2 Hz, 2H), 7.86 (d, *J* = 6.4 Hz, 1H), 8.48 (d, *J =* 6.4 Hz, 1H), 8.67 (s, 1H). |
| 68 | 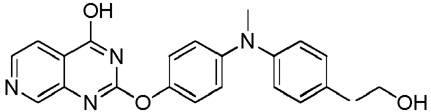 Prepared by the procedure of Example 29 | 417 | ¹H NMR (400 MHz, DMSO-*d*₆): δ 1.20 (s, 6H), 3.25 (s, 3H), 3.38 (s, 2H), 6.93 (d, *J =* 12.0 Hz, 2H), 7.01 *(d, J =* 11.2 Hz, 2H), 7.16 (d, *J* = 12.0 Hz, 2H), 7.31 (d, *J =* 11.2 Hz, 2H), 7.84 (d, *J=* 6.8 Hz, 1H), 8.48 (d, J= 6.4 Hz, 1H), 8.67(s, 1H). |
| 69 | 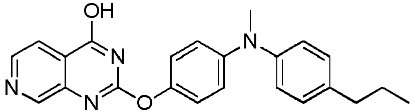 Prepared by the procedure of Example 29 | 387 | ¹H NMR (400 MHz, DMSO-*d*₆): δ 0.88 (t, *J=* 9.6 Hz, 3H), 1.53-1.57 (m, 2H), 2.49 (*J* = 6.8 Hz, 2H) 3.24 (s, 3H), 6.93 (d, *J =* 11.2 Hz, 2H), 7.01 (d, *J =* 11.2 Hz, 2H),7.12-7.17 (m, 4H), 7.85 (d, *J =* 6.4 Hz, 1H), 8.48 (d, *J*=6.4 Hz, 1H), 8.67 (s, 1H). |
| 70 | 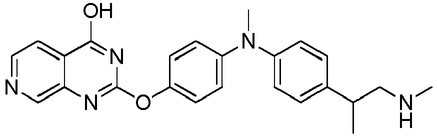 Prepared by the procedure of Example 30 | 416 | ¹H NMR (400 MHz, DMSO-*d*₆): δ 1.25 (d, *J=* 8.4 Hz,3H), 2.71 (s, 3H), 3.08 (d, *J =* 8.4 Hz, 2H), 3.21-3.23 (m, 1H), 3.32 (s, 3H), 7.05-7.26 (m, 8H), 7.24 (d, *J=* 7.2 Hz, 1H), 8.55 (d, *J =* 7.2 Hz, 1H), 8.87(s, 1H). |
| 71 | 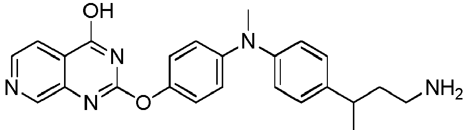 Prepared by the procedure of Example 30 | 416 | ¹H NMR (400 MHz, DMSO-*d*₆): δ 1.18 (d, *J=* 7.2 Hz, 3H), 1.76-1.79 (m, 2H), 2.52-2.58 (m, 1H), 2.69-2.72 (m, 2H), 3.23 (s, 3H), 6.88-7.11 (m, 8H),, 7.67 (d, *J*=4.8 Hz, 1H), 8.19 (d, *J*=4.8 Hz, 1H), 8.57 (s, 1H). |
| 72 | 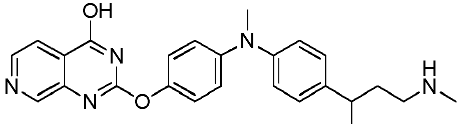 Prepared by the procedure of Example 30 | 430 | ¹H NMR (400 MHz, DMSO-*d*₆): δ 1.18 (d, *J =* 7.2 Hz, 3H), 1.76-1.79 (m, 2H), 2.48 (s, 3H), 2.58-2.72 (m, 3H), 3.26 (s, 3H), 6.88-7.11 (m, 8H), 7.67 (d, *J=* 7.2 Hz, 1H), 8.20 (d, *J=*7.2 Hz, 1H), 8.57 (s, 1H). |
| 73 | 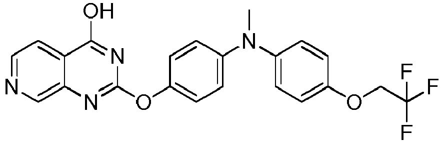 Prepared by the procedure of Example 5 | 443 | ¹H NMR (300 MHz, DMSO-*d*₆): δ 3.23 (s, 3H), 4.68-4.77 (m, 2H), 6.82 (d, *J =* 11.6 Hz, 2H), 7.06-7.15 (m, 6H), 7.87(d, J= 6.4 Hz, 1H), 8.48 (d, *J* = 6.0 Hz, 1H), 8.67 (s, 1H), 13.01 (s, 1H). |
| 74 | 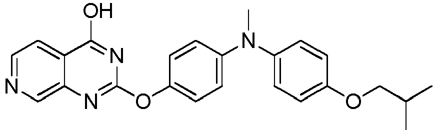 Prepared by the procedure of Example 5 | 417 | ¹H NMR (400 MHz, DMSO-*d*₆): δ 0.96 (d, *J*=9.2 Hz, 6H), 1.94-2.01 (m, 1H), 3.20 (s, 3H), 3.71 (d, *J*=8.4 Hz, 2H), 6.75 (d, *J*=12.4 Hz, 2H), 6.94 (d, *J*=12.0 Hz, 2H),7.06-7.10 (m, 4H), 7.81 (d, *J*=6.4 Hz, 1H), 8.43 (d, *J*=6.4 Hz, 1H), 8.64 (s, 1H). |
| 75 | 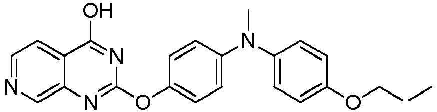 Prepared by the procedure of Example 5 | 431 | ¹H NMR (400 MHz, DMSO-*d*₆): δ 0.96 (s, 9H), 3.21 (s, 3H), 3.60 (s, 2H), 6.74 (d, *J =* 12.4 Hz, 2H), 6.94 (d, *J=* 12.0 Hz, 2H), 7.06-7.10 (m, 4H), 7.84 (d, *J=* 6.8 Hz, 1H ), 8.43 (d, *J* = 6.4 Hz, 1H), 8.66 (s, 1H). |
| 76 | 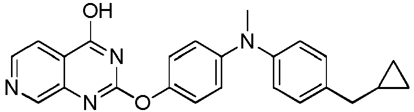 Prepared by the procedure of Example 26 | 399 | ¹H NMR (400 MHz, DMSO-*d*₆): δ 0.17-0.20 (m, 2H), 0.43-0.48 (m, 2H), 0.92-0.96 (m, 1H), 2.44 (s, 3H), 3.27 (d, *J*=9.2 Hz, 2H), 6.94 (d, *J*=12.0 Hz, 2H),7.03 (d, *J*=12.0 Hz, 2H), 7.15-7.23 (m, 4H), 7.86 (d, *J*=6.8 Hz, 1H), 8.49 (d, *J*=7.2 Hz, 1H), 8.68 (s, 1H), 13.02 (s, 1H). |
| 77 | 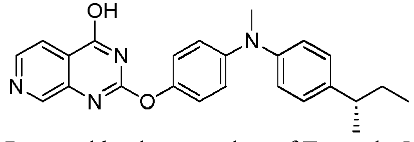 Prepared by the procedure of Example 5 | 401 | ¹H NMR (300 MHz, DMSO-*d*₆): δ 0.77 (t, *J =* 7.2 Hz, 3H), 1.17 (d, *J*=7.2 Hz, 3H), 1.50-1.55 (m, 2H), 2.48-2.55 (m, 1H), 3.27 (d, *J*=13.5 Hz, 3H), 6.92 (d, *J =* 9.3 Hz, 2H), 7.03 (d, *J=* 9.0 Hz, 2H), 7.15-7.29 (m, 4H), 7.86 (d, *J*=5.1 Hz, 1H), 8.49 (d, *J*=5.4 Hz, 1H), 8.68 (s, 1H), 13.02 (s, 1H). |
| 78 | 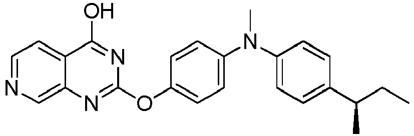 Prepared by the procedure of Example 5 | 401 | ¹H NMR (300 MHz, DMSO-*d*₆): δ 0.77 (t, *J =* 7.2 Hz, 3H), 1.17 (d, *J* = 7.2 Hz, 3H), 1.50-1.55 (m, 2H), 2.48-2.55 (m, 1H), 3.27 (d, *J=* 13.5 Hz, 3H), 6.92 (d, *J =* 9.3 Hz, 2H), 7.03 (d, *J=* 9.0 Hz, 2H), 7.15-7.29 (m, 4H), 7.86 (d, *J=* 5.1 Hz, 1H), 8.49 (d, *J=* 5.4 Hz, 1H), 8.68 (s, 1H), 13.02 (s, 1H). |
| 79 | 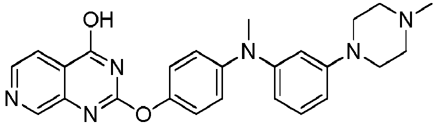 Prepared by the procedure of Example 22 | 442 | ¹H NMR (300 MHz, DMSO-*d*₆): δ 2.22 (s, 3H), 3.08-3.11 (m, 4H), 3.25 (s, 3H), 3.32-2.39 (m, 4H), 6.48 (d, J=8.4Hz, 1H), 6.93-6.96 (m, 2H),6.94 (d, *J*=9.3 Hz, 2H), 7.13-7.17 (m, 3H), 7.83 (d, *J*=5.1 Hz, 1H), 8.46 (d, *J*=5.1 Hz, 1H), 8.66(s, 1H). |
| 80 | 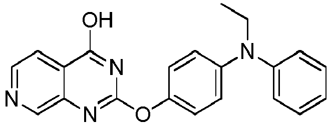 Prepared by the procedure of Example 5 | 358 | ¹H NMR (300 MHz, DMSO-*d*₆): δ 1.17 (t, *J =* 2.1 Hz, 3H), 3.74-3.81 (m, 2H), 6.95-7.03 (m, 5H), 7.20-7.23 (m, 2H), 7.31 (t, *J =* 7.2 Hz, 2H),7.87 (d, *J* = 5.1 Hz, 1H),8.51 (d, *J* = 5.1 Hz, 1H), 8.71 (s, 1H). |
| 81 | 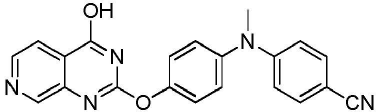 Prepared by the procedure of Example 1 | 369 | ¹H NMR (300 MHz, DMSO-*d*₆): δ 3.39 (s, 3H), 6.81 (d, *J =* 2.4 Hz, 2H), 7.33-7.43 (m, 4H), 7.54-7.59 (m, 2H), 7.87 (d, *J=* 4.8 Hz, 1H), 8.51 (brs, 1H), 8.71 (brs, 1H), 13.13 (brs, 1H). |
| 82 | 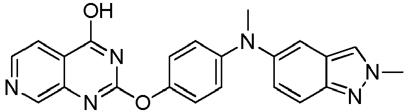 Prepared by the procedure of Example 5 | 398 | ¹H NMR (300 MHz, DMSO-*d*₆): δ 3.29 (s, 3H), 4.13 (s, 3H), 6.82 (d, *J =* 9.3 Hz, 2H), 7.03 (d, *J =* 9.3 Hz, 1H), 7.13 (d, *J*=9.0 Hz, 2H), 7.42 (s, 1H), 7.57 (d, *J*=9.0 Hz, 1H), 7.85 (d, *J= 5.1* Hz, 1H), 8.23 (s, 1H), 8.49 (d, *J =* 4.5 Hz, 1H), 8.68(s, 1H). |
| 83 | 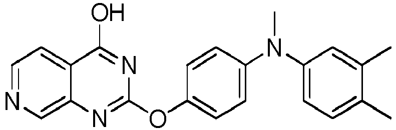 Prepared by the procedure of Example 18 | 372 | H NMR (300 MHz, DMSO-*d*₆): δ 2.17 (d, *J =* 2.4 Hz, 6H), 3.22 (s, 3H), 6.83-6.87 (m, 4H), 7.08-7.14 (m, 3H), 7.83 (d, *J =* 4.8 Hz, 1H), *8.47 (d, J* = 5.1 Hz, 1H), 8.66 (s, 1H). |
| 84 | 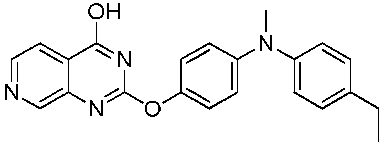 Prepared by the procedure of Example 18 | 372 | ¹H NMR (300 MHz, DMSO-*d*₆): δ 1.16 *(t, J=* 7.2 Hz, 3H), 2.55-2.60 (m, 2H), 3.23 (s, 3H), 6.90 (d, *J*=8.4 Hz, 2H), 7.03 (d, *J* = 8.4 Hz, 2H), 7.14-7.18 (m, 4H),7.85 (d, *J*=5.7 Hz, 1H), 8.48 (d, *J =* 5.7 Hz, 1H), 8.67 (s, 1H). |
| 85 | 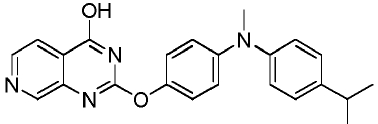 Prepared by the procedure of Example 18 | 386 | ¹H NMR (300 MHz, DMSO-*d*₆): δ 1.18 *(d, J=* 7.5 Hz, 6H), 2.80-2.89 (m, 1H), 3.24 (s, 3H), 6.92 (d, *J*=9.3 Hz, 2H), 7.02 (d, *J*=9.3 Hz, 2H), 7.14-7.21 (m, 4H),7.84 (d, *J*=5.1 Hz, 1H), 8.47 (d, *J* = 5.1 Hz, 1H), 8.67 (s, 1H). |
| 86 | 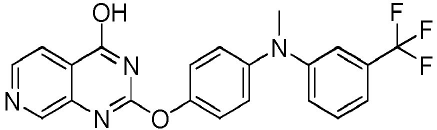 Prepared by the procedure of Example 5 | 412 | ¹H NMR (300 MHz, DMSO-*d*₆): δ 3.31 (s, 3H), 7.10-7.15 (m, 3H), 7.23 (d, *J=* 9.0 Hz, 2H), 7.35 (d, *J*=9.0 Hz, 2H), 7.44 (t, *J =* 7.8 Hz, 1H), 7.87 (d, *J =* 5.1 Hz, 1H), 8.50 (d, *J=* 4.5 Hz, 1H), 8.69 (s, 1H). |
| 87 | 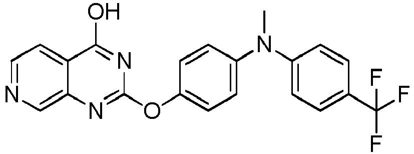 Prepared by the procedure of Example 5 | 412 | ¹H NMR (300 MHz, DMSO-*d*₆): δ 3.32 (s, 3H), 6.94 (d, *J*=6.6 Hz, 2H), 7.34 (d, *J =* 6.6Hz, 2H), 7.40 (d, *J*=6.3 Hz, 2H), 7.53(d, *J*=6.6 Hz, 2H), 7.90 (d, *J*=3.6 Hz, 1H), 8.53 (d, *J*=3.9 Hz, 1H), 8.73 (s, 1H). |
| 88 | 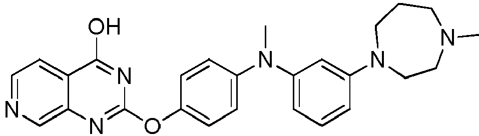 Prepared by the procedure of Example 22 | 456 | ¹H NMR (300 MHz, DMSO-*d*₆): δ 1.86-1.89 (m, 2H), 2.32 (s, 3H), 2.48-2.53 (m, 2H), 2.67 (m, 2H), 3.24 (s, 3H), 3.31-3.38 (m, 2H), 3.47-3.48 (m, 2H), 6.30-6.39 (m, 3H), 6.93 (d, *J=* 8.7 Hz, 2H), 7.08-7.13 (m, 3H), 7.80 (d, *J* = 4.5 Hz, 1H), 8.42 (d, *J* = 5.1 Hz, 1H), 8.64 (s, 1H). |
| 89 | 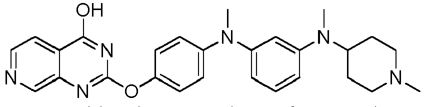 Prepared by the procedure of Example 22 | 470 | ¹H NMR (300 MHz, DMSO-*d*₆): δ 1.56-1.59 (m, 2H), 1.71-1.76 (m, 2H), 2.14-2.21 (m, 2H),2.25 (s, 3H), 2.66 (s, 3H), 2.92(d, *J =* 11.7 Hz, 2H), 3.23 (s, 3H), 3.36-3.38 (m, 1H), 6.34-6.37 (m, 1H), 6.43-6.49 (m, 2H), 6.91-6.94 (m, 2H), 7.07-7.13 (m, 3H), 7.81(d, *J*=5.1 Hz, 1H), 8.42 (d, *J*=5.1 Hz, 1H), 8.65 (s, 1H). |
| 90 | 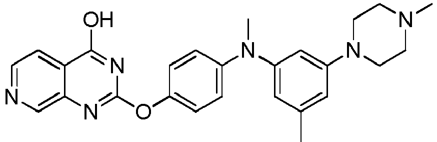 Prepared by the procedure of Example 22 | 456 | ¹H NMR (300 MHz, DMSO-*d*₆): δ 2.21 (s, 3H), 2.24 (s, 3H), 2.49-2.50 (m, 4H), 3.09-3.10 (m, 4H), 3.24 (s, 3H), 6.35 (s, 1H), 6.45 (d, *J*=4.8 Hz, 2H), 6.93 (d, *J =* 8.7 Hz, 2H),7.17 (d, *J=* 9.0 Hz, 2H), 7.86(d, *J*=4.8 Hz, 1H), 8.49 (d, *J=* 5.1 Hz, 1H), 8.68 (s, 1H). |
| 91 | 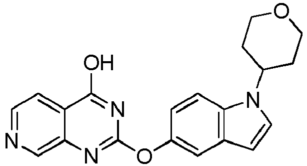 Prepared by the procedure of Example 31 | 363 | ¹H NMR (300 MHz, DMSO-*d*₆): δ 1.92-2.08 (m, 4H), 3.58 (t, *J*=8.7 Hz, 2H), 4.00-4.03 (m, 2H), 4.64-4.71 (m, 1H), 9.49 (s, 1H), 7.05 (d, *J*=1.5 Hz, 1H), 7.42 (s, 1H), 7.61-7.63 (m, 2H), 7.82(d, *J*=3.9 Hz, 1H), 8.43 (d, *J*=3.9 Hz, 1H), 8.60 (s, 1H). |
| 92 | 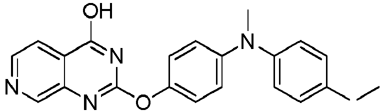 Prepared by the procedure of Example 18 | 400 | ¹H NMR (400 MHz, DMSO-*d*₆): δ 1.28 (s, 9H), 3.27 (s, 3H), 6.95 (d, *J*=8.0 Hz, 2H), 7.05 (d, *J*=8.0 Hz, 2H), 7.18 (d, *J*=8.0 Hz, 2H),7.36 (d, *J*=8.0 Hz, 2H), 7.87 (s, 1H), 8.47 (m, 1H), 8.72 (m, 1H), 13.05 (s, 1H). |
| 93 | 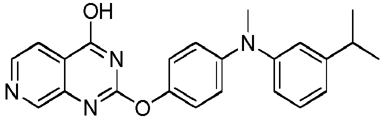 Prepared by the procedure of Example 18 | 386 | ¹H NMR (300 MHz, DMSO-*d*₆): δ 1.20 (d, *J=* 6.8 Hz, 6H), 2.85 (m, 1H), 3.29 (s, 3H), 6.89-7.01 (m, 5H), 7.20-7.25 (m, 3H), 7.88 (s, 1H), 8.52-8.73 (m, 2H), 13.07 (s, 1H). |
| 94 | 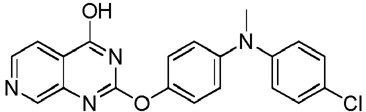 Prepared by the procedure of Example 18 | 379 | ¹H NMR (300 MHz, DMSO-*d*₆): δ 3.28 (s, 3H), 6.99 (d, *J*=9.0 Hz, 2H), 7.13 (d, *J*=9.0 Hz, 2H), 7.26 (d, *J* =9.0 Hz, 2H), 7.30 (d, *J*=9.0 Hz, 2H), 7.86 (d, J=5.1 Hz, 1H), 8.49 (d, J=5.1 Hz, 1H), 8.70 (s, 1H). |
| 95 | 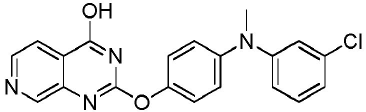 Prepared by the procedure of Example 18 | 379 | ¹H NMR (300 MHz, DMSO-*d*₆): δ 3.32 (s, 3H), 6.85-6.92 (m, 3H), 7.21-7.26 (m, 3H), 7.34 (d, *J*=5.1 Hz, 2H), 7.89 (d, *J =* 3.9 Hz, 1H), 8.52 (d, *J*=3.9 Hz, 1H), 8.72 (s, 1H), 13.11 (s, 1H). |
| 96 | 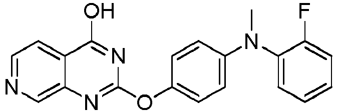 Prepared by the procedure of Example 18 | 363 | ¹H NMR (300 MHz, DMSO-*d*₆): δ 3.36 (s, 3H), 6.70 (d, *J*=8.1 Hz, 2H), 7.13 (d, *J*=8.1 Hz, 2H), 7.29-7.40 (m, 4H), 7.85 (s, 1H), 8.47-8.49 (br, 1H), 8.65-8.70 (br 1H). |
| 97 | 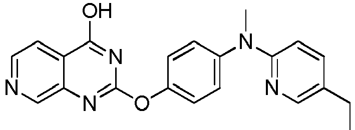 Prepared by the procedure of Example 34 | 374 | ¹H NMR (300 MHz, DMSO-*d*₆): δ 1.14 *(t, J=* 7.5 Hz, 3H), 2.45-2.47 (m, 2H), 3.36 (s, 3H), 8.64 (d, *J =* 8.7 Hz, 1H), 7.36-7.40 (m, 5H), 7.89 (d, *J =* 5.1 Hz, 1H), 8.03 (s, 1H), 8.53 (d, *J =* 4.5 Hz, 1H), 874 (s, 1H), 13.16 (s, 1H). |
| 98 | 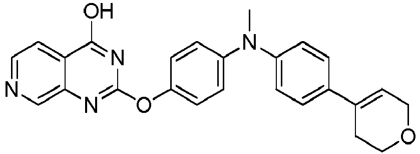 Prepared by the procedure of Example 34 | 427 | ¹H NMR (300 MHz, DMSO-*d*₆): δ 2.43-2.50 (m, 2H), 3.23 (s, 3H), 3.80-3.84 (m, 2H), 4.21-4.22 (m, 2H), 6.16 (s, 1H), 7.01-7.10 (m, 4H), 7.24-7.41 (m, 4H), 7.88 (d, *J*=4.8 Hz, 1H), 8.52 (d, *J*=5.1 Hz, 1H), 8.72 (s, 1H), 13.02 (s, 1H). |
| 99 | 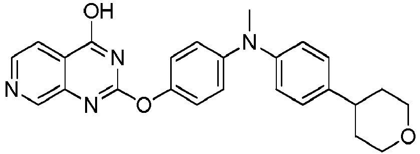 Prepared by the procedure of Example 34 | 429 | H NMR (300 MHz, DMSO-*d*₆): δ 1.65-1.68 (m, 4H), 2.49-2.51 (m, 1H), 3.26 (s, 3H), 3.40-3.41 (m, 2H), 3.91-3.94 (m, 2H), 6.93-7.05 (m, 4H), 7.16-7.22 (m, 4H), 7.85 (d, *J*=5.1 Hz, 1H), 8.49 (d, *J*=5.1 Hz, 1H), 8.68 (s, 1H), 13.07 (s, 1H). |
| 100 | 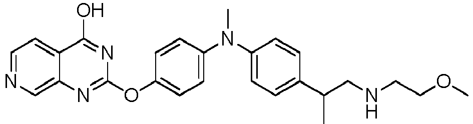 Prepared by the procedure of Example 37 | 460 | ¹H NMR (300 MHz, CD3OD-*d*₄): δ 1.19-1.26 (m, 3H), 2.64-2.70 (m, 1H), 3.07-3.66 (m, 12H), 7.07-7.20 (m, 8H), 8.00-8.05 (m, 1H), 8.47-8.50 (m, 1H), 8.75-8.77 (m, 1H). |
| 101 | 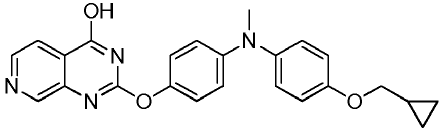 Prepared by the procedure of Example 5 | 415 | ¹H NMR (300 MHz, DMSO-*d*₆): δ 0.29-0.31 (m, 2H), 0.54-0.57 (m, 2H), 1.20-1.21 (m, 1H), 3.21 (s, 3H), 3.79 (d, *J=* 7.2 Hz, 2H), 6.74 (d, *J =* 9.0 Hz, 2H), 6.94 (d, *J =* 8.7 Hz, 2H), 7.09-7.12 (m, 4H), 7.85 (d, *J* = 5.1 Hz, 1H), 8.48 (d, *J =* 5.1 Hz, 1H), 8.67 (s, 1H), 13.00 (s, 1H). |
| 102 | 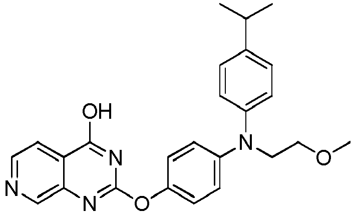 Prepared by the procedure of Example 5 | 413 | ¹H NMR (300 MHz, CD3OD-*d*₄): δ 1.25 (d, *J=* 6.9 Hz, 6H), 2.86-2.88 (m, 1H), 3.34 (s, 3H), 3.60 (t, *J=* 6.0 Hz, 2H), 3.90 (t, *J =* 5.7 Hz, 2H), 6.95 (d, *J=* 9.0 Hz, 2H), 7.06-7.13 (m, 4H), 7.20 (d, *J=* 8.7 Hz, 2H), 7.98 (d, *J* = 5.1 Hz, 1H), 8.47 (d, *J* = 5.1 Hz, 1H), 8.75 (s, 1H). |
| 103 | 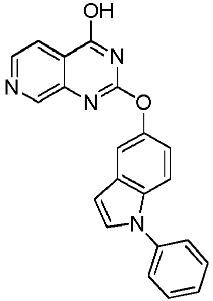 Prepared by the procedure of Example 32 | 355 | 1H NMR (400 MHz, DMSO-d6): δ 6.75 (d, *J =* 2.4 Hz, 1H), 7.16 (d, *J=* 8.4 Hz, 1H), 7.44 (t, *J=* 6.4 Hz, 1H), 7.61-7.65 (m, 6H), 7.76 (d, *J=* 3.2 Hz, 1H), 7.89 (s, 1H), 8.50-8.67 (m, 2H), 13.10 (s, 1H). |
| 104 | 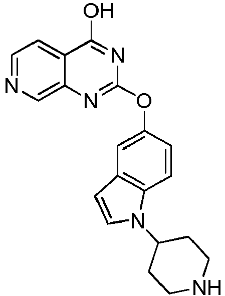 Prepared by the procedure of Example 31 | 362 | 1H NMR (400 MHz, DMSO-d6): δ 1.63-1.88 (m, 4H), 2.58-2.67 (m, 2H), 3.01-3.10 (m, 2H), 4.27-4.30 (m, 1H), 6.46 (d, *J=* 2.0 Hz, 1H), 7.01 (d, *J =* 8.8 Hz, 1H), 7.29 (s, 1H), 7.37 (d, *J* = 2.5 Hz, 1H), 7.62 (d, *J =* 8.8 Hz, 1H), 7.71 (d, *J =* 4.8 Hz, 1H), 8.25 (d, *J* = 4.8 Hz, 1H), 8.64 (s, 1H). |
| 105 | 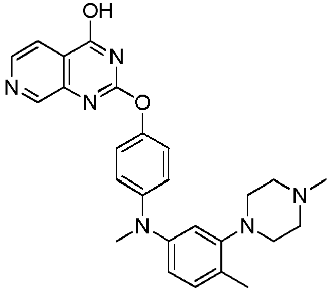 Prepared by the procedure of Example 23 | 457 | 1H NMR (300 MHz, DMSO-d6): δ 2.18 (s, 3H), 2.25 (s, 3H), 2.48-2.49 (m, 4H), 2.80-2.85 (m, 4H), 3.24 (s, 3H), 6.69-6.73 (m, 2H), 6.90 (d, *J =* 5.7 Hz, 2H), 7.09-7.14 (m, 3H), 7.83 (d, *J* = 5.1 Hz), 8.46 (d, *J* = 5.1 Hz), 8.66 (s, 1H). |
| 106 | 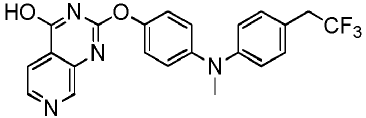 Prepared by the procedure of Example 39 | 426 | ¹H NMR (400 MHz, DMSO-*d*₆): δ ppm 3.28 - 3.31 (m, 3 H) 3.49 - 3.63 (m, 2 H) 6.98 - 7.06 (m, 2 H) 7.08 - 7.15 (m, 2 H) 7.26 (br. s., 4 H) 7.81 - 7.94 (m, 1 H) 8.44 - 8.58 (m, 1 H) 8.65 - 8.79 (m, 1 H) 12.78 - 13.25 (s, 1 H) |
| 107 | 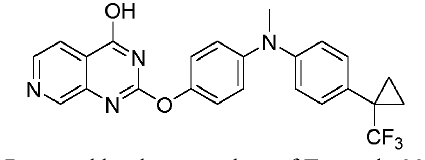 Prepared by the procedure of Example 39 | 452 | ¹H NMR (400 MHz, DMSO-*d*₆): δ ppm 1.02 - 1.13 (m, 2 H) 1.22 - 1.38 (m, 2 H) 2.48 - 2.50 (m, 3 H) 6.95 - 7.03 (m, 2 H) 7.13 - 7.19 (m, 2 H) 7.27 - 7.39 (m, 4 H) 7.85 - 7.93 (m, 1 H) 8.47 - 8.57 (m, 1 H) 8.70 - 8.75 (m, 1 H) 13.02 - 13.18 (s, 1 H) |
| 108 | 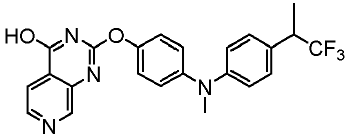 Prepared by the procedure of Example 5 | 440 | ¹H NMR (400 MHz, DMSO-*d*₆): δ ppm 1.38 - 1.47 (m, 3 H) 3.29 - 3.31 (s, 3 H) 3.62 - 3.79 (m, 1 H) 6.98 - 7.07 (m, 2 H) 7.09 - 7.17 (m, 2 H) 7.23 - 7.36 (m, 4 H) 7.84 - 7.93 (s, 1 H) 8.45 - 8.59 (s, 1 H) 8.65 - 8.79 (s, 1 H) 12.98 - 13.20 (s, 1 H) |
| 109 | 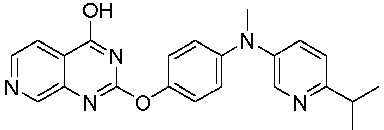 Prepared by the procedure of Example 5 | 387 | ¹H NMR (400 MHz, DMSO-*d*₆): δ ppm 1.19 - 1.27 (m, 6 H) 2.47 - 2.49 (m, 3 H) 2.93 - 3.09 (m, 1 H) 7.02 - 7.09 (m, 2 H) 7.21 - 7.30 (m, 3 H) 7.40 - 7.49 (m, 1 H) 7.85 - 7.93 (m, 1 H) 8.23 - 8.32 (m, 1 H) 8.48 - 8.58 (m, 1 H) 8.66 - 8.77 (m, 1 H) 13.01 - 13.16 (s, 1 H) |
| 110 | 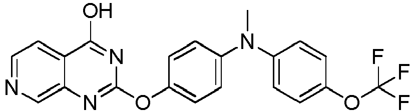 Prepared by the procedure of Example 26 | 429 | H NMR (300 MHz, DMSO-*d*₆): δ ppm 3.29 (s, 3 H) 7.02 - 7.05 (m, 2 H) 7.13 - 7.16 (m, 2 H) 7.23 -7.30 (m, 4 H) 7.86 - 7.88 (m, 1 H) 8.50 - 8.52 (m, 1 H) 8.70 (s, 1 H) 13.10 (s, 1 H) |
| 111 | 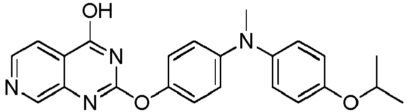 Prepared by the procedure of Example 5 | 403 | H NMR (300 MHz, DMSO-*d*₆): δ ppm 1.25 (d, *J*=8.0Hz, 6H), 3.21 (s, 3 H), 4.51-4.59 (m, 1H), 6.75 (d, *J*=12.0 Hz, 2H), 6.92 (d, *J*=12.0 Hz, 2H), 7.08 - 7.13 (m, 4 H), 7.85 (d, *J*=6.8 Hz,1 H), 8.48 (d, *J*=6.8 Hz,1 H) 8.67(s, 1 H) 12.98 (s, 1 H) |
| 112 | 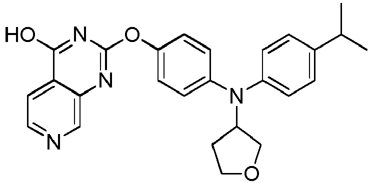 Prepared by the procedure of Example 47 | 422 | ¹H NMR (400 MHz, DMSO-*d*₆): δ 1.20 (d, *J=* 8.0 Hz, 6H), 1.81-1.83 (m, 1H), 2.19-2.21 (m, 1H), 2.87-2.92 (m, 1H), 3.58-3.65 (m, 3H), 3.90-3.95 (m, 1H), 4.58-4.62 (m, 1H), 6.76 (d, *J=* 12.0 Hz , 2H), 6.97 (d, *J =* 12.0 Hz, 2H), 7.19 (d, *J* = 12.0 Hz, 2H), 7.25 (d, *J=* 12.0 Hz , 2H), 7.87 (d, *J* = 6.4 Hz , 1H), 8.52 (dd, *J=* 2.0 Hz , 3.2 Hz, 1H), 8.69 (s, 1H), 13.02 (s, 1H). |
| 113 | 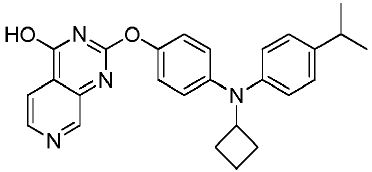 Prepared by the procedure of Example 47 | 426 | 1H NMR (300 MHz, DMSO-*d*₆): δ 1.22 (d, *J*=7.2 Hz, 6H), 1.58-1.77 (m, 4H), 2.20-2.24 (m, 2H), 2.87-2.91 (m, 1H), 4.29 (t, *J*=7.8 Hz, 1H),6.71 (d, *J*=8.7 Hz, 2H), 6.92 (d, *J*=8.1 Hz, 2H), 7.14 (d, *J*=8.7 Hz, 2H),7.26 (d, *J*=8.1 Hz, 2H), 7.86 (d, *J*=4.8 Hz, 1H), 8.48-8.50 (m, 1H), 8.69 (s, 1H), 13.01 (brs, 1H). |

### Preparation 114A: 2,2,2-trifluoro-N-(4-isopropyl-phenyl)-acetamide

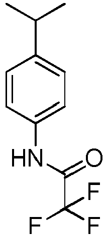

To a solution of 4-isopropyl-phenylamine (1.0 g, 7.4 mmol) in DCM (60ml) was added pyridine (1.8 g, 22.2 mmol) and TFAA (1.9 g, 8.9 mmol) at 0 °C, and the mixture was stirred for 2h at RT. The reaction was quenched with aqueous NaHCO₃ and the mixture was extracted with EA (80 mL × 3). The combined organics were dried over MgSO₄ and concentrated *in vacuo.* The resulting residue was purified by silica gel column chromatography (PE:EtOAc, 30:1) to give the title compound (1.6 g, 94%) as a yellow oil. [M+H] Calc'd for C₁₁H₁₂F₃NO, 232; Found, 232.

### Preparation 114B: (4-isopropyl-phenyl)-(2,2,2-trifluoro-ethyl)-amine

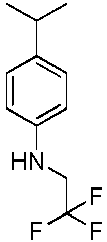

To a solution of 2,2,2-trifluoro-N-(4-isopropyl-phenyl)-acetamide (1.6 g, 7.0 mmol) in THF (70 mL) was added DMSB (7 mL, 14.0 mmol) and the mixture was refluxed for 2 h. The reaction was quenched with H₂O (70 mL), extracted with ether (80 mL × 3). The combined organics were dried over MgSO₄ and concentrated *in vacuo.* The resulting residue was purified by silica gel column chromatography (PE:EtOAc, 50:1) to give the title compound (800 mg, 53%) as a yellow oil. [M+H] Calc'd for C₁₁H₁₄F₃N, 218; Found, 218.

### Preparation 114C: 4-[(4-isopropyl-phenyl)-(2,2,2-trifluoro-ethyl)-amino]-phenol

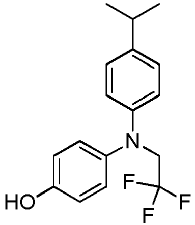

The title compound was prepared in 73% yield from 2-chloro-pyrido[3,4-d]pyrimidin-4-ol and (4-isopropyl-phenyl)-(2,2,2-trifluoro-ethyl)-amine according to the procedure of Preparation 33C. [M+H] Calc'd for C₁₇H₁₈F₃NO, 310; Found, 310.

### Example 114: 2-{4-[(4-isopropyl-phenyl)-(2,2,2-trifluoro-ethyl)-amino]-phenoxy}-pyrido[3,4-d]pyrimidin-4-ol

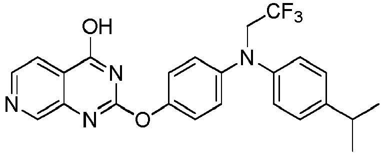

The title compound was prepared in 11% yield from 2-chloro-pyrido [3,4 d]pyrimidin-4-ol and 4-[(4-isopropyl-phenyl)-(2,2,2-trifluoro-ethyl)-amino]-phenol according to the procedure for the preparation of Example 1; ¹H NMR (300 MHz, DMSO-*d*₆): δ 1.20-1.22 (m, 6H), 2.88-2.89 (m, 1H), 4.59-4.62 (m, 2H), 6.94 (d, *J=* 5.4 Hz, 2H), 7.07-7.10 (d, *J=* 8.4 Hz, 2H), 7.25 (m, 4H), 7.86-7.88 (d, *J=* 4.5 Hz, 1H), 8.51 (d, *J=* 5.1 Hz, 1H) , 8.70 (s, 1 H). [M+H] Calc'd for C₂₄H₂₁F₃N₄O₂, 455; Found, 455.

### Preparation 115A: [4-(3-amino-1-methylpropyl)phenyl]methyl[4-(phenylmethoxy)phenyl]amine

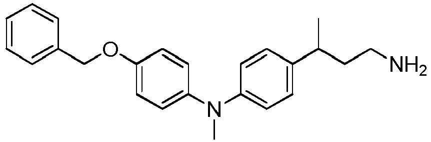

To a solution of 3-{4-[(4-benzyloxy-phenyl)-methyl-amino]-phenyl}-butyronitrile (1.0 g, 2.8 mmol) in THF (10 mL) was added LAH (2.3 mL, 2.4M) at 0 °C, and the mixture was stirred at RT for 2h. The reaction was quenched with water and extracted with EA (3x). The combined organics were dried over MgSO₄ and concentrated to give 0.68 g (68%) of the title compound. [M+H] Calc'd for C₂₄H₂₈N₂O, 361; Found, 361.

### Preparation 115B: (3-{4-[(4-benzyloxy-phenyl)-methyl-amino]-phenyl}-butyl)-carbamic acid tert-butyl ester

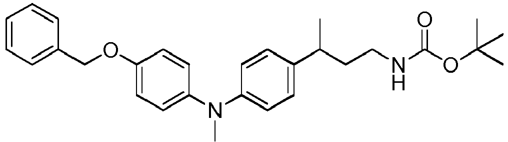

To a solution of [4-(3-amino-1-methylpropyl)phenyl]methyl[4-(phenylmethoxy) phenyl]amine (0.68g, 1.9mmol) in DCM (5mL) was added (Boc)₂O (0.5 g, 2.3 mmol) and TEA (0.38 g, 3.8 mmol), and the mixture was stirred at RT for 2h. The reaction was quenched with aqueous NH₄Cl and extracted with DCM (3x). The combined organics were dried over MgSO₄ and concentrated to give 0.83 g (95%) of the title compound. [M+H] Calc'd for C₂₉H₃₆N₂O₃, 461; Found, 461.

### Preparation 115C: (3-{4-[(4-benzyloxy-phenyl)-methyl-amino]-phenyl}-butyl)-methyl-carbamic acid tert-butyl ester

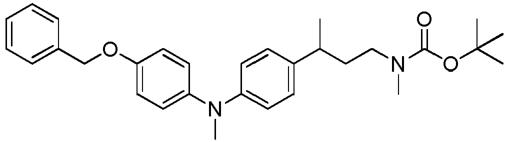

To a solution of (3-{4-[(4-benzyloxy-phenyl)-methyl-amino]-phenyl}-butyl)-carbamic acid tert-butyl ester (1.9 g, 4.1 mmol) in THF (20 mL) was added NaH (0.25 g, 6.15 mmol) at 0 °C, and the mixture was stirred at RT for 30 min at 0 °C. CH₃I (0.7 g, 4.92 mmol) was added and the mixture was stirred at 45°C for 2h. The reaction was quenched with aqueous NH₄Cl and extracted with EA (3x). The combined organics were dried over MgSO₄ and concentrated. The residue was purified by silica gel chromatography (EA:PE, 1:10) give 1.2 g (61%) of the title compound. [M+H] Calc'd for C₃₀H₃₈N₂O₃, 475; Found, 475.

### Preparation 115D: 4-{[4-(3-dimethylamino-1-methyl-propyl)-phenyl]-methyl-amino}-phenol

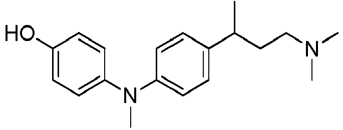

To a solution of (3-{4-[(4-benzyloxy-phenyl)-methyl-amino]-phenyl}-butyl)-methyl-carbamic acid tert-butyl ester (0.89 g, 1.88 mmol) in THF (10 mL) was added LAH (1.2 mL, 2.4M) at 0 °C, and the mixture was refluxed overnight. The reaction was quenched with water and extracted with EA (3x). The combined organics were dried over MgSO₄ and concentrated. The residue was dissolved in MeOH and Pd/C (30 mg) was added. The mixture was stirred overnight at RT under H₂ atmosphere and filtered. The filtrate was concentrated and the residue was purified by silica gel chromatography (MeOH:DCM, 1:10) to give 0.4 g (72%) of the title compound. [M+H] Calc'd for C₁₉H₂₆N₂O, 299; Found, 299.

### Example 115: 2-(4-{[4-(3-dimethylamino-l-methyl-propyl)-phenyl]-methyl-amino}-phenoxy)-pyrido[3,4-d]pyrimidin-4-ol

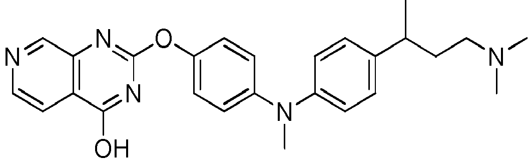

The title compound was prepared in 5% yield from 2-chloro-pyrido[3,4-d] pyrimidin-4-ol and N-(4-hydroxy-phenyl)-N-methyl-2-phenyl-acetamide according to the procedure for the preparation of Example 1. ¹H NMR (400 MHz, DMSO-*d*₆): δ1.02 (d, *J* = 8.4 Hz, 3H), 2.25-2.27 (m, 1H), 2.44-2.52 (m, 1H), 2.67-2.73 ( m, 1H), 2.88 (d, *J=* 26.0 Hz, 6H), 3.04-3.08 (m, 2H), 3.30 ( s, 3H), 7.00-7.20 (m, 8H), 7.87 (d, *J=* 6.8 Hz, 1H), 8.50 (d, *J=* 6.8 Hz, 1H), 8.67 (s, 1H). [M+H] Calc'd for C₂₆H₂₉N₅O₂, 444; Found, 444.

### Preparation 116A: 1-bromo-2-(2-methyl-allyloxy)-4-nitro-benzene

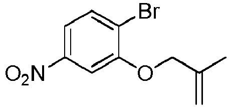

To a solution of 2-bromo-5-nitro-phenol (0.5 g, 2.3 mmol) in acetone (20 mL) was added 3-bromo-2-methyl-propene (465 mg, 3.4 mmol) and K₂CO₃ (633 mg, 4.6 mmol), and the mixture was refluxed overnight. The reaction mixture was cooled to RT and the solvent was concentrated. The residue was dissolved in EA, washed with water, washed with brine. The organics were concentrated and purified by silica gel chromatography (EA: PE, 1:20) to give 0.5 g (80%) of the title compound. [M+H] Calc'd for C₁₀H₁₀BrNO₃, 271; Found, 271.

### Preparation 116B: [4-bromo-3-(2-methyl-allyloxy)-phenyl]-carbamic acid tert-butyl ester

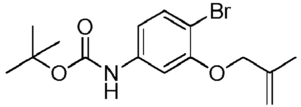

To a solution of 1-bromo-2-(2-methyl-allyloxy)-4-nitro-benzene (3.1 g, 114 mmol ) in methanol (100 mL) was added iron powder (1.95 g, 343 mmol ) and NH₄Cl (2.0 g, 37mmol ), and the mixture was refluxed overnight, the solvent was concentrated. The residue was adjusted with aqueous NaHCO₃ to PH~8, extracted with EA (3x). The combined organics were dried and concentrated. The residue was dissolved in THF (50 mL), (Boc)₂O ( 3.0 g, 137 mmol ) and TEA (2.5 g, 228 mmol ) were added, and the mixture was refluxed overnight. The reaction mixture was cooled to RT and the solvent was concentrated. The residue was purified by silica gel chromatography (EA:PE, 1:20) to give 3.0 g (77%) of the title compound. [M+H] Calc'd for C₁₅H₂₀BrNO₃, 342; Found, 342.

### Preparation 116C: (3,3-dimethyl-2,3-dihydro-benzofuran-6-yl)-carbamic acid tert-butyl ester

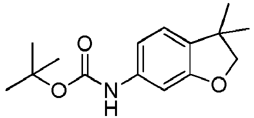

To a solution of [4-bromo-3-(2-methyl-allyloxy)-phenyl]-carbamic acid tert-butyl ester (340 mg, 1 mmol) in toluene (10 mL) was added was AIBI (16.5 mg, 0.1 mmol) and tributytin hydride (360 mg, 1.2 mmol), and the mixture was stirred overnight at 110°C. The reaction mixture was cooled to RT, EA and 10% KF solution were added and the mixture was stirred for 2h. The organic layer was washed with water, washed with brine and concentrated. The residue was purified by silica gel chromatography (PE:EA, 20:1) to give 150 mg (57%) of the title compound. [M+H] Calc'd for C₁₅H₂₁NO₃, 264; Found, 264.

### Preparation 116D: (3,3-dimethyl-2,3-dihydro-benzofuran-6-yl)-methyl-amine

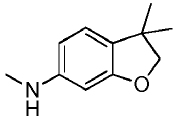

To a solution of (3,3-dimethyl-2,3-dihydro-benzofuran-6-yl)-carbamic acid *tert-*butyl ester (1.0 g, 3.8 mmol) in DMF (10 mL) was added NaH (0.23 g, 5.7 mmol) at 0 °C, an dthe mixture was stirred for 30 min at 0 °C. CH₃I (0.65 g, 4.5 mmol) was added and the mixture was stirred at RT for 2h, quenched with aqueous NH₄Cl, and extracted with EA (3x). The combined organics were washed with water, washed with brine, dried and concentrated. The residue was dissolved in DCM (10 mL), TFA (2 mL) was added and the mixture was stirred at RT for 1h. The organics were concentrated and the residue was purified by silica gel chromatography (EA:PE, 1:20) to give 600 mg (94%) of the title compound. [M+H] Calc'd for C₁₁H₁₅NO, 178; Found, 178.

### Preparation 116E: 4-[(3,3-dimethyl-2,3-dihydro-benzofuran-6-yl)-methyl-amino]-phenol

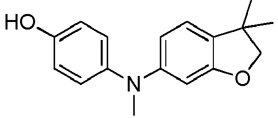

A solution of (3,3-dimethyl-2,3-dihydro-benzofuran-6-yl)-methyl-amine (300 mg, 1.7 mmol), 1-benzyloxy-4-bromo-benzene (535 mg, 2.0 mmol), S-Phos (35 mg, 0.085 mmol), Pd₂(dba)₃ (80 mg, 0.085 mmol), *t*-BuOK (475 mg, 4.25 mmol) in toluene (10 mL) was refluxed overnight under nitrogen atmosphere. The solvent was concentrated and the residue was purified by silica gel chromatography (EA:PE, 1:20) to give (4-benzyloxy-phenyl)-(3,3-dimethyl-2,3-dihydro-benzofuran-6-yl)-methyl-amine (600 mg, 99%). This benzyl protected product was in turn dissolved in THF/MeOH and Pd/C (50 mg) was added. The mixture was stirred overnight at RT under H₂ atmosphere. The mixture was filtered on celite and the filtrate was concentrated. The residue was purified by silica gel chromatography (EA:PE, 1:10) to give 430 mg (94%) of the title compound. [M+H] Calc'd for C₁₇H₁₉NO₂, 270; Found, 270.

### Example 116: (3,3-dimethyl-2,3-dihydro-benzofuran-6-yl)-methyl-[4-(4-methyl-pyrido [3,4-d]pyrimidin-2-yloxy)-phenyl]-amine

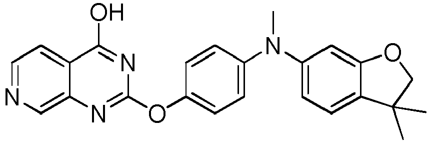

The title compound was prepared in 48% yield from 2-chloro-pyrido[3,4-d]pyrimidin-4-ol and N-(4-hydroxy-phenyl)-N-methyl-2-phenyl-acetamide according to the procedure for the preparation of Example 1. ¹H NMR (400 MHz, DMSO-*d*₆): δ 1.26 (s, 6H), 3.22 (s, 3H), 4.19 (s, 2H), 6.46-6.57 (m, 2H), 6.97 (d, *J* = 7.6 Hz, 1H), 7.10 (d, *J* = 10.8 Hz, ¹H ), 7.19 (d, *J* = 10.8 Hz, 2H ), 7.86 (d, *J* = 6.0 Hz, 1H), 8.50 (d, *J* = 6.0 Hz, 1H), 8.69 (s, 1H), 13.04 (s, 1H). [M+H] Calc'd for C₂₂H₁₈N₄O₃, 387; Found, 387.

### Preparation 117A: 4-(benzyloxy)-N-(4-isopropylphenyl)aniline

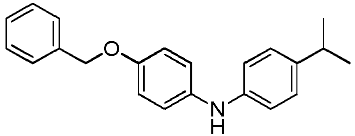

A mixture of 4-(benzyloxy)aniline (7.8 g, 39.03 mmol), 1-bromo-4-isopropylbenzene (8.5 g, 42.93 mmol), X-Phos (2.3 g, 4.68 mmol), Pd(OAc)₂ (0.53 g, 2.34 mmol) and CS₂CO₃ (50.9 g, 156.12 mmol) in toluene(150 mL) was purged with N₂ and then reflux overnight. The reaction mixture was cooled to RT and filtered. The filtrate was concentrated and purified by silica column chromatography (PE:EA, 20:1) to give 7.1 g (57%) of the title compound. ¹H NMR (CDCl₃, 300 MHz): δ 1.23 (d, *J=* 6.9 Hz, 6H), 2.77-2.92 (m, 1H), 5.04 (s, 2H), 6.87-6.94 (m, 4H), 7.01-7.04 (m, 2H), 7.08-7.10 (m, 2H), 7.31-7.46 (m, 5H). [M+H] Calc'd for C₂₂H₂₃NO, 318; Found, 318.

### Preparation 117B: 4-(benzyloxy)-N-ethyl-N-(4-isopropylphenyl)aniline

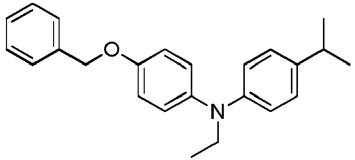

To a solution of compound 4-(benzyloxy)-N-(4-isopropylphenyl)aniline (0.5 g, 1.58 mmol) in DMF (5 mL) was added NaH (189 mg, 4.73 mmol) and the mixture was stirred at 0°C for 30 min. Iodoethane (761 mg, 4.9 mmol) was then added and the reaction mixture was stirred overnight at RT. The mixture was quenched with aqueous NH₄Cl solution and extracted with ethyl acetate (3 × 10 mL). The combined organic layers were washed with water (3 × 10 mL), washed with brine (10 mL), dried over Na₂SO₄ and concentrated to give 350 mg (64%) of the title compound as brown liquid. ¹H NMR (CDCl₃, 300 MHz): δ 1.19 (t, *J=* 6.9 Hz, 3H), 1.22 (d, *J* = 6.9 Hz, 6H), 2.76-2.90 (m, 1H), 3.69 (q, *J* = 6.9 Hz, 2H), 5.06 (s, 2H), 6.72-6.76 (m, 2H), 6.93-6.98 (m, 2H), 7.00-7.08 (m, 4H), 7.31-7.47 (m, 5H). [M+H] Calc'd for C₂₄H₂₇NO, 346; Found, 346.

### Preparation 117C: 4-(ethyl(4-isopropylphenyl)amino)phenol

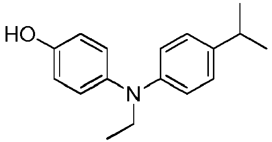

The title compound was prepared in 85% yield from 4-(benzyloxy)-N-ethyl-N-(4-isopropylphenyl)aniline according to the procedure of Preparation 26B. [M+H] Calc'd for C₁₇H₂₁NO, 256 Found, 256.

### Example 117: 2-(4-(ethyl(4-isopropylphenyl)amino)phenoxy)pyrido[3,4-d]pyrimidin-4-ol

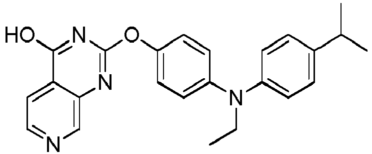

The title compound was prepared in 5% yield from 2-chloro-pyrido[3,4-d]pyrimidin-4-ol and 4-(ethyl(4-isopropylphenyl)amino)phenol according to the procedure for the preparation of Example 1. ¹H NMR (400 MHz, CDCl₃): δ 1.23-1.28 (m, 9H), 2.87-2.94 (m, 1H), 3.77 (q, *J=* 6.8 Hz, 2H), 6.88 (d, *J=* 8.0 Hz, 2H), 7.05-7.11 (m, 4H), 7.20 (d, *J=* 8.0 Hz, 2H), 8.01 (brs, 1H), 8.59 (brs, 1H), 8.93 (s, 1H), 9.69 (brs, 1H). [M+H] Calc'd for C₂₄H₂₄N₄O₂, 401; Found, 401.

### Preparation 118A: N-(4-isopropylphenyl)tetrahydro-2H-pyran-4-amine

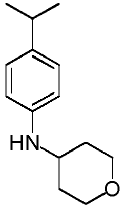

To a solution of compound 4-isopropylaniline (1.0 g, 7.4 mmol), dihydro-2H-pyran-4(3H)-one (1.5 g, 14.8 mmol) in DMF (10 mL) was added AcOH (2 mL). After stirring at RT for 30 min, the reaction mixture was cooled to 0 °C, and sodium triacetoxyborohydride (3.2 g, 14.8 mmol) was added slowly. The mixture was stirred at RT for 2h and then cooled to 0°C, quenched with Na₂CO₃ solution and extracted with EA (2 × 20 mL). The combined organics were washed with water (2 × 10 mL), washed with brine, dried over Na₂SO₄ and concentrated. The residue was purified by silica column chromatography (PE: EA = 10: 1) to give 1.5 g (93%) of the title compound as colorless oil. ¹H NMR (CDCl₃, 300 MHz): δ 1.20 (d, *J* = 7.2 Hz, 6H), 1.39-1.53 (m, 2H), 2.00-2.05 (m, 2H), 2.75-2.85 (m, 1H), 3.41-3.55 (m, 3H), 3.96-4.03 (m, 2H), 6.57 (d, *J=* 8.4 Hz, 2H), 7.04 (d, *J=* 8.4 Hz, 2H). [M+H] Calc'd for C₁₄H₂₁NO, 220; Found, 220.

### Preparation 118B: 4-((4-isopropylphenyl)(tetrahydro-2H-pyran-4-yl)amino)phenol

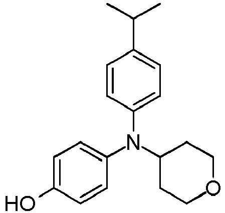

The title compound was prepared in 30% yield from N-(4-isopropylphenyl)tetrahydro-2H-pyran-4-amine according to the procedure of Preparation 5A. [M+H] Calc'd for C₂₀H_{2S}NO₂, 312 Found, 312.

### Example 118: 2-(4-((4-isopropylphenyl)(tetrahydro-2H-pyran-4-yl)amino)phenoxy)pyrido[3,4-d]pyrimidin-4-ol

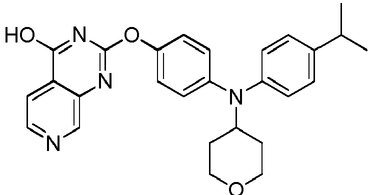

The title compound was prepared in 8% yield from 2-chloro-pyrido[3,4-d] pyrimidin-4-ol and 4-((4-isopropylphenyl)(tetrahydro-2H-pyran-4-yl)amino)phenol according to the procedure for the preparation of Example 1. ¹H NMR (400 MHz, CDCl₃): δ 1.28 (d, *J =* 6.8 Hz, 6H), 1.54-1.66 (m, 2H), 1.91-1.92 (m, 2H), 2.90-2.97 (m, 1H), 3.50-3.57 (m, 2H), 4.01-4.12 (m, 3H), 6.71 (d, *J=* 9.2 Hz, 2H), 6.97 (d, *J=* 8.4 Hz, 2H), 7.08 (d, *J=* 9.2 Hz, 2H), 7.24 (d, *J* = 8.4 Hz, 2H), 8.02 (d, *J* = 4.8 Hz, 1H), 8.58 (d,. *J =* 4.8 Hz, 1H), 8.90 (s, 1H), 9.09 (brs, 1H). [M+H] Calc'd for C₂₇H₂₈N₄O₃, 457; Found, 457.

| Ex. | Structure (prepared by procedure of cited Example) | MS (ESI) | NMR spectrum data |
|---|---|---|---|
| 119 | 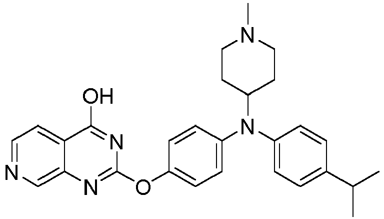 (Prepared by the procedure of Example 118.) | 470 | 1H NMR (400 MHz, DMSO): δ 1.18 (d, *J=* 9.2 Hz ,6H), 1.31-1.43 (m, 2H), 1.89 (d, *J =* 8.0Hz ,2H), 2.25-2.34 (m, 5H), 2.81-2.94 (m, 3H), 3.85-3.93 (m, 1H), 6.80 (d, *J =* 10.8 Hz ,2H), 6.87 (d, *J =* 11.2 Hz ,2H), 7.09 (d, *J =* 12.0 Hz ,2H), 7.22 (d, *J =* 10.8 Hz ,2H), 7.79 (d, *J* = 6.4 Hz ,1H), 8.40 (d, *J=* 6.4 Hz ,1H), 8.65 (s, 1H). |
| 120 | 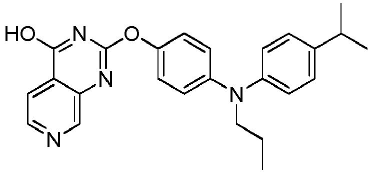 (Prepared by the procedure of Example 117.) | 415 | ¹H NMR (300 MHz, CDCl₃): δ 0.95 (t, *J =* 7.5 Hz, 3H), 1.26 (d, J= 6.9 Hz 6H), 1.65-1.77 (m, 2H), 2.85-2.95 (m, 1H), 3.64 (t, *J=* 7.8 Hz, 2H), 6.87 (d, *J =* 9 Hz, 2H), 7.04-7.11 (m, 4H), 7.20 (d, *J =* 8.1 Hz, 2H), 8.02 (d, *J =* 5.4 Hz, 1H), 8.59 (d, *J =* 5.4 Hz, 1H), 8.94 (s, 1H), 10.22 (s, 1H). |
| 121 | 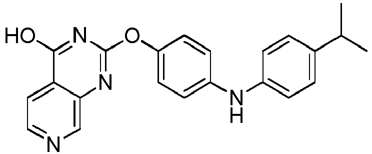 (Prepared by the procedure of Example 117.) | 373 | ¹H NMR (300 MHz, DMSO-*d*₆): δ 1.18 (d, *J* = 6.6 Hz, 6H), 2.80-2.85 (m, 1H), 7.13-7.17 (m, 8H), 8.06 (d, *J=* 6.9 Hz, 1H), 8.56 (d, *J* = 6.9 Hz, 1H), 8.87 (s, 1H). |
| 122 | 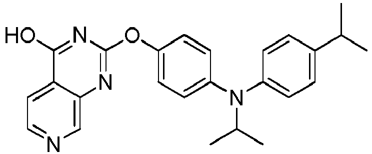 (Prepared by the procedure of Example 39.) | 415 | ¹H NMR (300 MHz, DMSO-*d*₆): δ 1.10 (d, *J* = 6.6 Hz, 6H), 1.22 (d, J= 6.9 Hz, 6H), 2.85-2.93 (m, 1H), 4.28-4.34 (m, 1H), 6.67 (d, *J=* 9.0 Hz, 2H), 6.93 (d, *J =* 8.1 Hz, 2H), 7.13 (d, *J=* 9.0 Hz, 2H), 7.27 (d, *J =* 8.1 Hz, 2H), 7.86 (d, *J =* 6.9 Hz, 1H), 8.50 (d, *J =* 6.9 Hz, 1H), 8.70 (s, 1H). |
| 123 | 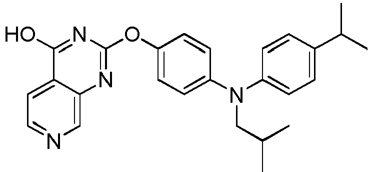 (Prepared by the procedure of Example 39.) | 429 | ¹H NMR (400 MHz, DMSO-*d*₆): δ 0.93 (d, *J=* 8.4 Hz ,6H), 1.20 (d, *J*=8.8 Hz ,6H), 1.88-1.96 (m, 1H), 2.81-2.90 (m, 1H), 3.50 (d, *J =* 9.2 Hz, 2H), 6.89 (d, *J =* 12.0 Hz, 2H), 7.01 (d, *J =* 12.0 Hz, 2H), 7.14-7.22 (m, 4H), 7.87 (d, *J =* 6.0 Hz, 1H), 8.50 (s, 1H), 8.70 (s, 1H). |
| 124 | 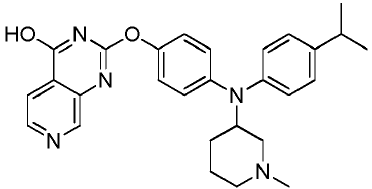 (Prepared by the procedure of Example 118.) | 470 | ¹H NMR (400 MHz, DMSO-*d*₆): δ 0.83-0.96 (m, 2H), 1.20 (d, *J =* 6.9 Hz ,6H), 1.67-1.93 (m, 4H), 2.27 (s, 3H), 2.77-2.92 (m, 2H), 3.15-3.18 (m, 1H), 3.99-4.02 (m, 1H), 6.67 *(d, J=* 9.0 Hz, 2H), 6.90 (d, *J =* 8.1 Hz, 2H), 7.12 (d, *J =* 9.0 Hz, 2H), 7.25 (d, *J =* 8.1 Hz, 2H), 7.84 (d, *J*=4.5 Hz, 1H), 8.46 (d, *J*=4.5 Hz, 1H), 8.67 (s, 1H). |
| 125 | 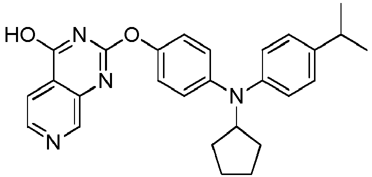 (Prepared by the procedure of Example 118.) | 441 | ¹H NMR (400 MHz,CDCl₃): δ 1.27 (d, *J* = 6.8 Hz, 6H), 1.44-1.47 (m, 2H), 1.58-1.61 (m, 4H), 1.96-2.02 (m, 2H), 2.89-2.96 (m, 1H), 4.20-4.28 (m, 1H), 6.74 (d, *J =* 9.2 Hz, 2H), 6.99 (d, *J =* 8.4 Hz, 2H), 7.07 (d, *J* = 9.2 Hz, 2H), 7.23 (d, *J=* 8.4 Hz, 2H), 8.00 (d, *J* = 12.4 Hz, 1H), 8.57 (d, *J =* 12.4 Hz, 1H), 8.89 (s, 1H), 9.20 (s ,1H). |
| 126 | 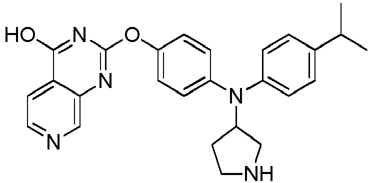 (Prepared by the procedure of Example 118.) | 442 | ¹H NMR (400 MHz, DMSO-*d*₆): δ 1.22 (d, *J=* 6.9 Hz ,6H), 1.63-1.71 (m, 1H), 2.21-2.28 (m, 1H), 2.89-3.17 (m, 4H), 3.57-3.59 (m, 1H), 4.67-4.72 (m, 1H), 6.82 (d, *J* = 9.0 Hz, 2H), 7.05 (d, *J* = 8.1 Hz, 2H), 7.12 (d, *J =* 9.0 Hz, 2H), 7.22 (d, *J =* 8.1 Hz, 2H), 7.89 (d, *J =* 5.4 Hz, 1H), 8.52 (d, *J =* 5.4 Hz, 1H), 8.71 (s, 1H). |
| 127 | 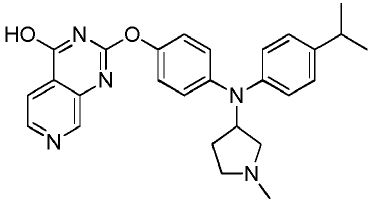 (Prepared by the procedure of Example 118.) | 456 | ¹H NMR (400 MHz, DMSO-*d*₆): δ 1.20 (d, *J=* 7.2 Hz ,6H), 1.70-1.81 (m, 1H), 2.14-2.20 (m, 1H), 2.28 (s, 3H), 2.54-2.59 (m, 3H), 2.83-2.91 (m, 2H), 4.52-4.57 (m, 1H), 6.81 (d, *J* = 9.0 Hz, 2H), 6.96 (d, *J =* 8.1 Hz, 2H), 7.13 (d, *J =* 9.0 Hz, 2H), 7.23 (d, *J =* 8.1 Hz, 2H), 7.83 (d, *J=* 5.1 Hz, 1H), 8.45 (d, *J* = 5.1 Hz, 1H), 8.67 (s, 1H). |
| 128 | 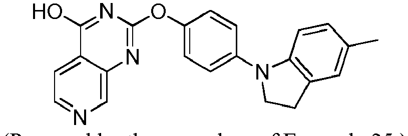 (Prepared by the procedure ot Example 25.) | 371 | ¹H NMR (400 MHz, DMSO-*d*₆): δ 2.27 (s, 3H), 3.07 (t, *J=* 6.3 Hz, 2H), 3.94 (t, *J =* 6.3 Hz, 2H), 6.87 (d, *J* = 6.6 Hz, 1H), 7.02-7.06 (m, 2H), 7.25-7.31 (m, 4H), 7.88 (d, *J* = 3.9 Hz, 1H), 8.51 (d, *J* = 3.9 Hz, 1H), 8.70 (s, 1H). |
| 129 | 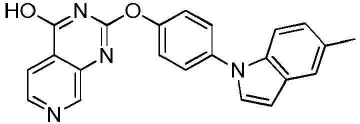 (Prepared by the procedure of Example 25.) | 369 | ¹H NMR (400 MHz, DMSO-*d*₆): δ 2.41 (s, 3H), 6.63 (d, *J=* 2.4 Hz, 1H), 7.04-7.06 (m, 1 H), 7.27-7.29 (m, 1H), 7.45-7.69 (m, 6H), 7.88 (d, *J =* 3.9 Hz, 1H), 8.50 (d, *J =* 3.9 Hz, 1H), 8.72 (s, 1H). |
| 130 | 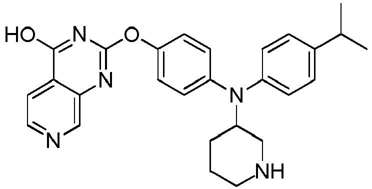 (Prepared by the procedure of Example 118.) | 456 | ¹H NMR (400 MHz, DMSO-*d*₆+D₂O): δ 1.20-1.22 (m, 7H), 1.78-1.90 (m, 2H), 1.98-2.03 (m, 1H), 2.62-2.71 (m, 2H), 2.88-2.92 (m, 1H), 3.20-3.23 (m, 1H), 3.50-3.54 (m, 1H), 4.20-4.27 (m, 1H), 6.72 (d, *J=* 8.7 Hz, 2H), 6.97 (d, *J* = 7.8 Hz, 2H), 7.19 (d, *J =* 9.7 Hz, 2H), 7.31 (d, *J* = 7.8 Hz, 2H), 7.89 (s, 1H), 8.52 (s, 1H), 8.68 (s, 1H). |
| 131 | 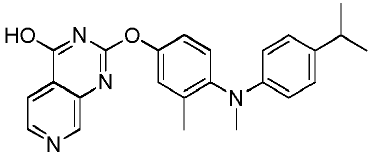 (Prepared by the procedure of Example 1.) | 401 | ¹H NMR (400 MHz, DMSO-*d*₆+D₂O): δ 1.15 (d, *J =* 7.2 Hz, 6H), 2.11 (s, 3H), 2.75-2.8 (m, 1H), 3.18 (s, 3H), 6.47 (d, J= 8.8 Hz, 2H), 7.04 (d, *J* = 8.8 Hz, 2H), 7.14 (d, *J=* 8.0 Hz, 1H), 7.22-7.24 (m, 1H), 7.3 (d, *J* = 2.0 Hz, 1H), 7.90 (d, *J=* 4.8 Hz, 1H), 8.53 (d, *J* = 4.8 Hz, 1H), 8.75 (s, 1H), 13.10 (s, 1H). |
| 132 | 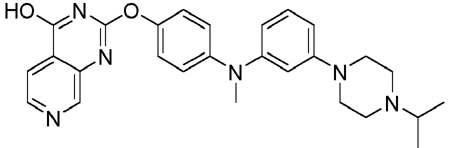 (Prepared by the procedure of Example 22.) | 470 | ¹H NMR (400 MHz, DMSO-d6): δ 1.03 (d, J = 6.4 Hz, 6H), 2.65-2.68 (m, 4H), 2.65-2.72-2.75 (m, 1H), 3.12-3.16 (m, 4H), 3.28 (s, 3H), 6.81-6.53 (d, *J=* 7.6 Hz, 1H), 6.25 (d, *J =* 6.4 Hz, 2H), 6.98 (t, *J* = 7.2, 2 Hz, 2H), 7.14-7.20 (m, 3H), 7.87 (d, *J=* 5.2 Hz, 1H),8.50 (d, *J =* 5.2 Hz, 1H), 8.69 (s, 1H). |
| 133 | 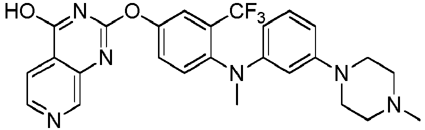 (Prepared by the procedure of Example 22.) | 511 | ¹H NMR (300 MHz, CDCl₃): δ 2.44 (s, 3H), 2.70-2.74 (m, 4H), 3.20-3.25 (m, 7H), 6.13 (s, 1H),6.18 (d, *J* = 8.7 Hz, 1H), 6.42 (d, *J =* 10.2 Hz, 1H), 7.11 (t, *J* = 7.8 Hz, 1H), 7.36 (d, *J* = 8.7 Hz, 1H), 7.50-7.54 (m, 1H), 7.67 (s, 1H), 7.99 (d, J = 5.1 Hz, 1H), 8.61 (d, *J=* 5.1 Hz, 1H), 8.89 (s, 1H). |
| 134 | 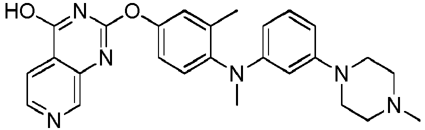 (Prepared by the procedure of Example 22.) | 457 | ¹H NMR (400 MHz, DMSO-d6): δ 2.09 (s,3H), 2.24 (s, 3H), 2.46-2.51 (m, 4H), 3.04-3.06 (m, 4H), 3.19 (s, 3H), 5.94 (d, *J* = 8.4 Hz, 1H), 6.08 (s, 1H), 6.30 (d, *J =* 1.2 Hz, 1H), 6.97 (t,*J* = 8.4 Hz, 1H), 7.18 (s, 2H), 7.23 (s, 1H), 7.84 (d, *J=* 5.6 Hz, 1H), 8.45 (d, *J* = 4.4 Hz, 1H), 8.67 (s, 1H). |
| 135 | 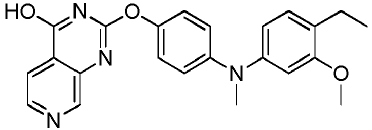 (Prepared by the procedure of Example 1.) | 403 | ¹H NMR (400 MHz, DMSO-*d*₆): δ 1.12 (t, *J* = 7.6 Hz, 3H), 2.50-2.56 (m, 2H), 3.16 (s, 3H), 3.75 (s, 3H), 6.63-6.65 (m, 1H), 6.71 (d, *J* = 2.4 Hz, 1H), 6.95 (d, *J* = 8.8 Hz, 2H), 7.10 (d, *J* = 8.4 Hz, 1H), 7.18 (d, *J* = 9.2 Hz, 2H), 7.87 (d, *J* = 5.2 Hz, 1H), 8.51 (d, *J=* 4.8 Hz, 1H), 8.69 (s, 1H). |
| 136 | 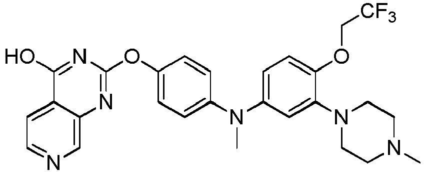 (Prepared by the procedure of Example 22.) | 541 | ¹H NMR (400 MHz, DMSO-*d*₆): δ 2.27 (s, 3H), 2.49-2.51 (m, 4H), 2.98-3.04 (m, 4H), 3.25 (s, 3H), 4.68-4.71 (m, 2H), 6.72 (s, 1H), 6.73 (d, *J=* 5.2 Hz, 1H), 6.85 (d, *J* = 9.2 Hz, 2H), 7.03 (d, *J* = 9.2 Hz, 1H), 7.14 (d, *J* = 9.2 Hz, 2H), 7.86 (d, *J* = 5.2 Hz, 1H), 8.50 (d, *J* = 5.2 Hz, 1H), 8.68 (s, 1H). |
| 137 | 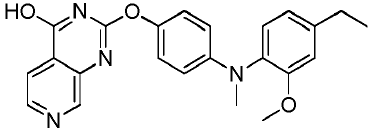 (Prepared by the procedure of Example 1.) | 403 | ¹H NMR (400 MHz, DMSO-d₆): δ 1.29 (t, *J=* 7.6 Hz, 3H), 2.69 (q, *J=* 8.0 Hz, 2H), 3.24 (s, 3H), 3.81 (s, 3H), 6.65-6.68 (m, 2H), 6.83 (d, *J=* 7.6 Hz, 2H), 7.03-7.05 (m, 2H), 7.14 (d, *J* = 8.0 Hz, 1H), 7.97 (s, 1H), 8.58 (s, 1H), 8.87 (s, 1H), 9.13 (s, 1H). |
| 138 | 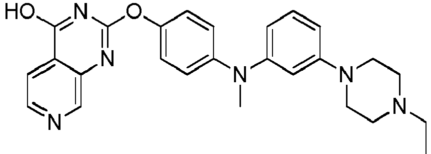 (Prepared by the procedure of Example 22.) | 457 | ¹H NMR (400 MHz, DMSO-*d*₆): δ 1.04 (t, *J=* 7.2 Hz, 3H), 2.43 (q, *J*=7.2 Hz, 2H), 2.54-2.56 (m, 4H), 3.12-3.14 (m, 4H), 3.27 (s, 3H), 6.50 (d, *J=* 8.0 Hz, 1H), 6.62-6.64 (m ,2H), 6.97 (d, *J=* 8.8 Hz, 2H), 7.14-7.20 (m, 3H), 7.87 (d, *J=* 4.8 Hz, 1H), 8.49 (d, *J* = 4.8 Hz, 1H), 8.69 (s, 1H). |
| 139 | 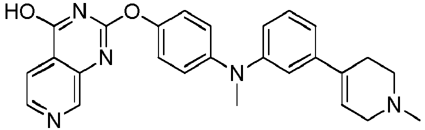 (Prepared by the procedure of Example 34.) | 440 | ¹H NMR (400 MHz, DMSO-*d*₆): δ 2.36 (s, 3H), 2.50 (s, 2H), 2.67-2.70 (m, 2H), 3.14 (s, 2H), 3.31 (s, 3H), 6.12 (s, 1H), 6.94-6.96 (m, 1H), 7.03-7.09 (m, 4H), 7.21 (d, *J*=8.8 Hz, 2H), 7.28 (t, *J*=8.8 Hz, 1H), 7.85 (d, *J*=4.8 Hz, 1H), 7.47 (d, *J*=4.8 Hz, 1H), 8.68 (s, 1H). |
| 140 | 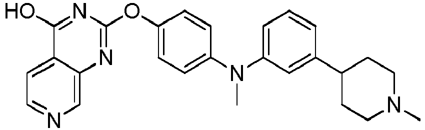 (Prepared by the procedure of Example 34.) | 442 | ¹H NMR (400 MHz, DMSO-*d*₆): δ 1.66-1.73 (m, 2H), 1.77-1.80 (m, 2H), 2.24-2.29 (m, 2H), 2.36 (s, 3H), 2.49-2.51 (m, 1H), 3.01-3.04 (m, 2H), 3.28 (s, 3H), 6.82-6.88 (m, 3H), 7.04 (d, *J=* 8.8 Hz, 2H), 7.18 (d, *J* = 8.8 Hz, 2H) 7.23 (m, 1H), 7.82 (d, *J* = 5.2 Hz, 1H), 8.42 (d, *J=* 5.2 Hz, 1H), 8.67 (s, 1H). |
| 141 | 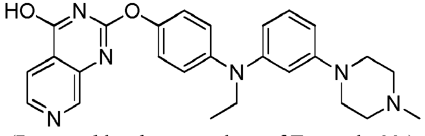 (Prepared by the procedure of Example 22.) | 457 | ¹H NMR (300 MHz, DMSO-*d*₆): δ 1.55 (t, *J=* 5.1 Hz, 3H), 2.23 (s, 3H), 2.45-2.51 (m, 4H), 3.09-3.11 (m, 4H), 3.72-3.77 (m, 2H), 6.47-6.50 (m, 1H), 6.59-6.63 (m, 2H), 6.92 (d, *J=* 5.1 Hz, 2H), 7.14-7.18 (m, 3H), 7.85 (d, *J*=3.6 Hz, 1H), 8.48 (d, *J=* 3.6 Hz, 1H), 8.69 (s, 1H). |
| 142 | 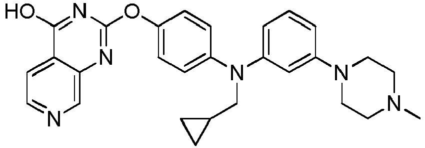 (Prepared by the procedure of Example 22.) | 483 | ¹H NMR (300 MHz, DMSO-*d*₆): δ 0.13-0.17 (m, 2H), 0.42-0.46 (m, 2H), 1.08-1.12 (m, 1H), 2.21 (s, 3H), 2.43-2.45 (m, 4H), 3.07-3.10 (m, 4H), 3.56 (d, *J* = 4.8 Hz, 2H), 6.47 (d, *J* = 6.9 Hz, 1H), 6.58-6.59 (m, 2H), 6.94 (d, *J* = 6.9 Hz, 2H), 7.10-7.15 (m, 3H), 7.79 (d, *J* = 3.9 Hz, 1H), 8.38 (d, *J* = 3.9 Hz, 1H), 8.64 (s, 1H). |
| 143 | 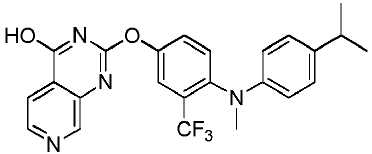 (Prepared by the procedure of Example 22.) | 455 | ¹H NMR (400 MHz, DMSO-d₆): δ 1.15 (d, *J* = 6.8 Hz, 6H), 2.76-2.78 (m, 1H), 3.13 (s, 3H), 6.48 (d, *J* = 8.8 Hz, 2H), 7.05 (d, *J* = 8.8 Hz, 2H), 7.41 (d, *J* = 8.4 Hz, 1H ), 7.62 (d, *J* = 8.4 Hz, 1H), 7.70 (s, 1H), 7.79 (d, *J* = 4.4 Hz, 1H), 8.37 (d, *J* = 8.8 Hz, 1H), 8.68 (s, 1H). |
| 144 | 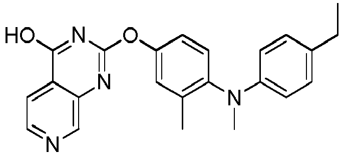 (Prepared by the procedure of Example 1.) | 387 | ¹H NMR (400 MHz, DMSO-*d*₆): δ 1.13 (t, *J=* 7.6 Hz, 3H), 2.10 (s, 3H), 2.48 (q, *J* = 7.6 Hz, 2H), 3.18 (s, 3H), 6.46 (d, *J=* 8.8 Hz, 2H), 7.01 (d, *J=* 8.8 Hz, 2H), 7.20-7.26 (m, 2H), 7.29 (s, 1H), 7.89 (d, *J* = 5.2 Hz, 1H), 8.53 (d, *J* = 5.2 Hz, 1H), 8.74 (s,1H). |
| 145 | 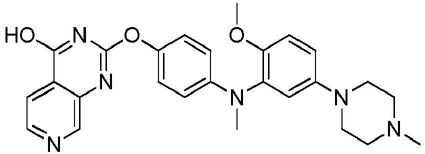 (Prepared by the procedure of Example 22.) | 473 | 1H NMR (400 MHz, CDCl3): 2.55 (s, 3H), 2.82-2.87 (m, 4H), 3.23 (s, 3H), 3.24-3.31 (m, 4H), 3.76 (s, 3H), 6.66 (d, *J=* 9.2 Hz, 2H), 6.83 (dd, *J*=8.8 Hz, 2.8 Hz, 1H), 6.88 (d, *J*=2.8 Hz, 1H), 6.93 (d, *J* = 8.8 Hz, 1H), 7.03 (d, *J* = 9.2 Hz, 2H), 7.96 (d, *J*=5.2 Hz, 1H), 8.55 (d, *J*=5.2 Hz, 1H), 8.87(s, 1H). |
| 146 | 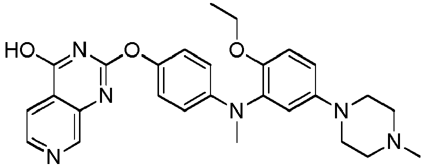 (Prepared by the procedure of Example 22.) | 487 | ¹H NMR (300 MHz, DMSO-*d*₆): δ 1.14 (t, *J=* 6.6 Hz, 3H), 2.24 (s, 3H), 2.50-2.55 (m, 4H), 3.01-3.09 (m, 4H), 3.18 (s, 3H), 3.87-3.94 (m, 2H), 6.58 (d, *J=* 9.0 Hz, 2H), 6.81 (d, *J=* 5.7 Hz, 2H), 6.98-7.07 (m, 3H), 7.85-7.86 (m, 1H), 8.48 (d, *J* = 5.1 Hz, 1H), 8.66 (s, 1H). |
| 147 | 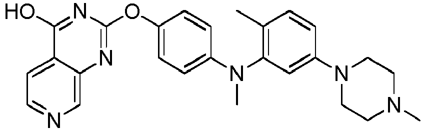 (Prepared by the procedure of Example 22.) | 457 | ¹H NMR (400 MHz, DMSO-*d*₆): 2.00 (s, 3H), 2.43 (s, 3H), 2.70-2.76 (m, 4H), 3.20 (s, 3H), 3.28-3.40 (m, 4H), 6.50 (d, *J* = 9.2 Hz, 2H), 6.77 (d, *J*=2.4 Hz, 1H), 6.85 (dd, *J* = 8.8 Hz, 2.4 Hz, 1H), 6.50 (d, *J* = 9.2 Hz, 2H), 7.19 (d, *J* = 8.8 Hz, 1H), 7.86 (dd, *J* = 5.2 Hz, 0.8 Hz, 1H), 8.50 (d, *J* = 5.2 Hz, 1H), 8.68(s, 1H). |
| 148 | 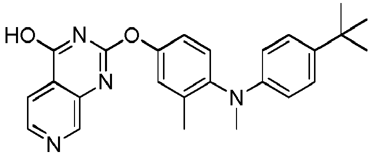 (Prepared by the procedure of Example 1.) | 415 | ¹H NMR (400 MHz, DMSO-*d*₆): δ 1.23 (s, 9H), 2.12 (s, 3H), 3.18 (s, 3H), 6.47 (d, *J*=8.8 Hz, 2H), 7.11-7.26 (m, 4H), 7.30 (s, 1H), 7.90 (d, *J*=5.2 Hz, 1H), 8.54 (d, *J*=5.2 Hz, 1H), 8.75 (s, 1H). |
| 149 | 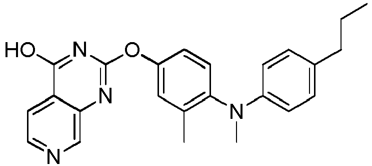 (Prepared by the procedure of Example 1.) | 401 | ¹H NMR (400 MHz, DMSO-*d*₆): δ 0.87 (t, *J*=7.6 Hz, 3H), 1.52 (m, 2H), 2.10 (s, 3H), 2.43 (t, *J*=7.6 Hz, 2H), 3.18 (s, 3H), 6.46 (d, *J*=8.8 Hz, 2H), 6.99 (d, *J*=8.8 Hz, 2H), 7.22-7.24 (m, 2H), 7.29 (s, 1H), 7.90 (d, *J*=4.8 Hz, 1H), 8.53 (d, *J*=4.8 Hz, 1H), 8.75 (s, 1H). |

### II. Biological Evaluation

### Example 1: In Vitro Enzyme Inhibition Assay

This assay determines the ability of a test compound to inhibit JMJD2C demethylase activity. Baculovirus expressed JMJD2C (GenBank Accession #BC143571, AA 2-372) was purchased from BPS Bioscience (Cat#50105).

### JMJD2C Assay

The ability of test compounds to inhibit the activity of JMJD2C was determined in 384-well plate-format under the following reaction conditions: 0.3 nM JMJD2C, 300 nM H3K9me3-biotin labeled peptide (Anaspec cat # 64360), 2 µM alpha-ketoglutaric acid in assay buffer of 50 mM HEPES, pH7.3, 0.005% Brij35, 0.5 mM TCEP, 0.2 mg/ml BSA, 50 µM sodium L-ascorbate, and 2 µM ammonium iron(II) sulfate. Reaction product was determined quantitatively by TR-FRET after the addition of detection reagent Phycolink Streptavidin-allophycocyanin (Prozyme) and Europium-anti-di-methylated histone H3 lysine 9 (H3K9me2) antibody (PerkinElmer) in the presence of 5 mM EDTA in LANCE detection buffer (PerkinElmer) at a final concentration of 50 nM and 1 nM, respectively.

The assay reaction was initiated by the following: 2 µl of the mixture of 900 nM H3K9me3-biotin labeled peptide and 6 µM alpha-ketoglutaric acid with 2 µl of 11-point serial diluted inhibitor in 3% DMSO were added to each well of the plate, followed by the addition of 2 µl of 0.9 nM JMJD2C to initiate the reaction. The reaction mixture was incubated at room temperature for 30 min, and terminated by the addition of 6 µl of 5 mM EDTA in LANCE detection buffer containing 100 nM Phycolink Streptavidin-allophycocyanin and 2 nM Europium-anti-H3K9me2 antibody. Plates were read by EnVisionMultilabel Reader in TR-FRET mode (excitation at 320nm, emission at 615nm and 665nm) after 1 hr incubation at room temperature. A ratio was calculated (665/615) for each well and fitted to determine inhibition constant (IC₅₀).

The ability of the compounds disclosed herein to inhibit demethylase activity was quantified and the respective IC₅₀ value was determined. Table 3 provides the IC₅₀ values of various compounds disclosed herein.

| Table 3. *In vitro* enzyme inhibition | | | |
|---|---|---|---|
| Ex. | Compound Name | | JMJD2C IC₅₀ |
| 1 | 2-[4-(methyl-pyridin-2-yl-amino)-phenoxy]-pyrido[3,4-d]pyrimidin-4-ol | | B |
| 2 | 2-(1-methyl-1H-indol-5-yloxy)-pyrido[3,4-d]pyrimidin-4-ol | | B |
| 3 | 2-(1-phenethyl-1H-indol-5-yloxy)-pyrido[3,4-d]pyrimidin-4-ol | | B |
| 4 | 2-(1-benzyl-1H-indol-5-yloxy)-pyrido[3,4-d]pyrimidin-4-ol | | B |
| 5 | 2-[4-(methyl-phenyl-amino)-phenoxy]-pyrido[3,4-d]pyrimidin-4-ol | | B |
| 6 | 2-[4-(benzyl-methyl-amino)-phenoxy]-pyrido[3,4-d]pyrimidin-4-ol | | B |
| 7 | 2-[3-(methyl-phenyl-amino)-phenoxy]-pyrido[3,4-d]pyrimidin-4-ol | | C |
| 8 | 2-(1-benzyl-1H-indol-6-yloxy)-pyrido[3,4-d]pyrimidin-4-ol | | C |
| 9 | 2-[3-(benzyl-methyl-amino)-phenoxy]-pyrido[3,4-d]pyrimidin-4-ol | | C |
| 10 | 2-[3-fluoro-4-(methyl-phenyl-amino)-phenoxy] -pyrido[3,4-d]pyrimidin-4-ol | | B |
| 11 | 2-(1-benzyl-1H-indazol-6-yloxy)-pyrido[3,4-d]pyrimidin-4-ol | | B |
| 12 | 2-(2-benzyl-2H-indazol-6-yloxy)-pyrido[3,4-d]pyrimidin-4-ol | | B |
| 13 | 2-{4-[methyl(2-phenylethyl)amino]phenoxy}pyridino[3,4-d]pyrimidin-4-ol | | B |
| 14 | 2-[2-benzyl-2H-indazol-5-yloxy]pyridino[3,4-d]pyrimidin-4-ol | | B |
| 15 | 2-(1-benzyl-1H-indazol-5-yloxy)-pyridino[3,4-d]pyrimidin-4-ol | | B |
| 16 | 2-{4-[(4-methoxy-phenyl)-methyl-amino]-phenoxy}-pyrido[3,4-d]pyrimidin-4-ol | | B |
| 17 | 2-{4-[(3-methoxy-phenyl)-methyl-amino]-phenoxy}-pyrido [3,4-d]pyrimidin-4-ol | | B |
| 18 | 2-{4-[methyl-(4-morpholin-4-yl-phenyl)-amino]-phenoxy}-pyrido [3,4-d]pyrimidin-4-ol | | B |
| 19 | 2-{4-[methyl-(3-morpholin-4-yl-phenyl)-amino]-phenoxy}-pyrido [3,4 d]pyrimidin-4-ol | | B |
| 20 | 2-[4-(methyl-p-tolyl-amino)-phenoxy]-pyrido[3,4-d]pyrimidin-4-ol | | B |
| 21 | 2-[4-(methyl-m-tolyl-amino)-phenoxy]-pyrido[3,4-d]pyrimidin-4-ol | | B |
| 22 | 2-(4-{methyl-[3-(4-methyl-piperazin-1-yl)-phenyl]-amino}-phenoxy)-pyrido[3,4-d] pyrimidin-4-ol | | A |
| 23 | 2-(4-{[4-(4-amino-piperidin-1-yl)-phenyl]-methyl-amino}-phenoxy)-pyrido[3,4-d]pyrimidin-4-ol | | B |
| 24 | N-[4-(4-hydroxy-pyrido[3,4-d]pyrimidin-2-yloxy)-phenyl]-N-methyl-2-phenyl-acetamide | | B |
| 25 | 2-[4-(3-phenyl-piperidin-1-yl)-phenoxy]-pyrido[3,4-d]pyrimidin-4-ol | | B |
| 26 | 2-[4-(2-phenyl-morpholin-4-yl)-phenoxy]-pyrido[3,4-d]pyrimidin-4-ol | | B |
| 27 | 2-{4-[methyl-(5-morpholin-4-yl-pyridin-3-yl)-amino]-phenoxy}-pyrido [3,4-d]pyrimidin-4-ol | | B |
| 28 | 2-{4-[methyl-(1,2,3,4-tetrahydro-naphthalen-1-ylmethyl)-amino]-phenoxy}-pyrido[3,4-d]pyrimidin-4-ol | | A |
| 29 | 2-(4-{[4-(2-methoxy-1-methyl-ethyl)-phenyl]-methyl-amino}-phenoxy)-pyrido[3,4-d]pyrimidin-4-ol | | B |
| 30 | 3-(4-{[4-(4-hydroxy-pyrido[3,4-d]pyrimidin-2-yloxy)-phenyl]-methyl-amino}-phenyl)-butyronitrile | | B |
| 31 | 2-(1-cyclopentyl-1H-indol-5-yloxy)-pyrido[3,4-d]pyrimidin-4-ol | | C |
| 32 | 2-(1-phenyl-2,3-dihydro-1H-indol-5-yloxy)-pyrido[3,4-d]pyrimidin-4-ol | | B |
| 33 | 2-(1-phenyl-1,2,3,4-tetrahydro-quinolin-6-yloxy)-pyrido[3,4-d]pyrimidin-4-ol | | C |
| 34 | 2-{4-[(5-isopropyl-pyridin-2-yl)-methyl-amino]-phenoxy}-pyrido [3,4-d]pyrimidin-4-ol | | A |
| 35 | 2-{4-[(4-isopropyl-3-morpholin-4-yl-phenyl)-methyl-amino]-phenoxy}-pyrido[3,4-d]pyrimidin-4-ol | | B |
| 36 | 2-(4-{[4-(1-methoxy-ethyl)-phenyl]-methyl-amino}-phenoxy)-pyrido[3,4-d]pyrimidin-4-ol | | B |
| 37 | 2-(4-{[4-(2-amino-1-methyl-ethyl)-phenyl]-methyl-amino}-phenoxy)-pyrido[3,4-d]pyrimidin-4-ol | | A |
| 38 | 2-{4-[(4-{2-[(2-methoxy-ethyl)-methyl-amino]-1-methyl-ethyl}-phenyl)-methyl-amino] -phenoxy } -pyrido[3,4-d]pyrimidin-4-ol | | B |
| 39 | 2-(4-{[4-(1-cyclopropyl-ethyl)-phenyl]-methyl-amino}-phenoxy)-pyrido[3,4-d]pyrimidin-4-ol | | C |
| 40 | 3-{[4-(4-hydroxy-pyrido[3,4-d]pyrimidin-2-yloxy)-phenyl]-methyl-amino}-benzonitrile | | B |
| 41 | 2-(4-{methyl-[3-(4-methyl-piperazin-1-ylmethyl)-phenyl] -amino } -phenoxy)-pyrido[3, 4-d]pyrimidin-4-ol | | A |
| 42 | 2-{4-[(4-cyclopropyl-phenyl)-methyl-amino]-phenoxy}-pyrido[3,4-d]pyrimidin-4-ol | | B |
| 43 | 2-[4-[methyl-(5-methylpyridin-2-yl)amino]phenoxy]pyrido[3,4-d]pyrimidin-4-ol | | B |
| 44 | 2-[4-[4-(dimethylamino)-N-methylanilino]phenoxy]pyrido[3,4-d]pyrimidin-4-ol | | A |
| 45 | 4-[3-[4-(4-hydroxypyrido[3,4-d]pyrimidin-2-yl)oxy-N-methylanilino] phenyl] -1-methylpiperazin-2-one | | B |
| 46 | 2-[4-[N-methyl-4-(4-methylpiperazin-1-yl)anilino]phenoxy]pyrido[3,4-d]pyrimidin-4-ol | | B |
| 47 | 2-[4-[3-(dimethylamino)-N-methylanilino]phenoxy]pyrido[3,4-d]pyrimidin-4-ol | | B |
| 48 | 2-[4-(N-methyl-3-pyrrolidin-1-ylanilino)phenoxy]pyrido[3,4-d]pyrimidin-4-ol | | B |
| 49 | 2-[4-(N-methyl-4-pyrrolidin-1-ylanilino)phenoxy]pyrido[3,4-d]pyrimidin-4-ol | | C |
| 50 | 2-[4-[3-(4-aminopiperidin-1-yl)-N-methylanilino]phenoxy]pyrido[3,4-d]pyrimidin-4-ol | | A |
| 51 | 2-[4-[methyl-[(1S)-1-phenylethyl]ammo]phenoxy]pyndo[3,4-d]pynmidin-4-ol | | B |
| 52 | 2-[4-[methyl-[(1R)-1-phenylethyl]ammo]phenoxy]pyndo[3,4-d]pynmidin-4-ol | | B |
| 53 | 2-[4-(3-fluoro-N,4-dimethylanilino)phenoxy]pyrido[3,4-d]pyrimidin-4-ol | | B |
| 54 | 2-[4-(3-phenylpyrrolidm-1-yl)phenoxy]pyndo[3,4-d]pyrimidin-4-ol | | C |
| 55 | 2-[4-(N,4-dimethyl-3-morpholin-4-ylanilino)phenoxy]pyrido[3,4-d]pyrimidin-4-ol | | B |
| 56 | 2-[4-(N-methyl-3-methylsulfonylanilino)phenoxy]pyrido[3,4-d]pyrimidin-4-ol | | B |
| 57 | 2-[4-(N-methyl-4-methylsulfonylanilino)phenoxy]pyrido[3,4-d]pyrimidin-4-ol | | B |
| 58 | 2-[4-[3-(3-aminopiperidin-1-yl)-N-methylanilino]phenoxy]pyrido[3,4-d]pyrimidin-4-ol | | A |
| 59 | 2-[4-(4-ethyl-N-methyl-3-morpholin-4-ylanilino)phenoxy]pyrido[3,4-d]pyrimidin-4-ol | | A |
| 60 | 2-[4-[4-ethyl-N-methyl-3-(4-methylpiperazin-1-yl)anilino] phenoxy]pyrido[3,4-d]pyrimidin-4-ol | | A |
| 61 | 2-[4-[methyl-(2-morpholin-4-ylpyridin-4-yl)amino]phenoxy]pyrido[3,4-d]pyrimidin-4-ol | | A |
| 62 | 2-[4-[2,3-dihydro-1H-inden-1-ylmethyl(methyl)amino]phenoxy]pyrido [3,4-d]pyrimidin-4-ol | | B |
| 63 | 2-[4-[N-methyl-4-(2-methylpropyl)anilino]phenoxy]pyrido [3,4-d]pyrimidin-4-ol | | B |
| 64 | 2-[4-[4-(2-hydroxypropan-2-yl)-N-methylanilino]phenoxy]pyrido[3,4-d]pyrimidin-4-ol | | B |
| 65 | 2-[4-[3-[2-(dimethylamino)ethoxy]-4-ethyl-N-methylanilino] phenoxy]pyrido[3,4-d]pyrimidin-4-ol | | A |
| 66 | 2-[4-[4-ethyl-3-(2-methoxyethoxy)-N-methylanilino]phenoxy] pyrido[3,4-d]pyrimidin-4-ol | | C |
| 67 | 2-[4-[4-(1-methoxy-2-methylpropan-2-yl)-N-methylanilino]phenoxy]pyrido[3,4-d]pyrimidin-4-ol | | C |
| 68 | 2-[4-[4-(1-hydroxy-2-methylpropan-2-yl)-N-methylanilino]phenoxy]pyrido[3,4-d]pyrimidin-4-ol | | B |
| 69 | 2-[4-(N-methyl-4-propylanilino)phenoxy]pyrido[3,4-d]pyrimidin-4-ol | | C |
| 70 | 2-[4-[N-methyl-4-[1-(methylamino)propan-2-yl]anilno] phenoxy]pyrido[3,4-d]pyrimidin-4-ol | | A |
| 71 | 2-[4-[4-(4-aminobutan-2-yl)-N-methylanilino]phenoxy]pyrido[3,4-d]pyrimidin-4-ol | | A |
| 72 | 2-[4-[N-methyl-4-[4-(methylamino)butan-2-yl]anilino] phenoxy]pyrido[3,4-d]pyrimidin-4-ol | | A |
| 73 | 2-[4-[N-methyl-4-(2,2,2-trifluoroethoxy)anilino]phenoxy]pyrido[3,4-d]pyrimidin-4-ol | | B |
| 74 | 2-[4-[N-methyl-4-(2-methylpropoxy)anilino]phenoxy]pyrido[3,4-d]pyrimidin-4-ol | | C |
| 75 | 2-[4-[4-(2,2-dimethylpropoxy)-N-methylanilino]phenoxy]pyrido[3,4-d]pyrimidin-4-ol | | C |
| 76 | 2-[4-[4-(cyclopropylmethyl)-N-methylanilino]phenoxy]pyrido[3,4-d]pyrimidin-4-ol | | C |
| 77 | 2-[4-[4-[(2S)-butan-2-yl]-N-methylanilino]phenoxy]pyrido[3,4-d]pyrimidin-4-ol | | C |
| 78 | 2-[4-[4-[(2R)-butan-2-yl]-N-methylanilino]phenoxy]pyrido[3,4-d]pyrimidin-4-ol | | C |
| 79 | 2-[4-[N-methyl-3-(4-methylpiperazin-1-yl)anilino]phenoxy]pyrido[3,4-d]pyrimidin-4-ol | | A |
| 80 | 2-[4-(N-ethylanilino)phenoxy]pyrido[3,4-d]pyrimidin-4-ol | | B |
| 81 | 4-[4-(4-hydroxypyrido[3,4-d]pyrimidin-2-yl)oxy-N-methylanilino]benzonitrile | | B |
| 82 | 2-[4-[methyl-(2-methylindazol-5-yl)amino]phenoxy]pyrido[3,4-d]pyrimidin-4-ol | | B |
| 83 | 2-[4-(N,3,4-trimethylanilino)phenoxy]pyrido[3,4-d]pyrimidin-4-ol | | C |
| 84 | 2-[4-(4-ethyl-N-methylanilino)phenoxy]pyrido[3,4-d]pyrimidin-4-ol | | B |
| 85 | 2-[4-(N-methyl-4-propan-2-ylanilino)phenoxy]pyrido[3,4-d]pyrimidin-4-ol | | C |
| 86 | 2-[4-[N-methyl-3-(trifluoromethyl)anilino]phenoxy]pyrido[3,4-d]pyrimidin-4-ol | | C |
| 87 | 2-[4-[N-methyl-4-(trifluoromethyl)anilino]phenoxy]pyrido[3,4-d]pyrimidin-4-ol | | C |
| 88 | 2-[4-[N-methyl-3-(4-methyl-1,4-diazepan-1-yl)anilino] phenoxy]pyrido[3,4-d]pyrimidin-4-ol | | A |
| 89 | 2-[4-[N-methyl-3-[methyl-(1-methylpiperidin-4-yl)amino]anilino]phenoxy] pyrido[3,4-d]pyrimidin-4-ol | | A |
| 90 | 2-[4-[N,3-dimethyl-5-(4-methylpiperazin-1-yl)anilino]phenoxy]pyrido [3,4-d]pyrimidin-4-ol | | A |
| 91 | 2-[1-(oxan-4-yl)indol-5-yl]oxypyrido[3,4-d]pyrimidin-4-ol | | B |
| 92 | 2-[4-(4-tert-butyl-N-methylanilino)phenoxy]pyrido[3,4-d]pyrimidin-4-ol | | B |
| 93 | 2-[4-(N-methyl-3-propan-2-ylanilino)phenoxy]pyrido[3,4-d]pyrimidin-4-ol | | B |
| 94 | 2-[4-(4-chloro-N-methylanilino)phenoxy]pyrido[3,4-d]pyrimidin-4-ol | | B |
| 95 | 2-[4-(3-chloro-N-methylanilino)phenoxy]pyrido[3,4-d]pyrimidin-4-ol | | C |
| 96 | 2-[4-(3-fluoro-N-methylanilino)phenoxy]pyrido[3,4-d]pyrimidin-4-ol | | B |
| 97 | 2-[4-[(5-ethylpyridin-2-yl)-methylamino]phenoxy]pyrido[3,4-d]pyrimidin-4-ol | | B |
| 98 | 2-[4-[4-(3,6-dihydro-2H-pyran-4-yl)-N-methylanilino] phenoxy]pyrido[3,4-d]pyrimidin-4-ol | | C |
| 99 | 2-[4-[N-methyl-4-(oxan-4-yl)anilino]phenoxy]pyrido[3,4-d]pyrimidin-4-ol | | B |
| 100 | 2-[4-[4-[1-(2-methoxyethylamino)propan-2-yl]-N-methylanilino]phenoxy]pyrido [3,4-d]pyrimidin-4-ol | | A |
| 101 | 2-[4-[4-(cyclopropylmethoxy)-N-methylanilino]phenoxy]pyrido[3,4-d]pyrimidin-4-ol | | C |
| 102 | 2-[4-[N-(2-methoxyethyl)-4-propan-2-ylanilino]phenoxy]pyrido[3,4-d]pyrimidin-4-ol | | B |
| 103 | 2-(1-phenylmdol-5-yl)oxypyndo[3,4-d]pyrimidin-4-ol | | C |
| 104 | 2-(1-piperidin-4-ylindol-5-yl)oxypyrido[3,4-d]pyrimidin-4-ol | | A |
| 105 | 2-[4-[N,4-dimethyl-3-(4-methylpiperazin-1-yl)anilino]phenoxy] pyrido[3,4-d]pyrimidin-4-ol | | A |
| 106 | 2-[4-[N-methyl-4-(2,2,2-trifluoroethyl)anilino]phenoxy]pyrido[3,4-d]pyrimidin-4-ol | | C |
| 107 | 2-[4-[N-methyl-4-[1-(trifluoromethyl)cyclopropyl]anilino] phenoxy] pyrido[3,4-d]pyrimidin-4-ol | | C |
| 108 | 2-[4-[N-methyl-4-(1,1,1-trifluoropropan-2-yl)anilino]phenoxy]pyrido[3,4-d] pyrimidin-4-ol | | B |
| 109 | 2-[4-[methyl-(6-propan-2-ylpyridin-3-yl)amino]phenoxy]pyrido[3,4-d]pyrimidin-4-ol | | B |
| 110 | 2-[4-[N-methyl-4-(trifluoromethoxy)anilino]phenoxy]pyrido[3,4-d]pyrimidin-4-ol | | C |
| 111 | 2-[4-(N-methyl-4-propan-2-yloxyanilino)phenoxy]pyrido[3,4-d]pyrimidin-4-ol | | B |
| 114 | 2-{4-[(4-isopropyl-phenyl)-(2,2,2-trifluoro-ethyl)-amino]-phenoxy}-pyrido[3,4-d]pyrimidin-4-ol | | B |
| 115 | 2-(4-{[4-(3-dimethylamino-1-methyl-propyl)-phenyl] -methyl-amino } -phenoxy)-pyrido[3,4-d]pyrimidin-4ol | | A |
| 116 | (3,3-dimethyl-2,3-dihydro-benzofuran-6-yl)-methyl-[4-(4-methyl-pyrido [3,4-d]pyrimidin-2-yloxy)-phenyl]-amine | | B |
| 117 | 2-(4-(ethyl(4-isopropylphenyl)amino)phenoxy)pyrido[3,4-d]pyrimidin-4-ol | | C |
| 118 | 2-(4-((4-isopropylphenyl)(tetrahydro-2H-pyran-4-yl)amino) phenoxy)pyrido[3,4-d]pyrimidin-4-ol | | B |
| 119 | 2-(4-((4-isopropylphenyl)(1-methylpiperidin-4-yl)amino)phenoxy) pyrido[3, 4-d]pyrimidin-4-ol | | A |
| 120 | 2-(4-((4-isopropylphenyl)(propyl)amino)phenoxy)pyrido[3,4-d]pyrimidin-4-ol | | B |
| 121 | 2-(4-((4-isopropylphenyl)amino)phenoxy)pyrido[3,4-d]pyrimidin-4-ol | | A |
| 122 | 2-(4-(isopropyl(4-isopropylphenyl)amino)phenoxy)pyrido[3,4-d]pyrimidin-4-ol | | C |
| 123 | 2-(4-(isobutyl(4-isopropylphenyl)amino)phenoxy)pyrido[3,4-d]pyrimidin-4-ol | | C |
| 124 | 2-(4-((4-isopropylphenyl)(1-methylpiperidin-3-yl)amino) phenoxy)pyrido[3,4-d]pyrimidin-4-ol | | A |
| 125 | 2-(4-(cyclopentyl(4-isopropylphenyl)amino)phenoxy)pyrido[3,4-d]pyrimidin-4-ol | | C |
| 126 | 2-(4-((4-isopropylphenyl)(pyrrolidin-3-yl)amino)phenoxy)pyrido[3,4-d]pyrimidin-4-ol | | A |
| 127 | 2-(4-((4-isopropylphenyl)(1-methylpyrrolidin-3-yl)amino) phenoxy)pyrido[3,4-d]pyrimidin-4-ol | | A |
| 128 | 2-(4-(5-methylindolin-1-yl)phenoxy)pyrido[3,4-d]pyrimidin-4-ol | | A |
| 129 | 2-(4-(5-methyl-1H-indol-1-yl)phenoxy)pyrido[3,4-d]pyrimidin-4-ol | | A |
| 130 | 2-(4-((4-isopropylphenyl)(piperidin-3-yl)amino)phenoxy)pyrido[3,4-d]pyrimidin-4-ol | | A |
| 131 | 2-(4-((4-isopropylphenyl)(methyl)amino)-3-methylphenoxy)pyrido[3,4-d]pyrimidin-4-ol | | B |
| 132 | 2-(4-((3-(4-isopropylpiperazin-1-yl)phenyl)(methyl)amino) phenoxy)pyrido[3,4-d]pyrimidin-4-ol | | A |

| Ex. | Compound Name | | JMJD2C IC₅₀ |
|---|---|---|---|
| 133 | 2-(4-(methyl(3-(4-methylpiperazin-1-yl)phenyl)amino)-3-(trifluoromethyl)phenoxy)pyrido[3,4-d]pyrimidin-4-ol | | A |
| 134 | 2-(3-methyl-4-(methyl(3-(4-methylpiperazin-1-yl)phenyl) amino)phenoxy)pyrido[3,4-d]pyrimidin-4-ol | | A |
| 135 | 2-(4-((4-ethyl-3-methoxyphenyl)(methyl)amino)phenoxy)pyrido[3,4-d]pyrimidin-4-ol | | B |
| 136 | 2-(4-(methyl(3-(4-methylpiperazin-1-yl)-4-(2,2,2-trifluoroethoxy) phenyl)amino)phenoxy)pyrido[3,4-d]pyrimidin-4-ol | | A |
| 137 | 2-(4-((4-ethyl-2-methoxyphenyl)(methyl)amino)phenoxy)pyrido[3,4-d]pyrimidin-4-ol | | B |
| 138 | 2-(4-((3-(4-ethylpiperazin-1-yl)phenyl)(methyl)amino) phenoxy)pyrido[3,4-d]pyrimidin-4-ol | | A |
| 139 | 2-(4-(methyl(3-(1-methyl-1,2,3,6-tetrahydropyridin-4-yl) phenyl)amino)phenoxy)pyrido[3,4-d]pyrimidin-4-ol | | A |
| 140 | 2-(4-(methyl(3-(1-methylpiperidin-4-yl)phenyl)amino) phenoxy)pyrido[3,4-d]pyrimidin-4-ol | | A |
| 141 | 2-(4-(ethyl(3-(4-methylpiperazin-1-yl)phenyl)amino) phenoxy)pyrido[3,4-d]pyrimidin-4-ol | | A |
| 142 | 2-(4-((cyclopropylmethyl)(3-(4-methylpiperazin-1-yl)phenyl)ammo) phenoxy)pyrido[3,4-d]pyrimidin-4-ol | | A |
| 143 | 2-(4-((4-isopropylphenyl)(methyl)amino)-3-(trifluoromethyl) phenoxy)pyrido[3,4-d]pyrimidin-4-ol | | C |
| 144 | 2-(4-((4-ethylphenyl)(methyl)amino)-3-methylphenoxy)pyrido[3,4-d]pyrimidin-4-ol | | A |
| 145 | 2-(4-((2-methoxy-5-(4-methylpiperazin-1-yl)phenyl)(methyl)amino) phenoxy)pyrido[3,4-d]pyrimidin-4-ol | | A |
| 146 | 2-(4-((2-ethoxy-5-(4-methylpiperazin-1-yl)phenyl)(methyl)amino) phenoxy)pyrido[3,4-d]pyrimidin-4-ol | | A |
| 147 | 2-(4-(methyl(2-methyl-5-(4-methylpiperazin-1-yl)phenyl)amino) phenoxy)pyrido[3,4-d]pyrimidin-4-ol | | A |
| 148 | 2-(4-((4-(tert-butyl)phenyl)(methyl)amino)-3-methylphenoxy) pyrido[3,4-d]pyrimidin-4-ol | | B |
| 149 | 2-(3-methyl-4-(methyl(4-propylphenyl)amino)phenoxy)pyrido[3,4-d]pyrimidin-4-ol | | B |
| Note: Biochemical assay IC₅₀ data are designated within the following ranges: | | | |
| A: ≤ 0.10 µM | | C: > 1.0 µM to ≤ 10 µM | |
| B: > 0.10 µM to ≤ 1.0 µM | | D: > 10 µM | |

### Example 2: In vitro cell-based assay

The primary cellular assay for JMJD2C inhibition is an assay that measures cellular proliferation via Bromodeoxyuridine (BrdU) incorporation after 168 hr of compound incubation. Cell lines tested include the JMJD2C gene amplified cell line KYSE-150. This is a quantitative ELISA assay measuring DNA incorporation of BrdU during S-phase as a direct readout of cellular proliferation.

Assay Principle: This is a colorimetric immunoassay for the quantification of cell proliferation. Cells treated for 168 hr with test compounds are assayed for their ability to go through S-phase as a measure of their proliferative potential.

Assay Method: The human KYSE-150 (SMAD4 mut, TP53 mut) esophageal carcinoma cell line was seeded at 2,000 cells/well on a 96-well tissue culture treated plate. After an overnight incubation, cells were treated with compound in an 11-point dilution series with final concentrations ranging from 100 µM to 2 nM. Cells were then incubated in the presence of compound for 168 hr. After compound incubation the cells were assayed using a BrdU Cell Proliferation ELISA (Roche). The cells were first incubated with BrdU labeling reagent for 2 hr. After 2 hr, the BrdU incorporated cells were fixed and denatured, probed with an anti-BrdU-Peroxidase antibody for 1.5 hr and washed. Finally, a tetramethylbenzidine peroxidase substrate was added to each well for 15 min. followed by a H₂SO₄ stop solution. The plate was read at 450 nm, and the raw optical density data was transferred into XLFit (IDBS) for IC₅₀ calculation using the formula: fit = (D+((Vmax^{∗}(x^n))/((x^n)+(Km^{^}n)))).

Table 4 provides the cellular IC₅₀ values of various compounds disclosed herein.

| Table 4. IC₅₀ values | | | | | | | |
|---|---|---|---|---|---|---|---|
| Ex. | Cellular IC₅₀ | Ex. | Cellular IC₅₀ | Ex. | Cellular IC₅₀ | Ex. | Cellular IC₅₀ |
| 1 | C | 47 | B | 77 | A | 107 | A |
| 2 | A | 48 | D | 78 | A | 108 | A |
| 3 | B | 49 | B | 79 | A | 110 | B |
| 4 | B | 50 | D | 80 | B | 111 | B |
| 5 | B | 51 | C | 81 | D | 115 | D |
| 6 | A | 52 | C | 82 | D | 117 | A |
| 7 | C | 53 | B | 83 | B | 118 | C |
| 12 | C | 54 | D | 84 | A | 119 | D |
| 13 | C | 55 | C | 85 | A | 120 | C |
| 15 | C | 56 | D | 86 | B | 121 | C |
| 19 | B | 57 | D | 87 | B | 124 | C |
| 23 | C | 58 | D | 88 | D | 125 | A |
| 24 | D | 59 | D | 89 | C | 126 | A |
| 26 | D | 60 | D | 90 | D | 127 | D |
| 27 | D | 61 | D | 91 | D | 128 | A |
| 29 | B | 62 | C | 92 | A | 129 | C |
| 30 | A | 64 | B | 94 | B | 130 | D |
| 32 | C | 65 | D | 95 | C | 131 | D |
| 36 | B | 66 | C | 96 | C | 132 | B |
| 37 | D | 67 | A | 97 | B | 133 | C |
| 38 | A | 68 | B | 98 | D | 134 | C |
| 39 | A | 69 | A | 99 | A | 135 | C |
| 40 | D | 70 | D | 100 | D | 136 | C |
| 41 | C | 71 | C | 101 | B | 137 | D |
| 42 | B | 72 | D | 102 | B | 138 | C |
| 43 | B | 73 | A | 103 | D | 140 | B |
| 44 | D | 74 | A | 104 | D | 141 | A |
| 45 | D | 75 | A | 105 | C | | |
| 46 | D | 76 | A | 106 | A | | |
| Note: Cell assay IC₅₀ data are designated within the following ranges: | | | | | | | |
| A: ≤ 0.10 µM | | | | C: > 1.0 µM to ≤ 10 µM | | | |
| B: > 0.10 µM to ≤ 1.0 µM | | | | D: > 10 µM | | | |

### Example 3: In vivo xenograph study

Time release pellets containing 0.72 mg 17-β Estradiol are subcutaneously implanted into nu/nu mice. MCF-7 cells are grown in RPMI containing 10% FBS at 5% CO₂, 37°C. Cells are spun down and re-suspended in 50% RPMI (serum free) and 50% Matrigel at 1×10⁷ cells/mL. MCF-7 cells are subcutaneously injected (100µL/animal) on the right flank 2-3 days post pellet implantation and tumor volume (length × width²/2) is monitored bi-weekly. When tumors reach an average volume of ~200 mm³ animals are randomized and treatment is started. Animals are treated with vehicle or compound daily for four weeks. Tumor volume and body weight are monitored bi-weekly throughout the study. At the conclusion of the treatment period, plasma and tumor samples are taken for pharmacokinetic and pharmacodynamic analyses, respectively.

### III. Preparation of Pharmaceutical Dosage Forms

### Example 1: Oral tablet

A tablet is prepared by mixing 48% by weight of a compound of Formula (I) or a pharmaceutically acceptable salt thereof, 45% by weight of microcrystalline cellulose, 5% by weight of low-substituted hydroxypropyl cellulose, and 2% by weight of magnesium stearate. Tablets are prepared by direct compression. The total weight of the compressed tablets is maintained at 250-500 mg.

## Claims

1. A substituted pyrido[3,4-d]pyrimidin-4-one derivative compound selected from the group consisting of:
2-(4-((4-isopropylphenyl)(1-methylpiperidin-4-yl)amino)phenoxy)pyrido [3,4 d] pyrimidin-4-ol;
2-(4-((4-isopropylphenyl)(propyl)amino)phenoxy)pyrido[3,4-d]pyrimidin-4-ol;
2-(4-((4-isopropylphenyl)amino)phenoxy)pyrido[3,4-d]pyrimidin-4-ol;
2-(4-(isopropyl(4-isopropylphenyl)amino)phenoxy)pyrido[3,4-d]pyrimidin-4-ol;
2-(4-(isobutyl(4-isopropylphenyl)amino)phenoxy)pyrido[3,4-d]pyrimidin-4-ol;
2-(4-((4-isopropylphenyl)(1-methylpiperidin-3-yl)amino)phenoxy)pyrido[3,4 d] pyrimidin-4-ol;
2-(4-(cyclopentyl(4-isopropylphenyl)amino)phenoxy)pyrido[3,4-d]pyrimidin-4-ol;
2-(4-((4-isopropylphenyl)(pyrrolidin-3-yl)amino)phenoxy)pyrido[3,4-d]pyrimidin-4-ol;
2-(4-((4-isopropylphenyl)(1-methylpyrrolidin-3-yl)amino)phenoxy)pyrido[3,4 d] pyrimidin-4-ol;
2-(4-(5-methylindolin-1-yl)phenoxy)pyrido[3,4-d]pyrimidin-4-ol;
2-(4-(5-methyl-1H-indol-1-yl)phenoxy)pyrido[3,4-d]pyrimidin-4-ol;
2-(4-((4-isopropylphenyl)(piperidin-3-yl)amino)phenoxy)pyrido[3,4-d]pyrimidin-4-ol;
2-(4-((4-isopropylphenyl)(methyl)amino)-3-methylphenoxy)pyrido[3,4-d]pyrimidin-4-ol;
2-(4-((3-(4-isopropylpiperazin-1-yl)phenyl)(methyl)amino)phenoxy)pyrido[3,4-d] pyrimidin-4-ol;
2-(4-(methyl(3-(4-methylpiperazin-1-yl)phenyl)amino)-3-(trifluoromethyl)phenoxy)pyrido[3,4-d] pyrimidin-4-ol;
2-(3-methyl-4-(methyl(3-(4-methylpiperazin-1-yl)phenyl)amino)phenoxy)pyrido[3,4 d] pyrimidin-4-ol;
2-(4-((4-ethyl-3-methoxyphenyl)(methyl)amino)phenoxy)pyrido[3,4-d]pyrimidin-4-ol;
2-(4-(methyl(3-(4-methylpiperazin-1-yl)-4-(2,2,2trifluoroethoxy)phenyl)amino)phenoxy) pyrido[3,4-d]pyrimidin-4-ol;
2-(4-((4-ethyl-2-methoxyphenyl)(methyl)amino)phenoxy)pyrido[3,4-d]pyrimidin-4-ol;
2-(4-((3-(4-ethylpiperazin-1-yl)phenyl)(methyl)amino)phenoxy)pyrido[3,4-d] pyrimidin-4-ol;
2-(4-(methyl(3-(1-methyl-1,2,3,6-tetrahydropyridin-4-yl)phenyl)amino)phenoxy)pyrido[3,4-d] pyrimidin-4-ol;
2-(4-(methyl(3-(1-methylpiperidin-4-yl)phenyl)amino)phenoxy)pyrido[3,4-d] pyrimidin-4-ol;
2-(4-(ethyl(3-(4-methylpiperazin-1-yl)phenyl)amino)phenoxy)pyrido[3,4-d]pyrimidin-4-ol;
2-(4-((cyclopropylmethyl)(3-(4-methylpiperazin-1-yl)phenyl)amino)phenoxy)pyrido[3,4-d] pyrimidin-4-ol;
2-(4-((4-isopropylphenyl)(methyl)amino)-3-(trifluoromethyl)phenoxy)pyrido[3,4-d] pyrimidin-4-ol;
2-(4-((4-ethylphenyl)(methyl)amino)-3-methylphenoxy)pyrido[3,4-d]pyrimidin-4-ol;
2-(4-((2-methoxy-5-(4-methylpiperazin-1-yl)phenyl)(methyl)amino)phenoxy)pyrido[3,4-d] pyrimidin-4-ol;
2-(4-((2-ethoxy-5-(4-methylpiperazin-1-yl)phenyl)(methyl)amino)phenoxy)pyrido[3,4-d] pyrimidin-4-ol;
2-(4-(methyl(2-methyl-5-(4-methylpiperazin-1-yl)phenyl)amino)phenoxy)pyrido[3,4 d] pyrimidin-4-ol;
2-(4-((4-(tert-butyl)phenyl)(methyl)amino)-3-methylphenoxy)pyrido[3,4-d]pyrimidin-4-ol; and
2-(3-methyl-4-(methyl(4-propylphenyl)amino)phenoxy)pyrido[3,4-d]pyrimidin-4-ol.

2. A pharmaceutical composition comprising a compound according to claim 1.

3. The compound according to claim 1 or the pharmaceutical composition according to claim 2 for use in the therapy of cancer

4. The compound according to claim 1 or the pharmaceutical composition according to claim 2 for use in the therapy of prostate cancer, breast cancer, bladder cancer, lung cancer and/or melanoma.

## Patentansprüche

1. Substituierte Pyrido[3,4-d]pyrimidin-4-on-Derivatverbindung, die aus der Gruppe ausgewählt ist, die aus Folgendem besteht:
2-(4-((4-Isopropylphenyl)(1-methylpiperidin-4-yl)amino)phenoxy)pyrido[3,4d]pyrimidin-4-ol;
2-(4-((4-Isopropylphenyl)(propyl)amino)phenoxy)pyrido[3,4-d]pyrimidin-4-ol;
2-(4-((4-Isopropylphenyl)amino)phenoxy)pyrido[3,4-d]pyrimidin-4-ol;
2-(4-(Isopropyl(4-isopropylphenyl)amino)phenoxy)pyrido[3,4-d]pyrimidin-4-ol;
2-(4-(Isobutyl(4-isopropylphenyl)amino)phenoxy)pyrido[3,4-d]pyrimidin-4-ol;
2-(4-((4-Isopropylphenyl)(1-methylpiperidin-3-yl)amino)phenoxy)pyrido[3,4 d]pyrimidin-4-ol;
2-(4-(Cyclopentyl(4-isopropylphenyl)amino)phenoxy)pyrido[3,4-d]pyrimidin-4-ol;
2-(4-((4-Isopropylphenyl)(pyrrolidin-3-yl)amino)phenoxy)pyrido[3,4-d]pyrimidin-4-ol;
2-(4-((4-Isopropylphenyl)(1-methylpyrrolidin-3-yl)amino)phenoxy)pyrido[3,4 d]pyrimidin-4-ol;
2-(4-(5-Methylindolin-1-yl)phenoxy)pyrido[3,4-d]pyrimidin-4-ol;
2-(4-(5-Methyl-1H-indol-1-yl)phenoxy)pyrido[3,4-d]pyrimidin-4-ol;
2-(4-((4-Isopropylphenyl)(piperidin-3-yl)amino)phenoxy)pyrido[3,4-d]pyrimidin-4-ol;
2-(4-((4-Isopropylphenyl)(methyl)amino)-3-methylphenoxy)pyrido[3,4-d]pyrimidin-4-ol;
2-(4-((3-(4-Isopropylpiperazin-1-yl)phenyl)(methyl)amino)phenoxy)pyrido[3,4-d]pyrimidin-4-ol;
2-(4-(Methyl(3-(4-methylpiperazin-1-yl)phenyl)amino)-3-(trifluormethyl)phenoxy)pyrido[3,4-d]pyrimidin-4-ol;
2-(3-Methyl-4-(methyl(3-(4-methylpiperazin-1-yl)phenyl)amino)phenoxy)pyrido[3,4 d]pyrimidin-4-ol;
2-(4-((4-Ethyl-3-methoxyphenyl)(methyl)amino)phenoxy)pyrido[3,4-d]pyrimidin-4-ol;
2-(4-(Methyl(3-(4-methylpiperazin-1-yl)-4-(2,2,2-trifluorethoxy)phenyl)amino)phenoxy)pyrido[3,4-d]pyrimidin-4-ol;
2-(4-((4-Ethyl-2-methoxyphenyl)(methyl)amino)phenoxy)pyrido[3,4-d]pyrimidin-4-ol;
2-(4-((3-(4-Ethylpiperazin-1-yl)phenyl)(methyl)amino)phenoxy)pyrido[3,4-d]pyrimidin-4-ol;
2-(4-(Methyl(3-(1-methyl-1,2,3,6-tetrahydropyridin-4-yl)phenyl)amino)phenoxy)pyrido[3,4-d]pyrimidin-4-ol;
2-(4-(Methyl(3-(1-methylpiperidin-4-yl)phenyl)amino)phenoxy)pyrido[3,4-d]pyrimidin-4-ol;
2-(4-(Ethyl(3-(4-methylpiperazin-1-yl)phenyl)amino)phenoxy)pyrido[3,4-d]pyrimidin-4-ol;
2-(4-((Cyclopropylmethyl)(3-(4-methylpiperazin-1-yl)phenyl)amino)phenoxy)pyrido[3,4-d]pyrimidin-4-ol;
2-(4-((4-Isopropylphenyl)(methyl)amino)-3-(trifluormethyl)phenoxy)pyrido[3,4-d]pyrimidin-4-ol;
2-(4-((4-Ethylphenyl)(methyl)amino)-3-methylphenoxy)pyrido[3,4-d]pyrimidin-4-ol;
2-(4-((2-Methoxy-5-(4-methylpiperazin-1-yl)phenyl)(methyl)amino)phenoxy)pyrido[3,4-d]pyrimidin-4-ol;
2-(4-((2-Ethoxy-5-(4-methylpiperazin-1-yl)phenyl)(methyl)amino)phenoxy)pyrido[3,4-d]pyrimidin-4-ol;
2-(4-(Methyl(2-methyl-5-(4-methylpiperazin-1-yl)phenyl)amino)phenoxy)pyrido[3,4 d]pyrimidin-4-ol;
2-(4-((4-(tert-Butyl)phenyl)(methyl)amino)-3-methylphenoxy)pyrido[3,4-d]pyrimidin-4-ol; und
2-(3-Methyl-4-(methyl(4-propylphenyl)amino)phenoxy)pyrido[3,4-d]pyrimidin-4-ol.

2. Pharmazeutische Zusammensetzung, die eine Verbindung nach Anspruch 1 umfasst.

3. Verbindung nach Anspruch 1 oder pharmazeutische Zusammensetzung nach Anspruch 2 zur Verwendung in der Therapie von Krebs.

4. Verbindung nach Anspruch 1 oder pharmazeutische Zusammensetzung nach Anspruch 2 zur Verwendung in der Therapie von Prostatakrebs, Brustkrebs, Blasenkrebs, Lungenkrebs und/oder Melanom.

## Revendications

1. Composé dérivé de pyrido[3,4-d]pyrimidin-4-one substitué choisi dans le groupe consistant en :
2-(4-((4-isopropylphényl)(1-méthylpipéridin-4-yl)amino)phénoxy)pyrido[3,4 d]pyrimidin-4-ol ;
2-(4-((4-isopropylphényl)(propyl)amino)phénoxy)pyrido[3,4-d]pyrimidine-4-ol ;
2-(4-((4-isopropylphényl)amino)phénoxy)pyrido[3,4-d]pyrimidine-4-ol ;
2-(4-(isopropyl(4-isopropylphényl)amino)phénoxy)pyrido[3,4-d]pyrimidine-4-ol ;
2-(4-(isobutyl(4-isopropylphényl)amino)phénoxy)pyrido[3,4-d]pyrimidin-4-ol ;
2-(4-((4-isopropylphényl)(1-méthylpipéridin-3-yl)amino)phénoxy)pyrido[3,4 d]pyrimidin-4-ol ;
2-(4-(cyclopentyl(4-isopropylphényl)amino)phénoxy)pyrido[3,4-d]pyrimidin-4-ol ;
2-(4-((4-isopropylphényl)(pyrrolidin-3-yl)amino)phénoxy)pyrido[3,4-d]pyrimidin-4-ol ;
2-(4-((4-isopropylphényl)(1-méthylpyrrolidin-3-yl)amino)phénoxy)pyrido[3,4 d]pyrimidin-4-ol ;
2-(4-(5-méthylindolin-1-yl)phénoxy)pyrido[3,4-d]pyrimidin-4-ol ;
2-(4-(5-méthyl-1H-indol-1-yl)phénoxy)pyrido[3,4-d]pyrimidin-4-ol ;
2-(4-((4-isopropylphényl)(pipéridin-3-yl)amino)phénoxy)pyrido[3,4-d]pyrimidin-4-ol ;
2-(4-((4-isopropylphényl)(méthyl)amino)-3-méthylphénoxy)pyrido[3,4-d]pyrimidin-4-ol ;
2-(4-((3-(4-isopropylpipérazin-1-yl)phényl)(méthyl)amino)phénoxy)pyrido[3,4-d]pyrimidin-4-ol ;
2-(4-(méthyl(3-(4-méthylpipérazin-1-yl)phényl)amino)-3-(trifluorométhyl)phénoxy)pyrido[3,4- d]pyrimidine-4-ol ;
2-(3-méthyl-4-(méthyl(3-(4-méthylpipérazin-1-yl)phényl)amino)phénoxy)pyrido[3,4 d] pyrimidin-4-ol ;
2-(4-((4-éthyl-3-méthoxyphényl)(méthyl)amino)phénoxy)pyrido[3,4-d]pyrimidin-4-ol ;
2-(4-(méthyl(3-(4-méthylpipérazin-1-yl)-4-(2,2,2
trifluoroéthoxy)phényl)amino)phénoxy)pyrido[3,4-d]pyrimidin-4-ol ;
2-(4-((4-éthyl-2-méthoxyphényl)(méthyl)amino)phénoxy)pyrido[3,4-d]pyrimidin-4-ol ;
2-(4-((3-(4-éthylpipérazin-1-yl)phényl)(méthyl)amino)phénoxy)pyrido[3,4-d] pyrimidin-4-ol ;
2-(4-(méthyl(3-(1-methyl-1,2,3,6-tétrahydropyridin-4-yl)phényl)amino)phénoxy)pyrido[3,4-d] pyrimidin-4-ol ;
2-(4-(méthyl(3-(1-méthylpipéridin-4-yl)phényl)amino)phénoxy)pyrido[3,4-d] pyrimidin-4-ol ;
2-(4-(éthyl(3-(4-méthylpipérazin-l-yl)phényl)amino)phénoxy)pyrido[3,4-d]pyrimidin-4-ol ;
2-(4-((cyclopropylméthyl)(3-(4-méthylpipérazin-1-yl)phényl)amino)phénoxy)pyrido[3,4-d] pyrimidin-4-ol ;
2-(4-((4-isopropylphényl)(méthyl)amino)-3-(trifluorométhyl)phénoxy)pyrido[3,4-d]pyrimidin-4-ol ;
2-(4-((4-éthylphényl)(méthyl)amino)-3-méthylphénoxy)pyrido[3,4-d]pyrimidin-4-ol ;
2-(4-((2-méthoxy-5-(4-méthylpipérazin-1-yl)phényl)(méthyl)amino)phénoxy)pyrido[3,4-d] pyrimidin-4-ol ;
2-(4-((2-éthoxy-5-(4-méthylpipérazin-1-yl)phényl)(méthyl)amino)phénoxy)pyrido[3,4-d] pyrimidin-4-ol ;
2-(4-(méthyl(2-méthyl-5-(4-méthylpipérazin-1-yl)phényl)amino)phénoxy)pyrido[3,4 d] pyrimidin-4-ol ;
2-(4-((4-(tert-butyl)phényl)(méthyl)amino)-3-méthylphénoxy)pyrido[3,4-d]pyrimidin-4-ol ; et
2-(3-méthyl-4-(méthyl(4-propylphényl)amino)phénoxy)pyrido[3,4-d]pyrimidin-4-ol.

2. Composition pharmaceutique comprenant un composé selon la revendication 1.

3. Composé selon la revendication 1 ou composition pharmaceutique selon la revendication 2 pour une utilisation dans le traitement d'un cancer.

4. Composé selon la revendication 1 ou composition pharmaceutique selon la revendication 2 pour une utilisation dans le traitement du cancer de la prostate, du cancer du sein, du cancer de la vessie, du cancer du poumon et/ou du mélanome.
